# EUROPEAN PATENT SPECIFICATION

(11) **EP 4 514 794 B1**
(45) Date of publication and mention of the grant of the patent: **25.03.2026**
(21) Application number: 23724630.1
(22) Date of filing: 25.04.2023
(51) Int. Cl.: C07D 471/04, C07D 519/00, A61P 35/00, A61K 31/445, A61K 31/444, A61K 31/55, A61K 31/506

(54) **FGFR2 INHIBITOR COMPOUNDS**
FGFR2-INHIBITORVERBINDUNGEN
COMPOSÉS INHIBITEURS DE FGFR2

(30) Priority: 25.04.2022 EP 22382389; 07.09.2022 EP 22382831
(43) Date of publication of application: 05.03.2025
(73) Proprietor: Eli Lilly and Company, Indianapolis, IN 46285 (US)
(72) Inventor: ADEVA BARTOLOMÉ, Marta, Indianapolis, Indiana 46206-6288 (US); FERNANDEZ FIGUEROA, Maria Carmen, Indianapolis, Indiana 46206-6288 (US); DEL PRADO CATALINA, Miriam Filadelfa, Indianapolis, Indiana 46206-6288 (US); GARCIA-CERRADA, Susana Maria, Indianapolis, Indiana 46206-6288 (US); GARCIA LOSADA, Pablo, Indianapolis, Indiana 46206-6288 (US); GARZÓN SANZ, Miguel, Indianapolis, Indiana 46206-6288 (US); GUTIERREZ SANFELICIANO, Sonia Maria, Indianapolis, Indiana 46206-6288 (US); MARTINEZ PEREZ, Jose Antonio, Indianapolis, Indiana 46206-6288 (US); METCALF, Andrew Terrance, Indianapolis, Indiana 46206-6288 (US); PEIRÓ CADAHIA, Jorge, Indianapolis, Indiana 46206-6288 (US); VALERO DE LA CRUZ, Alberto, Indianapolis, Indiana 46206-6288 (US); PRIETO VALLEJO, Maria Lourdes, Indianapolis, Indiana 46206-6288 (US)
(74) Representative: Eli Lilly and Company Limited
(86) International application number: PCT/US2023/066148
(87) International publication number: WO 2023/212535

(56) References cited:
- WO-A1-2017/011776
- WO-A1-2020/131627
- TURNER LEWIS D. ET AL: "From Fragment to Lead: De Novo Design and Development toward a Selective FGFR2 Inhibitor", JOURNAL OF MEDICINAL CHEMISTRY, vol. 65, no. 2, 15 November 2021 (2021-11-15), US, pages 1481 - 1504, XP093062588, ISSN: 0022-2623, Retrieved from the Internet <URL:https://pubs.acs.org/doi/pdf/10.1021/acs.jmedchem.1c01163> DOI: 10.1021/acs.jmedchem.1c01163

## Description

### Background

Fibroblast growth factor (FGF) has been recognized as an important mediator of many physiological processes, such as morphogenesis during development, fibrosis, and angiogenesis. The fibroblast growth factor receptor (FGFR) family consists of five members four of which (FGFR 1-4) are glycoproteins composed of extracellular immunoglobulin (Ig)-like domains, a hydrophobic transmembrane region and a cytoplasmic part containing a tyrosine kinase domain. FGF binding leads to FGFR dimerization, followed by receptor autophosphorylation and activation of downstream signaling pathways. Receptor activation is sufficient for the recruitment and activation of specific downstream signaling partners that participate in the regulation of diverse processes such as cell growth, cell metabolism and cell survival. Thus, the FGF/FGFR signaling pathway has pleiotropic effects on many biological processes critical to tumor cell proliferation, migration, invasion, and angiogenesis.

WO2020131627 discloses pyrazolo[1,5-a]pyridine compounds as inhibitors of FGFR tyrosine kinases.

### Summary

Provided herein are compounds of the formula: 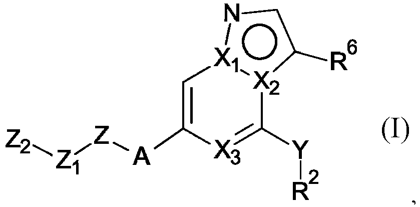 or a pharmaceutically acceptable salt thereof, wherein A, X₁, X₂, X₃, Y, Z, Z₁, Z₂, R² and R⁶ are as defined herein.

Provided herein are compounds of the formula: 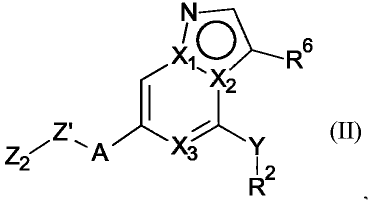 or a pharmaceutically acceptable salt thereof, wherein A, X₁, X₂, X₃, Y, Z', Z₂, R² and R⁶ are as defined herein.

Provided herein are compounds of the formula: 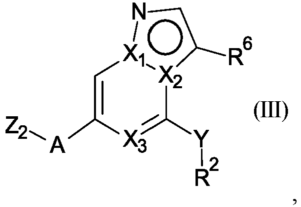 or a pharmaceutically acceptable salt thereof, wherein A, X₁, X₂, X₃, Y, Z₂, R² and R⁶ are as defined herein.

Provided herein are compounds of the formula: 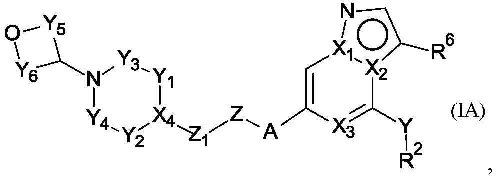 or a pharmaceutically acceptable salt thereof, wherein A, X₁, X₂, X₃, X₄, Y, Y₁, Y₂, Y₃, Y₄, Y₅, Y₆, Z, Z₁, R² and R⁶ are as defined herein.

Provided herein are compounds of the formula: 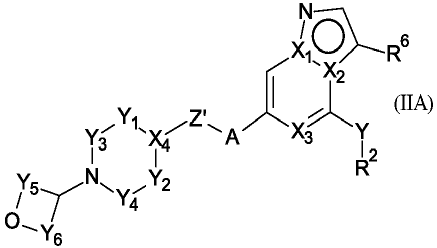 , or a pharmaceutically acceptable salt thereof, wherein A, X₁, X₂, X₃, X₄, Y, Y₁, Y₂, Y₃, Y₄, Y₅, Y₆, Z', R² and R⁶ are as defined herein.

Provided herein are compounds of the formula: 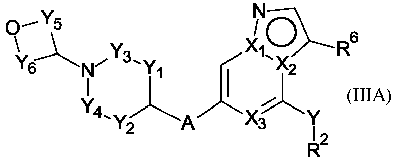 or a pharmaceutically acceptable salt thereof, wherein A, X₁, X₂, X₃, Y, Y₁, Y₂, Y₃, Y₄, Y₅, Y₆, R² and R⁶ are as defined herein.

Provided herein are pharmaceutical compositions comprising a compound of formula (I), (II), (III), (IA), (IIA) or (IIIA), or a pharmaceutically acceptable salt thereof, with one or more pharmaceutically acceptable carriers, diluents, or excipients.

Provided herein are methods of using the compounds of formula (I), (II), (III), (IA), (IIA) or (IIIA), or a pharmaceutically acceptable salt thereof, and pharmaceutical compositions thereof, to treat proliferative disorders such as cancer, particularly to treat FGFR2-associated cancer. The methods include administering an effective amount of a compound of formula (I), (II), (III), (IA), (IIA) or (IIIA), or a pharmaceutically acceptable salt thereof, to a patient in need.

Provided herein, are compounds of formula (I), (II), (III), (IA), (IIA) or (IIIA), or a pharmaceutically acceptable salt thereof, for use in therapy. Further provided herein, are the compounds of formula (I), (II), (III), (IA), (IIA) or (IIIA), or a pharmaceutically acceptable salt thereof, for use in the treatment of cancer, particularly for use in the treatment of FGFR2-associated cancer. The use of compounds of formula (I), (II), (III), (IA), (IIA) or (IIIA), or a pharmaceutically acceptable salt thereof, in the manufacture of a medicament for treating cancer, particularly for use in the treatment of FGFR2-associated cancer, is also provided.

### Description

Provided herein are compounds believed to have clinical use for the treatment of cancer and particularly for the treatment of FGFR2-associated cancer.

Certain compounds provided herein have superior FGFR2 potency compared to certain previously known FGFR inhibitors. Certain compounds provided herein have superior selectivity for FGFR2 over FGFR1 compared to certain previously known FGFR inhibitors, reducing potential dose limiting toxicity caused by inhibition of FGFR1 (e.g. hyperphosphatemia).

The compounds provided herein are of formula (I): 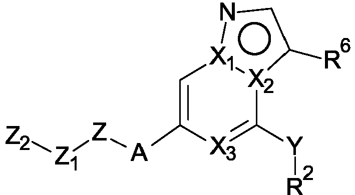 wherein
Z₂ is 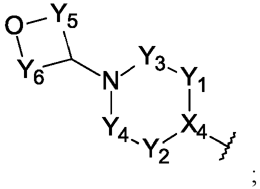
A is pyrazole, triazole, thiadiazole or oxadiazole, substituted with R¹ and R^{1A};
R¹ is hydrogen or C₁-C₃ alkyl;
R^{1A} is hydrogen, halo, CN or C₁-C₃ alkyl optionally substituted with one or more substituents independently selected from halo, OH, and OCH₃;
X₁ and X₂ are independently selected from N and C, wherein when one of X₁ or X₂ is N the other is C;
X₃ is N or CH;
X₄ is N or C-R⁹;
Y is NH, O, S or a bond;
Y₁ is a bond, CHR⁷, CH₂-CHR⁷, CHR⁷-CH₂, CF₂, CH₂-CF₂ or CF₂-CH₂;
Y₂ is a bond, CHR³, CH₂-CHR³, CHR³-CH₂, CF₂, CH₂-CF₂ or CF₂-CH₂;
Y₃ is CR⁴R⁵ or CF₂;
Y₄ is CR³R⁴ or CF₂;
Y₅ is CR¹³R¹⁴, CR¹³R¹⁴CH₂ or CH₂ CR¹³R¹⁴;
Y₆ is CR¹³R¹⁴, CR¹³R¹⁴CH₂ or CH₂ CR¹³R¹⁴;
Z is a bond, CHR^{9A}, CR⁴R^{4A}, CR⁴R^{4A}-CH₂, CH₂-CR⁴R^{4A}, cyclobutyl, cyclopentyl, cyclohexyl, bicyclo(1.1.1)pentane, bicyclo(2.1.1)hexane, azetidine, pyrrolidine or piperidine;
Z₁ is a bond when Z is a bond, CR⁴R^{4A}, CR⁴R^{4A}-CH₂, CH₂-CR⁴R^{4A}, cyclobutyl, cyclopentyl, cyclohexyl, bicyclo(1.1.1)pentane, bicyclo(2.1.1)hexane, azetidine, pyrrolidine or piperidine, or Z₁ is CH₂ or CH₂-CH₂ when Z is CHR^{9A};
Z₃ is a bond, C(O), SO₂ or -NR⁴C(O);
Z₄ is a bond, C(O), SO₂ or -NR⁴C(O);
R² is C₁-C₅ alkyl or R⁸, wherein C₁-C₅ alkyl is optionally substituted with one or more substituents independently selected from halo, OH, CN, oxo, -OC₁-C₄ alkyl, -OC₃-C₅ cycloalkyl, -Z₃-R¹¹ and R¹⁰, wherein C₁-C₄ alky and C₃-C₅ cycloalkyl are optionally substituted with one or more substituents independently selected from halo, OH, OCH₃, methylamine, N,N-dimethylamine and CN;
R³ is hydrogen, F, OH, OCH₃, C₁-C₃ alkyl, cyclopropyl, or one R³ is fused with R⁵ or R⁷ to form CH₂, CH₂-CH₂ or CH₂OCH₂;
R⁴ is hydrogen or C₁-C₃ alkyl;
R^{4A} is hydrogen, halo, OH or C₁-C₃ alkyl;
R⁵ is hydrogen, F, OH, OCH₃, C₁-C₃ alkyl, cyclopropyl or is fused with one R³ to form CH₂, CH₂-CH₂ or CH₂OCH₂;
R⁶ is hydrogen, halo, C₁-C₅ alkyl, CN, 3-6 membered cycloalkyl, 4-6 membered heterocycloalkyl, 5-6 membered aryl or 5-6 membered heteroaryl, wherein 3-6 membered cycloalkyl, 4-6 membered heterocycloalkyl, 5-6 membered aryl and 5-6 membered heteroaryl are optionally substituted with one or more substituents independently selected from halo, methyl, halomethyl, OH or OCH₃ and wherein C₁-C₅ alkyl is optionally substituted with one or more substituents independently selected from halo, OH and OCH₃;
R⁷ is hydrogen, F, OH, OCH₃, C₁-C₃ alkyl or is fused with one R³ to form CH₂, CH₂-CH₂ or CH₂OCH₂;
R⁸ is 3-6 membered cycloalkyl, 4-6 membered heterocycloalkyl, 5-6 membered aryl or 5-6 membered heteroaryl, optionally fused or substituted with R^{8A};
R^{8A} is 3-6 membered cycloalkyl, 4-6 membered heterocycloalkyl, 5-6 membered aryl or 5-6 membered heteroaryl;
R⁹ is hydrogen, C₁-C₃ alkyl, or is fused with R^{9A} to form CH₂ or CH₂-CH₂;
R¹⁰ is 3-6 membered cycloalkyl, 4-6 membered heterocycloalkyl, 5-6 membered aryl or 5-6 membered heteroaryl, optionally fused or substituted with R^{8A};
R₁₁ is C₁-C₄ alkyl, NH₂, NHC₁-C₃ alkyl, NHC₃-C₅ cycloalkyl or N(C₁-C₃ alkyl)₂, wherein C₁-C₄ alkyl, C₁-C₃ alkyl and C₃-C₅ cycloalkyl are optionally substituted with one or more substituents independently selected from halo, OH, OCH₃, methylamine, N,N-dimethylamine and CN;
R¹² is C₁-C₄ alkyl, C₃-C₅ cycloalkyl, NH₂, NHC₁-C₃ alkyl, NHC₃-C₅ cycloalkyl or N(C₁-C₃ alkyl)₂, wherein C₁-C₄ alky, C₁-C₃ alkyl and C₃-C₅ cycloalkyl are optionally substituted with one or more substituents independently selected from halo, OH, OCH₃, methylamine, N,N-dimethylamine and CN;
R¹³ is hydrogen, halo or C₁-C₃ alkyl;
R¹⁴ is hydrogen, halo or C₁-C₃ alkyl; and
R⁸, R¹⁰ and R^{8A} are optionally substituted with one or more substituents independently selected from halo, OH, CN, -OC₁-C₄ alkyl, -OC₃-C₅ cycloalkyl and -Z₄-R¹² wherein C₁-C₄ alky and C₃-C₅ cycloalkyl are optionally substituted with one or more substituents independently selected from halo, OH, OCH₃, methylamine, N,N-dimethylamine and CN;
or a pharmaceutically acceptable salt thereof.

In formula (II) and (IIA), A, X₁, X₂, X₃, Y, Y₁, Y₂, Y₃, Y₄, Y₅, Y₆, R² and R⁶ are as defined above for formula (I); and
X₄ is N or C-R⁹, wherein R⁹ is hydrogen or C₁-C₃ alkyl;
Z' is a bond, CR⁴R^{4A}, CR⁴R^{4A}-CH₂, CH₂-CR⁴R^{4A}, cyclobutyl, cyclopentyl, cyclohexyl, bicyclo(1.1.1)pentane, bicyclo(2.1.1)hexane, azetidine, pyrrolidine or piperidine.

In formula (III),
A, X₁, X₂, X₃, Y, Y₁, Y₂, Y₃, Y₄, Y₅, Y₆, Z₂, R² and R⁶ are as defined above for formula (I); and
X₄ is N or C-R⁹, wherein R⁹ is hydrogen or C₁-C₃ alkyl.

In formula (IIIA), A, X₁, X₂, X₃, Y, Y₁, Y₂, Y₃, Y₄, Y₅, Y₆, R² and R⁶ are as defined above for formula (I).

In the compounds of formula (I), (II), (III), (IA), (IIA) or (IIIA), X₁ can be C, and X₂ can be N; or X₁ can be N, and X₂ can be C.

In the compounds of formula (I), (II), (III), (IA), (IIA) or (IIIA), X₁ can be C, and X₂ can be N, forming: 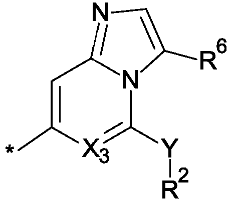 wherein * indicates the connection point to A in formula (I), (II), (III), (IA), (IIA) or (IIIA).

In the compounds of formula (I), (II), (III), (IA), (IIA) or (IIIA), X₁ can be N, and X₂ can be C, forming: 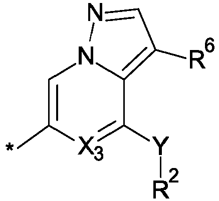 wherein * indicates the connection point to A in formula (I), (II), (III), (IA), (IIA) or (IIIA).

In the compounds of formula (I), (II), (III), (IA), (IIA) or (IIIA), X₁ can be C, X₂ can be N, and X₃ can be CH, forming: 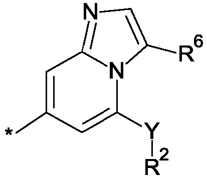 wherein * indicates the connection point to A in formula (I), (II), (III), (IA), (IIA) or (IIIA).

In the compounds of formula (I), (II), (III), (IA), (IIA) or (IIIA), X₁ can be N, X₂ can be C, and X₃ can be CH, forming: 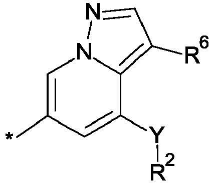 wherein * indicates the connection point to A in formula (I), (II), (III), (IA), (IIA) or (IIIA).

The specific chemical naming conventions used herein are intended to be familiar to one of skill in the chemical arts. Some terms are defined specifically for additional clarity.

As used herein, the term "alkyl" refers to a hydrocarbon chain that may be a straight chain or branched chain, containing the indicated number of carbon atoms. For example, the term "C₁-C₅ alkyl" as used herein refers to saturated linear or branched-chain monovalent hydrocarbon radicals of one, two, three, four or five carbon atoms. Examples of C₁-C₅ alkyl include, but are not limited to, methyl, ethyl, 1-propyl, isopropyl, 1-butyl, isobutyl, sec-butyl, tert-butyl, 2-methyl-2-propyl, pentyl and neopentyl. Examples of C₁-C₄ alkyl include, but are not limited to, methyl, ethyl, 1-propyl, isopropyl, 1-butyl, isobutyl, sec-butyl, tert-butyl and 2-methyl-2-propyl. Examples of C₁-C₃ alkyl include, but are not limited to, methyl, ethyl, 1-propyl or isopropyl.

As used herein, the term "cycloalkyl" means a saturated cyclic hydrocarbon group containing the indicated number of carbon atoms. For example, the term "3-6 membered cycloalkyl" as used herein refers to a saturated cyclic hydrocarbon group having three, four, five or six carbon atoms. Examples of 3-6 membered cycloalkyl include, cyclopropyl, cyclobutyl, cyclopentyl and cyclohexyl. Examples of 3-5 membered cycloalkyl include, cyclopropyl, cyclobutyl and cyclopentyl.

As used herein, the term "heterocycloalkyl" means a saturated cyclic group containing the indicated number of atoms selected from C(O)₀₋₁, N, O and S(O)₀₋₂. For example, the term "5-6 membered heterocycloalkyl" as used herein refers to a saturated cyclic ring system having five or six ring atoms, one, two or three of which are selected from N, O and S(O)₀₋₂, the remainder being C(O)₀₋₁. Examples of 4-6 membered heterocycloalkyl groups include, but are not limited to, azetidinyl, pyrrolidinyl, piperidinyl, piperazinyl, pyrrolidinyl, pyrrolidin-2-onyl, dioxanyl, morpholinyl, oxetanyl, tetrahydrofuranyl, tetrahydrothiophenyl, tetrahydropyranyl, oxazolidinyl, isothiazolidinyl oxozolid-2-onyl and isothiazolid-2-onyl. Examples of 5-6 membered heterocycloalkyl groups include, but are not limited to, piperidinyl, piperazinyl, pyrrolidinyl, pyrrolidin-2-onyl, dioxanyl, morpholinyl, tetrahydrofuranyl, tetrahydrothiophenyl, tetrahydropyranyl, oxazolidinyl, isothiazolidinyl oxozolid-2-onyl and isothiazolid-2-onyl.

As used herein, the term "aryl" refers to an aromatic cyclic hydrocarbon group having the indicated number of carbon atoms. For example, the term "5-6 membered aryl" as used herein refers to an aromatic cyclic hydrocarbon group having five or six carbon atoms. Examples of 5-6 membered aryls include cyclopentadienyl and phenyl.

As used herein, the term "heteroaryl" refers to an aromatic cyclic group having the indicated number of atoms selected from C, N, O and S. For example, the term "5-6 membered heteroaryl" as used herein refers to an aromatic cyclic group having five or six ring atoms, one, two or three of which are selected from N, O and S, the remainder being C. Examples of 5-6 membered heteroaryls include, but are not limited to, pyridinyl, pyrazinyl, pyrimidinyl, pyridazinyl, furanyl, pyrrolyl, thiophenyl, imidazolyl, pyrazolyl, triazolyl, oxazolyl, isoxazolyl, thiazolyl, isothiazolyl, oxadiazolyl and thiadiazolyl. Examples of 6 membered heteroaryls include, but are not limited to, pyridinyl, pyrazinyl, pyrimidinyl and pyridazinyl.

As used herein the term "halogen" or "halo" refers to F (fluoro), Cl (chloro), Br (bromo) and I (iodo).

As used herein the term "halomethyl" refers to -CH₃, in which one or more hydrogen atoms is/are replaced with an independently selected halo.

As used herein the term "oxo" refers to the substitution of CH₂ with O to form C(O).

As used herein the term "N(C₁-C₃ alkyl)₂" allows the independent selection of each C₁-C₃ alkyl substituent, for example, N may be substituted by methyl and ethyl.

As used herein the substituent -NR⁴C(O) is connected to R² through N.

In the compounds of formula (I), (II), (III), (IA), (IIA) or (IIIA), A can be pyrazole, 1,2,3 triazole, 1,2,4 triazole, 1,2,3 thiadiazole, 1,2,4 thiadiazole, 1,2,5 thiadiazole, 1,3,4 thiadiazole, 1,2,3 oxadiazole, 1,2,4 oxadiazole, 1,2,5 oxadiazole or 1,3,4 oxadiazole, substituted with R¹ and R^{1A}.

In the compounds of formula (I), (II), (III), (IA), (IIA) or (IIIA), A can be pyrazole, 1,2,3 triazole or 1,2,4 triazole, substituted with R¹ and R^{1A}.

In the compounds of formula (I), (II), (III), (IA), (IIA) or (IIIA), A can be pyrazole, 1,2,3 triazole or 1,2,4 triazole, substituted with R¹ and R^{1A}, wherein R^{1A} is hydrogen and R¹ is C₁-C₃ alkyl.

In the compounds of formula (I), (II), (III), (IA), (IIA) or (IIIA), A can be pyrazole, 1,2,3 triazole or 1,2,4 triazole, substituted with R¹ and R^{1A}, wherein R^{1A} is hydrogen and R¹ is CH₃.

In the compounds of formula (I), (II), (III), (IA) or (IIA), A can be: 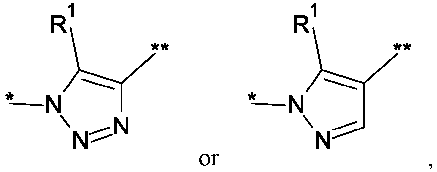 wherein * indicates the connection point to Z, Z' or Z₂ and ** indicates the other connection point from A in formula (I), (II), (III), (IA) or (IIA); and R¹ can be C₁-C₃ alkyl;

In the compounds of formula (IIIA), A can be: 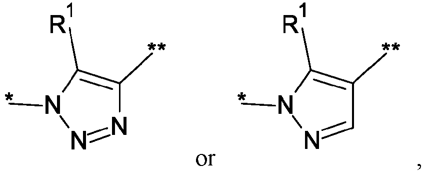 wherein * indicates the connection point to the substituent comprising Y₁ and ** indicates the other connection point from A in formula (IIIA); and R¹ can be C₁-C₃ alkyl;

In the compounds of formula (I), Z can be CHR^{9A}, cyclobutyl, azetidine, pyrrolidine or piperidine.

In the compounds of formula (I) or (IA), Z can be a bond, 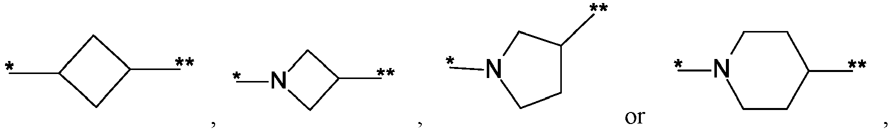 wherein * indicates the connection point to Z₁ and ** indicates the connection point to A in formula (I) or (IA).

In the compounds of formula (I) or (IA), Z can be a bond, 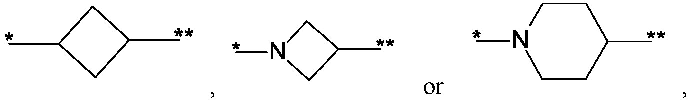 wherein * indicates the connection point to Z₁ and ** indicates the connection point to A in formula (I) or (IA).

In the compounds of formula (I) or (IA), Z can be CHR^{9A}, Z₁ can be selected from CH₂ or CH₂-CH₂, and R⁹ can be fused with R^{9A} to form CH₂ or CH₂-CH₂.

In the compounds of formula (I) or (IA), Z can be CHR^{9A}, Z₁ can be CH₂, and R⁹ can be fused with R^{9A} to form CH₂ or CH₂-CH₂.

In the compounds of formula (I) or (IA), Z can be CHR^{9A}, Z₁ can be CH₂-CH₂, and R⁹ can be fused with R^{9A} to form CH₂ or CH₂-CH₂.

In the compounds of formula (I) or (IA), Z can be CHR^{9A}, Z₁ can be selected from CH₂ or CH₂-CH₂, and R⁹ can be fused with R^{9A} to form CH₂.

In the compounds of formula (I) or (IA), Z can be CHR^{9A}, Z₁ can be selected from CH₂ or CH₂-CH₂, and R⁹ can be fused with R^{9A} to form CH₂-CH₂.

In the compounds of formula (I) or (IA), Z can be CHR^{9A}, Z₁ can be CH₂, and R⁹ can be fused with R^{9A} to form CH₂.

In the compounds of formula (I) or (IA), Z can be CHR^{9A}, Z₁ can be CH₂-CH₂, and R⁹ can be fused with R^{9A} to form CH₂-CH₂.

In the compounds of formula (II) or (IIA), Z' can be: 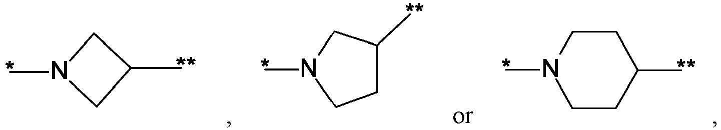 wherein ** indicates the connection point to A and * indicates the other connection point from Z' in formula (II) or (IIA).

In the compounds of formula (II) or (IIA), Z' can be: 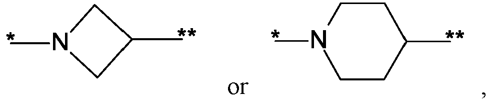 wherein ** indicates the connection point to A and * indicates the other connection point from Z' in formula (II) or (IIA).

In the compounds of formula (I) or (IA), Z can be a bond.

In the compounds of formula (II) or (IIA), Z' can be a bond.

In the compounds of formula (I), (II), (IA) or (IIA), Z₁ can be a bond.

In the compounds of formula (I), (II), (III), (IA), (IIA) or (IIIA), Y can be NH or O.

In the compounds of formula (I), (II), (III), (IA), (IIA) or (IIIA), Y can be O.

In the compounds of formula (I), (II), (III), (IA), (IIA) or (IIIA), Y₁ can be a bond, CHR⁷, CH₂-CHR⁷ or CHR⁷-CH₂, wherein R⁷ is selected from hydrogen, F, OH and CH₃; and Y₂ can a bond, CHR³, CH₂-CHR³ or CHR³-CH₂, wherein R³ is selected from hydrogen, F, OH and CH₃.

In the compounds of formula (I), (II), (III), (IA), (IIA) or (IIIA), Y₁ can be a bond or CHR⁷, wherein R⁷ is hydrogen, F, OH or CH₃; and Y₂ can a bond or CHR³, wherein R³ is hydrogen, F, OH or CH₃.

In the compounds of formula (I), (II), (III), (IA) or (IIA), Y₁ can be a bond, CHR⁷, CH₂-CHR⁷ or CHR⁷-CH₂, wherein R⁷ is hydrogen, F, OH or CH₃; and Y₂ can a bond, CHR³, CH₂-CHR³ or CHR³-CH₂, wherein R³ is hydrogen, F, OH or CH₃, forming: 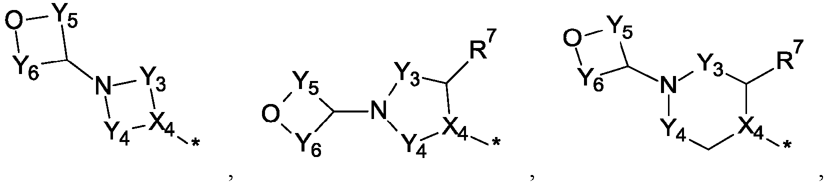 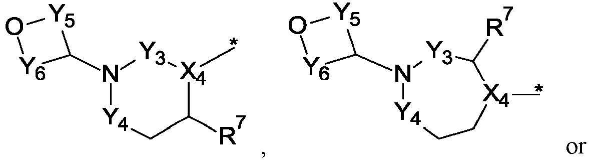 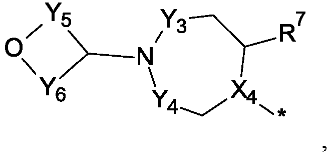 wherein * indicates the connection point to Z₁, in formula (I) or (IA); Z' in formula (II) or (IIA); or A in formula (III).

In the compounds of formula (I), (II), (III), (IA) or (IIA), Y₁ can be a bond or CHR⁷, wherein R⁷ is hydrogen, F, OH or CH₃; and Y₂ can a bond or CHR³, wherein R³ is hydrogen, F, OH or CH₃, forming: 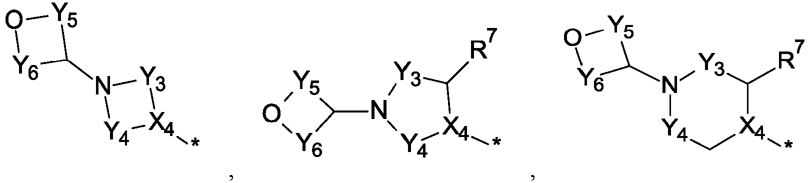 or 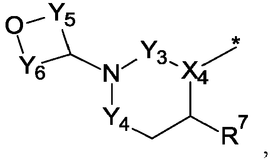 wherein * indicates the connection point to Z₁ in formula (I) or (IA); Z' in formula (II) or (IIA); or A in formula (III).

In the compounds of formula (I), (II), (III), (IA), (IIA) or (IIIA), Y₁ can be a bond, CHR⁷, CH₂-CHR⁷ or CHR⁷-CH₂, wherein R⁷ is hydrogen, F, OH or CH₃; and Y₂ can a bond, CHR³, CH₂-CHR³ or CHR³-CH₂, wherein R³ is hydrogen, F, OH or CH₃, forming: 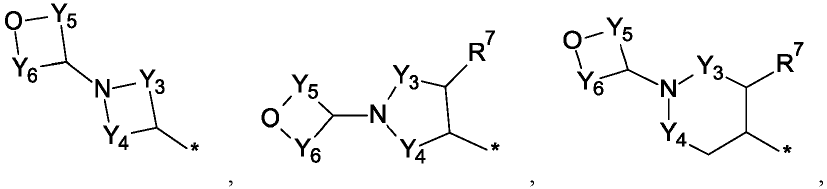 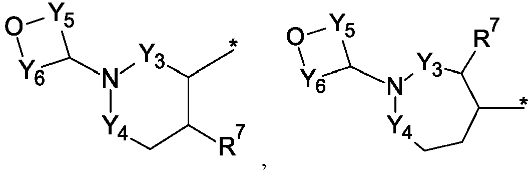 or 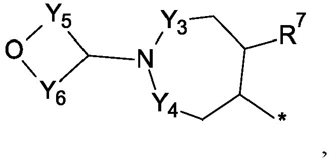 wherein * indicates the connection point to A in formula (I), (II), (III), (IA), (IIA) or (IIIA).

In the compounds of formula (I), (II), (III), (IA), (IIA) or (IIIA), Y₁ can be a bond or CHR⁷, wherein R⁷ is hydrogen, F, OH or CH₃; and Y₂ can a bond or CHR³, wherein R³ is hydrogen, F, OH or CH₃, forming: 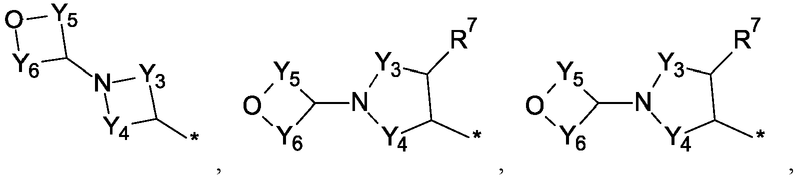 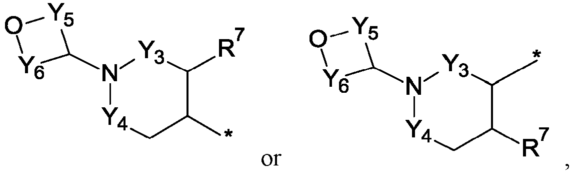 wherein * indicates the connection point to A in formula (I), (II), (III), (IA), (IIA) or (IIIA), or wherein * indicates the connection point to Z₁ in formula (I) or (IA); Z' in formula (II) or (IIA); or A in formula (III) or (IIIA).

In the compounds of formula (I), (II), (III), (IA), (IIA) or (IIIA), R^{1A} can be hydrogen or C₁-C₃ alkyl optionally substituted with one or more substituents independently selected from halo, OH and OCH₃.

In the compounds of formula (I), (II), (III), (IA), (IIA) or (IIIA), R^{1A} can be hydrogen or CH₃.

In the compounds of formula (I), (II), (III), (IA), (IIA) or (IIIA), R^{1A} can be hydrogen.

In the compounds of formula (I), (II), (III), (IA), (IIA) or (IIIA), R¹ can be methyl, ethyl or propyl.

In the compounds of formula (I), (II), (III), (IA), (IIA) or (IIIA), R¹ can be methyl.

In the compounds of formula (I), (II), (III), (IA), (IIA) or (IIIA), R² can be C₁-C₃ alkyl optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH3, methylamine, N,N-dimethylamine and CN.

In the compounds of formula (I), (II), (III), (IA), (IIA) or (IIIA), R² can be C₁-C₃ alkyl optionally substituted with one or more substituents independently selected from F, OH, CN, oxo, -OCH₃, -OC₃ cycloalkyl and R¹⁰.

In the compounds of formula (I), (II), (III), (IA), (IIA) or (IIIA), R² can be: 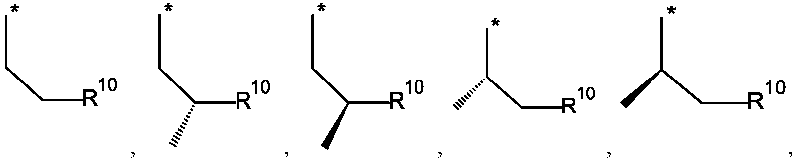 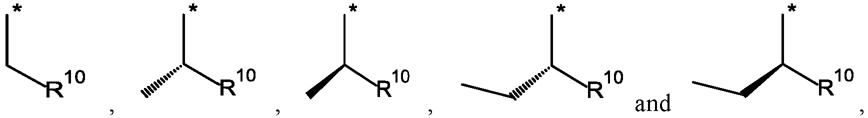 optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, wherein * indicates the connection point to Y in formula (I), (II), (III), (IA), (IIA) or (IIIA).

In the compounds of formula (I), (II), (III), (IA), (IIA) or (IIIA), R² can be: 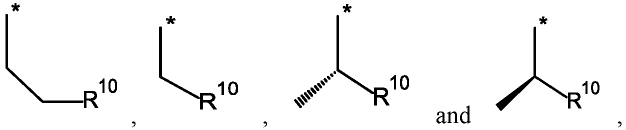 optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, wherein * indicates the connection point to Y in formula (I), (II), (III), (IA), (IIA) or (IIIA).

In the compounds of formula (I), (II), (III), (IA), (IIA) or (IIIA), R² can be: 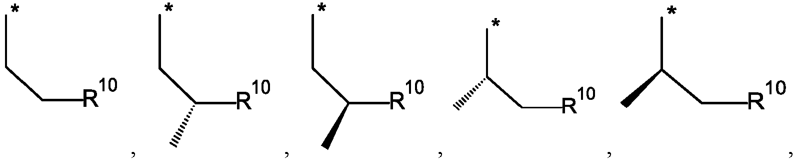 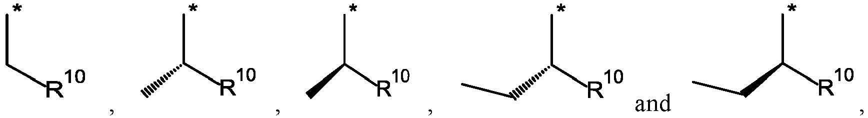 optionally substituted with one, two, three or four substituents independently selected from F, OH, CN, oxo, -OCH₃, -OC₃ cycloalkyl and R¹⁰, wherein * indicates the connection point to Y in formula (I), (II), (III), (IA), (IIA) or (IIIA).

In the compounds of formula (I), (II), (III), (IA), (IIA) or (IIIA), R² can be: 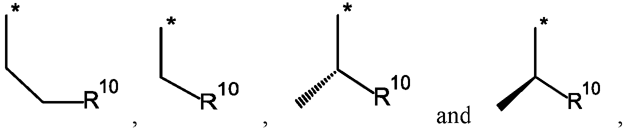 optionally substituted with one, two, three or four substituents independently selected from F, OH, CN, oxo, -OCH₃ and -OC₃ cycloalkyl and R¹⁰, wherein * indicates the connection point to Y in formula (I), (II), (III), (IA), (IIA) or (IIIA).

In the compounds of formula (I), (II), (III), (IA), (IIA) or (IIIA), Y₁ can be a bond, CHR⁷, CH₂-CHR⁷ or CHR⁷-CH₂, wherein R⁷ can be selected from hydrogen, F, OH and CH₃.

In the compounds of formula (I), (II), (III), (IA), (IIA) or (IIIA), Y₂ can be a bond, CHR³, CH₂-CHR³ or CHR³-CH₂, wherein R³ can be selected from hydrogen, F, OH and CH₃.

In the compounds of formula (I), (II), (III), (IA), (IIA) or (IIIA), Y₃ can be CR⁴R⁵ or CF₂, wherein R⁴ is hydrogen or CH₃ and R⁵ is hydrogen, F, OH or CH₃; and Y₄ is CR³R⁴ or CF₂ wherein R⁴ is hydrogen or CH₃, and R³ is hydrogen, F, OH or CH₃.

In the compounds of formula (I), (II), (III), (IA), (IIA) or (IIIA), Y₃ can be CR⁴R⁵, wherein R⁴ is hydrogen and R⁵ is fused with one R³ to form CH₂, CH₂-CH₂ or CH₂OCH₂; and Y₄ is CR³R⁴ wherein R⁴ is hydrogen, and R³ is fused with R⁵ to form CH₂, CH₂-CH₂ or CH₂OCH₂.

In the compounds of formula (I), (II), (III), (IA) or (IIA), Y₃ can be CR⁴R⁵, wherein R⁴ is hydrogen and R⁵ is fused with one R³ to form CH₂, CH₂-CH₂ or CH₂OCH₂; and Y₄ is CR³R⁴ wherein R⁴ is hydrogen, and R³ is fused with R⁵ to form CH₂, CH₂-CH₂ or CH₂OCH₂, forming: 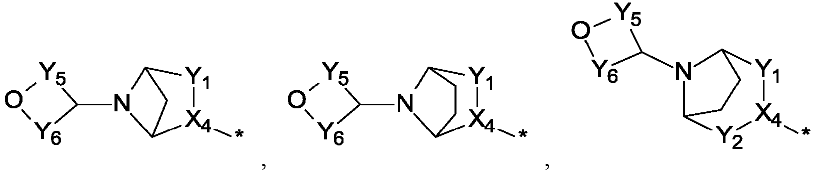 or 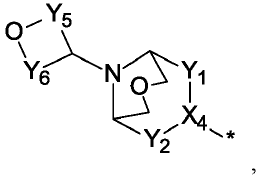 wherein * indicates the connection point to Z₁ in formula (I) or (IA); Z' in formula (II) or (IIA); or A in formula (III).

In the compounds of formula (IIIA), Y₃ can be CR⁴R⁵, wherein R⁴ is hydrogen and R⁵ is fused with one R³ to form CH₂, CH₂-CH₂ or CH₂OCH₂; and Y₄ is CR³R⁴ wherein R⁴ is hydrogen, and R³ is fused with R⁵ to form CH₂, CH₂-CH₂ or CH₂OCH₂, forming: 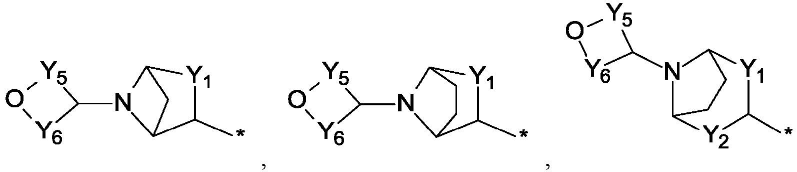 or 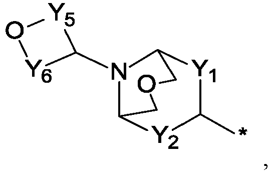 wherein * indicates the connection point A in formula (IIIA), or , or wherein * indicates the connection point to Z₁ in formula (I) or (IA); Z' in formula (II) or (IIA); or A in formula (III) or (IIIA).

In the compounds of formula (I), (II), (III), (IA), (IIA) or (IIIA), Y₅ can be CR¹³R¹⁴, CR¹³R¹⁴-CH₂ or CH₂-CR¹³R¹⁴, wherein R¹³ and R¹⁴ are independently selected from H and CH₃; and Y₆ can be CR¹³R¹⁴, CR¹³R¹⁴-CH₂ or CH₂-CR¹³R¹⁴, wherein R¹³ and R¹⁴ are independently selected from H and CH₃.

In the compounds of formula (I), (II), (III), (IA), (IIA) or (IIIA), Y₅ can be CH₂ or CH₂-CH₂ and Y₆ can be CH₂ or CH₂-CH₂.

In the compounds of formula (I), (II), (III), (IA), (IIA) or (IIIA), Y₅ and Y₆ can be CH₂.

In the compounds of formula (I), (II), (III), (IA) or (IIA), X₄ can be N or C-R⁹ wherein R⁹ is hydrogen or CH₃.

In the compounds of formula (I) or (IA), X₄ can be C-R⁹ wherein R⁹ is fused with R^{9A} to form CH₂ or CH₂-CH₂; and Z₁ is CH₂ or CH₂-CH₂.

In the compounds of formula (I), (II), (III), (IA) or (IIA), X₄ can be N or CH.

In the compounds of formula (I), (II), (III), (IA), (IIA) or (IIIA), R⁶ can be CN, F, Cl, CH₃, CF₃ or cyclopropyl.

In the compounds of formula (I), (II), (III), (IA), (IIA) or (IIIA), R⁶ can be CN, F or Cl.

In the compounds of formula (I), (II), (III), (IA), (IIA) or (IIIA), R⁶ can be CN or Cl.

In the compounds of formula (I), (II), (III), (IA), (IIA) or (IIIA), R⁶ can be CN.

In the compounds of formula (I), (II), (III), (IA), (IIA) or (IIIA), R⁶ can be Cl.

In the compounds of formula (I), (II), (III), (IA), (IIA) or (IIIA), R⁸ can be 5-6 membered cycloalkyl, 5-6 membered heterocycloalkyl, 5-6 membered aryl or 5-6 membered heteroaryl, optionally fused or substituted with R^{8A}.

In the compounds of formula (I), (II), (III), (IA), (IIA) or (IIIA), R⁸ can be 5-6 membered cycloalkyl or 5-6 membered heterocycloalkyl, optionally fused with R^{8A}.

In the compounds of formula (I), (II), (III), (IA), (IIA) or (IIIA), R⁸ can be cyclopentyl, cyclohexyl, piperidinyl, pyrrolidinyl, tetrahydrofuranyl, tetrahydropyranyl, phenyl or pyridinyl, optionally fused with R^{8A}.

In the compounds of formula (I), (II), (III), (IA), (IIA) or (IIIA), R⁸ can be cyclopentyl, cyclohexyl, piperidinyl, pyrrolidinyl, tetrahydrofuranyl or tetrahydropyranyl, fused with R^{8A}.

In the compounds of formula (I), (II), (III), (IA), (IIA) or (IIIA), R⁸ can be cyclopentyl, cyclohexyl, piperidinyl, pyrrolidinyl, tetrahydrofuranyl or tetrahydropyranyl, fused with R^{8A}, wherein R^{8A} can be phenyl or 6 membered heteroaryl.

In the compounds of formula (I), (II), (III), (IA) or (IIA), R⁹ can be hydrogen.

In the compounds of formula (I), (II), (III), (IA), (IIA) or (IIIA), R¹⁰ can be 3-6 membered cycloalkyl, 5-6 membered heterocycloalkyl, 5-6 membered aryl or 5-6 membered heteroaryl, optionally fused with R^{8A}.

In the compounds of formula (I), (II), (III), (IA), (IIA) or (IIIA), R¹⁰ can be 3-6 membered cycloalkyl, 5-6 membered heterocycloalkyl, phenyl or 5-6 membered heteroaryl, optionally fused or substituted with R^{8A}.

In the compounds of formula (I), (II), (III), (IA), (IIA) or (IIIA), R¹⁰ can be cyclopropyl, cyclobutyl, cyclopentyl, cyclohexyl, piperidinyl, piperazinyl, pyrrolidinyl, pyrrolidin-2-onyl, dioxanyl, morpholinyl, tetrahydrofuranyl, tetrahydrothiophenyl, tetrahydropyranyl, oxazolidinyl, isothiazolidinyl, oxozolid-2-onyl, isothiazolid-2-onyl, phenyl, pyridinyl, pyrazinyl, pyrimidinyl, pyridazinyl, furanyl, pyrrolyl, thiophenyl, imidazolyl, pyrazolyl, triazolyl, oxazolyl, isoxazolyl, thiazolyl, isothiazolyl, oxadiazolyl or thiadiazolyl, optionally fused or substituted with R^{8A}.

In the compounds of formula (I), (II), (III), (IA), (IIA) or (IIIA), R¹⁰ can be cyclopropyl, cyclobutyl, cyclopentyl, cyclohexyl, tetrahydrofuranyl, tetrahydropyranyl, phenyl, pyridinyl, pyrazinyl, pyrimidinyl, pyridazinyl, pyrazolyl, triazolyl, oxazolyl, isoxazolyl, thiazolyl, isothiazolyl, oxadiazolyl or thiadiazolyl, optionally fused or substituted with R^{8A}.

In the compounds of formula (I), (II), (III), (IA), (IIA) or (IIIA), R¹⁰ can be cyclopropyl, cyclobutyl, cyclopentyl, cyclohexyl, tetrahydrofuranyl, tetrahydropyranyl, phenyl, pyridinyl, pyrazinyl, pyrimidinyl, pyridazinyl, oxazolyl, isoxazolyl, thiazolyl, isothiazolyl, oxadiazolyl or thiadiazolyl, optionally fused or substituted with R^{8A}.

In the compounds of formula (I), (II), (III), (IA), (IIA) or (IIIA), R¹⁰ can be cyclopropyl, cyclobutyl, phenyl, pyridinyl, oxazolyl, isoxazolyl, thiazolyl or isothiazolyl, optionally fused or substituted with R^{8A}.

In the compounds of formula (I), (II), (III), (IA), (IIA) or (IIIA), R¹⁰ can be cyclopropyl, cyclobutyl, cyclopentyl, cyclohexyl, piperidinyl, piperazinyl, pyrrolidinyl, pyrrolidin-2-onyl, dioxanyl, morpholinyl, tetrahydrofuranyl, tetrahydrothiophenyl, tetrahydropyranyl, oxazolidinyl, isothiazolidinyl, oxozolid-2-onyl, isothiazolid-2-onyl, phenyl, pyridinyl, pyrazinyl, pyrimidinyl, pyridazinyl, furanyl, pyrrolyl, thiophenyl, imidazolyl, pyrazolyl, triazolyl, oxazolyl, isoxazolyl, thiazolyl, isothiazolyl, oxadiazolyl or thiadiazolyl.

In the compounds of formula (I), (II), (III), (IA), (IIA) or (IIIA), R¹⁰ can be cyclopropyl, cyclobutyl, cyclopentyl, cyclohexyl, tetrahydrofuranyl, tetrahydropyranyl, phenyl, pyridinyl, pyrazinyl, pyrimidinyl, pyridazinyl, pyrazolyl, triazolyl, oxazolyl, isoxazolyl, thiazolyl, isothiazolyl, oxadiazolyl or thiadiazolyl.

In the compounds of formula (I), (II), (III), (IA), (IIA) or (IIIA), R¹⁰ can be cyclopropyl, cyclobutyl, cyclopentyl, cyclohexyl, tetrahydrofuranyl, tetrahydropyranyl, phenyl, pyridinyl, pyrazinyl, pyrimidinyl, pyridazinyl, oxazolyl, isoxazolyl, thiazolyl, isothiazolyl, oxadiazolyl or thiadiazolyl.

In the compounds of formula (I), (II), (III), (IA), (IIA) or (IIIA), R¹⁰ can be cyclopropyl, cyclobutyl, phenyl, pyridinyl, oxazolyl, isoxazolyl, thiazolyl or isothiazolyl.

In the compounds of formula (I), (II), (III), (IA), (IIA) or (IIIA), R¹⁰ can be cyclopentyl, cyclohexyl, tetrahydrofuranyl, tetrahydropyranyl, phenyl, pyridinyl, pyrazinyl, pyrimidinyl, pyridazinyl, pyrazolyl, triazolyl, oxazolyl, isoxazolyl, thiazolyl, isothiazolyl, oxadiazolyl or thiadiazolyl, fused or substituted with R^{8A}.

In the compounds of formula (I), (II), (III), (IA), (IIA) or (IIIA), R¹⁰ can be cyclopentyl, cyclohexyl, piperidinyl, pyrrolidinyl, tetrahydrofuranyl, tetrahydropyranyl, phenyl or pyridinyl, fused or substituted with R^{8A}.

In the compounds of formula (I), (II), (III), (IA), (IIA) or (IIIA), R¹⁰ can be cyclopentyl, cyclohexyl, piperidinyl, pyrrolidinyl, tetrahydrofuranyl, tetrahydropyranyl, phenyl or pyridinyl, fused or substituted with R^{8A}.

In the compounds of formula (I), (II), (III), (IA), (IIA) or (IIIA), R¹⁰ can be cyclopentyl, cyclohexyl, piperidinyl, pyrrolidinyl, tetrahydrofuranyl, tetrahydropyranyl, phenyl or pyridinyl, fused or substituted with R^{8A}.

In the compounds of formula (I), (II), (III), (IA), (IIA) or (IIIA), R¹⁰ can be cyclopentyl, cyclohexyl, phenyl or pyridinyl, fused or substituted with R^{8A}.

In the compounds of formula (I), (II), (III), (IA), (IIA) or (IIIA), R¹⁰ can be phenyl or pyridinyl, fused with R^{8A} wherein R^{8A} can be 5-6 membered heterocycloalkyl or 5-6 membered heteroaryl.

In the compounds of formula (I), (II), (III), (IA), (IIA) or (IIIA), R¹⁰ can be phenyl or pyridinyl, fused with R^{8A} wherein R^{8A} can be pyrrolidinyl, pyrrolidin-2-onyl, dioxanyl, tetrahydrofuranyl, tetrahydrothiophenyl, tetrahydropyranyl, oxazolidinyl, isothiazolidinyl, oxozolid-2-onyl, isothiazolid-2-onyl furanyl, pyrrolyl, thiophenyl, imidazolyl, pyrazolyl, triazolyl, oxazolyl, isoxazolyl, thiazolyl, isothiazolyl, oxadiazolyl or thiadiazolyl.

In the compounds of formula (I), (II), (III), (IA), (IIA) or (IIIA), R¹⁰ can be phenyl or pyridinyl, fused with R^{8A} wherein R^{8A} can be tetrahydrofuranyl, tetrahydrothiophenyl, tetrahydropyranyl, oxazolidinyl, isothiazolidinyl, oxozolid-2-onyl, isothiazolid-2-onyl, furanyl, pyrrolyl, thiophenyl, imidazolyl, pyrazolyl, triazolyl, oxazolyl, isoxazolyl, thiazolyl, isothiazolyl, oxadiazolyl or thiadiazolyl.

In the compounds of formula (I), (II), (III), (IA), (IIA) or (IIIA), R¹⁰ can be phenyl or pyridinyl, fused with R^{8A} wherein R^{8A} can be tetrahydrofuranyl, tetrahydrothiophenyl, tetrahydropyranyl, oxazolidinyl, isothiazolidinyl, oxozolid-2-only or isothiazolid-2-onyl.

In the compounds of formula (I) or (IA), where both Z and Z₁ are a bond, together they form a single bond.

In the compounds of formula (I) or (IA), Z can be CHR^{9A}, Z₁ can be CH₂, X₄ can be C-R⁹, and R⁹ can be fused with R^{9A} to form CH₂, forming: 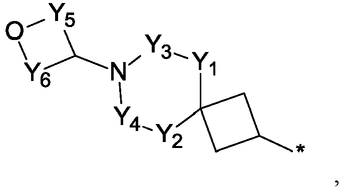 wherein * indicates the connection point to A.

In the compounds of formula (I), (II), (III), (IA) or (IIA), R⁵ can be fused with one R³ to form CH₂-CH₂, for example forming: 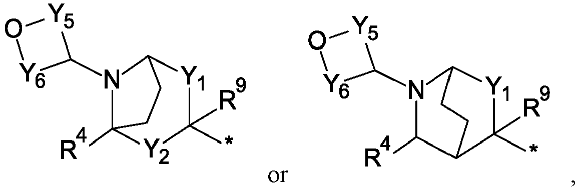 wherein * indicates the connection point to Z₁ in formula (I) or (IA); or Z' in formula (II) or (IIA); or A in formula (III).

In the compounds of formula (I), (II), (III), (IA), (IIA) or (IIIA), R⁵ can be fused with one R³ to form CH₂-CH₂, for example forming: 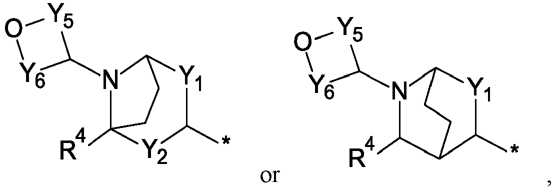 wherein * indicates the connection point to Z₁ in formula (I) or (IA); or Z' in formula (II) or (IIA); or A in formula (III) or (IIIA).

In the compounds of formula (I), (II), (III), (IA), (IIA) or (IIIA), R⁸ can be cyclopentyl, fused with R^{8A}, wherein R^{8A} can be pyridinyl, for example forming: 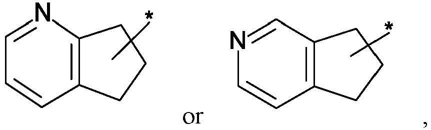 wherein * indicates the connection point to Y.

In the compounds of formula (I), (II), (III), (IA), (IIA) or (IIIA), A can be pyrazole, 1,2,3 triazole or 1,2,4 triazole, substituted with R¹ and R^{1A}; and Y can be NH or O.

In the compounds of formula (I), (II), (III), (IA), (IIA) or (IIIA), A can be pyrazole, 1,2,3 triazole or 1,2,4 triazole, substituted with R¹ and R^{1A}, wherein R^{1A} is hydrogen and R¹ is C₁-C₃ alkyl; and Y can be NH or O.

In the compounds of formula (I), (II), (III), (IA) or (IIA), A can be: 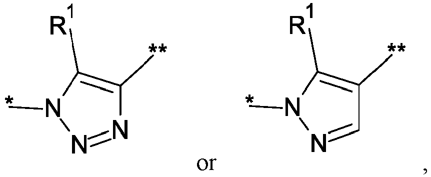 wherein * indicates the connection point to Z, Z' or Z₂ and ** indicates the other connection point from A in formula (I), (II), (III), (IA) or (IIA); R¹ can be C₁-C₃ alkyl; and Y can be NH or O.

In the compounds of formula (IIIA), A can be: 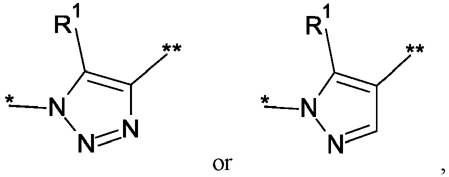 wherein * indicates the connection point to the substituent comprising Y₁ and ** indicates the other connection point from A in formula (IIIA); R¹ can be C₁-C₃ alkyl; and Y can be NH or O.

In the compounds of formula (I), (II), (III), (IA), (IIA) or (IIIA), A can be pyrazole, 1,2,3 triazole or 1,2,4 triazole, substituted with R¹ and R^{1A}; and Y can be O.

In the compounds of formula (I), (II), (III), (IA), (IIA) or (IIIA), A can be pyrazole, 1,2,3 triazole or 1,2,4 triazole, substituted with R¹ and R^{1A}, wherein R^{1A} is hydrogen and R¹ is C₁-C₃ alkyl; and Y can be O.

In the compounds of formula (I), (II), (III), (IA) or (IIA), A can be: 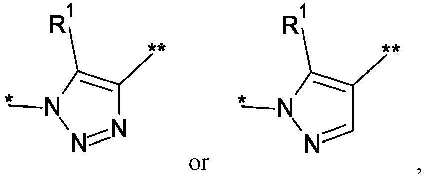 wherein * indicates the connection point to Z, Z' or Z₂ and ** indicates the other connection point from A in formula (I), (II), (III), (IA) or (IIA); R¹ can be C₁-C₃ alkyl; and Y can be O.

In the compounds of formula (IIIA), A can be: 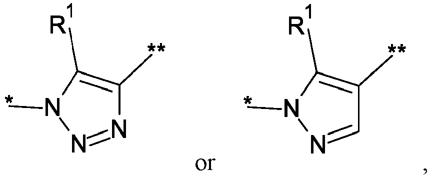 wherein * indicates the connection point to the substituent comprising Y₁ and ** indicates the other connection point from A in formula (IIIA); R¹ can be C₁-C₃ alkyl; and Y can be O.

In the compounds of formula (I), (II), (III), (IA), (IIA) or (IIIA), A can be pyrazole, 1,2,3 triazole or 1,2,4 triazole, substituted with R¹ and R^{1A}; and R⁶ can be CN, F, Cl, CH₃, CF₃ or cyclopropyl.

In the compounds of formula (I), (II), (III), (IA), (IIA) or (IIIA), A can be pyrazole, 1,2,3 triazole or 1,2,4 triazole, substituted with R¹ and R^{1A}, wherein R^{1A} is hydrogen and R¹ is C₁-C₃ alkyl; and R⁶ can be CN, F, Cl or CF₃.

In the compounds of formula (I), (II), (III), (IA) or (IIA), A can be: 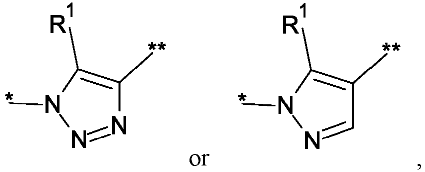 wherein * indicates the connection point to Z, Z' or Z₂ and ** indicates the other connection point from A in formula (I), (II), (III), (IA) or (IIA); and R⁶ can be CN, F, Cl, CH₃, CF₃ or cyclopropyl.

In the compounds of formula (IIIA), A can be: 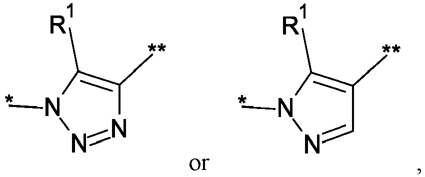 wherein * indicates the connection point to the substituent comprising Y₁ and ** indicates the other connection point from A in formula (IIIA) and R⁶ can be CN, F, Cl, CH₃, CF₃ or cyclopropyl.

In the compounds of formula (I), (II), (III), (IA) or (IIA), A can be: 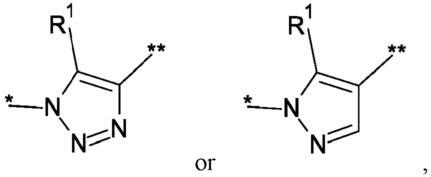 wherein * indicates the connection point to Z, Z' or Z₂ and ** indicates the other connection point from A in formula (I), (II), (III), (IA) or (IIA); and R⁶ can be CN, F, Cl or CF₃.

In the compounds of formula (IIIA), A can be: 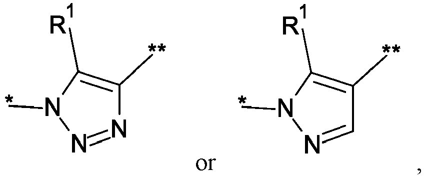 wherein * indicates the connection point to the substituent comprising Y₁ and ** indicates the other connection point from A in formula (IIIA) and R⁶ can be CN, F, Cl, CH_{3 or} CF₃.

In the compounds of formula (I), (II), (III), (IA) or (IIA), A can be: 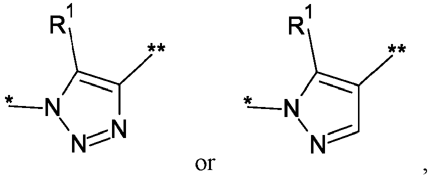 wherein * indicates the connection point to Z, Z' or Z₂ and ** indicates the other connection point from A in formula (I), (II), (III), (IA) or (IIA); R¹ can be C₁-C₃ alkyl; and R⁶ can be CN or Cl.

In the compounds of formula (IIIA), A can be: 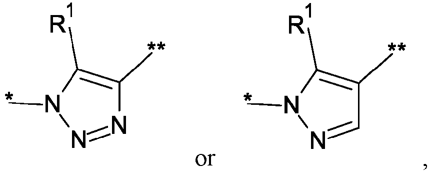 wherein * indicates the connection point to the substituent comprising Y₁ and ** indicates the other connection point from A in formula (IIIA); R¹ can be C₁-C₃ alkyl; and R⁶ can be CN or Cl.

In the compounds of formula (I), (II), (III), (IA), (IIA) or (IIIA), A can be pyrazole, 1,2,3 triazole or 1,2,4 triazole, substituted with R¹ and R^{1A}; R⁶ can be CN, F, Cl, CH₃, CF₃ or cyclopropyl; and Y can be NH or O.

In the compounds of formula (I), (II), (III), (IA), (IIA) or (IIIA), A can be pyrazole, 1,2,3 triazole or 1,2,4 triazole, substituted with R¹ and R^{1A}, wherein R^{1A} is hydrogen and R¹ is C₁-C₃ alkyl; R⁶ can be CN, F, Cl or CF₃; and Y can be NH or O.

In the compounds of formula (I), (II), (III), (IA) or (IIA), A can be: 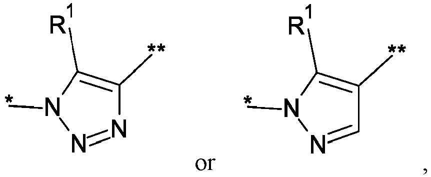 wherein * indicates the connection point to Z, Z' or Z₂ and ** indicates the other connection point from A in formula (I), (II), (III), (IA) or (IIA); R⁶ can be CN, F, Cl, CH₃, CF₃ or cyclopropyl; and Y can be NH or O.

In the compounds of formula (IIIA), A can be: 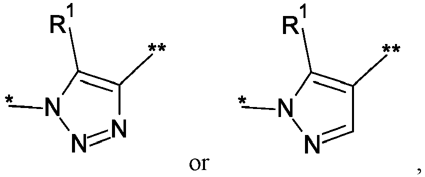 wherein * indicates the connection point to the substituent comprising Y₁ and ** indicates the other connection point from A in formula (IIIA); R⁶ can be CN, F, Cl, CH₃, CF₃ or cyclopropyl; and Y can be NH or O.

In the compounds of formula (I), (II), (III), (IA) or (IIA), A can be: 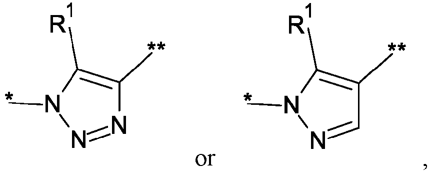 wherein * indicates the connection point to Z, Z' or Z₂ and ** indicates the other connection point from A in formula (I), (II), (III), (IA) or (IIA); R⁶ can be CN, F, Cl or CF₃; and Y can be NH or O.

In the compounds of formula (IIIA), A can be: 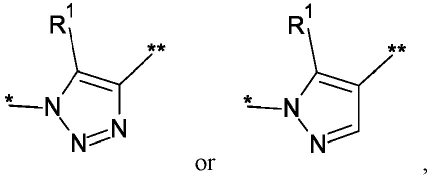 wherein * indicates the connection point to the substituent comprising Y₁ and ** indicates the other connection point from A in formula (IIIA); R⁶ can be CN, F, Cl or CF₃; and Y can be NH or O.

In the compounds of formula (I), (II), (III), (IA), (IIA) or (IIIA), A can be pyrazole, 1,2,3 triazole or 1,2,4 triazole, substituted with R¹ and R^{1A}; R⁶ can be CN, F, Cl, CH₃, CF₃ or cyclopropyl; and Y can be O.

In the compounds of formula (I), (II), (III), (IA), (IIA) or (IIIA), A can be pyrazole, 1,2,3 triazole or 1,2,4 triazole, substituted with R¹ and R^{1A}, wherein R^{1A} is hydrogen and R¹ is C₁-C₃ alkyl; R⁶ can be CN, F, Cl or CF₃; and Y can be O.

In the compounds of formula (I), (II), (III), (IA) or (IIA), A can be: 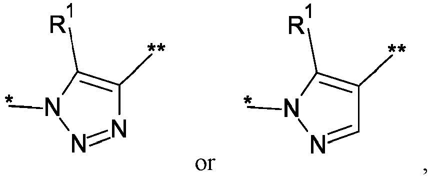 wherein * indicates the connection point to Z, Z' or Z₂ and ** indicates the other connection point from A in formula (I), (II), (III), (IA) or (IIA); R⁶ can be CN, F, Cl, CH₃, CF₃ or cyclopropyl; and Y can be O.

In the compounds of formula (IIIA), A can be: 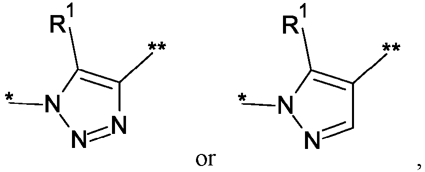 wherein * indicates the connection point to the substituent comprising Y₁ and ** indicates the other connection point from A in formula (IIIA); R⁶ can be CN, F, Cl, CH₃, CF₃ or cyclopropyl; and Y can be O.

In the compounds of formula (I), (II), (III), (IA) or (IIA), A can be: 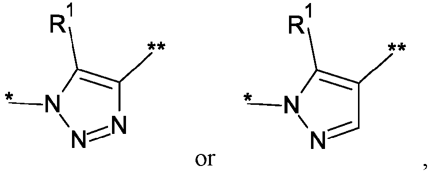 wherein * indicates the connection point to Z, Z' or Z₂ and ** indicates the other connection point from A in formula (I), (II), (III), (IA) or (IIA); R⁶ can be CN, F, Cl or CF₃; and Y can be O.

In the compounds of formula (IIIA), A can be: 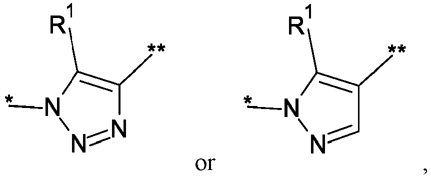 wherein * indicates the connection point to the substituent comprising Y₁ and ** indicates the other connection point from A in formula (IIIA); R⁶ can be CN, F, Cl or CF₃; and Y can be O.

In the compounds of formula (I), (II), (III), (IA) or (IIA), A can be: 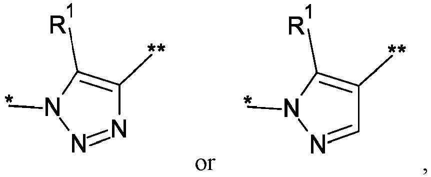 wherein * indicates the connection point to Z, Z' or Z₂ and ** indicates the other connection point from A in formula (I), (II), (III), (IA) or (IIA); R¹ can be C₁-C₃ alkyl; R⁶ can be CN or Cl; and Y can be O.

In the compounds of formula (IIIA), A can be: 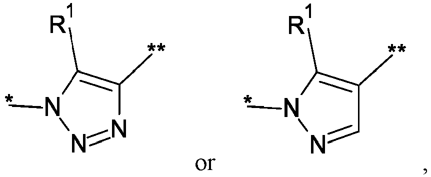 wherein * indicates the connection point to the substituent comprising Y₁ and ** indicates the other connection point from A in formula (IIIA); R⁶ can be CN, F, Cl or CF₃; and Y can be O.

In the compounds of formula (I), (II), (III), (IA), (IIA) or (IIIA), A can be pyrazole, 1,2,3 triazole or 1,2,4 triazole, substituted with R¹ and R^{1A}; R⁶ can be CN, F, Cl, CH₃, CF₃ or cyclopropyl; Y can be NH or O; and R² can be C₁-C₃ alkyl optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN.

In the compounds of formula (I), (II), (III), (IA), (IIA) or (IIIA), A can be pyrazole, 1,2,3 triazole or 1,2,4 triazole, substituted with R¹ and R^{1A}; R⁶ can be CN, F, Cl, CH₃, CF₃ or cyclopropyl; Y can be NH or O; and R² can be C₁-C₄ alkyl optionally substituted with one or more substituents independently selected from F, OH, CN, oxo, - OCH₃, -OC₃ cycloalkyl and R¹⁰.

In the compounds of formula (I), (II), (III), (IA), (IIA) or (IIIA), A can be pyrazole, 1,2,3 triazole or 1,2,4 triazole, substituted with R¹ and R^{1A}; R⁶ can be CN, F, Cl or CF₃; Y can be NH or O; and R² can be: 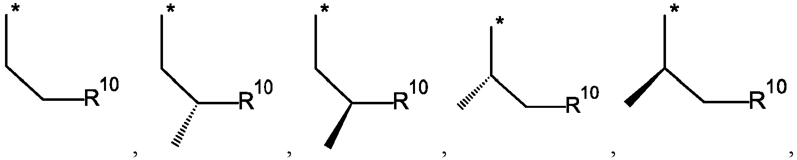 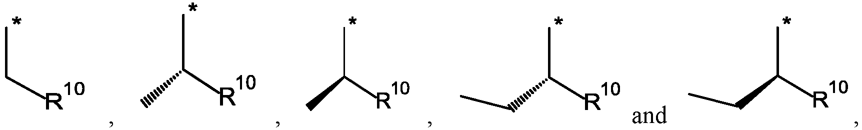 optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, wherein * indicates the connection point to Y in formula (I), (II), (III), (IA), (IIA) or (IIIA).

In the compounds of formula (I), (II), (III), (IA), (IIA) or (IIIA), A can be pyrazole, 1,2,3 triazole or 1,2,4 triazole, substituted with R¹ and R^{1A}; R⁶ can CN, F, Cl or CF₃; Y can be NH or O; and R² can be: 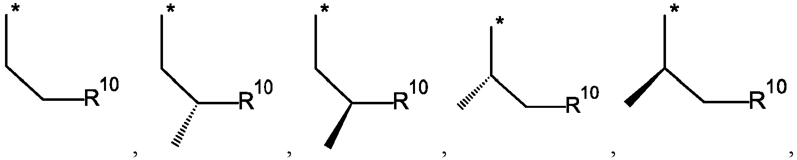 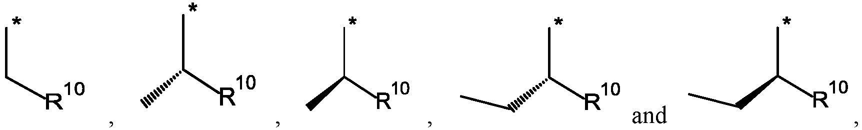 optionally substituted with one, two, three or four substituents independently selected from F, OH, CN, oxo, -OCH₃, -OC₃ cycloalkyl and R¹⁰, wherein * indicates the connection point to Y in formula (I), (II), (III), (IA), (IIA) or (IIIA).

In the compounds of formula (I), (II), (III), (IA), (IIA) or (IIIA), A can be pyrazole, 1,2,3 triazole or 1,2,4 triazole, substituted with R¹ and R^{1A}; R⁶ can be CN, F, Cl or CF₃; Y can be NH or O; and R² can be: 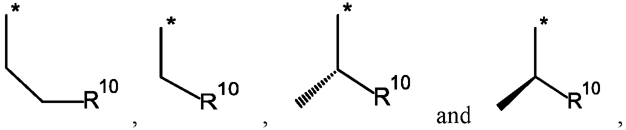 optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, wherein * indicates the connection point to Y in formula (I), (II), (III), (IA), (IIA) or (IIIA).

In the compounds of formula (I), A can be pyrazole, 1,2,3 triazole or 1,2,4 triazole, substituted with R¹ and R^{1A}; R⁶ can CN, F, Cl or CF₃; Y can be NH or O; and R² can be: 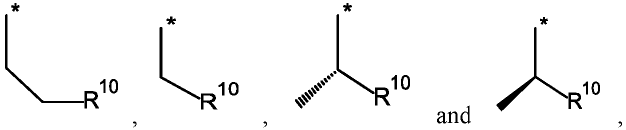 optionally substituted with one, two, three or four substituents independently selected from F, OH, CN, oxo, -OCH₃, -OC₃ cycloalkyl and R¹⁰, wherein * indicates the connection point to Y in formula (I), (II), (III), (IA), (IIA) or (IIIA).

In the compounds of formula (I), (II), (III), (IA) or (IIA), A can be: 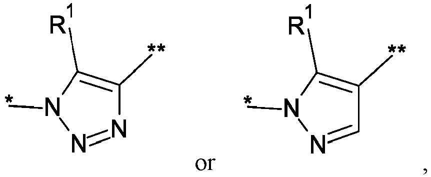 wherein * indicates the connection point to Z, Z' or Z₂and ** indicates the other connection point from A in formula (I), (II), (III), (IA) or (IIA); R⁶ can be CN, F, Cl, CH₃, CF₃ or cyclopropyl; Y can be NH or O; and R² can be C₁-C₃ alkyl optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN.

In the compounds of formula (I), (II), (III), (IA) or (IIA), A can be: 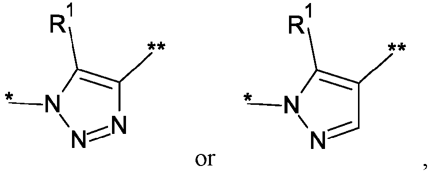 wherein * indicates the connection point to Z, Z' or Z₂ and ** indicates the other connection point from A in formula (I), (II), (III), (IA) or (IIA); R⁶ can be CN, F, Cl, CH₃, CF₃ or cyclopropyl; Y can be NH or O; and R² can be C₁-C₄ alkyl optionally substituted with one or more substituents independently selected from F, OH, CN, oxo, -OCH₃, -OC₃ cycloalkyl and R¹⁰.

In the compounds of formula (I), (II), (III), (IA) or (IIA), A can be: 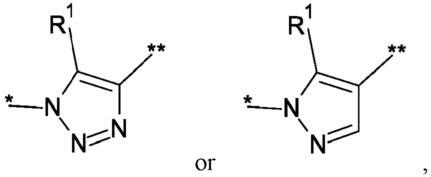 wherein * indicates the connection point to Z, Z' or Z₂ and ** indicates the other connection point from A in formula (I), (II), (III), (IA) or (IIA); R⁶ can be CN, F, Cl or CF₃; Y can be NH or O; and R² can be: 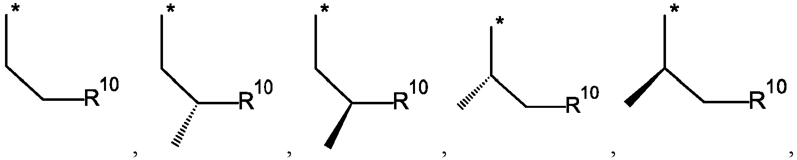 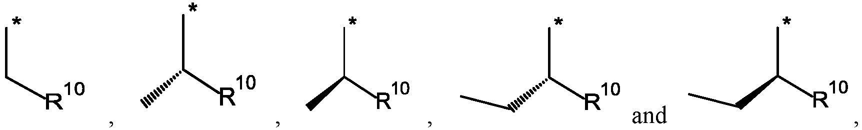 optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, wherein * indicates the connection point to Y in formula (I), (II), (III), (IA) or (IIA).

In the compounds of formula (I), (II), (III), (IA) or (IIA), A can be: 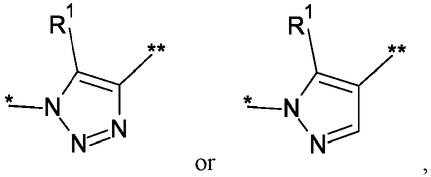 wherein * indicates the connection point to Z, Z' or Z₂ and ** indicates the other connection point from A in formula (I), (II), (III), (IA) or (IIA); R⁶ can CN, F, Cl or CF₃; Y can be NH or O; and R² can be: 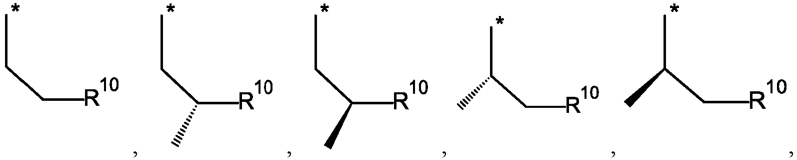 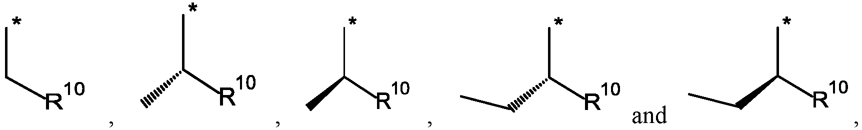 optionally substituted with one, two, three or four substituents independently selected from F, OH, CN, oxo, -OCH₃, -OC₃ cycloalkyl and R¹⁰, wherein * indicates the connection point to Y in formula (I), (II), (III), (IA) or (IIA).

In the compounds of formula (I), (II), (III), (IA) or (IIA), A can be: 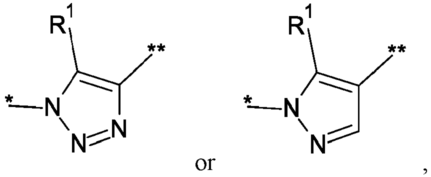 wherein * indicates the connection point to Z, Z' or Z₂ and ** indicates the other connection point from A in formula (I), (II), (III), (IA) or (IIA); R⁶ can be CN, F, Cl or CF₃; Y can be NH or O; and R² can be: 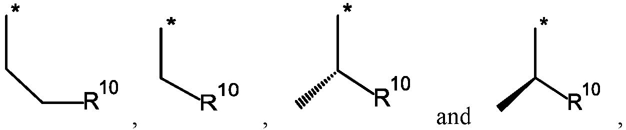 optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, wherein * indicates the connection point to Y in formula (I), (II), (III), (IA) or (IIA).

In the compounds of formula (I), (II), (III), (IA) or (IIA), A can be: 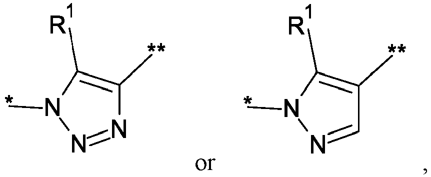 wherein * indicates the connection point to Z, Z' or Z₂ and ** indicates the other connection point from A in formula (I), (II), (III), (IA) or (IIA); R⁶ can CN, F, Cl or CF₃; Y can be NH or O; and R² can be: 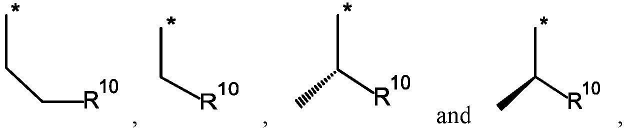 optionally substituted with one, two, three or four substituents independently selected from F, OH, CN, oxo, -OCH₃, -OC₃ cycloalkyl and R¹⁰, wherein * indicates the connection point to Y in formula (I), (II), (III), (IA) or (IIA).

In the compounds of formula (IIIA), A can be: 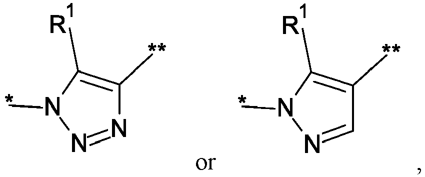 wherein * indicates the connection point to the substituent comprising Y₁ and ** indicates the other connection point from A in formula (IIIA); R⁶ can CN, F, Cl or CF₃; Y can be NH or O; and R² can be: 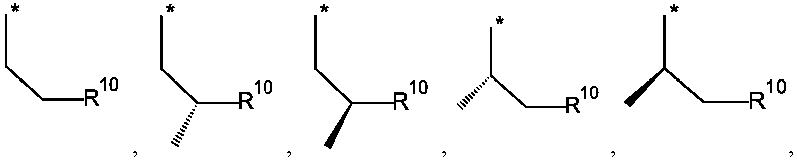 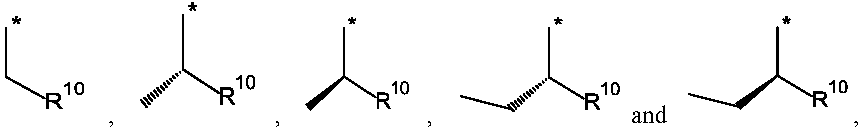 optionally substituted with one, two, three or four substituents independently selected from F, OH, CN, oxo, -OCH₃, -OC₃ cycloalkyl and R¹⁰, wherein * indicates the connection point to Y in formula (IIIA).

In the compounds of formula (IIIA), A can be: 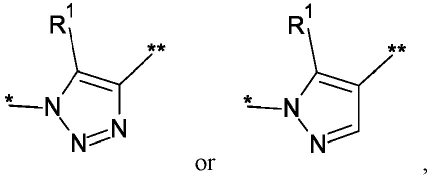 wherein * indicates the connection point to the substituent comprising Y₁ and ** indicates the other connection point from A in formula (IIIA); R⁶ can be CN, F, Cl or CF₃; Y can be NH or O; and R² can be: 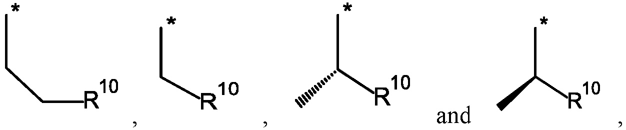 optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, wherein * indicates the connection point to Y in formula (IIIA).

In the compounds of formula (IIIA), A can be: 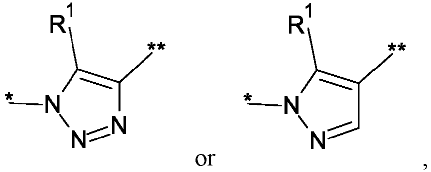 wherein * indicates the connection point to the substituent comprising Y₁ and ** indicates the other connection point from A in formula (IIIA); R⁶ can CN, F, Cl or CF₃; Y can be NH or O; and R² can be: 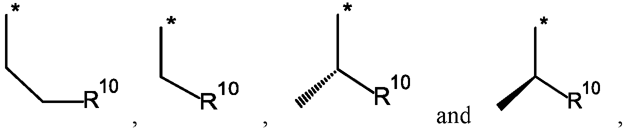 optionally substituted with one, two, three or four substituents independently selected from F, OH, CN, oxo, -OCH₃, -OC₃ cycloalkyl and R¹⁰, wherein * indicates the connection point to Y in formula (IIIA).

In the compounds of formula (I), (II), (III), (IA) or (IIA), A can be: 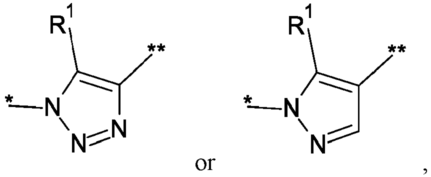 wherein * indicates the connection point to Z, Z' or Z₂ and ** indicates the other connection point from A in formula (I), (II), (III), (IA) or (IIA); R⁶ can be CN, F, Cl, CH₃, CF₃ or cyclopropyl; Y can be NH or O; and R² can be C₁-C₃ alkyl optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN; Y₅ is CR¹³R¹⁴, CR¹³R¹⁴-CH₂ or CH₂-CR¹³R¹⁴, wherein R¹³ and R¹⁴ are independently selected from H and CH₃; and Y₆ is CR¹³R¹⁴, CR¹³R¹⁴-CH₂ or CH₂-CR¹³R¹⁴, wherein R¹³ and R¹⁴ are independently selected from H and CH₃.

In the compounds of formula (I), (II), (III), (IA) or (IIA), A can be: 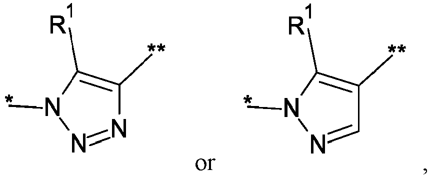 wherein * indicates the connection point to Z, Z' or Z₂ and ** indicates the other connection point from A in formula (I), (II), (III), (IA) or (IIA); R⁶ can be CN, F, Cl, CH₃, CF₃ or cyclopropyl; Y can be NH or O; and R² can be C₁-C₄ alkyl optionally substituted with one or more substituents independently selected from F, OH, CN, oxo, -OCH₃, -OC₃ cycloalkyl and R¹⁰; Y₅ is CR¹³R¹⁴, CR¹³R¹⁴-CH₂ or CH₂-CR¹³R¹⁴, wherein R¹³ and R¹⁴ are independently selected from H and CH₃; and Y₆ is CR¹³R¹⁴, CR¹³R¹⁴-CH₂ or CH₂-CR¹³R¹⁴, wherein R¹³ and R¹⁴ are independently selected from H and CH₃.

In the compounds of formula (I), (II), (III), (IA) or (IIA), A can be: 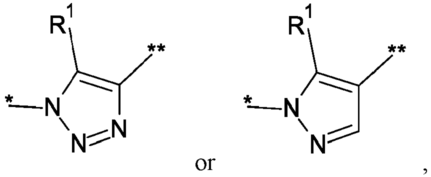 wherein * indicates the connection point to Z, Z' or Z₂ and ** indicates the other connection point from A in formula (I), (II), (III), (IA) or (IIA); R⁶ can be CN, F, Cl or CF₃; Y can be NH or O; and R² can be: 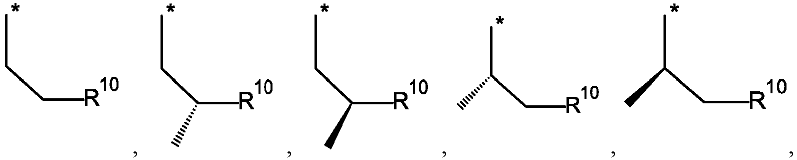 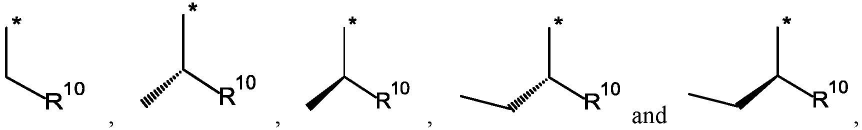 optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, wherein * indicates the connection point to Y in formula (I), (II), (III), (IA) or (IIA); Y₅ is CR¹³R¹⁴, CR¹³R¹⁴-CH₂ or CH₂-CR¹³R¹⁴, wherein R¹³ and R¹⁴ are independently selected from H and CH₃; and Y₆ is CR¹³R¹⁴, CR¹³R¹⁴-CH₂ or CH₂-CR¹³R¹⁴, wherein R¹³ and R¹⁴ are independently selected from H and CH₃.

In the compounds of formula (I), (II), (III), (IA) or (IIA), A can be: 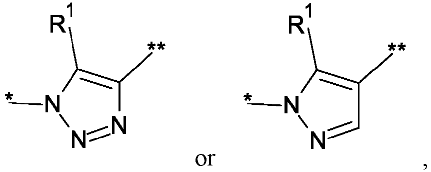 wherein * indicates the connection point to Z, Z' or Z₂ and ** indicates the other connection point from A in formula (I), (II), (III), (IA) or (IIA); R⁶ can CN, F, Cl or CF₃; Y can be NH or O; and R² can be: 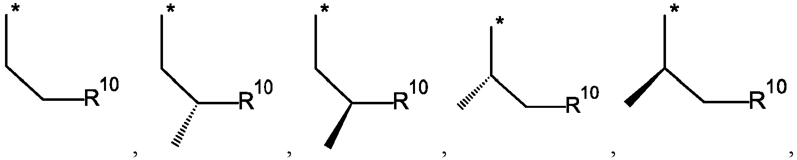 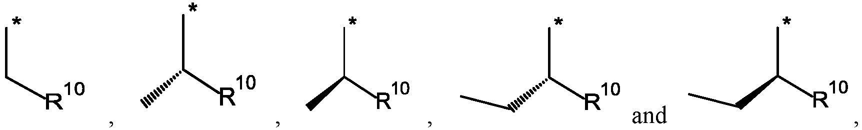 optionally substituted with one, two, three or four substituents independently selected from F, OH, CN, oxo, -OCH₃, -OC₃ cycloalkyl and R¹⁰, wherein * indicates the connection point to Y in formula (I), (II), (III), (IA) or (IIA); Y₅ is CR¹³R¹⁴, CR¹³R¹⁴-CH₂ or CH₂-CR¹³R¹⁴, wherein R¹³ and R¹⁴ are independently selected from H and CH₃; and Y₆ is CR¹³R¹⁴, CR¹³R¹⁴-CH₂ or CH₂-CR¹³R¹⁴, wherein R¹³ and R¹⁴ are independently selected from H and CH₃.

In the compounds of formula (I), (II), (III), (IA) or (IIA), A can be: 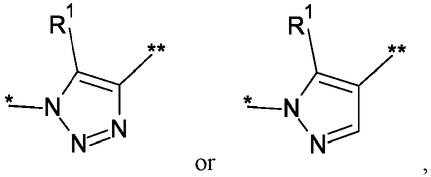 wherein * indicates the connection point to Z, Z' or Z₂ and ** indicates the other connection point from A in formula (I), (II), (III), (IA) or (IIA); R⁶ can be CN, F, Cl or CF₃; Y can be NH or O; and R² can be: 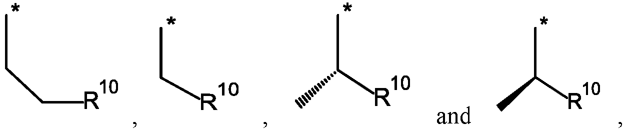 optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, wherein * indicates the connection point to Y in formula (I), (II), (III), (IA) or (IIA); Y₅ is CR¹³R¹⁴, CR¹³R¹⁴-CH₂ or CH₂-CR¹³R¹⁴, wherein R¹³ and R¹⁴ are independently selected from H and CH₃; and Y₆ is CR¹³R¹⁴, CR¹³R¹⁴-CH₂ or CH₂-CR¹³R¹⁴, wherein R¹³ and R¹⁴ are independently selected from H and CH₃.

In the compounds of formula (I), (II), (III), (IA) or (IIA), A can be: 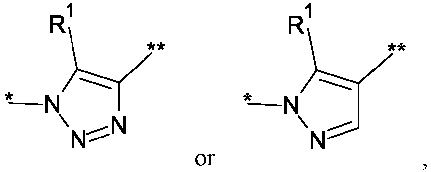 wherein * indicates the connection point to Z, Z' or Z₂ and ** indicates the other connection point from A in formula (I), (II), (III), (IA) or (IIA); R⁶ can CN, F, Cl or CF₃; Y can be NH or O; and R² can be: 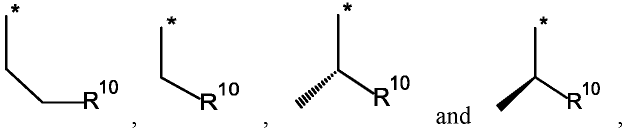 optionally substituted with one, two, three or four substituents independently selected from F, OH, CN, oxo, -OCH₃, -OC₃ cycloalkyl and R¹⁰, wherein * indicates the connection point to Y in formula (I), (II), (III), (IA) or (IIA); Y₅ is CR¹³R¹⁴, CR¹³R¹⁴-CH₂ or CH₂-CR¹³R¹⁴, wherein R¹³ and R¹⁴ are independently selected from H and CH₃; and Y₆ is CR¹³R¹⁴, CR¹³R¹⁴-CH₂ or CH₂-CR¹³R¹⁴, wherein R¹³ and R¹⁴ are independently selected from H and CH₃.

In the compounds of formula (IIIA), A can be: 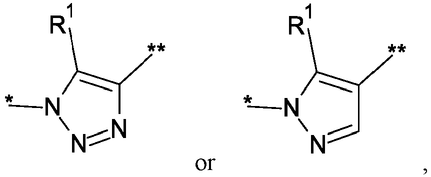 wherein * indicates the connection point to the substituent comprising Y₁ and ** indicates the other connection point from A in formula (IIIA); R⁶ can CN, F, Cl or CF₃; Y can be NH or O; and R² can be: 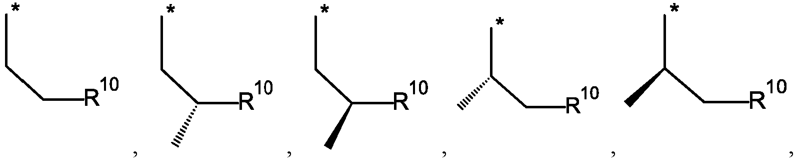 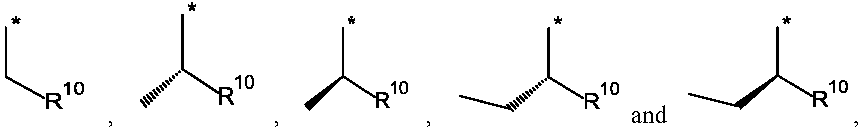 optionally substituted with one, two, three or four substituents independently selected from F, OH, CN, oxo, -OCH₃, -OC₃ cycloalkyl and R¹⁰, wherein * indicates the connection point to Y in formula (IIIA); Y₅ is CR¹³R¹⁴, CR¹³R¹⁴-CH₂ or CH₂-CR¹³R¹⁴, wherein R¹³ and R¹⁴ are independently selected from H and CH₃; and Y₆ is CR¹³R¹⁴, CR¹³R¹⁴-CH₂ or CH₂-CR¹³R¹⁴, wherein R¹³ and R¹⁴ are independently selected from H and CH₃.

In the compounds of formula (IIIA), A can be: 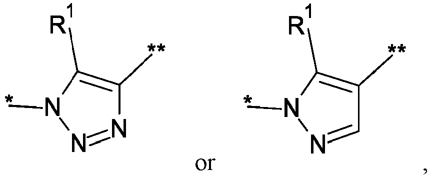 wherein * indicates the connection point to the substituent comprising Y₁ and ** indicates the other connection point from A in formula (IIIA); R⁶ can be CN, F, Cl or CF₃; Y can be NH or O; and R² can be: 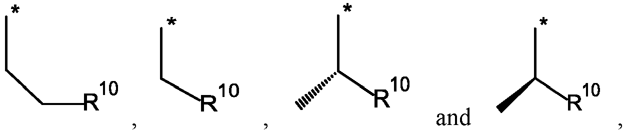 optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, wherein * indicates the connection point to Y in formula (IIIA); Y₅ is CR¹³R¹⁴, CR¹³R¹⁴-CH₂ or CH₂-CR¹³R¹⁴, wherein R¹³ and R¹⁴ are independently selected from H and CH₃; and Y₆ is CR¹³R¹⁴, CR¹³R¹⁴-CH₂ or CH₂-CR¹³R¹⁴, wherein R¹³ and R¹⁴ are independently selected from H and CH₃.

In the compounds of formula (IIIA), A can be: 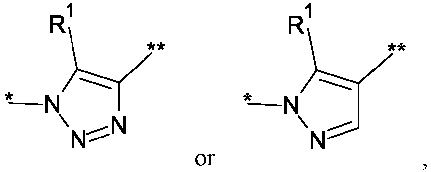 wherein * indicates the connection point to the substituent comprising Y₁ and ** indicates the other connection point from A in formula (IIIA); R⁶ can CN, F, Cl or CF₃; Y can be NH or O; and R² can be: 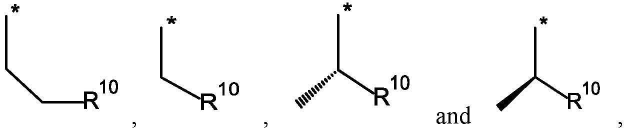 optionally substituted with one, two, three or four substituents independently selected from F, OH, CN, oxo, -OCH₃, -OC₃ cycloalkyl and R¹⁰, wherein * indicates the connection point to Y in formula (IIIA); Y₅ is CR¹³R¹⁴, CR¹³R¹⁴-CH₂ or CH₂-CR¹³R¹⁴, wherein R¹³ and R¹⁴ are independently selected from H and CH₃; and Y₆ is CR¹³R¹⁴, CR¹³R¹⁴-CH₂ or CH₂-CR¹³R¹⁴, wherein R¹³ and R¹⁴ are independently selected from H and CH₃.

In one embodiment, the compounds of Formula (I) are selected from the group consisting of: 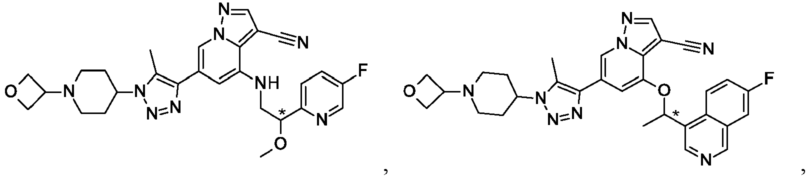 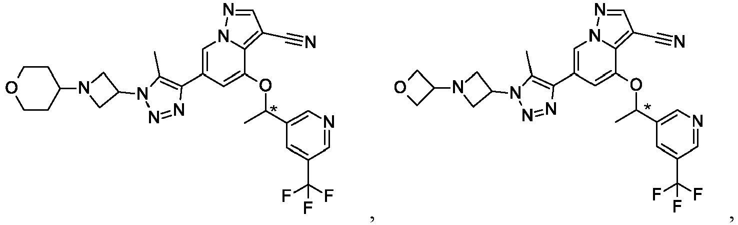 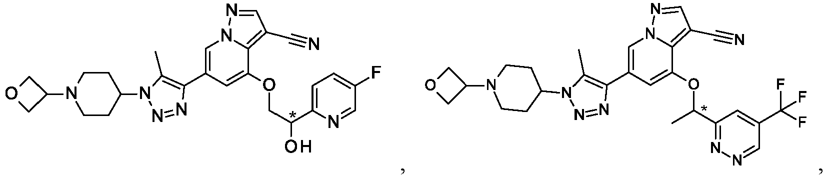 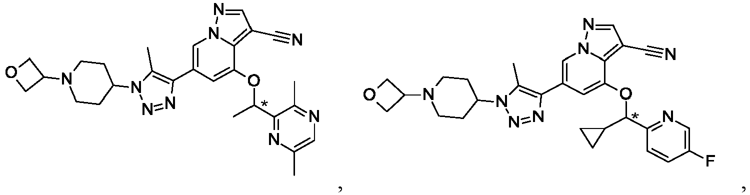 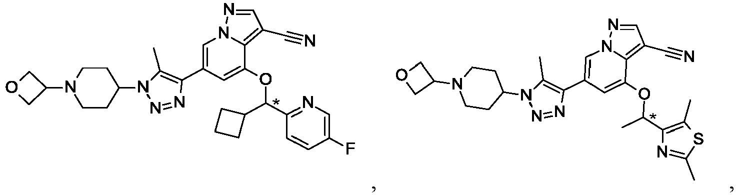 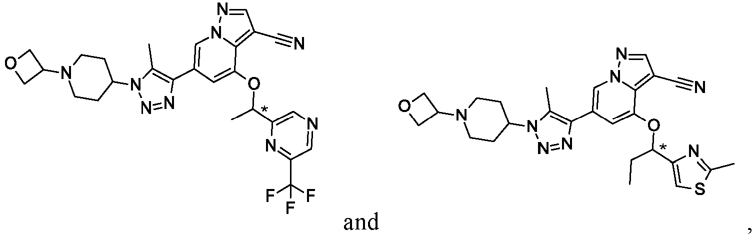 or a pharmaceutically acceptable salt thereof,
wherein the bond at the * position is as represented,

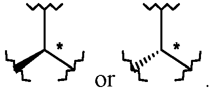

For example, for the compound of formula: 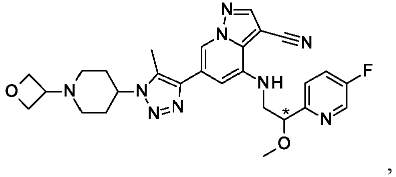 where the bond at the * position is as represented, 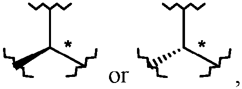 forms the compounds:

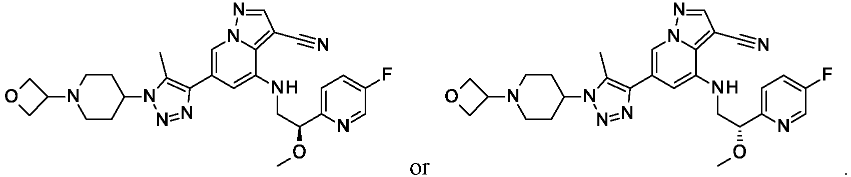

In a further embodiment, the compounds of Formula (I) are selected from the group consisting of: 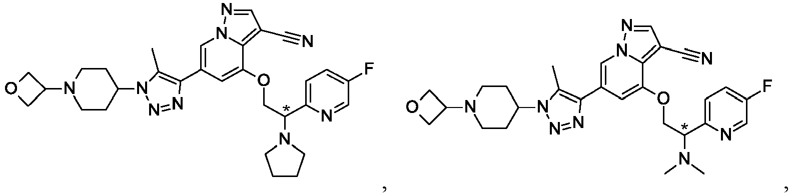 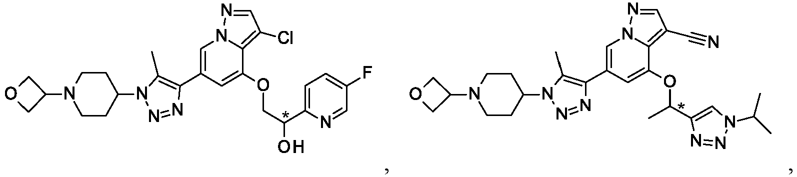 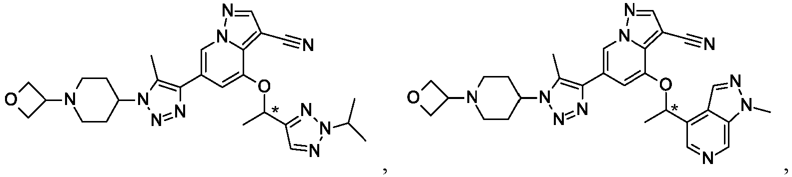 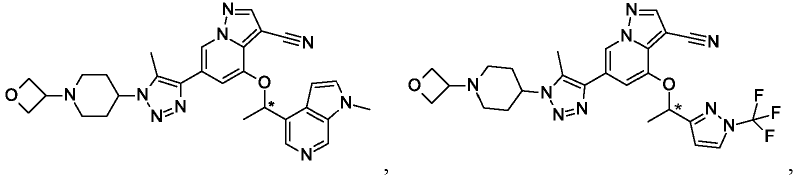 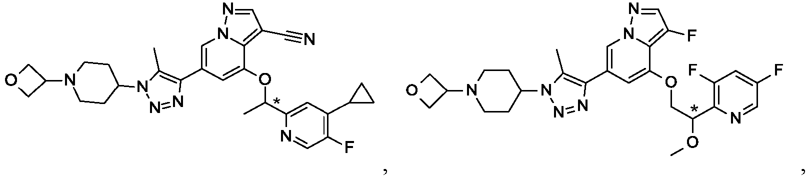 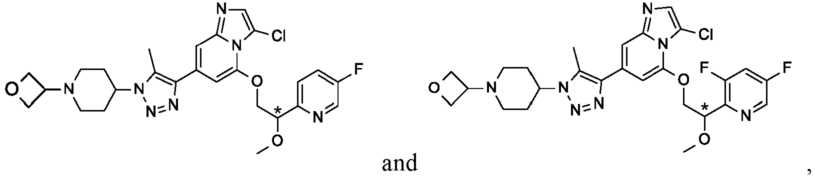 or a pharmaceutically acceptable salt thereof, where the bond at the * position is as represented,

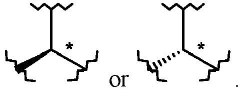

In a further embodiment, the compounds of Formula (I) are selected from the group consisting of: 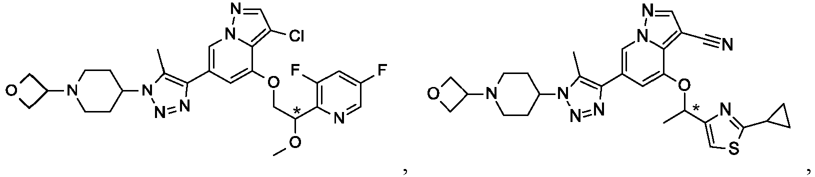 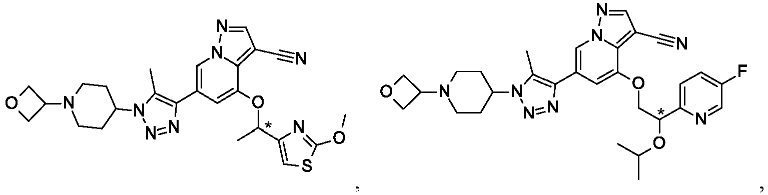 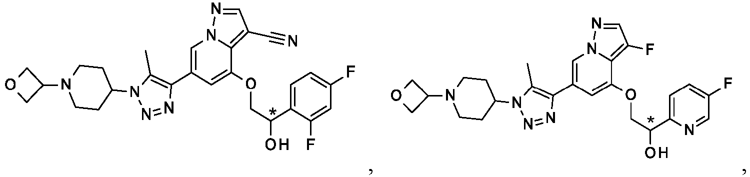 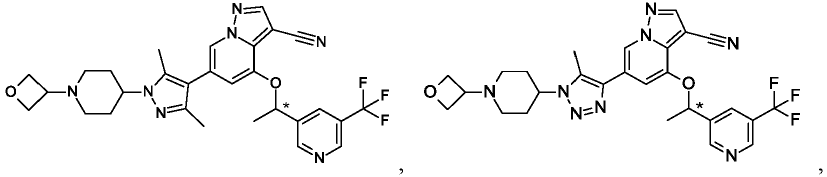 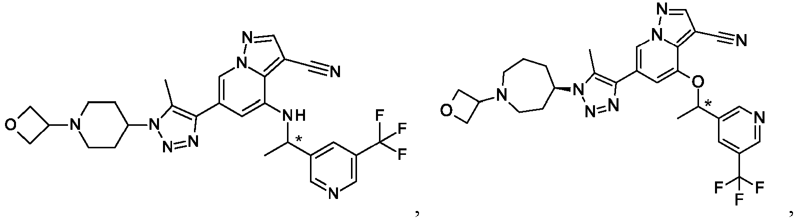 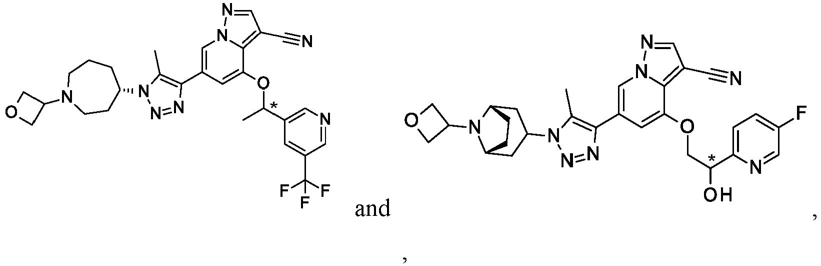 or a pharmaceutically acceptable salt thereof, where the bond at the * position is as represented,

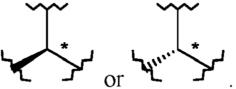

In a further embodiment, the compounds of Formula (I) are selected from the group consisting of: 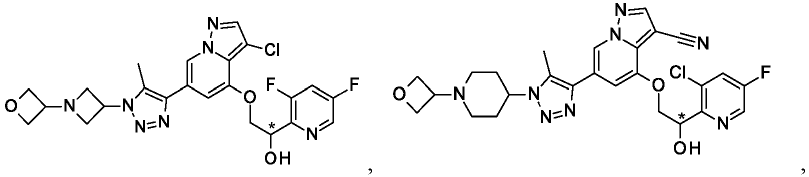 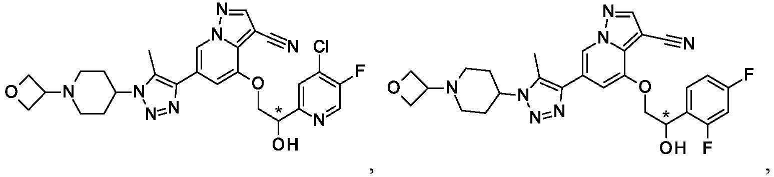 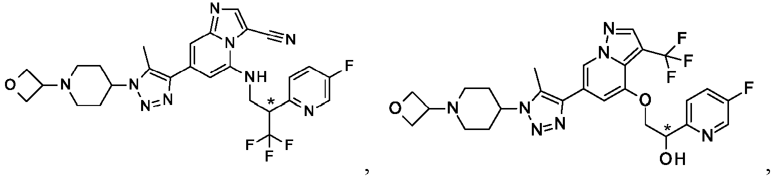 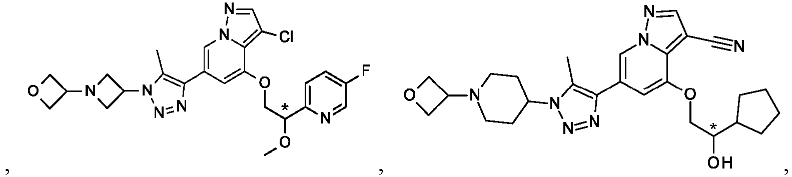 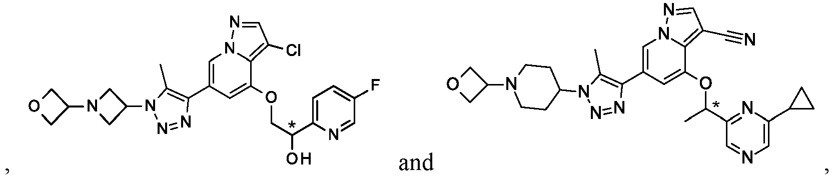 or a pharmaceutically acceptable salt thereof, where the bond at the * position is as represented,

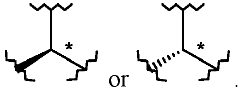

In a further embodiment, the compounds of Formula (I) are selected from the group consisting of: 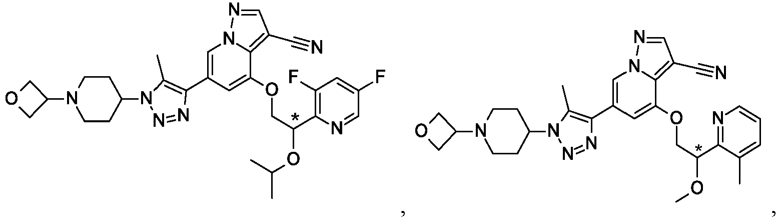 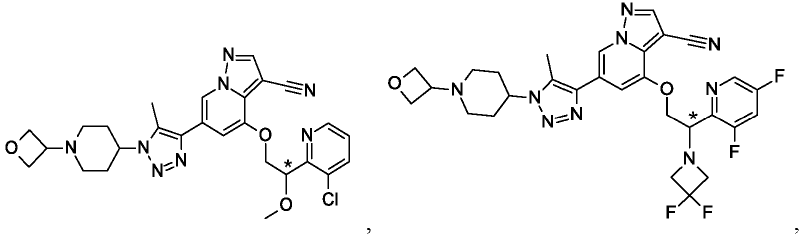 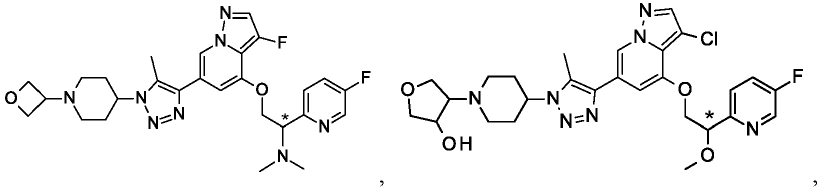 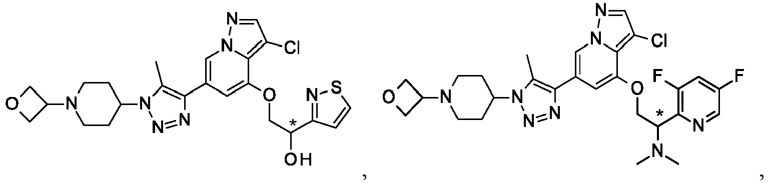 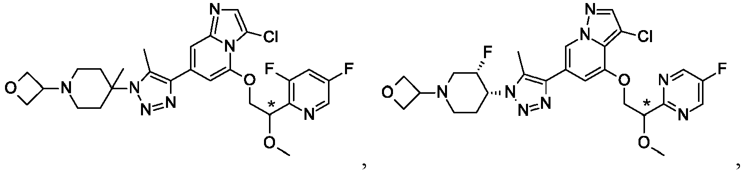 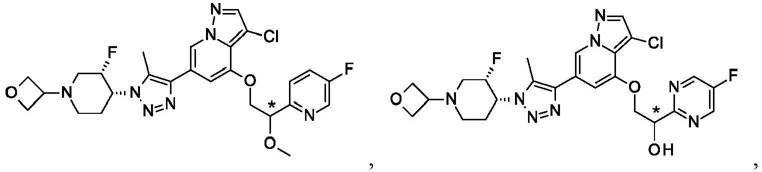 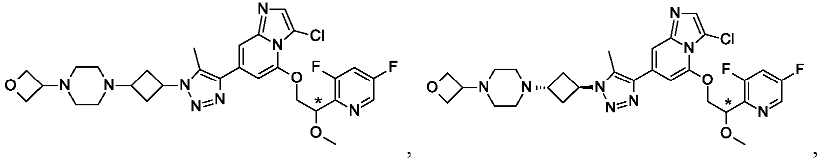 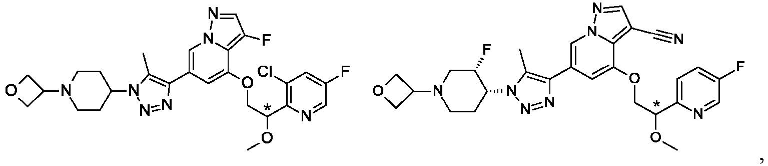 and 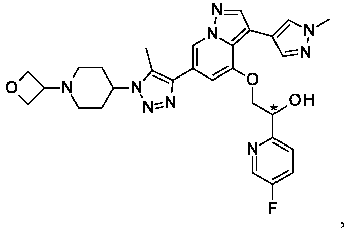 or a pharmaceutically acceptable salt thereof, where the bond at the * position is as represented,

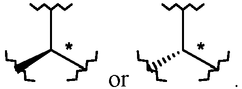

In a further embodiment, the compounds of Formula (I) are selected from the group consisting of: 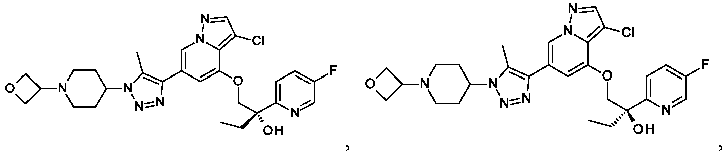 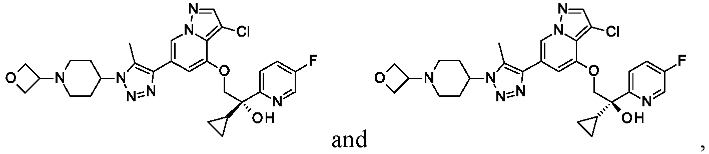 or a pharmaceutically acceptable salt thereof.

In a further embodiment, the compounds of Formula (I) are selected from the group consisting of: 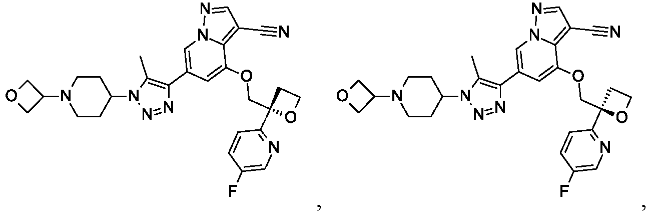 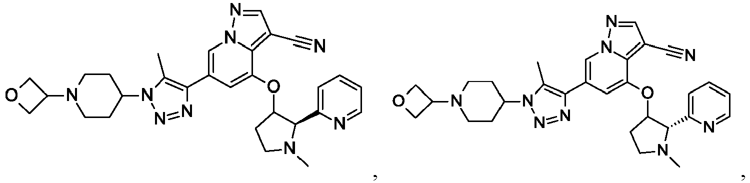 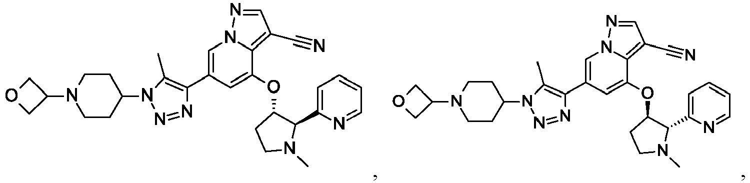 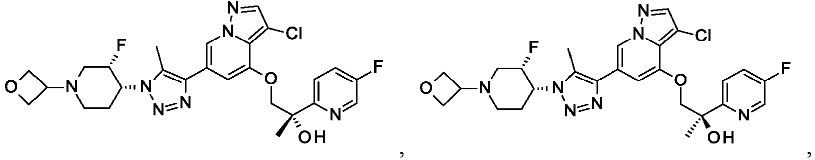 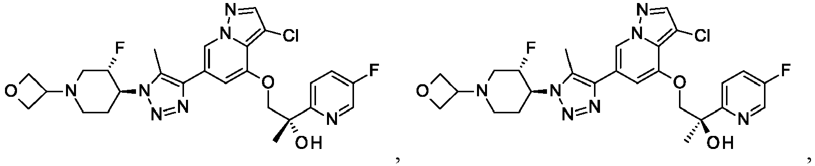 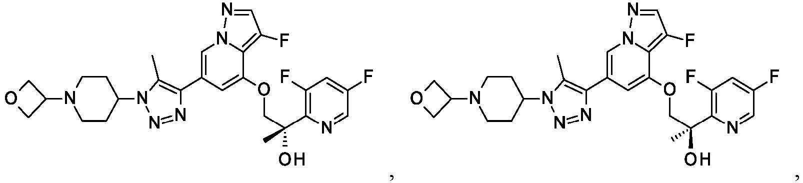 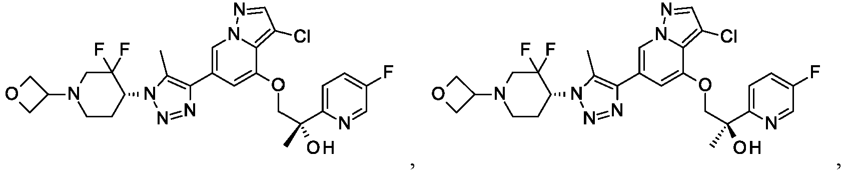 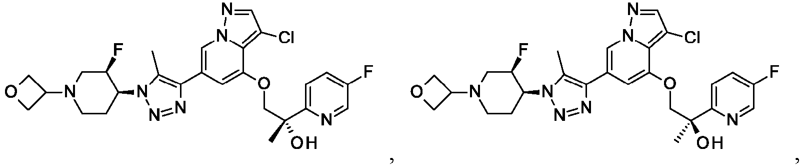 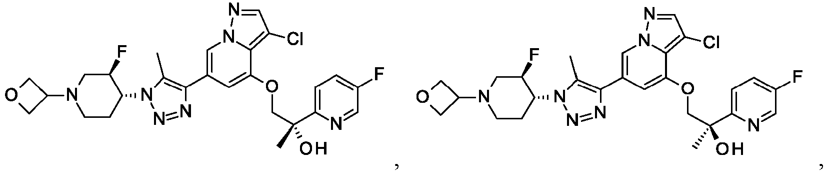 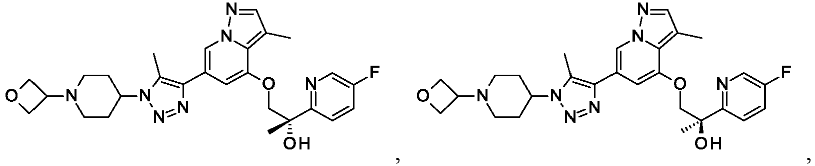 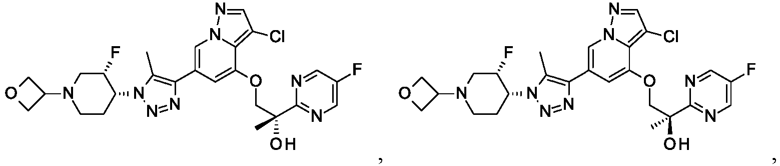 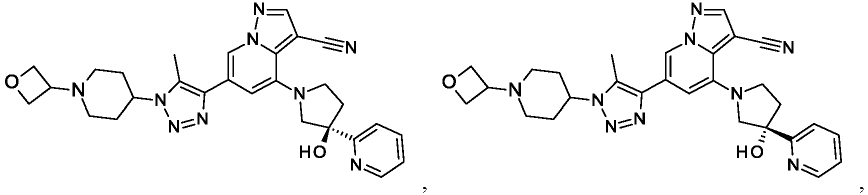 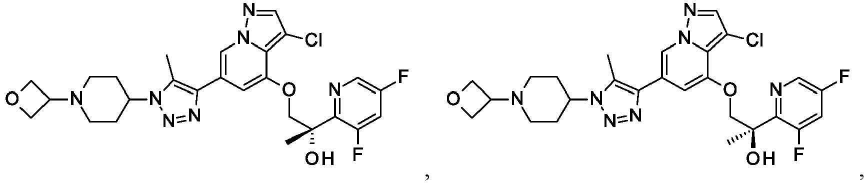 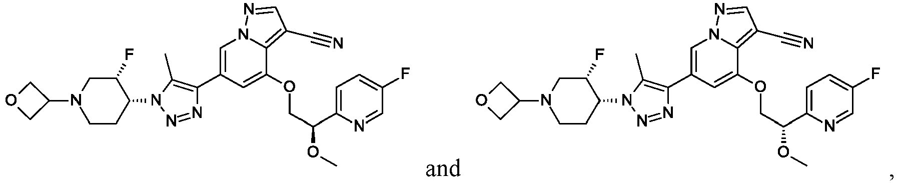 or a pharmaceutically acceptable salt thereof.

In a further embodiment, the compounds of Formula (I) are selected from the group consisting of: 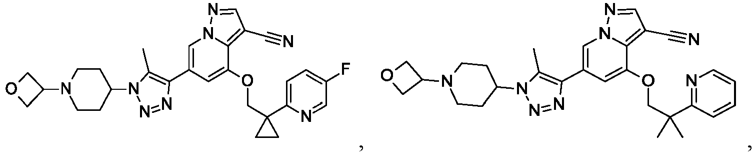 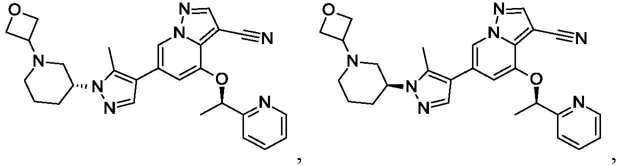 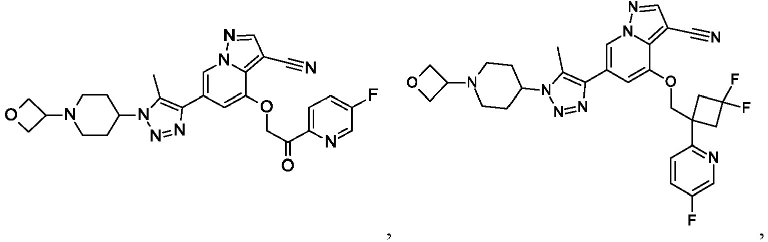 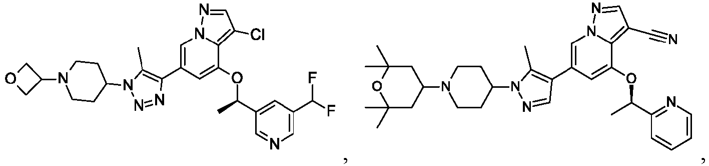 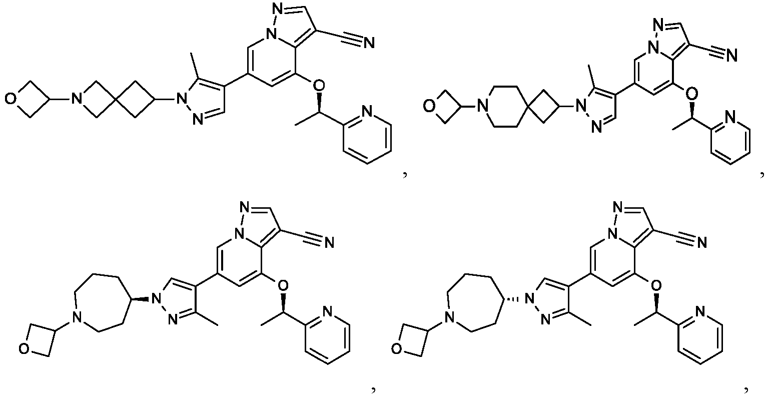 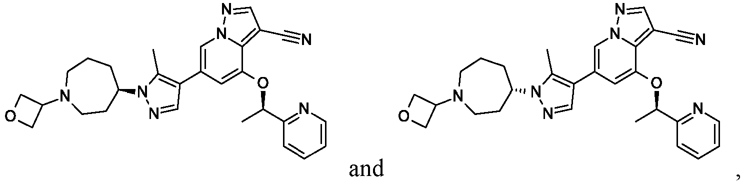 or a pharmaceutically acceptable salt thereof.

In a further embodiment, the compounds of Formula (I) are selected from the group consisting of: 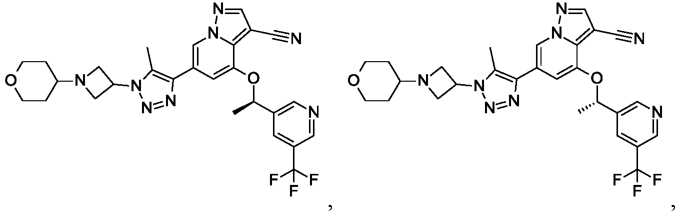 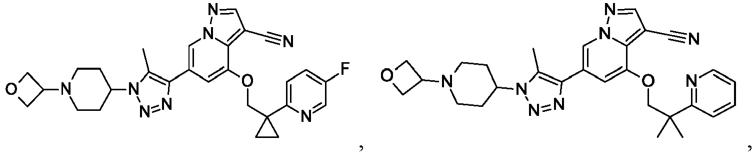 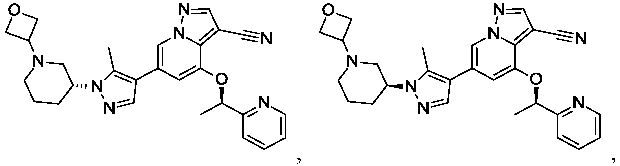 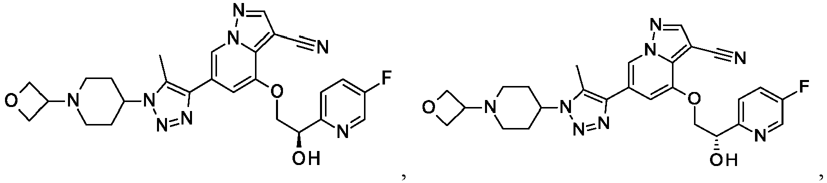 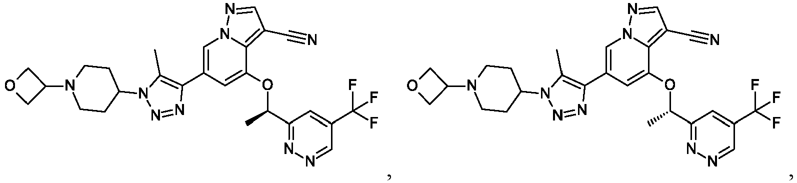 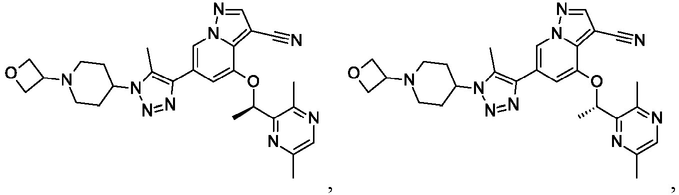 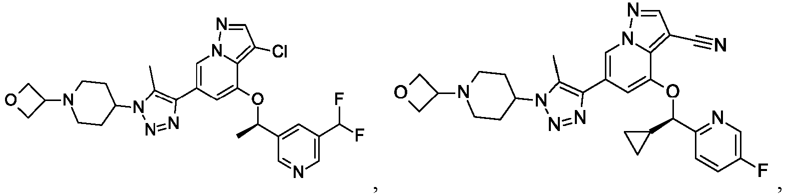 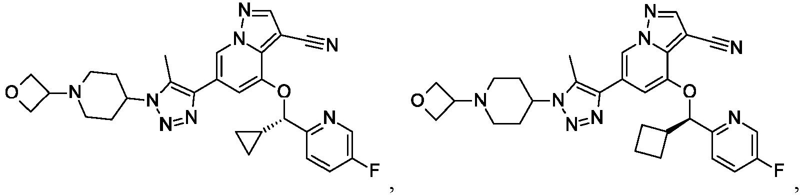 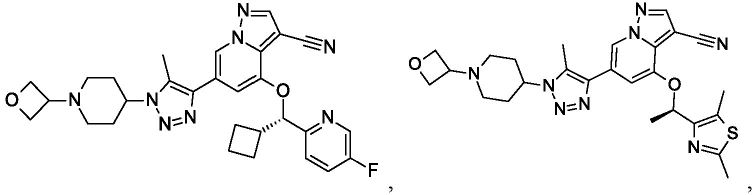 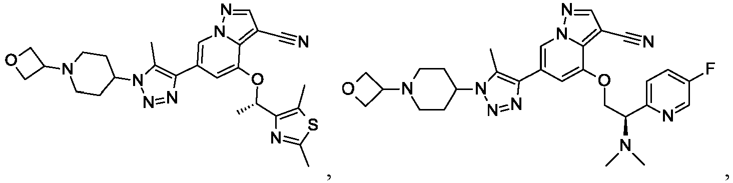 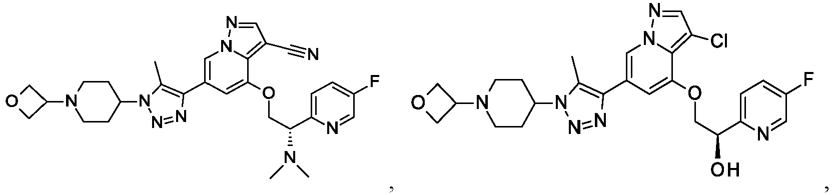 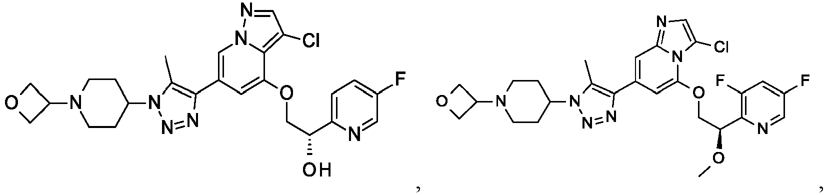 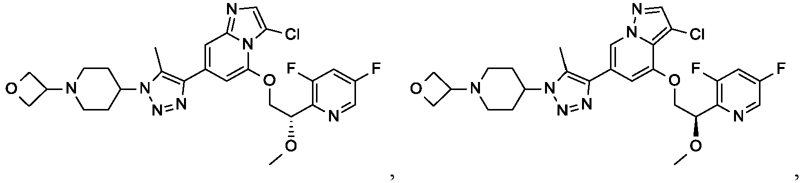 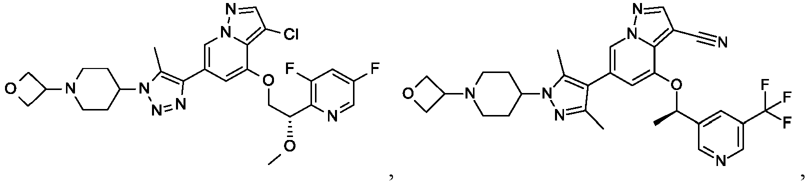 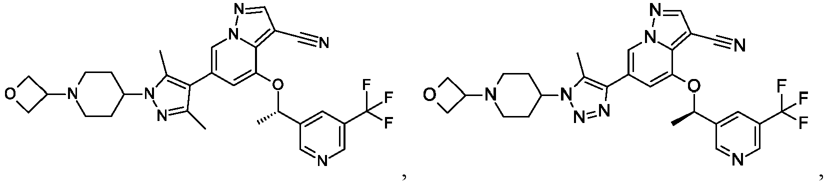 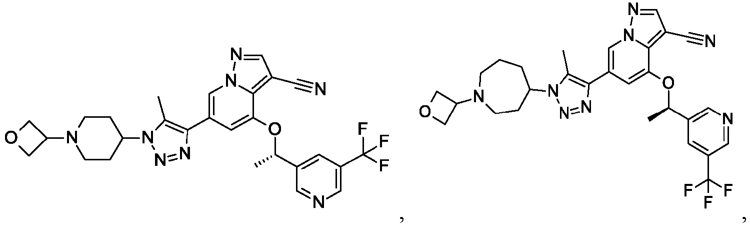 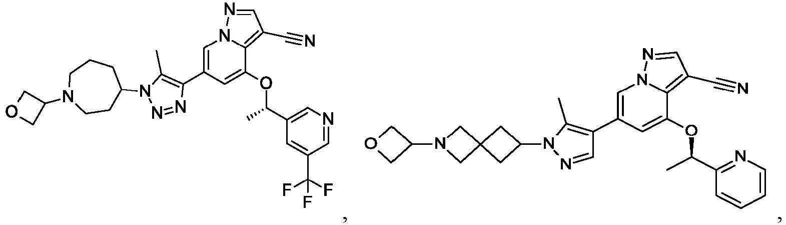 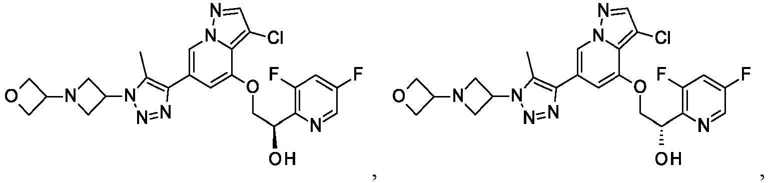 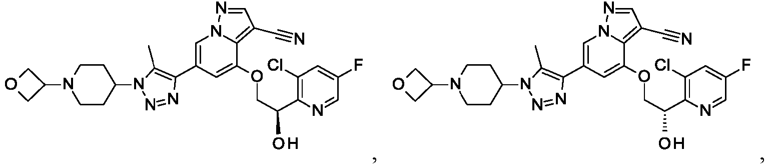 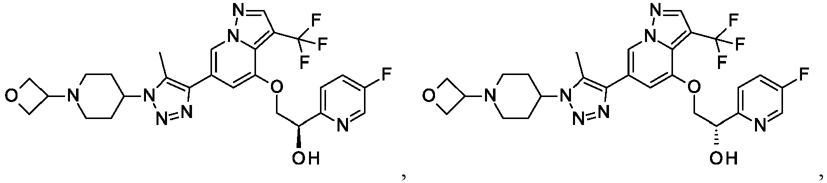 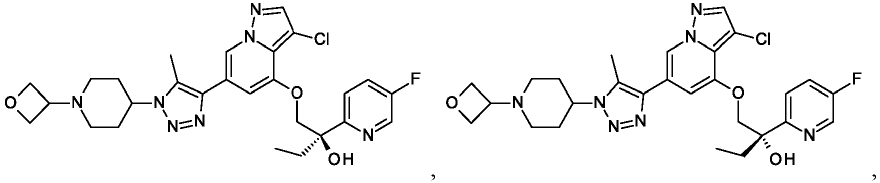 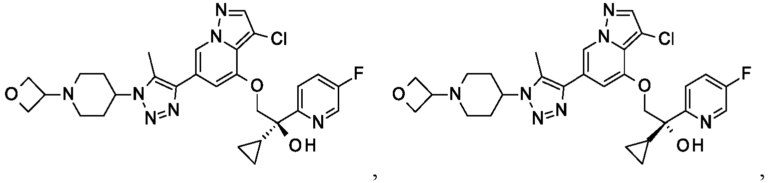 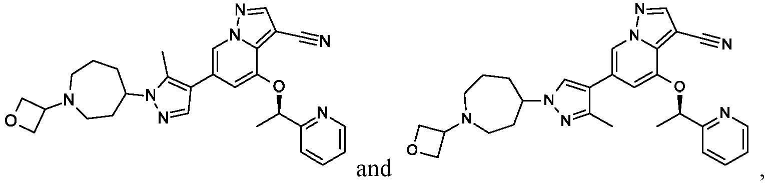 or a pharmaceutically acceptable salt thereof.

In a further embodiment, the compounds of Formula (I) are selected from the group consisting of: 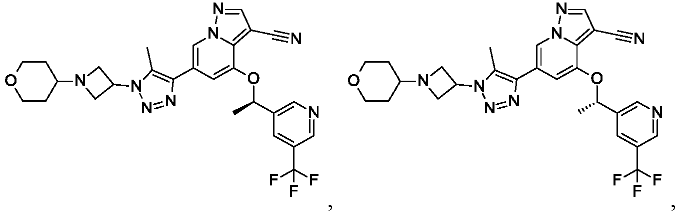 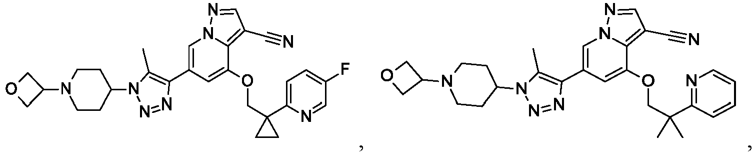 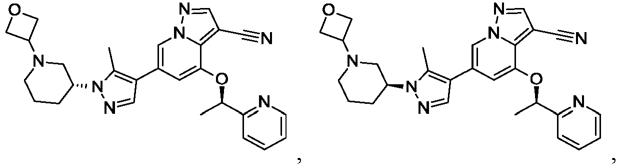 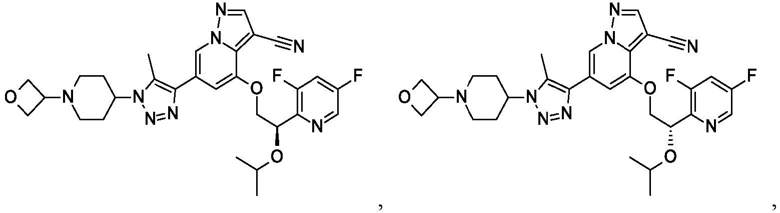 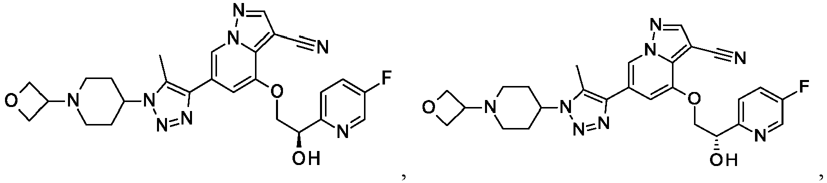 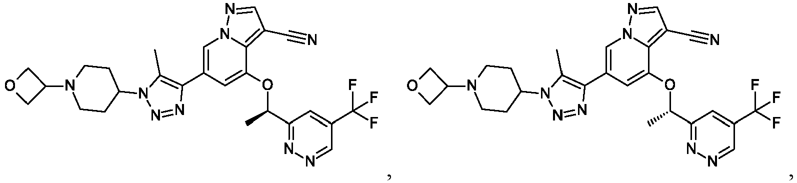 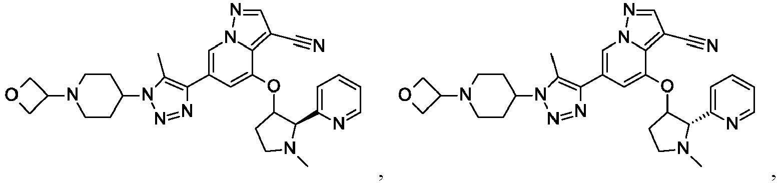 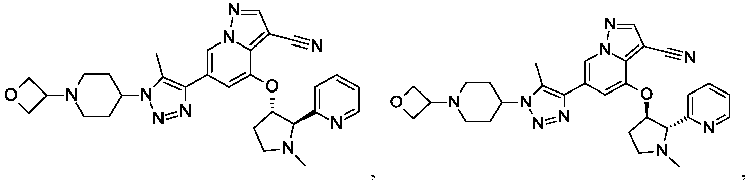 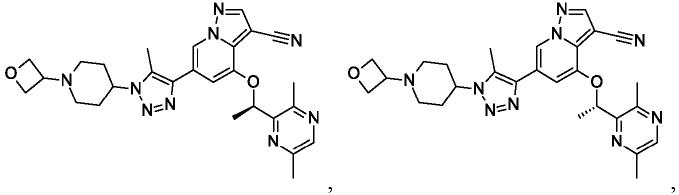 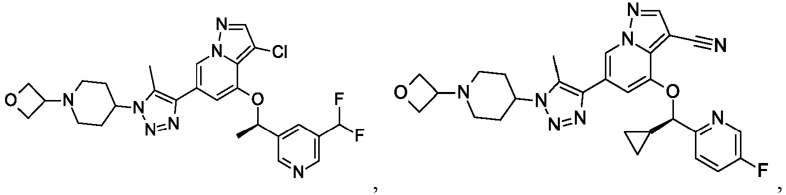 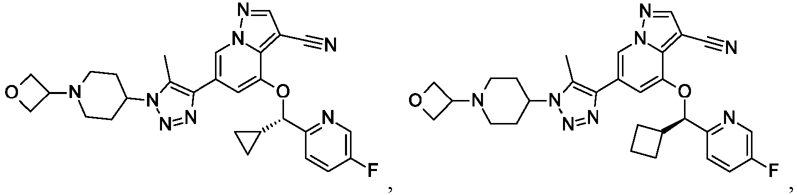 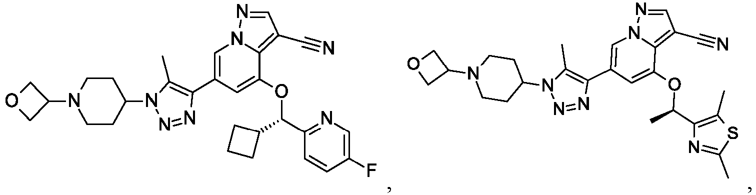 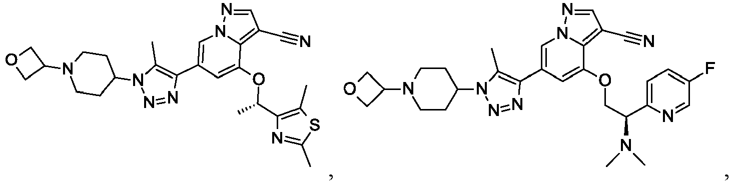 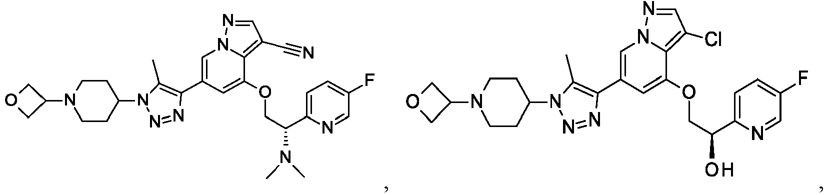 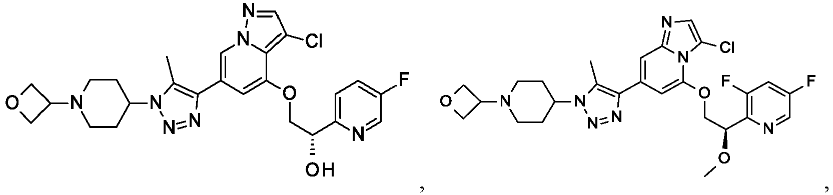 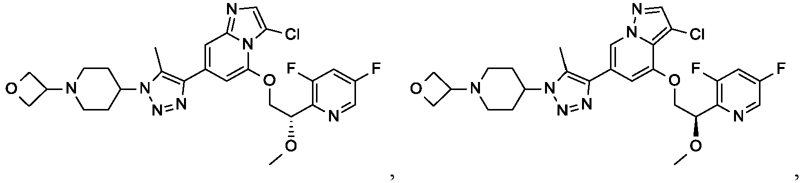 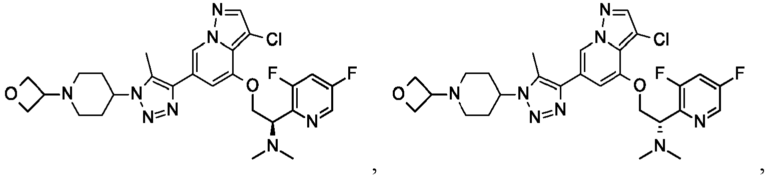 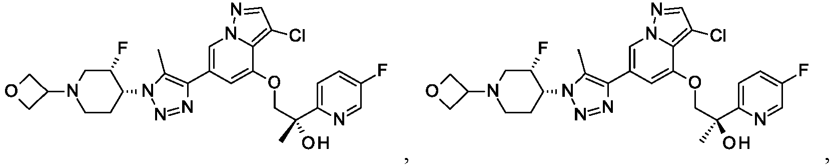 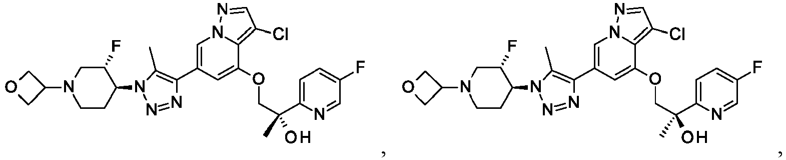 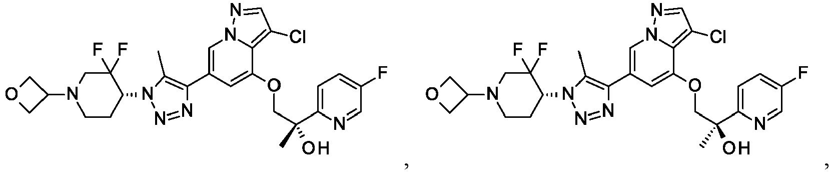 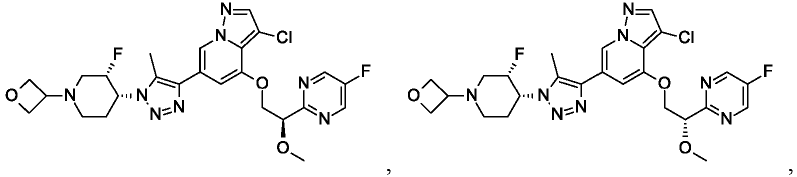 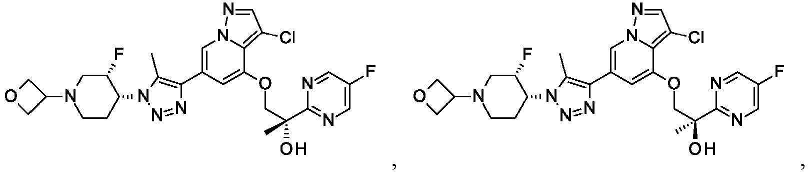 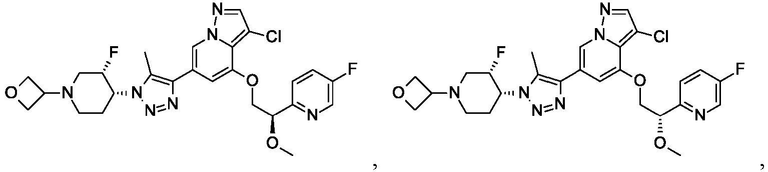 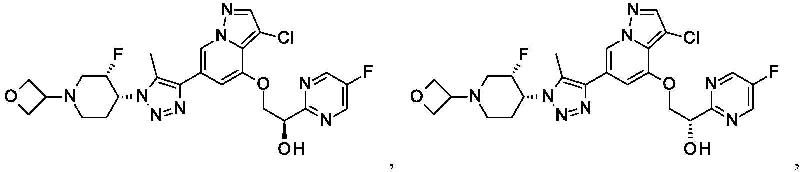 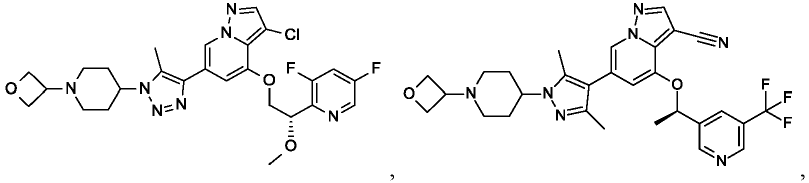 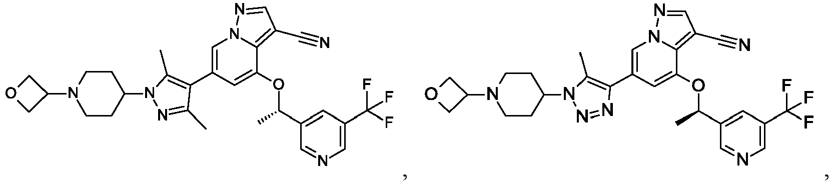 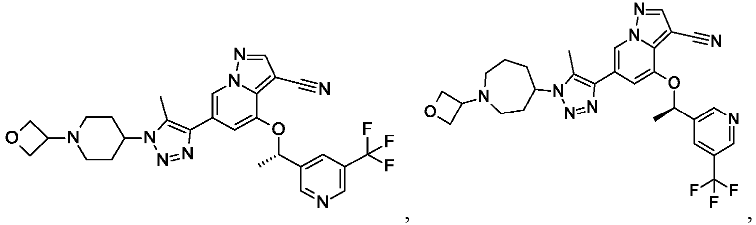 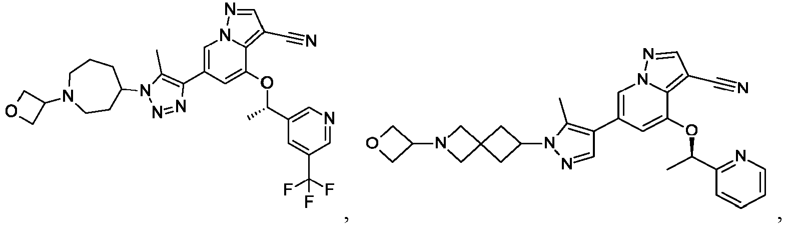 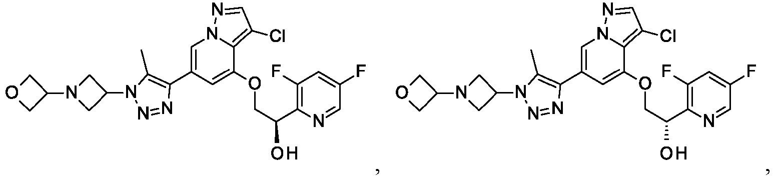 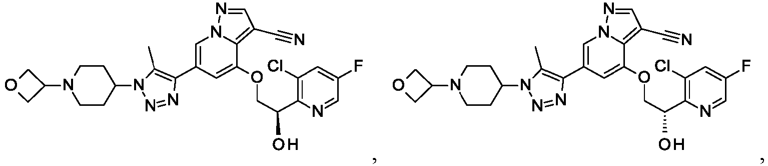 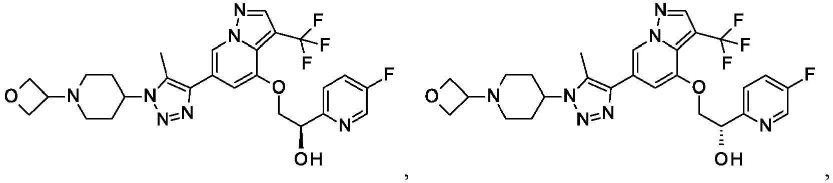 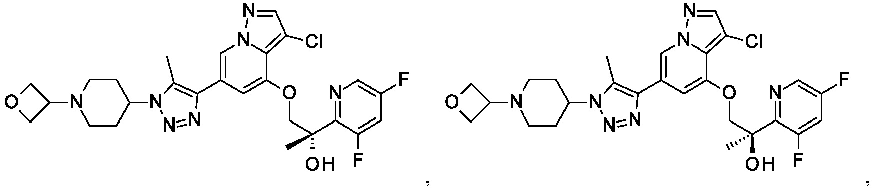 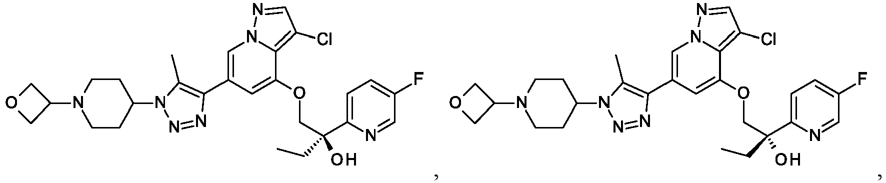 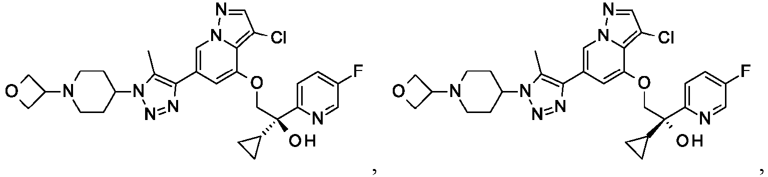 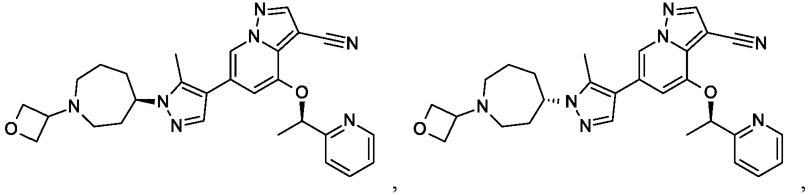 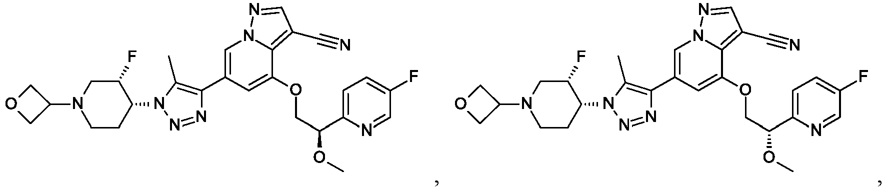 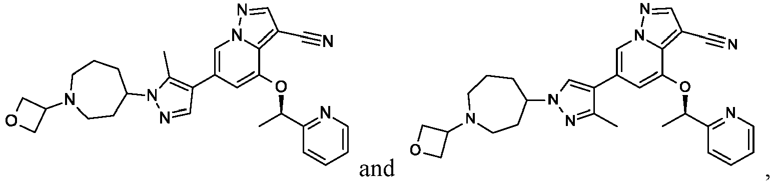 or a pharmaceutically acceptable salt thereof.

The compounds of formula (I), (II), (III), (IA), (IIA) or (IIIA), provided herein, or a pharmaceutically acceptable salt thereof, any or all hydrogens present in the compound, or in a particular group or moiety within the compound, may be replaced by a deuterium or a tritium. Thus, a recitation of alkyl includes deuterated alkyl, where from one to the maximum number of hydrogens present may be replaced by deuterium. For example, ethyl refers to both C₂H₅ or C₂H₅ where from 1 to 5 hydrogens are replaced by deuterium, such as in C₂DₓH₅₋ₓ.

The compounds of formula (I), (II), (III), (IA), (IIA) or (IIIA) provided herein may form pharmaceutically acceptable salts. The Examples provided herein may form pharmaceutically acceptable salts. Such pharmaceutically acceptable salts are intended to be included. Pharmaceutically acceptable salts and common methodology for preparing them are well known in the art (*see, e.g.,* P. Stahl, et al. Handbook of Pharmaceutical Salts: Properties, Selection and Use, 2nd Revised Edition (Wiley-VCH, 2011); S.M. Berge, et al., "Pharmaceutical Salts," Journal of Pharmaceutical Sciences, Vol. 66, No. 1, January 1977).

The compounds of formula (I), (II), (III), (IA), (IIA) or (IIIA) provided herein, or a pharmaceutically acceptable salt thereof, can be mixed with one or more pharmaceutically acceptable carriers, diluents, or excipients. More particularly, the compounds of formula (I), (II), (III), (IA), (IIA) or (IIIA) provided herein, or a pharmaceutically acceptable salt thereof, can be formulated as pharmaceutical compositions. Such pharmaceutical compositions and processes for preparing the same are well known in the art (*see, e.g.,* Remington: The Science and Practice of Pharmacy (A. Gennaro, et al., eds., 21st ed., Mack Publishing Co., 2005)).

The compounds of formula (I), (II), (III), (IA), (IIA) or (IIIA) provided herein, or a pharmaceutically acceptable salt thereof, and their pharmaceutical compositions can be administered by a variety of routes. Such routes of administration include oral and intravenous.

The compounds of formula (I), (II), (III), (IA), (IIA) or (IIIA) provided herein, or a pharmaceutically acceptable salt thereof, can be combined with one or more other therapeutic agents.

The compounds of formula (I), (II), (III), (IA), (IIA) or (IIIA) provided herein, or a pharmaceutically acceptable salt thereof, can be a component in a pharmaceutical composition for the treatment of cancer with one or more pharmaceutically acceptable carriers, diluents, or excipients, and optionally with one or more additional therapeutic agents.

The compounds of formula (I), (II), (III), (IA), (IIA) or (IIIA) provided herein, or a pharmaceutically acceptable salt thereof, can be a component in a pharmaceutical composition for the treatment of cancer with one or more pharmaceutically acceptable carriers, diluents, or excipients, and optionally with one or more additional therapeutic agents.

The compounds of formula (I), (II), (III), (IA), (IIA) or (IIIA) provided herein, or a pharmaceutically acceptable salt thereof, can be combined with one or more other therapeutic agents for simultaneous, separate or sequential administration.

The compounds of formula (I), (II), (III), (IA), (IIA) or (IIIA)provided herein, or a pharmaceutically acceptable salt thereof, and their pharmaceutical compositions can be used in the methods described herein.

The compounds of formula (I), (II), (III), (IA), (IIA) or (IIIA) provided herein, or a pharmaceutically acceptable salt thereof, are generally effective over a wide dosage range. For example, dosages per day normally fall within the range of about 0.5 to about 100 mg/kg of body weight. In some instances, dosage levels below the lower limit of the aforesaid range may be more than adequate, while in other cases still larger doses may be employed without causing any harmful side effect, and therefore the above dosage range is not intended to limit the scope of the invention in any way. It will be understood that the amount of the compound actually administered will be determined by a physician, in the light of the relevant circumstances, including the condition to be treated, the chosen route of administration, the actual compound or compounds administered, the age, weight, and response of the individual patient, and the severity of the patient's symptoms.

Certain compounds of formula (I), (II), (III), (IA), (IIA) or (IIIA), or a pharmaceutically acceptable salt thereof, selectively target FGFR2. For example, certain compounds of formula (I), (II), (III), (IA), (IIA) or (IIIA), or a pharmaceutically acceptable salt thereof, selectively target FGFR2 over another FGFR. For example, certain compounds of formula (I), (II), (III), (IA), (IIA) or (IIIA), or a pharmaceutically acceptable salt thereof, selectively target FGFR2 over FGFR1. For example, certain compounds of formula (I), (II), (III), (IA), (IIA) or (IIIA), or a pharmaceutically acceptable salt thereof, are at least about 3 fold (e.g. at least about 4-, 5-, 6-, 7-, 8-, 9-, 10-, 15-, 20-, 30-, 40-, 50-fold, or more) more selective for FGFR2 than for FGFR1.

As used herein, the term "selectivity" of a compound refers to the compound having more potent activity at the first target than the second target. A fold selectivity can be calculated by any method known in the art. For example, a fold selectivity can be calculated by dividing the IC₅₀ value of a compound for the second target (e.g., FGFR1) by the IC₅₀ value of the same compound for the first target (e.g., FGFR2). An IC₅₀ value can be determined by any method known in the art. For example, an IC₅₀ value can be determined as described in the assays below.

As used herein, the term "cancer" refers to or describes the physiological condition in patients that is typically characterized by unregulated cell proliferation. Included in this definition are benign and malignant cancers, primary and metastatic cancers.

As used herein, the term "FGFR2-associated cancer" refers to cancers associated with or having a dysregulation of the FGFR2 gene, the FGFR2 kinase protein, or expression or activity, or level of any of the same. Non-limiting examples of FGFR2-associated cancer are described herein. As used herein an "FGFR2-associated cancer" includes but is not limited to stomach cancer, hepatobiliary cancer, cancer of unknown primary, gallbladder cancer (e.g. gallbladder adenocarcinoma), bile duct cancer (e.g. intrahepatic bile duct cancer, extrahepatic bile duct cancer), sarcoma, esophagogastric cancer (e.g. gastroesophageal junction adenocarcinoma, gastric remnant adenocarcinoma), esophageal cancer (e.g. esophageal squamous cell cancer, esophageal adenocarcinoma), glioma (e.g. astrocytoma, oligodendroglioma, ependymoma), Non-Hodgkin Lymphoma (e.g. B-cell Non-Hodgkin Lymphoma), gastrointestinal stromal tumor, breast cancer (e.g. invasive ductal cancer, invasive lobular cancer), lung cancer (e.g. non-small-cell lung cancer, lung adenocarcinoma, squamous cell lung cancer and small-cell lung cancer), urothelial cancer, bladder cancer (e.g. urothelial bladder cancer, non-muscle invasive bladder cancer, muscle invasive bladder cancer), gastric cancer (e.g. gastric adenocarcinoma), pancreatic cancer (e.g. pancreatic adenocarcinoma), prostate cancer (e.g. prostate adenocarcinoma), colorectal cancer (e.g. colorectal adenocarcinoma, colon adenocarcinoma,), multiple myeloma, liver cancer (e.g. hepatocellular cancer, fibrolamellar hepatocellular cancer), skin cancer (e.g. squamous cell skin cancer), melanoma (e.g. cutaneous melanoma), head and neck cancer (e.g. head and neck squamous cell cancer, hypopharyngeal cancer, laryngeal cancer, lip and oral cavity cancer, salivary gland cancer), glioblastoma, endometrial cancer (e.g. endometrial endometrioid adenocarcinoma), cervical cancer and ovarian cancer (e.g. epithelial ovarian cancer).

As used herein, the term "treating" (or "treatment") refers to restraining, slowing, stopping, or reversing the progression or severity of an existing symptom, condition or disorder.

As used herein, the term "patient" refers to a mammal, particularly a human.

Provided herein, are compounds of formula (I), (II), (III), (IA), (IIA) or (IIIA), or a pharmaceutically acceptable salt thereof, for use in therapy.

Provided herein, are compounds of formula (I), (II), (III), (IA), (IIA) or (IIIA), or a pharmaceutically acceptable salt thereof, for use in the treatment of cancer.

Provided herein, are the use of compounds of formula (I), (II), (III), (IA), (IIA) or (IIIA), or a pharmaceutically acceptable salt thereof, in the manufacture of a medicament for treating cancer.

Provided herein, are compounds of formula (1), (II), (III), (IA), (IIA) or (IIIA), or a pharmaceutically acceptable salt thereof, for use in methods of treating cancer, comprising administering to a patient in need of such treatment an effective amount of the compounds of formula (I), (II), (III), (IA), (IIA) or (IIIA), or a pharmaceutically acceptable salt thereof.

Provided in the methods and uses herein, the cancer is selected from the group consisting of stomach cancer, hepatobiliary cancer, cancer of unknown primary, gallbladder cancer (e.g. gallbladder adenocarcinoma), bile duct cancer (e.g. intrahepatic bile duct cancer, extrahepatic bile duct cancer), sarcoma, esophagogastric cancer (e.g. gastroesophageal junction adenocarcinoma, gastric remnant adenocarcinoma), esophageal cancer (e.g. esophageal squamous cell cancer, esophageal adenocarcinoma), glioma (e.g. astrocytoma, oligodendroglioma, ependymoma), Non-Hodgkin Lymphoma (e.g. B-cell Non-Hodgkin Lymphoma), gastrointestinal stromal tumor, breast cancer (e.g. invasive ductal cancer, invasive lobular cancer), lung cancer (e.g. non-small-cell lung cancer, lung adenocarcinoma, squamous cell lung cancer and small-cell lung cancer), urothelial cancer, bladder cancer (e.g. urothelial bladder cancer, non-muscle invasive bladder cancer, muscle invasive bladder cancer), gastric cancer (e.g. gastric adenocarcinoma), pancreatic cancer (e.g. pancreatic adenocarcinoma), prostate cancer (e.g. prostate adenocarcinoma), colorectal cancer (e.g. colorectal adenocarcinoma, colon adenocarcinoma,), multiple myeloma, liver cancer (e.g. hepatocellular cancer, fibrolamellar hepatocellular cancer), skin cancer (e.g. squamous cell skin cancer), melanoma (e.g. cutaneous melanoma), head and neck cancer (e.g. head and neck squamous cell cancer, hypopharyngeal cancer, laryngeal cancer, lip and oral cavity cancer, salivary gland cancer), glioblastoma, endometrial cancer (e.g. endometrial endometrioid adenocarcinoma), cervical cancer and ovarian cancer (e.g. epithelial ovarian cancer). Particularly, the cancer is selected from the group consisting of stomach cancer, hepatobiliary cancer, cancer of unknown primary, gallbladder cancer (e.g. gallbladder adenocarcinoma), bile duct cancer (e.g. intrahepatic bile duct cancer, extrahepatic bile duct cancer), esophagogastric cancer (e.g. gastroesophageal junction adenocarcinoma, gastric remnant adenocarcinoma), esophageal cancer (e.g. esophageal squamous cell cancer, esophageal adenocarcinoma), glioma (e.g. astrocytoma, oligodendroglioma, ependymoma), breast cancer (e.g. invasive ductal cancer, invasive lobular cancer), lung cancer (e.g. non-small-cell lung cancer, lung adenocarcinoma, squamous cell lung cancer and small-cell lung cancer), gastric cancer (e.g. gastric adenocarcinoma), pancreatic cancer (e.g. pancreatic adenocarcinoma), colorectal cancer (e.g. colorectal adenocarcinoma, colon adenocarcinoma,), liver cancer (e.g. hepatocellular cancer, fibrolamellar hepatocellular cancer), skin cancer (e.g. squamous cell skin cancer), melanoma (e.g. cutaneous melanoma), head and neck cancer (e.g. head and neck squamous cell cancer, hypopharyngeal cancer, laryngeal cancer, lip and oral cavity cancer, salivary gland cancer), glioblastoma, endometrial cancer (e.g. endometrial endometrioid adenocarcinoma) and ovarian cancer (e.g. epithelial ovarian cancer). More particularly, the cancer is selected from the group consisting of hepatobiliary cancer, cancer of unknown primary, gallbladder cancer (e.g. gallbladder adenocarcinoma), bile duct cancer (e.g. intrahepatic bile duct cancer, extrahepatic bile duct cancer), breast cancer (e.g. invasive ductal cancer, invasive lobular cancer), liver cancer (e.g. hepatocellular cancer, fibrolamellar hepatocellular cancer), skin cancer (e.g. squamous cell skin cancer), melanoma (e.g. cutaneous melanoma) and endometrial cancer (e.g. endometrial endometrioid adenocarcinoma). Most particularly, the cancer is selected from the group consisting of hepatobiliary cancer, gallbladder cancer (e.g. gallbladder adenocarcinoma), bile duct cancer (e.g. intrahepatic bile duct cancer, extrahepatic bile duct cancer), breast cancer (e.g. invasive ductal cancer, invasive lobular cancer), liver cancer (e.g. hepatocellular cancer, fibrolamellar hepatocellular cancer and endometrial cancer (e.g. endometrial endometrioid adenocarcinoma).

The compounds provided herein can be prepared as illustrated in the preparations and examples below.

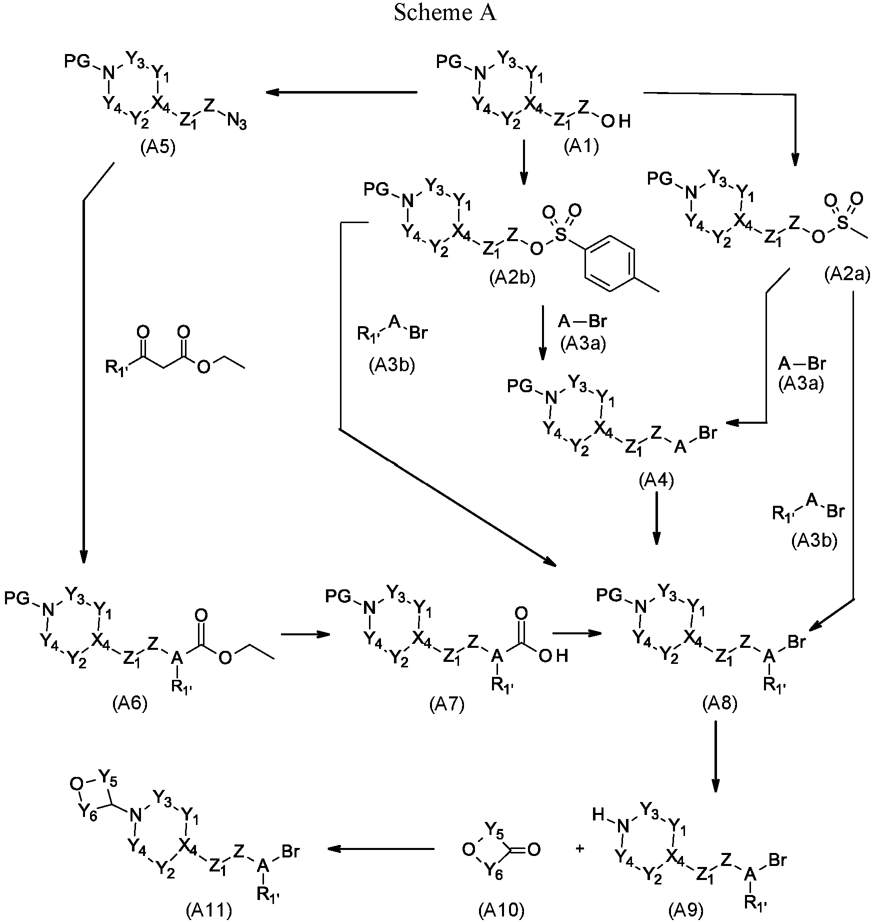

Scheme A depicts the preparation of compound (A8), where R₁' is defined as C₁-C₃ alkyl, through multiple synthetic routes that lead to compound (A11) and will be further elaborated to Formula 1. Alcohol (A1) may react with mesyl chloride or tosyl chloride to afford compound (A2a) or (A2b), that may be further reacted with (A3a) to provide (A4). Treatment of compound (A4) with LDA and an appropriate alkylating agent may afford compound (A8). Alternatively, compound A8 may be directly synthesized by alkylating compound A3b with mesylate A2a or tosylate A2b to afford compound A8.

Compound (A8) may also be synthesized through an alternative route as depicted in Scheme 1. Alcohol (A1) may react under Mitsunobu conditions to provide azide (A5). Azide (A5) may be condensed with a beta-keto ester to afford triazole ester (A6) that can undergo saponification to provide carboxylic acid (7). Treatment of carboxylic acid (A7) with bromine in the presence of base affords compounds of (A8).

Scheme 1 further depicts the preparation of compounds of (A11). Compounds of (A8) may be deprotected to give (A9). Reacting compound (A9) with an appropriate ketone (A10) under reductive amination conditions affords compounds of (A11). R_{1'} is defined as C₁-C₃ alkyl.

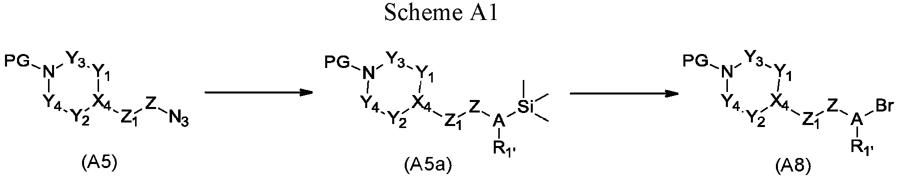

Scheme A1 depicts an alternative route to obtain compounds of (A8). Reaction of (A5) in the presence of trimethyl(prop-1-yn-1-yl)silane under microwave conditions may afford trimethylsilyl analogs of (A5a). Treatment of (A5a) with NBS in the presence of SiO₂ may afford compounds of (A8).

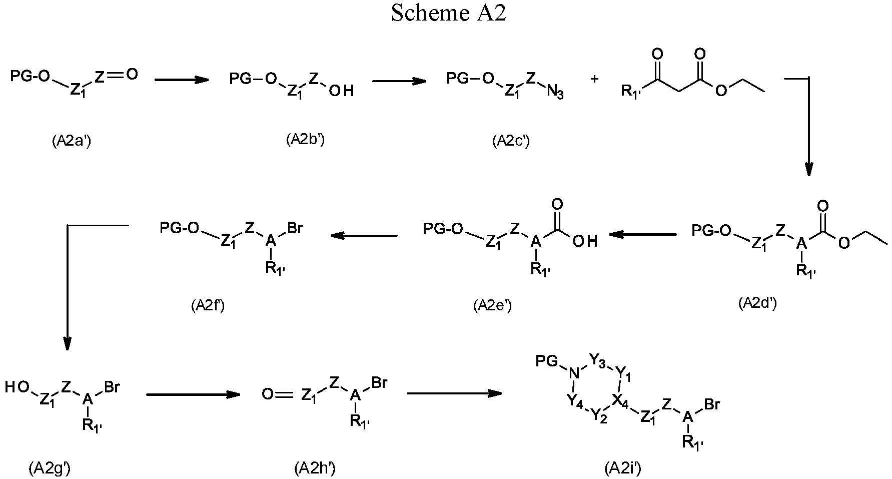

Scheme A2 depicts the preparation of compound (A2i'), where R₁' is defined as C₁-C₃ alkyl, that will be further elaborated to Formula 1. O-protected cyclobutan-1-one (A2a') may be reacted in the presence of NaBH₄ to afford alcohol (A2b'). Formation of the azide (A2c') may be accomplished by reacting the alcohol (A2b') with PPh₃ and DIAD followed by DPPA. Reaction of azide (A2c') with ethyl acetoacetate may yield ester (A2d'). Hydrolysis of (A2d') under basic conditions may afford acid (A2e') which then may be subjected to bromination under basic conditions to provide bromide (A2f'). Removal of the protecting group from (A2f') may afford alcohol (A2g'). Oxidation of alcohol (A2g') may afford ketone (A2h') which may then be reacted under reductive amniation conditions to afford (A2i').

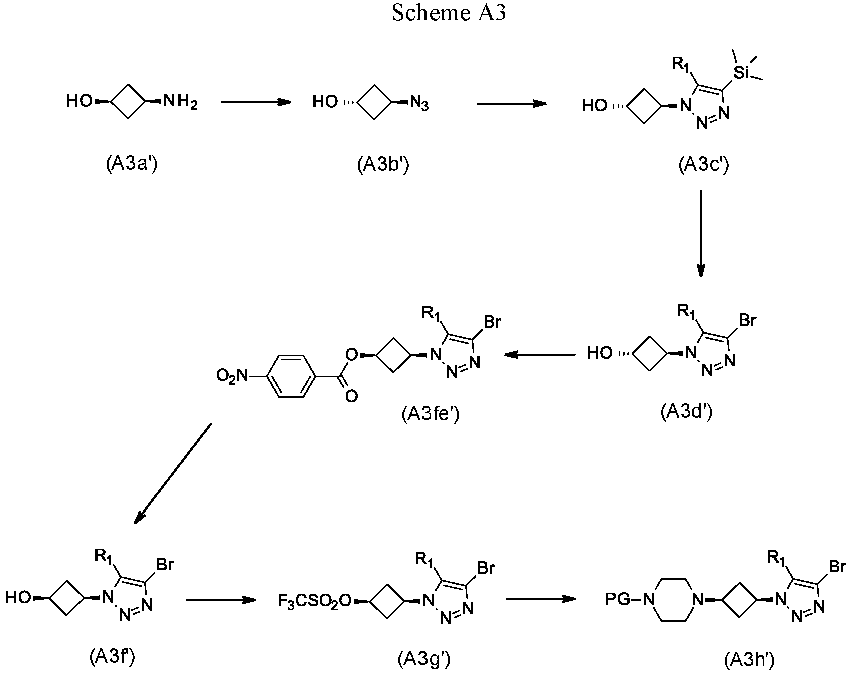

Scheme A3 depicts the preparation of compound (A3h') that will be further elaborated to Formula 1. A solution of (A3a') may be reacted with N-diazo-1,1,1-trifluoro-methanesulfonamide in the presence of copper sulfate and NaHCO₃ to afford azide (A3b'). Treatment of the azide in the presence of trimethyl(prop-1-yn-1-yl)silane under microwave conditions may provide (A3c'). Formation of bromide (A3d') may be accomplished by treatment of (A3c') with NBS in the presence of SiO₂. Subjecting (A3d') to Mitsunobu conditions with p-nitrobenzoic acid may yield ester (A3f'). Hydrolysis of the ester (A3e') under basic conditions may provide alcohol (A3f'). Formation of triflate (A3g') may result when the alcohol (A3f') is treated with (trifluoromethane)sulfonyl trifluoromethanesulfonate in the presence of a base. Displacement of triflate (A3g') with an appropriate amine may afford (A3h')

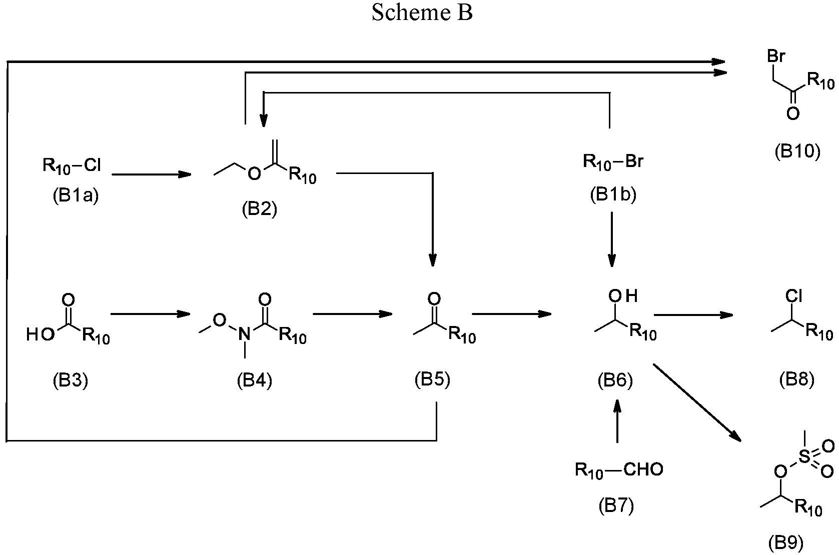

Scheme B depicts the preparation of compounds B6, B8, B9 and B10 that will be further elaborated to Formula 1. Compounds of (B10) and (B10a) may be further elaborated to Formula 2, Formula 3, Formula 4 or Formula 4a.

Compound (B2) may be synthesized starting from either halide (B1a) or (B1b) and reacting either one of these halides with tributyl(1-ethoxyethenyl)stannane under palladium catalyzed conditions. Hydrolysis of ethoxyvinly (B2) may afford ketone (B5). Ketone (B5) may be reduced to afford alcohol (B6) which is then reacted with MsCl to afford mesylate (B8) or chloride (B9).

Alternatively, compounds of (B6) may be synthesized by reacting aldehyde (B7) with the appropriate Grignard reagent to provide alcohol (B6). A third alternative route to compounds of (B6) may be accomplished by treatment of halide (B1b) with lithium dibutyl(methyl) magnesite followed by the addition of acetaldehyde to afford alcohol (B6).

Treatment of ketone (B5) with phenyltrimethylammonium bromide may provide the alpha-bromo ketone (B10). Alternatively, the alpha-bromo ketone (B10) may be formed by the reaction of ethoxyvinyl (B2) in the presence of NBS in aq THF.

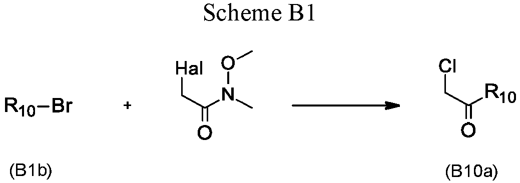

Scheme B1 depicts an alternative route to alpha-halo ketones of (B10a) that will be further elaborated to Formula 1. Treatment of (B1b) with i-PrMgCl followed by the addition of chloro-N-methoxy-N-methylacetamide may afford (B10a) which may be further elaborated to compounds of Formula (I).

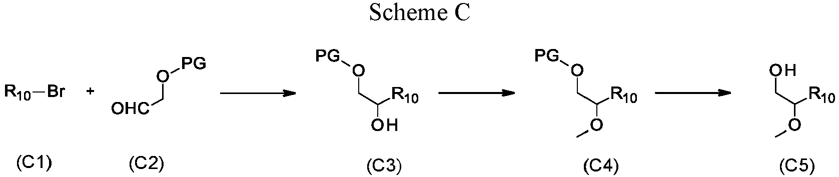

Scheme C depicts the preparation of compounds (C5) that will be further elaborated to Formula 1. Treatment of compound (C1) with i-PrMgCl followed by the addition of aldehyde (C2) may provide alcohol (C3). Reaction of alcohol (C3) by the addition of potassium t-butoxide in the presence of methyl iodide may afford compound (C4) which is then deprotected to provide alcohol (C5).

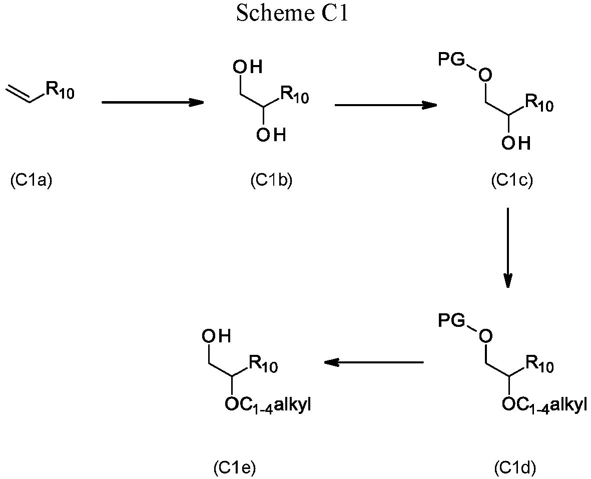

Scheme C1 depicts the preparation of compounds (C1e) that will be further elaborated to Formula 1. Reaction of ethenyl (C1a) in the presence of K₂OsO₄ and NMO may afford the bis alcohol (C1b). The primary alcohol of (C1a) may be protected by treatment with SEM-Cl in the presence of a base which may provide (C1b). The secondary alchol of (C1b) may be alkyalted with methyl iodide under silver catalyzed conditions which may afford the ether (C1d). Removal of the protecting group of (C1d) under acidic conditions may provide the primary alcohol (C1e).

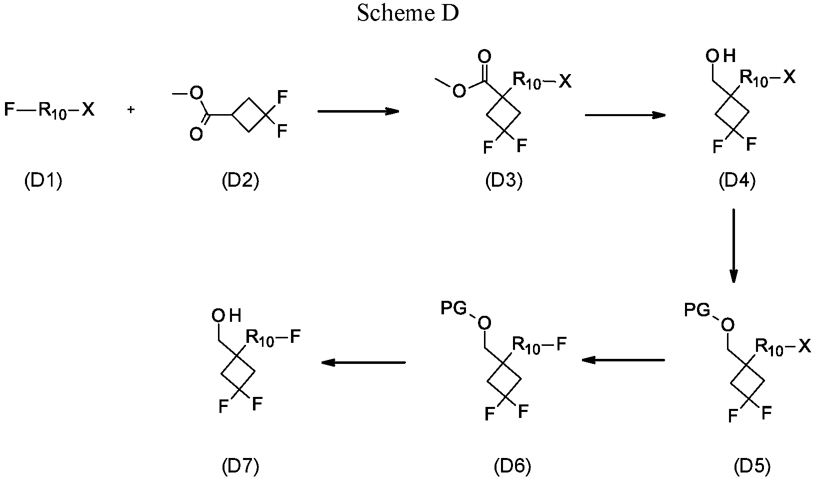

Scheme D depicts the preparation of compound (D7) that will be further elaborated to Formula 1. Treatment of methyl 3,3-difluorocyclobutane-1-carboxylate with KHMDS in the presence of fluoro (D1), where X = Br, may provide ester (D2). The reduction of ester (D2) to alcohol (D4) is accomplished by treatment with LiBH₄. Protection of alcohol (D4) with DHP and a catalytic amount of TsOH may afford the tetrahydropyran (D5). Reaction of tetrahydropyran (D5) with n-BuLi followed by addition of NFSI may afford (D6). Removal of the protecting group under acidic conditions may afford alcohol (D7).

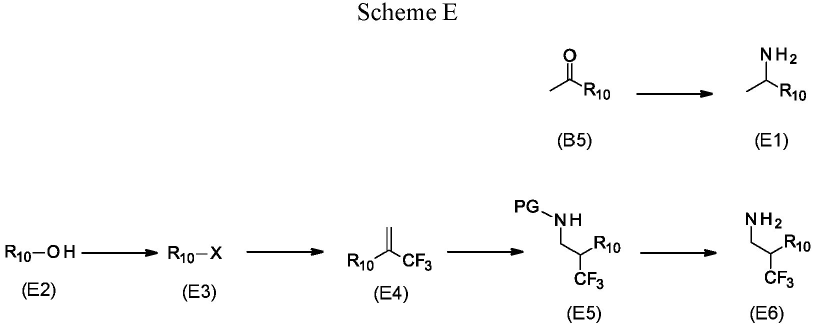

Scheme E depicts the preparation of compounds (E1) and (E6) that will be further elaborated to Formula 1. Compounds of (E6) may be synthesized by reaction of (E2) with POCl₃ to provide halogenated (E3). Treatment of (E3) with tributyl(1-ethoxyethenyl)stannane under palladium catalyzed conditions may afford ethoxyvinyl (E4). Reaction of benylamine with (E4) may provide N-benzyl protected (E5). Deprotection of (E5) may provide amine (F6).

Compounds of (E1) may be synthesized by treatment of (B5) with NH₄OAc.

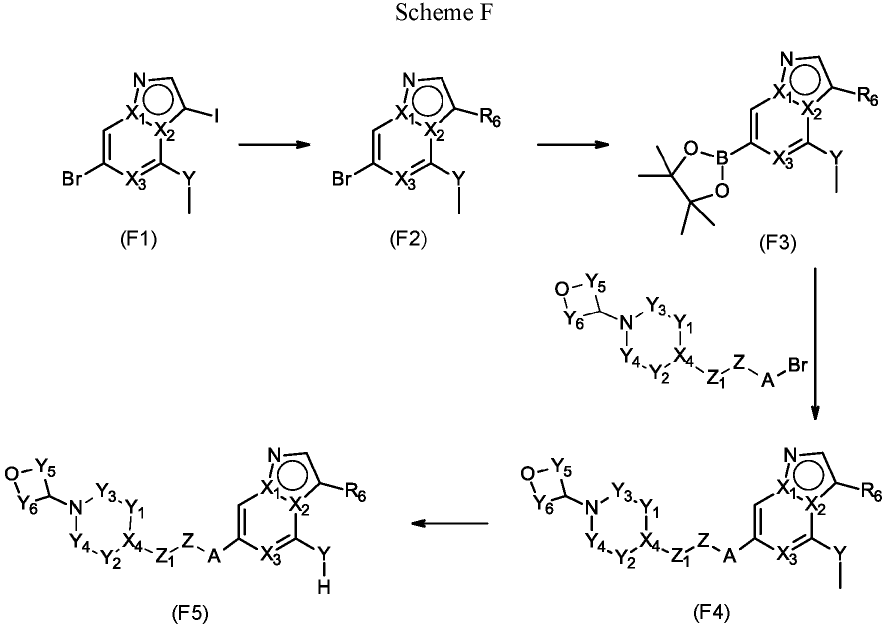

Scheme F depicts the preparation of compound (F5) that will be further elaborated to Formula 1. Reaction of (F1) with methyl 2,2-difluoro-2-(fluorosulfonyl)acetate in the presence of CuI may provide (F2). Treatment of (F2) with bis(pinacolato)diboron under palladium catalyzed conditions may afford boronate ester (F3). Palladium catalyzed coupling with the appropriate bromide with boronate ester (F3) may provide (F4) which is then demethylated with NDM to afford (F5).

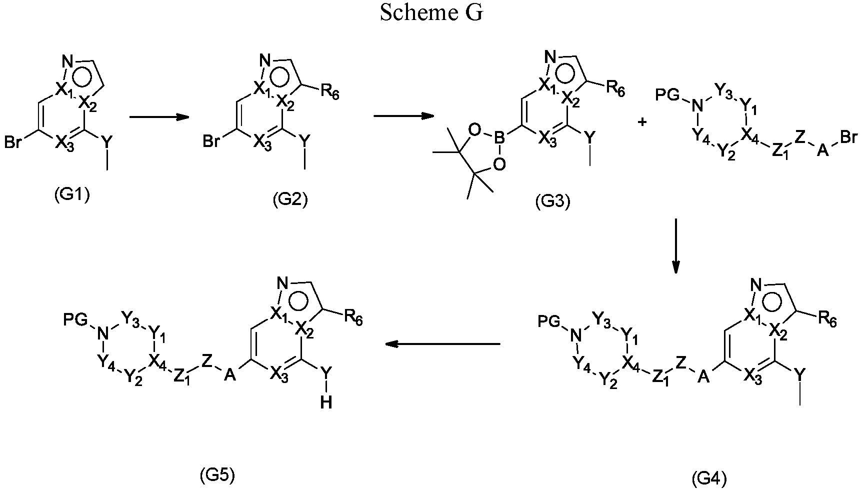

Scheme G depicts the preparation of compound (G5) that will be further elaborated to Formula 1. Reaction of (G1) with 1-chloromethyl-4-fluoro-1,4-diazoniabicyclo[2.2.2]octane bis(tetrafluoroborate) may provide (G2), wherein R₆ is defined as R₆ = F. Treatment of (G2) with bis(pinacolato)diboron under palladium catalyzed conditions may afford boronate ester (F3). The coupling of boronate ester (F3) with the appropriate bromide in the presence of a palladium catalyst may provide (G4). Demethylation of (G4) with NDM may afford (G5).

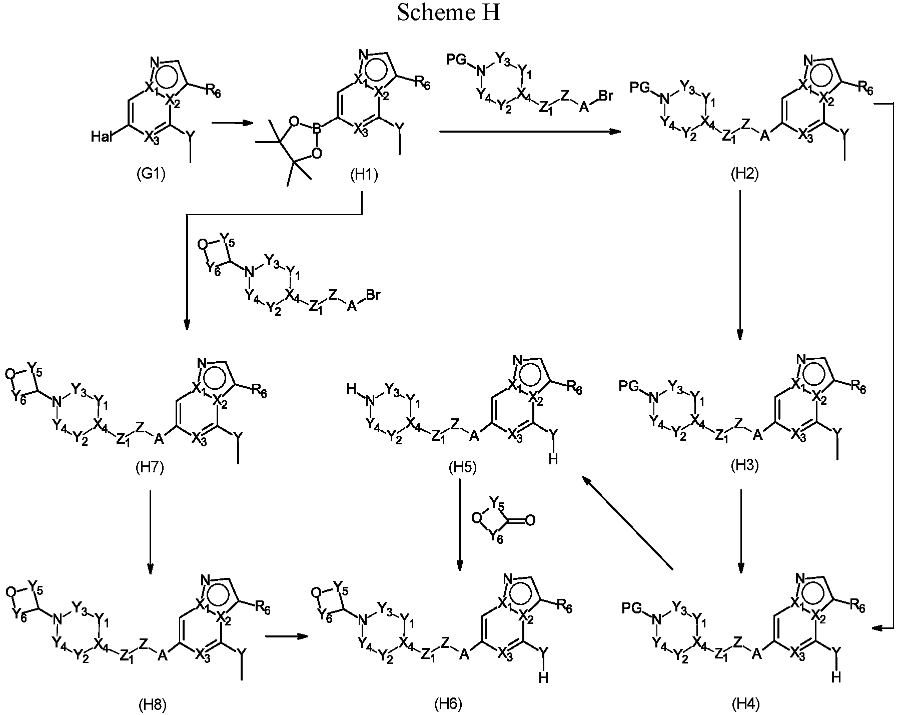

Scheme H depicts multiple methods for the preparation of the compounds (H4) and (H6), that will be further elaborated to Formula 1. Compound (G1), where Hal is defined as Hal = halogen, may be reacted with bis(pinacolato)diboron under palladium catalyzed conditions to provide boronate ester (H1). The boronate ester (H1) is coupled with the appropriate bromide in the presence of a palladium catalyst to provide (H2). Chlorination of (H2) with NSC may provide (H3), where R₆ is defined as R₆ = Cl, which is then demethylated with NDM to afford (H4). Deprotection of (H4) under acidic conditions may yield (H5), which may then be subjected to reductive amination conditions with the appropriate ketone to provide (H6). Alternatively, boronate ester (H1), where R₆ is defined as R₆ = H, may be coupled with the appropriate bromide to provide (H7). Chlorination of (H7) may be accomplished by treatment with NCS to afford (H8), where R₆ is defined as R₆ = Cl. Demethylation of (H8) with NDM may afford (H6).

Scheme H also depicts multiple methods for the preparation of the compounds (H4) and (H6), where R₆ is defined as R₆ = CN, and Hal is defined as Hal = Br, that will be further elaborated to Formula (I). Compound (G1) may be reacted with bis(pinacolato)diboron under palladium catalyzed conditions to provide boronate ester (H1). The boronate ester (H1) is coupled with the appropriate bromide in the presence of a palladium catalyst to provide (H2). Subjecting (H2) with NDM may afford demethylated (H4). Deprotection of (H4) under acidic conditions may yield (H5), which may then be subjected to reductive amination conditions with the appropriate ketone to provide (H6). Alternatively, boronate ester (H1) may be coupled with the appropriate bromide to provide (H7). Demethylation of (H7) with NDM may afford (H6).

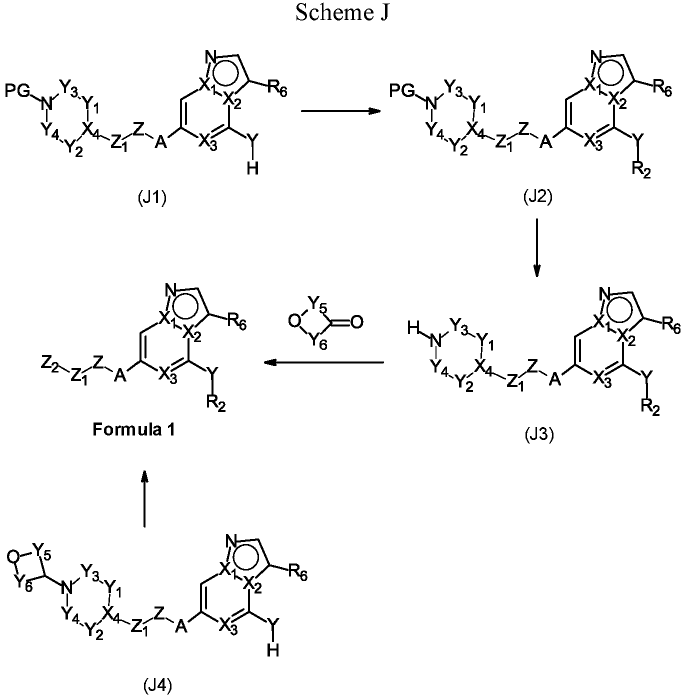

Scheme J depicts the preparation of compounds of Formula 1. (J1) may be alkylated with halogens of (B8), (B10), (B10a) or with mesylates of (B9) to afford (K2). Alternatively, reaction of alcohols of (C1e), (C5) or (D7) under Mitsunobu conditions may also provide (J2). Deprotection of (J2) under acidic conditions may yield (J3) which is then subjected to reductive amination conditions with the appropriate ketone to afford compounds of Formula 1. Alternatively, compounds of Formula 1 may be synthesized by alkylation of (J4) with halogens of (B8), (B10), (B10a) or with mesylates of (B9) or alternatively, with reaction of alcohols of (C1e), (C5) or (D7) under Mitsunobu conditions. One skilled in the art may recognize that compound (J1) may be interchanged with compounds of (G5) or (H4). Additionally, one skilled in the art may recognize that compound (J4) may be interchanged with compounds of (F5) or (H6).

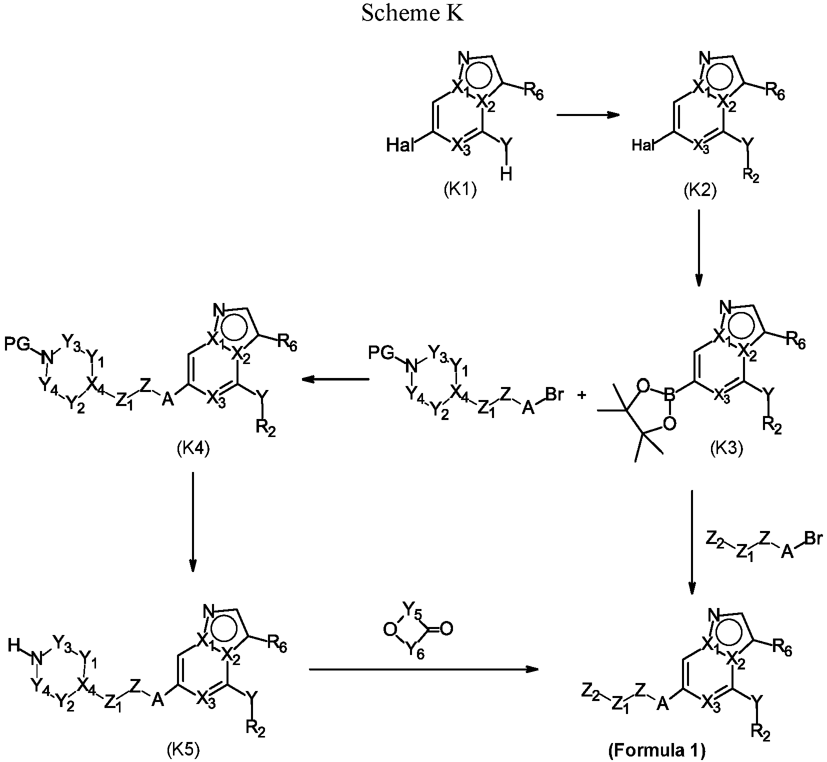

Scheme K depicts the preparation of compounds of Formula 1. Reaction of (K1) and Hal is defined as Hal = Br, may be alkylated with halides of (B8), (B10), (B10a) or mesylates of (B9) to provide (L2). Treatment of (K2) with bis(pinacolato)diboron under palladium catalyzed conditions may afford boronate ester (K3). Palladium catalyzed coupling of (K3) with the appropriate bromide, (A2i') or (A3h') may provide compounds of Formula (I). Alternatively, compounds of Formula (I) may be synthesized by the palladium catalyzed coupling of (K3) with the appropriate bromide to yield (K4). Deprotection of (K4) under acidic conditions may afford (K5) which may be subjected to reductive amination conditions with the appropriate ketone to provide compounds of Formula 1.

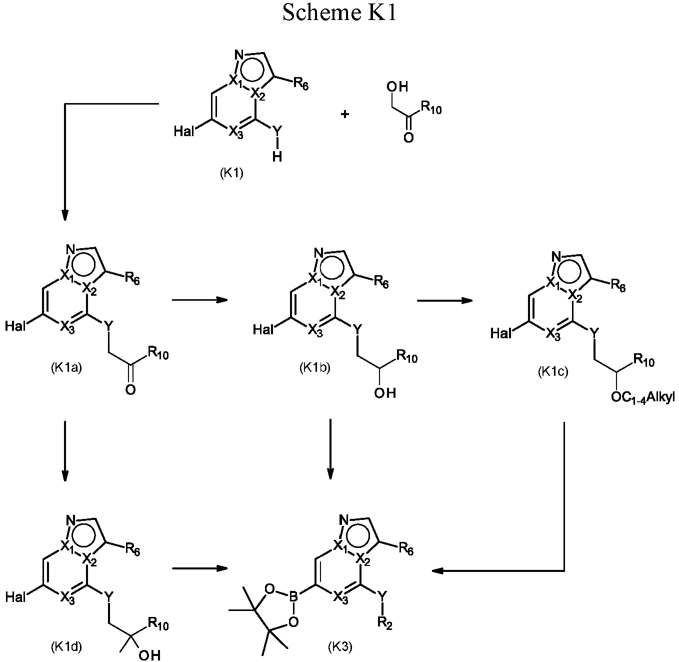

Scheme K1 depicts an alternative preparation of compound (K3) that may be taken on to Formula 1. Reaction of (K1) under Mitsunobu conditions may afford ketone (K1a). Reduction of the ketone (K1a) may afford alcohol (K1b) which may be alkylated with the appropriate alkyating agent to afford ether (K1c). Treatment of (K1b) or (K1c) with bis(pinacolato)diboron under palladium catalyzed conditions may afford (K3). Alternatively, (K1a) may be reacted with the appropriate Grignard reagent to provide (K1d). Treatment of (K1d) with bis(pinacolato)diboron under palladium catalyzed conditions may afford (K3).

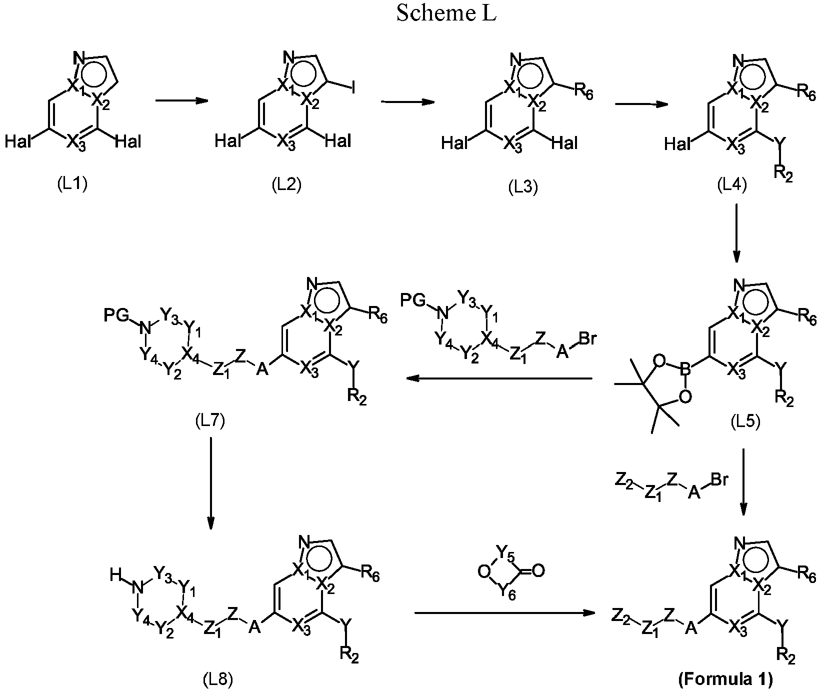

Scheme L depicts two methods for the preparation of compounds of Formula 1. In the first method, treatment of (L1) with NIS may afford (L2) which is then reacted with CuCN to provide (L3). Reaction of (L3) with amine (F4) in the presence of an organic base may yield (L4). Treatment of (L4) with bis(pinacolato)diboron under palladium catalyzed conditions may afford boronate ester (L5). Palladium catalyzed coupling of (L5) with the appropriate bromide may provide compounds of Formula 1. Alternatively, in the second method, compounds of Formula 1 may be synthesized by the palladium catalyzed coupling of (L5) with the appropriate bromide to yield (L7). Deprotection of (L7) under acidic conditions may afford (L8) which may be subjected to reductive amination conditions with the appropriate ketone to provide compounds of Formula 1. For compounds of Formula 1 the enantiomers may be separated by chiral chromatography.

Scheme L also depicts the preparation of compounds of Formula 1. Treatment of (L3) with amine (F4) or (F5) under palladium catalyzed conditions may yield (L4). Reaction of (L4) with bis(pinacolato)diboron under palladium catalyzed conditions may afford boronate ester (L5). Palladium catalyzed coupling of (L5) with the appropriate bromide may provide compounds of Formula 1. For compounds of Formula 1 the enantiomers may be separated by chiral chromatography.

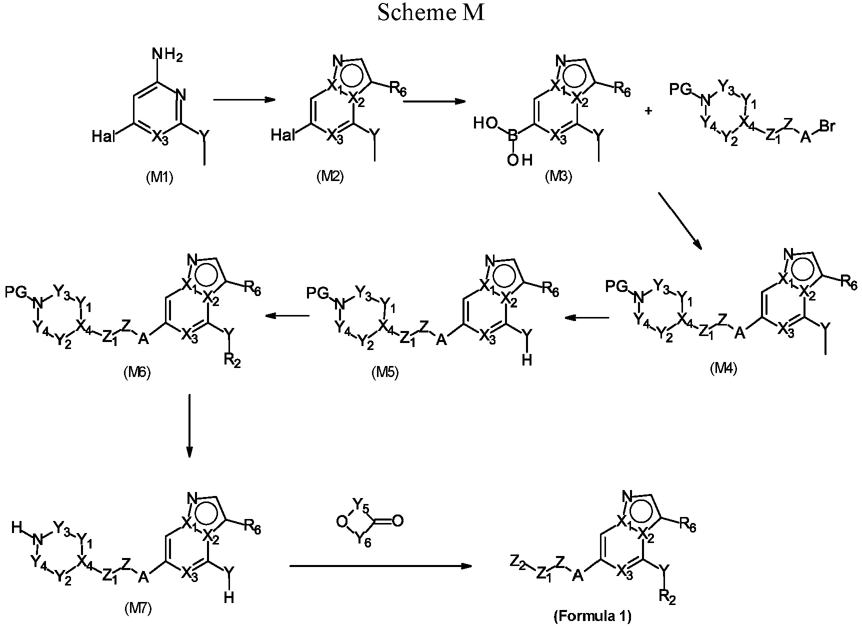

Scheme M depicts the preparation of compounds of Formula 1 where R₆ is defined as R₆ = Cl and X₁, X₂ and X₃ are defined as X₂ = N, X₁ and X₃ = C. Treatment of (M1), where Hal is defined as Cl and Y is defined O, with chloroacetaldehyde in the presence of a base may result in (M2). Reaction of (M2) with bis(pinacolato)diboron under palladium catalyzed conditions may afford the boronic acid (M3). Palladium catalyzed coupling of (M3) with the appropriate bromide may yield (M4). Demethylation of (M4) with NDM may afford (M5) which may then be reacted with alcohols of (C1e) or (C5) under Mitsunobu conditions to provide (M6). Deprotection of (M6) under acidic conditions may afford (M7) which is then subjected to reductive amination conditions with the appropriate ketone to provide compounds of Formula 1. For compounds of Formula 1 the enantiomers may be separated by chiral chromatography.

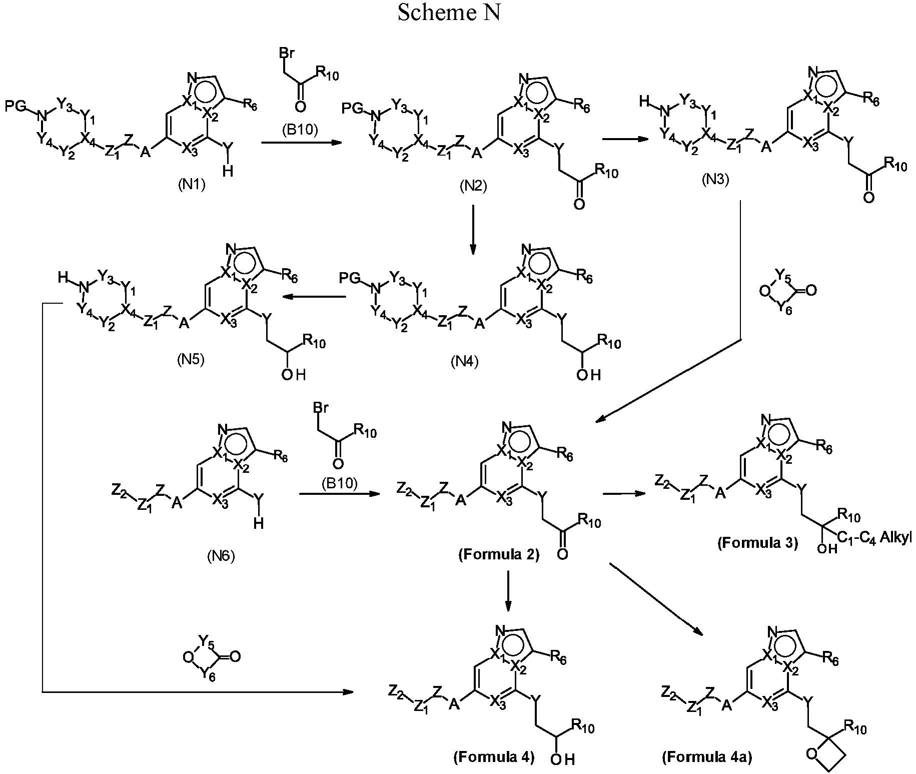

Scheme N depicts two methods for the preparation of compounds of Formula 2. In the first method (N1) may be alkylated with alpha-bromo ketone (B10) or (B10a) to afford (N2). Subsequent deprotection of (N2) under acidic conditions may yield (N3) which then may be reacted with an appropriate ketone to provide compounds of Formula 2. In the second method (N6) may be alkylated with alpha-bromo ketone (B10) or (B10a). For compounds of Formula 2 the enantiomers may be separated by chiral chromatography.

Compounds of Formula 3 may be synthesized by the treatment of compounds of Formula 2 with the appropriate Grignard reagent to provide compounds of Formula 3. For compounds of Formula 3 the enantiomers may be separated by chiral chromatography.

Compounds of Formula 4 may be synthesized by two routes. In the first route, reduction of the ketone for compounds of Formula 2 may be accomplished by treatment with NaBH₄ to provide compounds of Formula 4. In the second route, the ketone (N2) may be reduced with NaBH₄ to provide (N4) which may be deprotected under acidic conditions to afford (N5). Treatment of (N5) with the appropriate ketone under reductive amination conditions may yield compounds of Formula 4. For compounds of Formula 4 the enantiomers may be separated by chiral chromatography.

One skilled in the art may recognize that compounds of (N1) may be interchanged with compounds of (G5) or (H4). Additionally, one skilled in the art may also recognize that compounds of (N6) may be interchanged with compounds of (F5).

Compounds of Formula (4a) may be synthesized by reacting trimethylsulfoxonium iodide in the presence of a base followed by the addition of compounds of Formula 4.

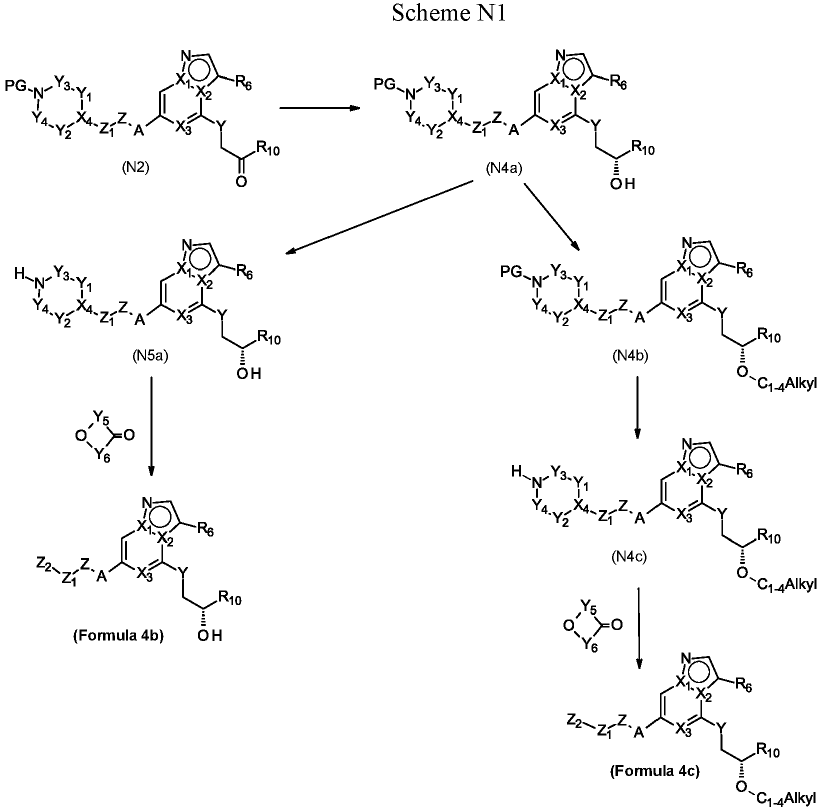

Scheme N1 depicts the asymetric synthesis of compounds of Formula 4b and 4c. The asymetric reduction of ketone (N2) may be accomplished by treatment with chloro(n-[(1R,2R)-2-[(S)-[2-[[1,2,3,4,5,6-η)-4-methylphenyl]methoxy]ethyl]amino]-1,2-diphenyl ethylmethanesulfonamidato) ruthenium(II) to afford alcohol (N4a). Deprotection of (N4a) under acidic conditions may yield the amine (N5a). Reductive amination of (N5a) with the appropriate ketone may afford compounds of Formula 4b.

Alkylation of alcohol (N4a) with the appropriate alkylating agent may provide alkyl ether (N4b). Subsequent deprotection of (N4b) under acidic conditions may yield amine (N4c). Redcutive amination of (N4c) with the apporpriate ketone may provide compounds of Formula 4c.

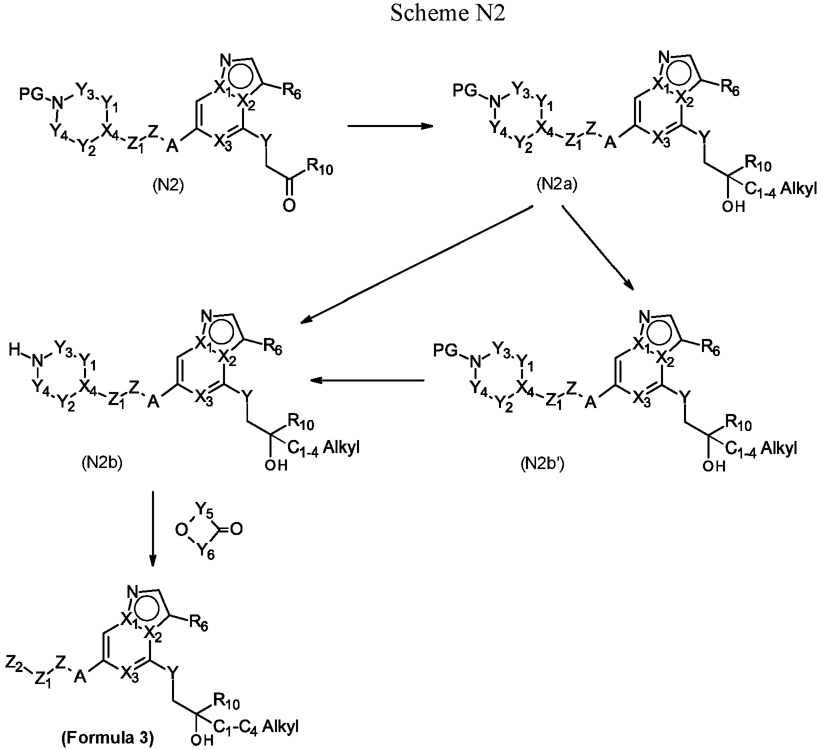

Scheme N2 depicts an alternative preparation for compounds of Formula 3. Ketone (N2) may be reacted with a Grignard reagent to afford the tertiary alcohol (N2a). Removal of the protecting group of (N2a) under acidic conditions may afford the amine (N2b). Subjecting (N2b) under reductive amination conditions with an appropriate ketone may afford compounds of Formula 3.

In cases where R₆ = H for (N2a), treatment with NCS may provide compounds where R₆ = Cl for (N2b'). Subsequent treatment of (N2b') under acidic conditions may afford the amine (N2b).

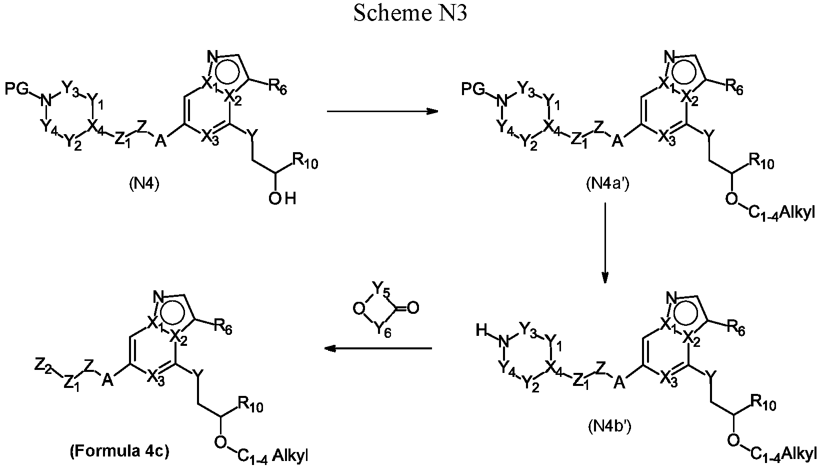

Scheme N3 depicts an alternative route for the synthesis of compounds of Formula 4c. Alkylation of the alcohol (N4) with an appropriate alkylating agent may afford the alkyl ether (N4a'). Deprotection of (N4a') under acidic conditions may provide the amine (N4b') which is then reacted with the appropriate ketone under reductive amination conditions to afford compounds of Formula 4c.

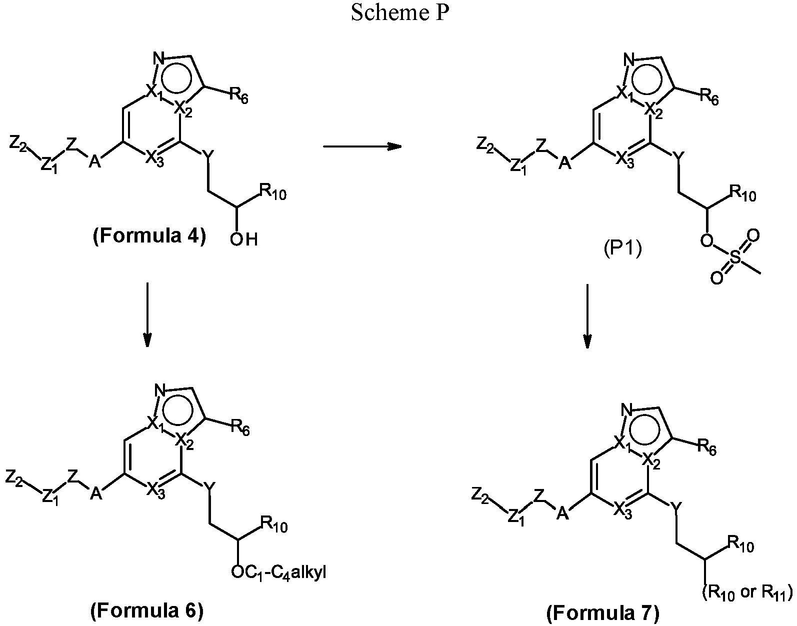

Scheme P depicts the preparation of compounds of Formula 6 or Formula 7. For the preparation of compounds of Formula 6, compounds of Formula 4 may be reacted with NaH followed by treatment with the appropriate alkylating agent to afford compounds of Formula 6. For the preparation of compounds of Formula 6, compounds of Formula 4 may be reacted with MsCl to provide (P1). Subsequent treatment of (P1) with an appropriate amine provides compounds of Formula 7.

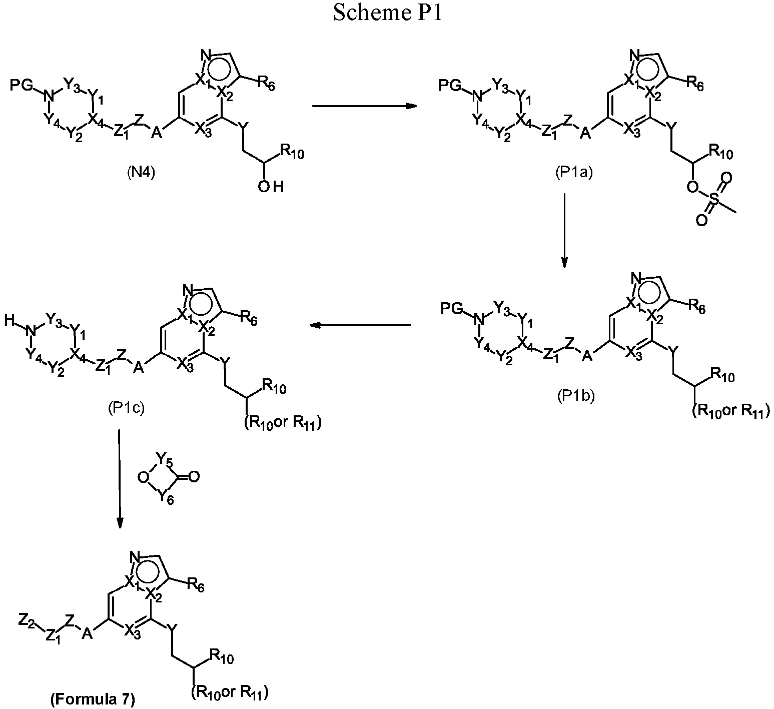

Scheme P1 depicts an alternative preparation for compounds of Formula 7. The mesylate (P1a) may be prepared by the reaction of (N4) with methane sulfonyl chloride. Displacement of the mesylate (P1a) with an amine may afford the alkyl amino (P1b). Deprotection of (P1b) under acidic conditions may lead to the amine (P1c) which is the reacted with the appropriate ketone under reductive amination conditions to afford compounds of Formula 7.

Certain stereochemical centers have been left unspecified and certain substituents have been eliminated in the following schemes for the sake of clarity and are not intended to limit the teaching of the schemes in any way. Furthermore, individual isomers, enantiomers, and diastereomers may be separated or resolved by one of ordinary skill in the art at any convenient point in the synthesis of compounds of the invention, by methods such as selective crystallization techniques or chiral chromatography (See for example, J. Jacques, et al., "Enantiomers, Racemates, and Resolutions", John Wiley and Sons, Inc., 1981, and E.L. Eliel and S.H. Wilen," Stereochemistry of Organic Compounds", Wiley-Interscience, 1994). The designations "isomer 1" and "isomer 2" refer to the compounds that elute from chiral chromatography first and second, respectively, under the conditions described herein and if chiral chromatography is initiated early in the synthesis, the same designation is applied to subsequent intermediates and examples. Additionally, the intermediates described in the following schemes may contain a number of nitrogen or oxygen protecting groups. The variable protecting group may be the same or different in each occurrence depending on the particular reaction conditions and the particular transformations to be performed. The protection and deprotection conditions are well known to the skilled artisan and are described in the literature (See for example *"*Greene's Protective Groups in Organic Synthesis", Fourth Edition, by Peter G.M. Wuts and Theodora W. Greene, John Wiley and Sons, Inc. 2007).

The designation "isomer 1" was also used where ketones had been subjected to asymmetric reduction using ruthenium catalysts which are described herein. The same designation was applied to subsequent intermediates and examples unless the examples had been subjected to chiral chromatography. The asymmetric reduction of ketones to secondary alchols using ruthenium catalysts is known in the literature (See for example "J. Am. Chem. Soc. 2011, 133, 14960-14963).

"AcOH" refers to acetic acid; "AcCN" refers to acetonitrile; "NH₄OAc" refers to ammonium acetate; "NH₄OH" refers to ammonium hydroxide; "aq" refers to aqueous; "BPR" refers to back pressure regulator; "NBS" refers to N-bromosuccinimide; "nBuOH" refers to 1-butanol; "n-BuLi" refers to n-butyl lithium; "DCDMH" refers to 1,3-dichloro-5,5-dimethyl-2,4-imidazolidinedione; "NCS" refers to N-chloro succinimide; "conc" refers to concentrated; "cHex" refers to cyclohexane; "DE" refers to diatomaceous earth; "DHP" refers to 3,4-dihydropyran; "DIAD" refers to diisopropyl azodicarboxylate; "DDQ" refers to 2,3-dichloro-5,6-dicyano-1,4-benzoquinone; "DCE" refers to 1,2-dichloroethane; "DCM" refers to dichloromethane; "DEA" refers to diethylamine; "Et₂O" refers to diethyl ether; "DIPEA" refers to diisopropylethylamine; "DIEA" refers to diisopropylethylamine; "ex" refers to example; "DMA" refers to dimethylacetamide; "DME" refers to 1,2-dimethoxyethane; "HATU" refers to N-[(dimethylamino)-1H-1,2,3-triazolo-[4,5-b]pyridin-1-ylmethylene]-N-methylmethanaminium hexafluorophosphate N-oxide; "NDM" refers to 1-dodecanethiol; "DMEA" refers to dimethylethyl amine: "NMO" refers to N-methylmorpholine N-oxide; "Et₂O" refers to diethyl ether; "DMF" refers to N,N-dimethylformamide; "DMSO" refers to dimethyl sulfoxide; "dppf" refers to 1'-bis(diphenylphosphino)ferrocene; "DPPA" refers to diphenylphosphoryl azide; "EtOH" refers to ethanol; "EA" refers to ethyl acetate; "EtMgBr" refers to ethylmagnesium bromide; "NFSI" refers to N -fluorobenzene sulfonimide; "FA" refers to formic acid; "h" refers to hour(s); "hal" refers to halogen; "NIS" refers to N-iodo succinimide; "IPA" refers to isopropyl alcohol; "IPAm" refers to isopropylamine; "i-PrMgCl" refers to isopropyl magnesium chloride; "L" refers to liter(s); "LDA" refers to lithium diisopropylamide; "MsCl" refers to methanesulfonyl chloride; "MeMgBr" refers to methylmagnesium bromide; "MTBE" refers to methyl tert-butyl ether; "MeTHF" refers to 2-methyltetrahydrofuran; "ml" refers to milliliter; "min" refers to minute(s); 'M' refers to molar; "PdCl₂(DtBPF)" refers to [1,1'-bis(di-tert-butyl phosphino)ferrocene] dichloropalladium(II); "KHMDS" refers to potassium bis(trimethylsilyl)amide; "Pd(PPh₃)₄" refers tetetrakis(triphenylphosphine) palladium (0); "Pd(dppf)Cl2" refers to (1,1'-bis (diphenylphosphino)ferrocene) palladium(II) dichloride; "Pd₂(dba)₃" refers to tris (dibenzylidene acetone)dipalladium (0): "PE" refers to petroleum ether; "POCl₃" refers to phosphorus oxychloride; "KOAc" refers to potassium acetate; "t-BuOK" refers to potassium t-butoxide; "RT" refers to room temperature; "t_{R}" refers to retention time; "sat" refers to saturated; "NaOMe" refers to sodium methoxide; "Na(OAc)₃BH" sodium triacetoxyborohydride; "sat." refers to saturated; "soln" refers to solution; "SFC" refers to supercritical fluid chromatography; "THF" refers to tetrahydrofuran; "SOCl₂" refers to thionyl chloride; "TsOH" refers to p-toluenesulfonic acid; "NEt₃" and "Et₃N" refers to triethylamine; "Et₃Si" refers to triethylsilane; "TFA" refers to trifluoroacetic acid; "SEM-Cl" refers to 2-(trimethylsilyl)ethoxymethyl chloride; "PPh₃" refers to triphenylphosphine; "Dess-Martin" refers to 1,1,1-Tris(acetyloxy)-1,1-dihydro-1,2-benziodoxol-3-(1H)-one; "Xphos Palladacycle Gen 4" refers to chloro(2-dicyclohexyl phosphino-2',4',6'-triisopropyl-1,1'-biphenyl)[2-(2-aminoethyl)phenyl)] palladium(II); "XPhos Pd G4" refers to dicyclohexyl-[2-[2,4,6-tri(propan-2-yl)phenyl]phenyl] phosphanium; methanesulfonic acid;N-methyl-2-phenylaniline; palladium; "ACN" refers to acetonitrile; "CAN" refers to acetonitrile; "NSC" refers to to N-chloro succinimide.

**Table A: Analytical chiral chromatography methods.**

| Analytical Method | Column | Dimensions | Elution Conditions |
|---|---|---|---|
| A | Chiralpak AD | 3 x 100 mm, 3µm | 40% IPA (0.2% IPAm) in CO₂ |
| B | Chiralpak IA | 3 x 100 mm, 3µm | 25% to 50% EtOH (0.2% IPAm) in CO₂ for 2.5 min then 50% EtOH (0.2% IPAm) in CO₂ |
| C | Chiralpak AD | 3 x 100 mm, 3µm | 10% to 50% EtOH (0.2% IPAm) in CO₂ for 0.40 min then 50% EtOH (0.2% IPAm) in CO₂ |
| D | Chiralcel OJ | 3 x 100 mm, 3µm | 10% to 50% MeOH (0.2% IPAm) in CO₂ for 2.50 min then 50% EtOH (0.2% IPAm) in CO₂ |
| E | Chiralcel OJ | 4.6 x 150 mm, 5µm | MeOH (0.2% DMEA) |
| F | Chiralcel OJ | 3 x 100 mm, 3µm | 10% to 50% MeOH (0.2% IPAm) in CO₂ for 0.40 min then 50% MeOH (0.2% IPAm) in CO₂ |
| G | Chiralpak IA | 3 x 100 mm, 3µm | 40% IPA (0.2% IPAm) in CO₂ |
| H | Chiralpak AD | 3 x 100 mm, 3µm | 10% to 50% IPA (0.2% IPAm) in CO₂ for 0.40 min then 50% IPA (0.2% IPAm) in CO₂ |
| I | Chiralpak AD | 3 x 100 mm, 3µm | 45% EtOH (0.2% IPAm) in CO₂ |
| J | Chiralpak AD | 3 x 100 mm, 3µm | 10% to 50% EtOH (0.2% IPAm) in CO₂ for 2.50 min then 50% EtOH (0.2% IPAm) in CO₂ |
| K | Chiralpak IH | 3 x 100 mm, 3µm | 10% to 50% EtOH (0.2% IPAm) in CO₂ for 2.50 min then 50% EtOH (0.2% IPAm) in CO₂ |
| L | Chiralpak AD | 3 x 100 mm, 3µm | 45% IPA (0.2% IPAm) in CO₂ |
| M | Chiralcel OD | 3 x 100 mm, 3µm | 10% to 50% IPA (0.2% IPAm) in CO₂ for 2.5min then 50% IPA (0.2% IPAm) in CO₂ |
| N | Chiralpak IH | 3 x 100 mm, 3µm | 10% to 50% MeOH (0.2% IPAm) in CO₂ for 2.5min then 50% MeOH (0.2% IPAm) in CO₂ |
| P | Chiralpak IA | 3 x 100 mm, 3µm | 25% to 50% IPA (0.2% IPAm) in CO₂ for 2.5min then 50% IPA (0.2% IPAm) in CO₂ |
| Q | Chiralpak AD | 3 x 100 mm, 3µm | 40% MeOH (0.2% IPAm) in CO₂ |
| R | Chiralcel OD | 3 x 100 mm, 3µm | 45% MeOH (0.2% IPAm) in CO₂ |
| S | Chiralpak AD | 3 x 100 mm, 3µm | 10% to 50% IPA (0.2% IPAm) in CO₂ for 2.5min then 50% IPA (0.2% IPAm) in CO₂ |

### Preparation 1

### tert-Butyl 6-hydroxy-2-azaspiro [3.3]heptane-2-carboxylate

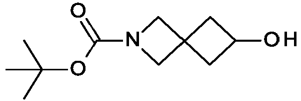

A MeOH soln (50 ml) of tert-butyl 6-oxo-2-azaspiro[3.3]heptane-2-carboxylate (3.6 g, 17 mmol) was cooled to 0°C and treated in portions with NaBH₄ (1.0 g, 26 mmol). Allowed the reaction to stir overnight slowly warming to RT. The reaction was diluted with EA (100 ml), washed with saturated aq NaHCO₃ (2 x 100 ml), and brine (100 ml). The organic layer was collected, dried over MgSO4, filtered, and concentrated to obtain the title compound (3.1 g, 85%) as a white solid. MS ES+ *m*/*z* 158 [MH-^{t}Bu]⁺.

The following compounds were prepared in a manner essentially analogous to the method of Preparation 1 using the appropriate reagents, adjusting reaction time to determine completion of the reaction, and adjusting the purification system as appropriate.

**Table 1**

| Prep # | Chemical Name | Structure | ¹H NMR (400 MHz, CDCl₃) δ |
|---|---|---|---|
| 2 | Tert-Butyl 4-hydroxyazepane-1-carboxylate | 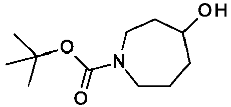 | 3.90 (brs, 1H), 3.56 - 3.17 (m, 4H), 2.06 - 1.81 (m, 3H), 1.80 - 1.61 (m, 3H), 1.48 (s, 9H), 1.40 - 1.35 (m, 1H). |
| 3 | tert-Butyl (1R,5S)-3-hydroxy-8-azabicyclo[3.2.1]octane-8-carboxylate | 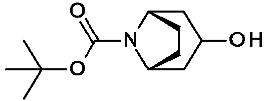 | 4.36 - 4.01 (m, 3H), 2.25 - 1.85 (m, 5H), 1.72 (d, J = 14.7 Hz, 1H), 1.67 - 1.58 (m, 2H), 1.48 (d, J = 5.2 Hz, 9H), 1.46 - 1.36 (m, 1H) |

### Preparation 4

### tert-Butyl 6-methylsulfonyloxy-2-azaspiro[3.3]heptane-2-carboxylate

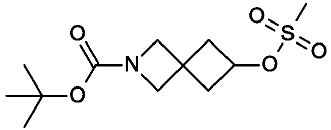

A soln of tert-butyl 6-hydroxy-2-azaspiro[3.3]heptane-2-carboxylate (3.1 g, 15 mmol) in DCM (35 ml) cooled to 0°C was treated with Et₃N (3.6 ml, 26 mmol) followed by the dropwise addition of methanesulfonyl chloride (1.5 ml, 19 mmol). The reaction was allowed to stir at 0°C and slowly warm to RT. After stirring for 1 h, the reaction was diluted with EA (75 ml), washed with 50% brine (100 mL), collected, dried over MgSO₄, filtered, and concentrated to obtain the title compound (4.3 g, 87%) as a white solid which was used without purification. MS ES+ *m*/*z* 236 [MH-^{t}Bu]⁺.

The following compounds were prepared in a manner essentially analogous to the method of Preparation 4 using the appropriate reagents, adjusting reaction time to determine completion of the reaction, and adjusting the purification system as appropriate.

**Table 2**

| Prep # | Chemical Name | Structure | MS ES+ m/z |
|---|---|---|---|
| 5 | tert-Butyl 2-methylsulfonyloxy-7-azaspiro[3.5]nonane-7-carboxylate | 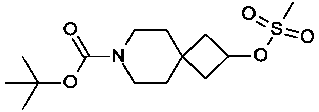 | |
| 6 | tert-Butyl 4-methylsulfonyloxyazepane-1-carboxylate | 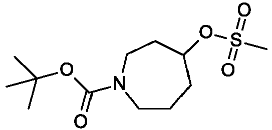 | 238 [M+H-tBu]⁺ |
| 7 | [(1S)-1-[5-(Trifluoromethyl)-3-pyridyl]ethyl] methanesulfonate | 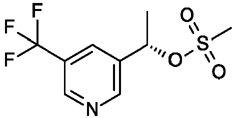 | 270 [M+H]⁺ |

### Preparation 8

### Benzyloxycyclobutanol

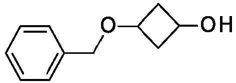

A mixture of 3-(benzyloxy)cyclobutan-1-one (20 g, 113.5 mmol) and NaBH₄ (4.29 g, 113.5 mmol) in MeOH (50 ml) was stirred for 2 h at RT under N₂. The reaction was quenched with H₂O at 0 °C, extracted with EA (3 x 100 ml), washed with brine (2 x 100 ml), dried over Na₂SO₄, and filtered. The filtrate was concentrated under reduced pressure to afford the title compound (20 g, 99%) as a light-yellow oil. ¹H NMR (400 MHz, DMSO-d₆) δ 7.42-7.21 (m, 5H), 4.45(s, 2H), 3.93-3.85 (m, 1H), 3.71-3.62 (m, 1H), 2.82-2.63 (m, 2H), 1.97-1.92 (m, 2H).

### Preparation 9

### tert-Butyl (1R,5S)-3-azido-8-azabicyclo[3.2.1]octane-8-carboxylate

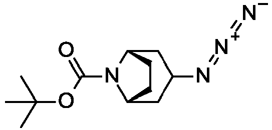

A mixture of tert-butyl (1R,5S)-3-(p-tolylsulfonyloxy)-8-azabicyclo[3.2.1]octane-8-carboxylate (4.5 g, 12 mmol) in DMSO (79 ml) was sonicated to help solubilize the solid and then treated with NaN₃ (4 M in H₂O, 4.1 ml, 17 mmol). 80 mL of this soln was then passed through a Uniqsis Flowsyn system which consisted of a 14 ml Teflon reactor (1/16") with a residence time of 40 min, a temperature of 135 °C, and a BPR of 10 bar. The reaction was diluted with H₂O (200 mL) and extracted with EA (2 x 100 ml). The combined organics were dried over MgSO₄, filtered, and concentrated to obtain the title compound as a soln in DMSO which was used in the next synthetic step without purification and assuming 100% conversion.

### Preparation 10

### tert-Butyl (4R)-4-azido-3,3-difluoro-piperidine-1-carboxylate

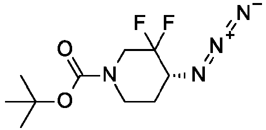

A solution of tert-butyl (4R)-4-amino-3,3-difluoro-piperidine-1-carboxylate (2 g, 8.47 mmol) and K₂CO₃ (1.99 g, 14.39 mmol) in MeOH (20 ml) was treated with CuSO4·5H₂O (0.21 g, 0.85 mmol) and 1H-imidazole-1-sulfonyl azide hydrochloride (2.13 g, 10.16 mmol) at RT under N₂ and stirred overnight. The reaction was diluted with H₂O (50 ml) and extracted with EA (2 x 100 ml). The combined organic layers were washed with brine (2 x 50 ml), dried over Na₂SO₄, filtered, and concentrated to afford the title compound as a yellow oil (3.4 g, crude). ¹H NMR (300 MHz, CDCl₃) δ (ppm): 4.25 - 4.14 (m, 1H), 4.08 - 3.90 (m, 1H), 3.76 - 3.71 (m, 1H), 3.59 - 3.37 (m, 1H), 3.20 (d, 1H), 2.04 - 1.91 (m, 1H), 1.69 -1.54 (m, 1H), 1.41 (s, 9H).

The following compounds were prepared in a manner essentially analogous to the method of Preparation 10 using the appropriate reagents, adjusting reaction time to determine completion of the reaction, and adjusting the purification system as appropriate.

**Table 3**

| Prep # | Chemical Name | Structure | ¹H NMR (300 MHz, CDCl₃), δ |
|---|---|---|---|
| 11 | tert-Butyl 4-azido-4-methylpiperidine-1-carboxylate | 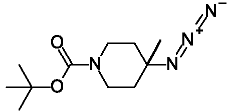 | 3.89 - 3.77 (m, 2H), 3.18 - 3.02 (m, 2H), 1.72 -1.61 (m, 2H), 1.59 - 1.50 (m, 2H), 1.47 (s, 9H), 1.37 (s, 3H). |

### Preparation 12

### N-Diazo-1,1,1-trifluoro-methanesulfonamide

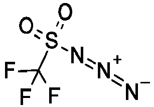

To a solution of NaN₃ (9.22 g, 142 mmol) and hydrogen tetra(but-1-yl)ammonium sulfate (0.48 g, 1.42 mmol) in distilled H₂O (30 mL) cooled to 0 °C was added slowly a solution of (CF₃SO₂)₂O (8.00 g, 28.4 mmol) in heptane (25 mL). The reaction was stirred 1-2 hr at 0 °C. Heptane (25 ml) was added to the reaction and the layers were separated. The aqueous layer was extracted with heptane (3 x 10 ml). The combined organic layers were dried over NaOH pellets. The organic layer was decanted, and the solution was used immediately in the subsequent reaction.

### Preparation 13

### (1r,3r)-3-Azidocyclobutanol

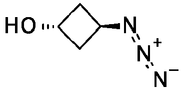

A solution of (1r,3r)-3-aminocyclobutan-1-ol (1.23 g, 14.2 mmol), NaHCO₃ (4.05 g, 48.2 mmol), and CuSO₄·5H₂O (1.77 g, 7.08 mmol) in MeOH (15 mL) and H₂O (15 mL) (v/v) was treated with a freshly prepared stock solution of N-diazo-1,1,1-trifluoro-methanesulfonamide in heptane (4.96 g, 28.3 mmol). Additional MeOH was added to the reaction in 5 mL increments until a homogeneous mixture resulted (20 mL total added). The reaction was stirred overnight at RT. EA was added and the layers were separated. The aqueous layer was extracted with EA (3x). The combined organic layers were concentrated in vacuo to afford a dark green solution (1.60 g, 100%, assumed quantitative yield). ¹H NMR(400 MHz, DMSO-d₆) δ 2.05 - 2.31 (m, 4 H) 4.07 - 4.18 (m, 1 H) 4.29 (br s, 1 H) 5.14 - 5.32 (m, 1 H).

### Preparation 14

### tert-Butyl (3R,4R)-4-azido-3-fluoro-piperidine-1-carboxylate

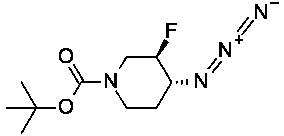

To tert-butyl (3R,4S)-3-fluoro-4-hydroxypiperidine-1-carboxylate (4 g, 18.24 mmol) and PPh₃ (6.22 g, 23.72 mmol) in THF (40 ml) was added DIAD (5.53 g, 27.37 mmol) dropwise at 0 °C under N₂ followed by the dropwise addition of DPPA (5.52 g, 20.07 mmol). The reaction was stirred for 3 h at RT then concentrated in vacuo. The residue was purified by silica gel column chromatography eluting with PE / EA (10: 1) to afford the title compound (1.7 g, 38%) as a light-yellow solid. ¹H NMR (300 MHz, DMSO-d₆) δ 4.63 - 4.24 (m, 1H), 4.09 - 3.83 (m, 2H), 3.80 - 3.55 (m, 1H), 3.20 - 2.84 (m, 2H), 2. 06 - 1.78 (m, 1H), 1.42 - 1.36 (m, 10H).

The following compounds were prepared in a manner essentially analogous to the method of Preparation 14 using the appropriate reagents, adjusting reaction time to determine completion of the reaction, and adjusting the purification system as appropriate.

**Table 4**

| Prep No. | Chemical Name | Structure | ¹H NMR (400 MHz, CDCl₃), δ |
|---|---|---|---|
| 15^{1, 2} | tert-Butyl (3R,4S)-4-azido-3-fluoro-piperidine-1-carboxylate | 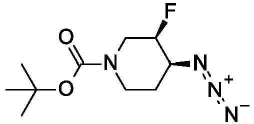 | 4.89-4.82(m, 1H), 4.09-4.01(m, 1H), 3.95 - 3.60 (m, 2H), 3.15 (dd, 2H), 1.90 - 1.64 (m,2H), 1.40 (s, 9H) |
| 16^{2, 3} | tert-Butyl (3S,4S)-4-azido-3-fluoro-piperidine-1-carboxylate | 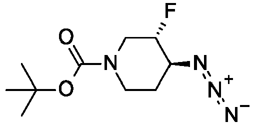 | 4.41 - 4.36 (m, 1H), 4.06 - 3.85 (m, 2H), 3.68 - 3.57 (m, 1H), 3.06 - 2.97 (m, 2H), 1.93 - 1.83 (m, 1H), 1.40 (s, 10H) |
| 17^{4, 5} | tert-Butyl (3S,4R)-4-azido-3-fluoro-piperidine-1-carboxylate | 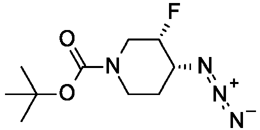 | 4.98 - 4.76 (m, 1H), 4.12 - 3.96 (m, 1H), 3.97 - 3.64 (m, 2H), 3.26 - 2.82 (m, 2H), 1.85 - 1.63 (m, 2H), 1.39 (s, 9 |
| 18 | 3-(Azidocyclobutoxy)methyl benzene | 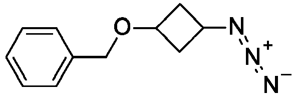 | 2.44 - 2.27 (m, 4H), 4.16 - 4.08(m, 1H), 4.28 - 4.21(m, 1H), 4.41 (s, 2H), 7.39 - 7.28 (m, 5H) |

| | | | |
|---|---|---|---|
| Purified by reverse phase chromatography; C18 column; eluting with 40% to 50% ACN in H₂O. ^{2 1}H NMR (300 MHz, DMSOd₆). ³ Purified by silica gel chromatography, eluting with PE / EA (20:1). ⁴ Purified by silica gel chromatography, eluting with PE / EA (15:1). ^{5 1}H NMR (400 MHz, DMSOd₆). | | | |

### Preparation 19

### tert-Butyl 4-methyl-4-(5-methyl-4-trimethylsilyl-triazol-1-yl)piperidine-1-carboxylate

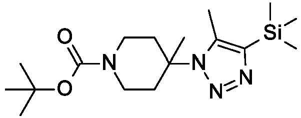

A mixture of tert-butyl 4-azido-4-methyl-piperidine-1-carboxylate (4 g, crude) and trimethyl(prop-1-yn-1-yl)silane (5.61 g, 49.94 mmol) was irradiated with microwave radiation for 1 h at 150 °C. Once cooled to RT, the resulting mixture was concentrated in vacuo to afford the title compound as a yellow solid (4.1 g, crude). MS ES+ *m*/*z* 353 [M+H]⁺.

The following compounds were prepared in a manner essentially analogous to the method of Preparation 19 using the appropriate reagents, adjusting reaction time to determine completion of the reaction, and adjusting the purification system as appropriate.

**Table 5**

| Prep # | Chemical Name | Structure | MS ES+ m/z |
|---|---|---|---|
| 20^{1,2} | tert-Butyl (4R)-3,3-difluoro-4-(5-methyl-4-trimethylsilyl-triazol-1-yl)piperidine-1-carboxylate | 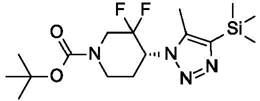 | 375 [M+H]⁺ |
| 21 | tert-Butyl (3R,4R)-3-fluoro-4-(5-methyl-4-trimethylsilyl-triazol-1-yl)piperidine-1-carboxylate | 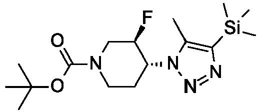 | 357 [M+H]⁺ |
| 22 | tert-Butyl (3R,4S)-3-fluoro-4-(5-methyl-4-trimethylsilyl-triazol-1-yl)piperidine-1-carboxylate | 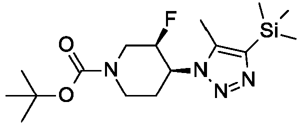 | 357 [M+H]⁺ |
| 23 | tert-Butyl (3S,4S)-3-fluoro-4-(5-methyl-4-trimethylsilyl-triazol-1-yl)piperidine-1-carboxylate | 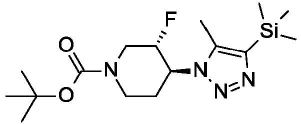 | 357 [M+H]⁺ |
| 24 | tert-Butyl (3S,4R)-3-fluoro-4-(5-methyl-4-trimethylsilyl-triazol-1-yl)piperidine-1-carboxylate | 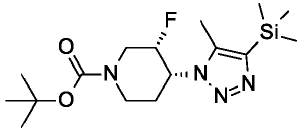 | 357 [M+H]+ |
| 25^{3,4} | (1r,3r)-3-(5-Methyl-4-(trimethylsilyl)-1H-1,2,3-triazol-1-yl)cyclobutan-1-ol | 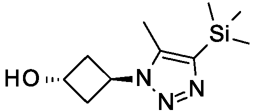 | a |

| | | | |
|---|---|---|---|
| ¹ Toluene used as solvent for this transformation. ² Purified by silica gel chromatography eluting with PE / EA (5:1 - 3:1). ³ Purified by silica gel chromatography eluting with 0% to 100% EA in heptane. ⁴ Reaction was run in toluene. ^{a 1}H NMR (400 MHz, DMSO-d₆) δ 0.26 (s, 9 H) 2.24 (s, 3 H) 2.36 - 2.47 (m, 2 H) 2.65 - 2.75 (m, 2 H) 4.42 - 4.59 (m, 1 H) 4.97 (ttd, J=8.38, 8.38, 5.14, 5.14, 0.73 Hz, 1 H) 5.31 (d, J=4.89 Hz, 1 H). | | | |

### Preparation 26

### tert-Butyl 4-(4-bromo-5-methyl-triazol-1-yl)-4-methyl-piperidine-1-carboxylate

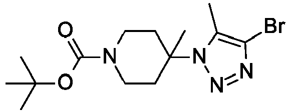

A mixture of tert-butyl 4-methyl-4-(5-methyl-4-trimethylsilyl-triazol-1-yl)piperidine-1-carboxylate (2.3 g, crude) and SiO₂ (0.78 g, 13.05 mmol) in ACN (15 ml) was treated with NBS (1.74 g, 9.79 mmol) at RT under N₂. The resulting mixture was stirred for 2 h at 80 °C under N₂. Once cooled to RT, the reaction was quenched with H₂O and extracted with EA (2 x 200 ml). The combined organic layers were washed with brine (2 x 100 ml), dried over Na₂SO₄, filtered, and concentrated in vacuo. The residue was purified by silica gel column chromatography eluting with PE / EA (4:1) to afford title compound as a yellow solid (2 g, 85%). MS ES+ m/z (⁷⁹Br/⁸¹Br) 359/361 [M+H]⁺.

The following compounds were prepared in a manner essentially analogous to the method of Preparation 26 using the appropriate reagents, adjusting reaction time to determine completion of the reaction, and adjusting the purification system as appropriate.

**Table 6**

| Prep # | Chemical Name | Structure | MS ES+ m/z (⁷⁹Br/⁸¹Br) |
|---|---|---|---|
| 27¹ | tert-Butyl (4R)-4-(4-bromo-5-methyl-triazol-1-yl)-3,3-difluoro-piperidine-1-carboxylate | 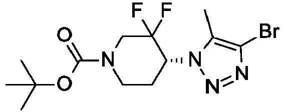 | 381/383 [M+H]⁺ |
| 28² | tert-Butyl (3R,4R)-4-(4-bromo-5-methyl-triazol-1-yl)-3-fluoro-piperidine-1-carboxylate | 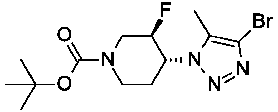 | 363/365 [M+H]⁺ |
| 29¹ | tert-Butyl (3R,4S)-4-(4-bromo-5-methyl-triazol-1-yl)-3-fluoro-piperidine-1-carboxylate | 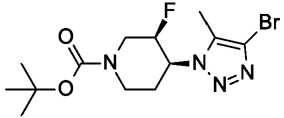 | 363/365 [M+H]⁺ |
| 30² | tert-Butyl (3S,4S)-4-(4-bromo-5-methyl-triazol-1-yl)-3-fluoro-piperidine-1-carboxylate | 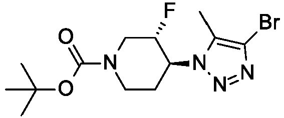 | 363/365 [M+H]⁺ |
| 31¹ | tert-Butyl (3S,4R)-4-(4-bromo-5-methyl-triazol-1-yl)-3-fluoro-piperidine-1-carboxylate | 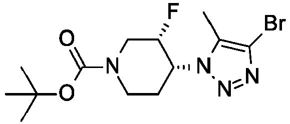 | 404.0/406.0 [M+H+ACN]⁺ |
| 32³ | (1r,3r)-3-(4,5-Dimethyl-1H-1,2,3-triazol-1-yl)cyclobutan-1-ol | 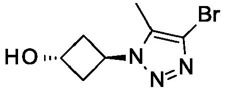 | a |

| | | | |
|---|---|---|---|
| ¹ Purified by silica gel chromatography eluting with PE / EA (3:1). ² Purified by silica gel chromatography, eluting with PE / EA (4:1). ³ Purified by silica gel chromatography eluting with 0% to 100% EA in heptane. ^{a 1}H NMR (400 MHz, DMSO-d₆) δ 2.20 (s, 3 H) 2.37 - 2.47 (m, 2 H) 2.69 - 2.77 (m, 2 H) 4.41 - 4.49 (m, 1 H) 4.99 - 5.09 (m, 1 H) 5.09 - 5.67 (m, 1 H). | | | |

### Preparation 33

### tert-Butyl 4-(4-ethoxycarbonyl-5-methyl-triazol-1-yl)azepane-1-carboxylate

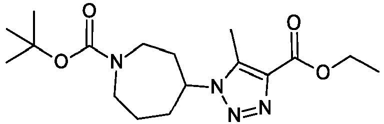

A solution of tert-butyl 4-(p-tolylsulfonyloxy)azepane-1-carboxylate (15 g, 38.57 mmol) in DMSO (120 ml) was stirred under N₂. A solution of NaN₃ (2.8 g, 42 mmol) in H₂O (16.8 ml) was added. After stirring 30 min at RT a solution had resulted. The solution was subjected to the following flow chemistry conditions: Reactor size 20 ml (Teflon type mas T: 150°C); flow rate: 0.666 ml/mi; BPR 1.2 bar; temperature: 100 °C; residence time: 30 min. The intermediate azide was isolated as a solution in DMSO/H₂O (8:1)(0.28 M, 130 ml total). The soln of the azide intermediate was used in the next step.

To the above soln of the azide intermediate was added ethyl acetoacetate (5 ml, 39.30 mmol) and K₂CO₃ (12 g, 86.83 mmol). The reaction was stirred 15 min at RT then heated at 80°C overnight. The reaction was cooled to RT then diluted with H₂O (100 ml). After 15 min of stirring all the solids had dissolved. MTBE was added (100 ml) and mixture was stirred 15 min at RT. The organic layer was separated and the aq layer was extracted twice with MTBE (2 x 50 ml). The organic layers were combined, dried over Na₂SO₄, filtered and the filtrate was concentrated to afford the title compound (3.9 g, 25%, 75% purity). MS ES+ m/z 353 [M+H]⁺.

### Preparation 34

### tert-Butyl (1R,5S)-3-(4-ethoxycarbonyl-5-methyl-triazol-1-yl)-8-azabicyclo[3.2.1]octane-8-carboxylate

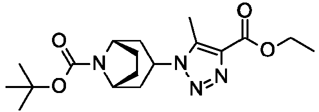

To a stirred solution of tert-butyl(3-endo)-3-azido-8-azabicyclo[3.2.1]octane-8-carboxylate (3.63 g, 14.4 mmol) and ethyl 3-oxobutanoate (2.25 g, 17.3 mmol) in DMSO (29 ml) was added K₂CO₃ (5.97 g, 43.2 mmol) at RT under N₂. The reaction was stirred at 85 °C for 6 h. Upon cooling to RT the reaction was poured into ice / H₂O (200 ml) with stirring. The resultant cream colored precipatate was collected by filtration, washed with H₂O (100 ml) and dried at 40 °C overnight to afford the title compound (3.10g, 59%). MS ES+ m/z 365 [MH-^{t}Bu]⁺.

### Preparation 35

### Ethyl 1-(3-benzyloxycyclobutyl)-5-methyl-triazole-4-carboxylate

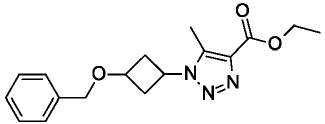

To a stirred mixture of 3-(azidocyclobutoxy)methylbenzene (21 g, 103.32 mmol) and ethyl acetoacetate (14.79 g, 113.66 mmol) in DMF (100 ml) was added K₂CO₃ (42.84 g, 309.97 mmol) at RT under N₂. The resulting mixture was stirred for 2 h at 80 °C under N₂. Upon cooling to RT the reaction was concentrated in vacuo. The mixture was diluted with H₂O (300 ml) and extracted with EA (3 x 200ml). The combined organic layers were washed with brine (3 x 200 ml), dried over Na₂SO₄, filtered and concetrated in vacuo. The residue was purified by silica gel column chromatography and eluted with 50% EA in PE, to afford the title compound (29 g, 93%) as a brownish yellow oil. ES+ m/z 316 [M+H]⁺.

### Preparation 36

### 1-[(1R,5S)-8-tert-Butoxycarbonyl-8-azabicyclo[3.2.1]octan-3-yl]-5-methyl-triazole-4-carboxylic acid

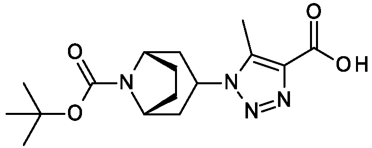

A soln of tert-butyl (1R,5S)-3-(4-ethoxycarbonyl-5-methyl-triazol-1-yl)-8-azabicyclo[3.2.1]octane-8-carboxylate (1.5 g, 4.1 mmol) in H₂O (8 ml) was treated with KOH (0.28 g, 5 mmol) and stirred at RT for 1 h, then stirred at 55 °C for 2 h, and then at 40 °C overnight. The reaction was allowed to cool to RT, quenched with 2 M aq HCl until the pH was ~ 2, and extracted with EA (2 x 30 ml). The combined organic layers were dried over Na₂SO₄, filtered, and concentrated to obtain the title compound (1.16 g, 84%) which was used in the next synthetic step without purification or analysis.

The following compounds were prepared in a manner essentially analogous to the method of Preparation 36 using the appropriate reagents and adjusting the reaction times to determine completion of the reactions. MeOH can be used as a cosolvent.

**Table 7**

| Prep # | Chemical Name | Structure | ES/MS *m*/*z* [M+H]⁺ |
|---|---|---|---|
| 37 | 1-(3-Benzyloxycyclobutyl)-5-methyl-triazole-4-carboxylic acid | 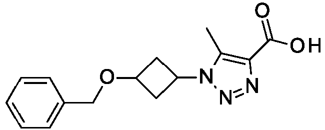 | 288 |

### Preparation 38a

### tert-Butyl 6-(4-bromo-5-methyl-pyrazol-1-yl)-2-azaspiro[3.3]heptane-2-carboxylate

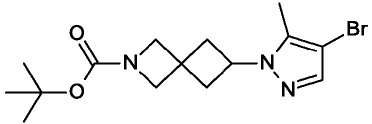

and

### Preparation 38b

### tert-Butyl 6-(4-bromo-3-methyl-pyrazol-1-yl)-2-azaspiro[3.3]heptane-2-carboxylate

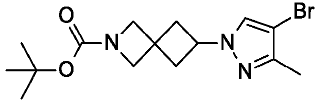

A DMSO suspension (10 ml) of tert-butyl 6-methylsulfonyloxy-2-azaspiro[3.3]heptane-2-carboxylate (2.5 g, 8.6 mmol) and 4-bromo-5-methyl-1H-pyrazole (1.2 g, 7.5 mmol) was treated with Cs₂CO₃ (5.7 g, 17 mmol) and the reaction stirred at 50 °C for 12 h and then placed into a refrigerator. After 4 days, the reaction was poured into ice and H₂O which resulted in the formation of a precipitate. The insoluble material was removed by filtration and washed with H₂O to obtain the title compounds (2.7 g, 44%) as a mixture. MS ES+ m/z 300/302 [M+H-tBu]⁺.

### Preparation 39a

### tert-Butyl 2-(4-bromo-5-methyl-pyrazol-1-yl)-7-azaspiro[3.5]nonane-7-carboxylate

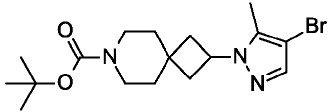

and

### Preparation 39b

### tert-Butyl 2-(4-bromo-3-methyl-pyrazol-1-yl)-7-azaspiro[3.5]nonane-7-carboxylate

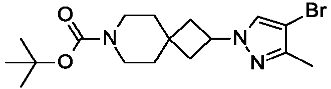

tert-Butyl 2-methylsulfonyloxy-7-azaspiro[3.5]nonane-7-carboxylate (1.41 g, 4.41 mmol) and 4-bromo-5-methyl-1H-pyrazole (0.67 g, 4.14 mmol) in DMF (10 ml) was treated with Cs₂CO₃ (2.02 g, 6.2 mmol) at RT. After stirring at 90 °C for 3.5 h, the reaction was allowed to stir over the weekend at RT. Another portion of 4-bromo-5-methyl-1H-pyrazole (0.67 g, 4.14 mmol) and Cs₂CO₃ (2.02 g, 6.2 mmol) was added, and the reaction stirred at 90 °C for 2 h. Another portion of 4-bromo-5-methyl-1H-pyrazole (0.67 g, 4.14 mmol) was added and stirred at 90 °C for 1 h. After cooling to RT, the reaction was diluted with H₂O (50 ml) and extracted with EA (2x). The organic layers were combined, dried over MgSO₄, filtered, and concentrated. The resulting oil was purified by silica gel chromatography eluting with 33% EA in hexanes to obtain the title compound (1.68 g) as a mixture. MS ES+ m/z 328/330 [M+H-tBu]⁺.

The following compounds were prepared in a manner essentially analogous to the method of Preparation 39a and 39b using the appropriate reagents, adjusting reaction time to determine completion of the reaction, and adjusting the purification system as appropriate.

**Table 8**

| Prep # | Chemical Name | Structure | MS ES+ m/z |
|---|---|---|---|
| 40a | tert-Butyl 4-(4-bromo-5-methyl-pyrazol-1-yl)azepane-1-carboxylate | 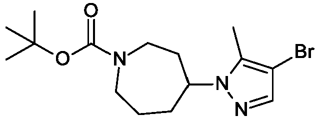 | (⁷⁹Br/⁸¹Br) 302/304 [M+H-tBu]⁺ |
| 40b | tert-Butyl 4-(4-bromo-3-methyl-pyrazol-1-yl)azepane-1-carboxylate | 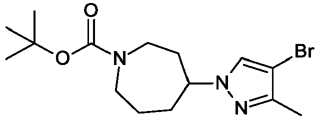 | (⁷⁹Br/⁸¹Br) 302/304 [M+H-^{t}Bu]⁺ |
| 41 | tert-Butyl 4-(4-bromo-3,5-dimethyl-pyrazol-1-yl)piperidine-1-carboxylate | 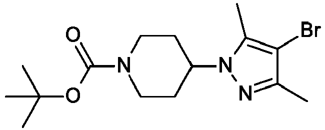 | (⁷⁹Br/⁸¹Br) 358/360 [M+H]⁺ |

### Preparation 42

### 1-(1-tert-Butoxycarbonylazetidin-3-yl)-5-methyl-triazole-4-carboxylic acid

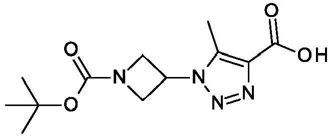

To a soln of ethyl 1-(1-tert-butoxycarbonylazetidin-3-yl)-5-methyl-triazole-4-carboxylate (12.15 g, 39.14 mmol) in THF (75 ml) was added aq LiOH (75 ml, 75 mmol, 1M) and the reaction was stirred overnight at RT. After stirring 18 h, aq HCl (1N) was added until pH was between 5 and 6. The mixture was extracted with EA and DCM / MeOH (9:1) (5x). The combined organic layers were dried on MgSO₄, filtered and the filtrate was concentrated to afford the title compound (10.3 g, 93%) of a pale brown solid. MS ES+ m/z 283 [M+H]⁺.

### Preparation 43

### 1-(1-tert-Butoxycarbonylazepan-4-yl)-5-methyl-triazole-4-carboxylic acid

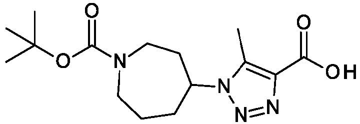

To a stirred solution of tert-butyl 4-(4-ethoxycarbonyl-5-methyl-triazol-1-yl)azepane-1-carboxylate (3.1 g, 8.8 mmol) in THF (15 ml) was added aq LiOH (15 ml, 15 mmol, 1M) and the reaction was stirred at RT for 210 minutes. Aq HCl (1N) was added until pH was approximately 5-6. The reaction was extracted twice with EA and twice with 10% MeOH in DCM. The organic layers were combined, dried on MgSO₄, filtered and the filtrate was concentrated in vacuo to afford the title compound (2.89 g, 96%, 95 mass %). MS ES+ m/z 296 [M+H]⁺.

### Preparation 44

### tert-Butyl (3S)-3-(4-bromo-5-methyl-pyrazol-1-yl)piperidine-1-carboxylate

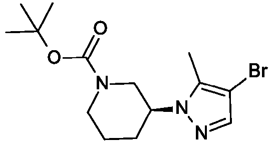

To tert-butyl (3S)-3-(4-bromopyrazol-1-yl)piperidine-1-carboxylate (500 mg, 1.51 mmol) in THF (5 ml) was added LDA in hexanes (2.3 ml, 4.6 mmol, 2 M) at -70°C and stirred for 0.5 h. Next, CH₃I (0.19 mL, 3.1 mmol) was added, and the stirring continued for 30 min. The reaction was quenched with sat. aq NH₄Cl (50 ml) and concentrated. The mixture was extracted with EA (3 x 20 ml). The combined organic layers were wash with brine (3 x 20 ml), dried over Na₂SO₄, filtered, and concentrated. The residue was purified by silica gel chromatography eluting with petroleum ether / EA (1:0 to 10:1) to afford the title compound (0.4 g, 70% Yield) as a yellow oil. MS ES+ m/z (⁷⁹Br/⁸¹Br) 344/346 [M+H]⁺.

The following compounds were prepared in a manner essentially analogous to the method of Preparation 44 using the appropriate reagents, adjusting reaction time to determine completion of the reaction, and adjusting the purification system as appropriate.

**Table 9**

| Prep # | Chemical Name | Structure | MS ES+ m/z |
|---|---|---|---|
| 45 | tert-Butyl (3R)-3-(4-bromo-5-methyl-pyrazol-1-yl)piperidine-1-carboxylate | 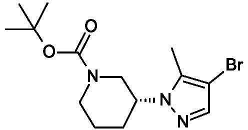 | (⁷⁹Br/⁸¹Br) 344/346 [M+H]⁺ |

### Preparation 46

### tert-Butyl 4-(4-bromo-5-methyl-triazol-1-yl)piperidine-1-carboxylate

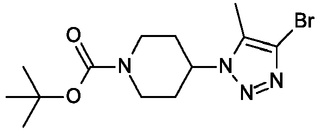

A soln of 1-(1-tert-butoxycarbonyl-4-piperidyl)-5-methyl-triazole-4-carboxylic acid (17 g, 55 mmol) and KOH (3.7 g, 66 mmol) in H₂O (60 ml) was treated in portions with Br₂ (10.4 g, 66 mmol) at RT. After stirring at RT for 3 h at RT, the reaction was extracted with EA (3 x 30 ml). The combined organic layers were washed with brine (2 x 150 mL), dried over anhydrous Na₂SO₄, filtered, and concentrated to obtain the title compound (13 g, 68%) as a yellow solid. MS ES+ m/z 345 [M+H]⁺.

The following compounds were prepared in a manner essentially analogous to the method of Preparation 46 using the appropriate reagents, adjusting reaction time to determine completion of the reaction, and adjusting the purification system as appropriate.

**Table 10**

| Prep # | Chemical Name | Structure | MS ES+ m/z |
|---|---|---|---|
| 47 | tert-Butyl 3-(4-bromo-5-methyl-triazol-1-yl)azetidine-1-carboxylate | 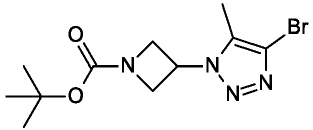 | (⁷⁹Br/⁸¹Br) 317/319 [M+H]⁺ |
| 48 | tert-Butyl (1R,5S)-3-(4-bromo-5-methyl-triazol-1-yl)-8-azabicyclo [3.2.1]octane-8-carboxylate | 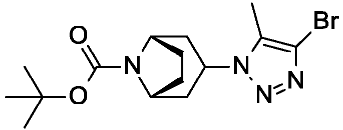 | (⁷⁹Br/⁸¹Br) 371/373 [M+H]⁺ |
| 49¹ | tert-Butyl 4-(4-bromo-5-methyl-triazol-1-yl)azepane-1-carboxylate | 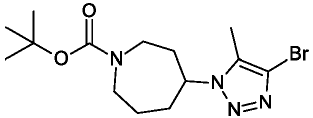 | (⁷⁹ Br/⁸¹Br) 359/361 [M+H]⁺ |
| 50 | 1-(3-Benzyloxycyclobutyl)-4-bromo-5-methyl-triazole | 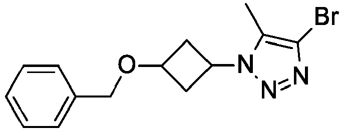 | a |

| | | | |
|---|---|---|---|
| ¹ Prior to workup reaction was diluted with aq NaOH (2N). ^{a 1}H NMR (400 MHz, DMSO-d₆) δ 7.40-7.34 (m, 4H), 7.33 - 7.27 (m, 1H), 5.14 - 5.06 (m, 1H), 4.45 (s, 2H), 4.42-4.34 (m, 1H), 2.81 - 2.72 (m, 2H), 2.66 - 2.56 (m, 2H), 2.22 (s, 3H). | | | |

### Preparation 51

### (1s, 3s)-[3-(4-Bromo-5-methyl-triazol-1-yl)cyclobutyl] 4-nitrobenzoate

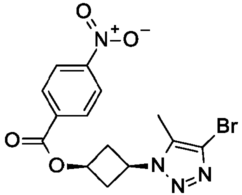

To a solution of DIAD (4.18 g, 20.68 mmol) in THF (40 ml) was added PPh₃ (5.88 g, 22.41 mmol) dropwise at 0 °C under N₂. The reaction was stirred for 30 min at 0 °C. Next, (1r,3r)-3-(4,5-dimethyl-1H-1,2,3-triazol-1-yl)cyclobutan-1-ol (4.0 g, 17.24 mmol) and p-nitrobenzoic acid (3.46 g, 20.68 mmol) were added in portions at RT under N₂. The mixture was stirred for 2 h at RT then concetrated in vacuo. The residue was purified by silica gel column chromatography, eluted with PE / EA (5: 1) to afford the title compound (8 g, crude) as a white solid. MS ES+ m/z (⁷⁹Br/⁸¹Br) 381/383 [M+H]⁺.

### Preparation 52

### (1s,3s)-3-(4-Bromo-5-methyl-1H-1,2,3-triazol-1-yl)cyclobutan-1-ol

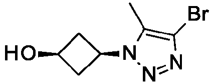

To a stirred solution of (1s, 3s)-[3-(4-bromo-5-methyl-triazol-1-yl)cyclobutyl] 4-nitrobenzoate (8.0 g, crude) in THF (50 ml) was added LiOH·H₂O (0.79 g, 18.89 mmol) in portions at RT under N₂. The reaction was stirred for 2 h at RT then concentrated in vacuo. The residue was purified by reversed flash chromatography with the following conditions: column, C18; mobile phase, 10% to 50% ACN in H₂O (0.1% FA), to afford the title compound (2.5 g, 51%) as a yellow oil. MS ES+ m/z (⁷⁹Br/⁸¹Br) 232/234 [M+H]⁺. ¹H NMR (300 MHz, DMSO-d₆) δ 5.46 - 5.28 (m, 1H), 4.55 - 4.40 (m, 1H), 4.14 - 3.90 (m, 1H), 2.87 - 2.76 (m, 2H), 2.49 - 2.41 (m, 2H), 2.25 - 2.19 (m, 3H).

### Preparation 53

### 3-(4-Bromo-5-methyl-triazol-1-yl)cyclobutanol

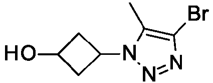

A mixture of 1-(3-benzyloxycyclobutyl)-4-bromo-5-methyl-triazole (8.5 g, 26.4 mmol) and FeCl₃ (8.56 g, 52.8 mmol) in DCM (100 ml) was stirred for 2 h at 50 °C under N₂. Upon cooling to RT the mixture was diluted with H₂O (50 ml) and extracted with EA (3 x 100 ml). The combined organic layers were washed with brine (2 x 100 ml), dried over Na₂SO₄, filtered and the filtrate was concentrated in vacuo to afford the title compound (8 g, crude) as a brown solid. ¹H NMR (400 MHz, DMSO-d₆) δ 5.09 - 4.99 (m, 1H), 4.55 - 4.42 (m, 1H), 2.80 - 2.71 (m, 2H), 2.48 - 2.37 (m, 2H), 2.21 (s, 3H).

### Preparation 54

### 4-(4-Bromo-5-methyl-triazol-1-yl)piperidine hydrochloride

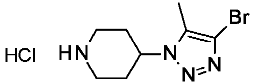

A soln of tert-butyl 4-(4-bromo-5-methyl-triazol-1-yl)piperidine-1-carboxylate (58 g, 159.6 mmol) in 2-propanol (60 ml) was treated with HCl in 2-propanol (250 ml; 1250 mmol; 4.99 M) and allowed to stir overnight. The reaction was diluted with MTBE (250 ml) and the resulting suspension was stirred at RT for 1 h. The suspension was filtered to afford the title compound (41.5 g, 91%) as a white solid. MS ES+ m/z (⁷⁹Br/⁸¹Br) 245/247 [M+H]⁺.

### Preparation 55

### 4-(4-Bromo-3,5-dimethyl-pyrazol-1-yl)piperidine

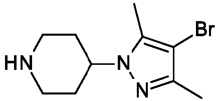

A DCM soln (15 ml) of tert-butyl 4-(4-bromo-3,5-dimethyl-pyrazol-1-yl)piperidine-1-carboxylate (0.97 g, 2.70 mmol) was treated with TFA (4 ml) and the reaction stirred at RT. After stirring for 45 min, the reaction was loaded onto an SCX column pretreated with MeOH. The column was washed with MeOH. The title compound was eluted with 2 M NH₃ in MeOH and concentrated to obtain the title compound (557 mg, 80%) as a colorless oil. MS ES+ *m*/*z* (⁷⁹Br/⁸¹Br) 258/260 [M+H]⁺.

The following compounds were prepared in a manner essentially analogous to the method of Preparation 55 using the appropriate reagents, adjusting reaction time to determine completion of the reaction, and adjusting the purification system as appropriate.

**Table 11**

| Prep # | Chemical Name | Structure | MS ES+ m/z |
|---|---|---|---|
| 56¹ | 4-(4-Bromo-5-methyl-triazol-1-yl)azepane | 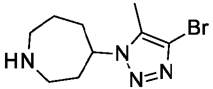 | (⁷⁹Br/⁸¹Br) 259/261 [M+H]⁺ |

| | | | |
|---|---|---|---|
| ¹ Crude material loaded onto an SCX cartridge. Non-basic impurities were washed off the cartridge with MeOH then the title compound was eluted with methanolic ammonia (2N). | | | |

### Preparation 57

### 3-(4-Bromo-5-methyl-triazol-1-yl)cyclobutanone

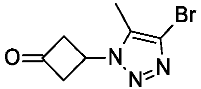

A mixture of 3-(4-bromo-5-methyl-triazol-1-yl)cyclobutanol (4.00 g, 17.23 mmol) and Dess-Martin (10.97 g, 25.85 mmol) in DCM (40 mL) was stirred for 2 h at RT under N₂. The mixture was diluted with H₂O (100 mL), extracted with EA (3 x 100 mL), washed with brine (2 x 100 mL), dried over Na₂SO₄, and filtered. The filtrate was concentrated in vacuo. The residue was purified by reversed phase chromatography with the following conditions: column, C18; mobile phase, 20% to 40% ACN in H₂O (0.1% FA) to afford the title compound (2.10 g, 52.96%) as a white solid. ES/MS m/z (⁷⁹Br/⁸¹Br) 230/232 [M+H]⁺.

### Preparation 58

### (1s, 3s)-[3-(4-Bromo-5-methyl-triazol-1-yl)cyclobutyl] trifluoromethanesulfonate

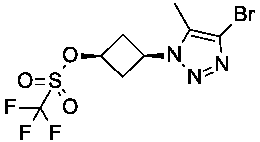

To (1s,3s)-3-(4-bromo-5-methyl-1H-1,2,3-triazol-1-yl)cyclobutan-1-ol (1.1 g, 4.74 mmol) and DIEA (3.06 g, 23.70 mmol) in DCM (10 ml) was added (trifluoromethane)sulfonyl trifluoromethanesulfonate (2.01 g, 7.11 mmol) dropwise at -70 °C under N₂. The reaction was stirred for 1 h at -70 °C. The mixture was used in the next step without further purification and assuming 100% conversion. ES/MS m/z (⁷⁹Br/⁸¹Br) 364/366 [M+H]⁺.

### Preparation 59

### tert-Butyl 4-((1r,3r)-3-(4-bromo-5-methyl-1H-1,2,3-triazol-1-yl)cyclobutyl)piperazine-1-carboxylate

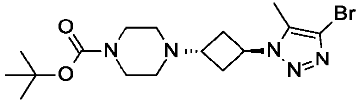

To (1s, 3s)-[3-(4-Bromo-5-methyl-triazol-1-yl)cyclobutyl] trifluoromethanesulfonate (1.7 g, 4.67 mmol) and DIEA (1.81 g, 14.01 mmol) in DCM (10 ml) was added tert-butyl piperazine-1-carboxylate (1.74 g, 9.34 mmol) in portions at - 70 °C under N₂. The reaction was stirred for overnight at RT then concentrated in vacuo. The residue was purified by silica gel column chromatography, eluted with PE / EA (10:1) to afford the title compound (1.1 g, 59%) as a white solid. ES/MS *m*/*z* (⁷⁹Br/⁸¹Br) 400/402 [M+H]⁺. ¹H NMR (300 MHz, DMSO-d₆) δ 5.02 - 4.90 (m, 1H), 3.38 - 3.35 (m, 4H), 3.08 - 2.96 (m, 1H), 2.65 - 2.52 (m, 4H), 2.32 - 2.23 (m, 4H), 2.23 - 2.20 (m, 3H), 1.40 (s, 9H).

### Preparation 60

### tert-Butyl 4-[3-(4-bromo-5-methyl-triazol-1-yl)cyclobutyl] piperazine-1-carboxylate

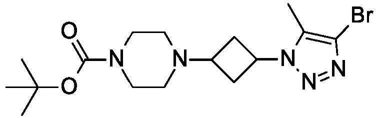

To 3-(4-bromo-5-methyl-triazol-1-yl)cyclobutanone (1.67 g, 7.25 mmol) and tert-butyl piperazine-1-carboxylate (0.90 g, 4.83 mmol) in MeOH (5 ml) was added CH₃CO₂H (0.29 g, 4.83 mmol) in portions at RT. The resulting mixture was stirred for 30 min at 50 °C under N₂. Next, NaBH₃CN (0.61 g, 9.66 mmol) was added in portions at RT under N₂. The reaction was stirred at 50 °C for 2 h. Upon cooling to RT, the reaction was quenched with H₂O (100 ml) and pH adjusted to approximately 7 with saturated aq NaHCO₃. The mixture was extracted with EA (3 x 100 ml). The organic layers were combined, washed with brine (2 x 100 ml), dried over Na₂SO₄, filtered and concentrated in vacuo. The residue was purified by silica gel column chromatography, eluted with 1% to 50% EA in PE to afford the title compound (1.4 g, 72%) as an oil. ES/MS m/z (⁷⁹Br/⁸¹Br) 400/402 [M+H]⁺.

### Preparation 61

### 4-(4-Bromo-5-methyl-triazol-1-yl)-1-(oxetan-3-yl)piperidine

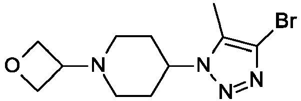

A soln (700 ml) of 4-(4-bromo-5-methyl-triazol-1-yl)piperidine hydrochloride 41 g, 142.69 mmol) in MeOH (700 ml) was treated with oxetan-3-one (35 g, 485.7 mmol) under N₂ and allowed to stir at RT for 5 min. The reaction was treated with AcOH (11.53 g, 192 mmol), allowed to stir for 5 min, then treated in portions with NaBH₃CN (35 g, 556.95 mmol) over 1 h. The reaction was stirred at RT for 30 min then stirred at 30 °C overnight. After cooling to RT, the reaction was quenched with H₂O (50 ml) and the pH was adjusted to 9 with 2M aq K₃PO₄ (75 ml). The organic solvent removed in vacuo. The resulting suspension was diluted with H₂O (30 ml), stirred for 30 min, and filtered to obtain the title compound (30 g, 62%) as a pale white solid. MS ES+ m/z 301/303 [M+H]⁺.

### Preparation 62

### 4-(4-Bromo-3,5-dimethyl-pyrazol-1-yl)-1-(oxetan-3-yl)piperidine

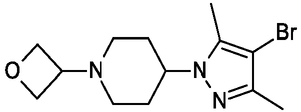

NaBH₃CN (0.51 g) was added to a soln of 4-(4-bromo-3,5-dimethyl-pyrazol-1-yl)piperidine (0.56 g, 2.18 mmol), oxetan-3-one (292 mg, 4.05 mmol) and AcOH (0.15 ml, 2.62 mmol) in MeOH (10 ml). The resulting mixture was stirred at RT for 15 min then at 50°C for 18 h. Upon cooling to RT, the reaction was concentrated in vacuo. The residue was suspended in DCM and washed with a sat. soln of NaHCO₃. The organic phase was separated, and the aq. phase was extracted with DCM (2x). The combined organic layers were dried over MgSO₄, filtered and concentrated. The residue was purified by silica gel chromatography eluting with a gradient of 0% to 100% EA in cHex followed by 0% to 20% MeOH in DCM to afford the title compound (510 mg, 60%) as a white solid. MS ES+ m/z (⁷⁹Br/⁸¹Br) 314/316 [M+H]⁺.

The following compounds were prepared in a manner essentially analogous to the method of Preparation 62 using the appropriate reagents, adjusting reaction time to determine completion of the reaction, and adjusting the purification system as appropriate.

**Table 12**

| Prep # | Chemical Name | Structure | MS ES+ m/z |
|---|---|---|---|
| 63¹ | 4-(4-Bromo-5-methyl-triazol-1-yl)-1-(oxetan-3-yl)azepane | 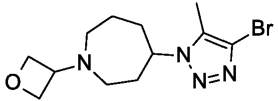 | (⁷⁹ Br/⁸¹Br) 315/317 [M+H]⁺ |

| | | | |
|---|---|---|---|
| ¹ Purified by silica gel chromatography eluting with 0% to 10% MeOH in DCM. | | | |

### Preparation 64

### (1R,5S)-3-(4-Bromo-5-methyl-triazol-1-yl)-8-(oxetan-3-yl)-8-azabicyclo[3.2.1]octane

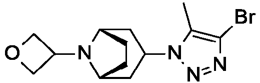

To a solution of tert-butyl (1R,5S)-3-(4-bromo-5-methyl-triazol-1-yl)-8-azabicyclo[3.2.1]octane-8-carboxylate (1.03 g, 2.77 mmol) in 1,4-dioxane (5 ml, 58.57 mmol) was added HCl in 1,4-dioxane (3 ml, 12 mmol, 4 mol/L) and the mixture was stirred for 1 h at RT, then heated at 50 °C for 1 h and stirred overnight at RT. The reaction was concentrated in vacuo to afford the intermediate (1R,5S)-3-(4-bromo-5-methyl-triazol-1-yl)-8-azoniabicyclo[3.2.1]octane hydrochloride as a solid. This material was used in the next step without further purification or characterization.

To a solution of (1R,5S)-3-(4-bromo-5-methyl-triazol-1-yl)-8-azoniabicyclo[3.2.1]octane hydrochloride (0.75 g, 2.44 mmol) in MeOH (10 ml) was added 3-oxetanone (0.7 mL, 10 mmol). The mixture was stirred at RT for 5 min then NaBH₃CN (0.7 g, 10 mmol) was added. The reaction was stirred overnight at RT. The reaction was heated at 50 °C for 9 h. Additional 3-oxetanone (170 µl, 2.79 mmol,) and NaBH₃CN (170 mg, 2.70 mmol) were added, and the reaction was stirred overnight at 50 °C. The reaction was quenched with H₂O and extracted with EA (3 x 25 ml). The combined organic layers were dried over Na₂SO₄, filtered, and concentrated in vacuo. The residue was purified by silica gel chromatography eluting with 0% to 20% EA in DCM followed by 0% to 5% MeOH in DCM. To afford the title compound (630 mg, 69%) as an orange solid. ES+ *m*/*z* (⁷⁹Br/⁸¹Br) 327/329[M+H]⁺.

### Preparation 65

### 2-Chloro-4-cyclopropyl-5-fluoropyridine

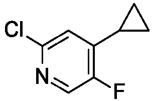

A soln of 2-chloro-5-fluoro-4-iodopyridine (1.50 g, 5.827 mmol) and Pd(PPh₃)₄ (674 mg, 0.583 mmol) in THF (5 ml) was degassed by three freeze-pump-thaw cycle. A soln of cyclopropylzinc bromide (30 ml, 15.150 mmol, 0.5M in THF) was added dropwise at 0 °C. The reaction was stirred overnight allowing it to slowly warm to RT. The reaction was quenched with H₂O and extracted with EA. The combined organic layers were washed with brine, dried over MgSO₄, filtered, concentrated under in vacuo. The residue was purified by silica gel chromatography eluting with EA in heptane to afford the title compound as a colorless oil (758 mg, 76%). ES+ m/z 172.1 [M+H]⁺.

### Preparation 66

### 3-Chloro-5-(trifluoromethyl)pyridazine

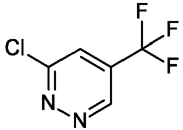

A soln of 4-(trifluoromethyl)-1H-pyridazin-6-one (10 g, 60.94 mmol) and POCl₃ (46.72 g, 304.72 mmol) in ACN (80 ml) was stirred overnight at 80 °C under N₂. The mixture was allowed to cool to RT, poured into H₂O / ice (300 ml), and basified to pH 8 with NaHCO₃. The resulting mixture was extracted with EA (3 x 100 ml). The combined organic layers were washed with brine (1 x 100 ml), dried over Na₂SO₄, filtered and concentrated. The residue was purified by silica gel chromatography eluting with PE / EA (40:1 to 10:1) to obtain the title compound (5 g, 45%) as a yellow oil. MS ES+ m/z 183 [M+H]⁺.

### Preparation 67

### 5-Fluoro-2-[ 1-(trifluoromethyl)vinyl]pyridine

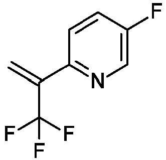

A soln of 2-bromo-5-fluoropyridine (8 g, 45.46 mmol) and 4,4,6-trimethyl-2-[1-(trifluoromethyl)vinyl]-1,3,2-dioxaborinane (11.10 g, 50.00 mmol) in DME (40 ml) and H₂O (10 ml) was treated with K₂CO₃ (18.85 g, 136.37 mmol) and Pd(PPh₃)₄ (1.05 g, 0.91 mmol) under N₂. After stirring for 2 h at 100°C, the mixture was diluted with H₂O (100 ml) and extracted with EA (3 x 100 ml). The combined organic layers were washed with brine (3 x 100 ml), dried over anhydrous Na₂SO₄, filtered, and concentrated. The residue was purified by silica gel chromatography, eluting with PE / EA (20 to 10:1) to obtain the title compound (3.4 g, 39%) as an off-white oil. MS ES+ m/z 192 [M+H]⁺.

### Preparation 68

### 3-(1-Ethoxyvinyl)-5-(trifluoromethyl)pyridazine

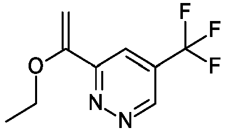

To a mixture of tributyl(1-ethoxyethenyl)stannane (11.87 g, 32.87 mmol) and 3-chloro-5-(trifluoromethyl)pyridazine (5.00 g, 27.39 mmol) in 1,4-dioxane (40 ml) was added Pd(PPh₃)₄ (0.95 g, 0.82 mmol) at RT under a N₂. The reaction was stirred for 2 h at 100 °C. After cooling to RT, the mixture was concentrated and purified by silica gel chromatography eluting with PE / EA (30:1 to 12:1) to afford the title compound (4.6 g, 77%) as a yellow oil. MS ES+ m/z 219 [M+H]⁺.

The following compounds were prepared in a manner essentially analogous to the method of Preparation 68 using the appropriate reagents, adjusting reaction time to determine completion of the reaction, and adjusting the purification system as appropriate.

**Table 13**

| Prep # | Chemical Name | Structure | MS ES+ m/z |
|---|---|---|---|
| 69¹ | 3-(1-Ethoxyvinyl)-2,5-dimethyl-pyrazine | 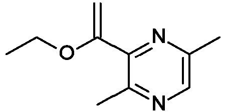 | 179 [M+H]⁺ |
| 70^{2, 3} | 3-Chloro-2-(1-ethoxyvinyl)-5-fluoro-pyridine | 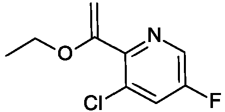 | 202 [M+H]⁺ |
| 71³ | 4-Chloro-2-(1-ethoxyvinyl)-5-fluoropyridine | 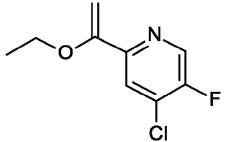 | 201.9 [M]⁺ |

| | | | |
|---|---|---|---|
| ¹ Purified by silica gel chromatography eluting with PE / EA 10:1 to 5:1. ² Reaction quenched with sat. aq KF. ³ Purified by silica gel chromatography eluting with EA in heptane | | | |

### Preparation 72

### N-Methoxy-N-methyl-1-(trifluoromethyl)pyrazole-3-carboxamide

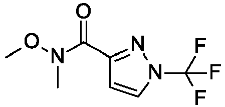

1-(Trifluoromethyl)-1H-pyrazole-3-carboxylic acid (780 mg, 4.33 mmol) was dissolved in SOCl₂ (10 ml) and stirred at reflux for 2 h. The reaction was concentrated in vacuo then re-dissolved in DCM (8.5 ml) at 0 °C and DIPEA (2.2 ml, 12.84 mmol) was added followed by N,O-dimethylhydroxylamine hydrochloride (840 mg, 8.56 mmol). The reaction was allowed to warm to RT then stirred 1 h. The mixture was diluted with DCM and washed with sat. aq NaHCO₃ soln, the combined organic phases were dried over MgSO₄, filtered, and the filtrate was concentrated in vacuo to afford the title compound as a pale-yellow oil (809 mg, 85%). MS ES+ m/z 224.1 [M+H]⁺.

### Preparation 73

### N-Methoxy-N-methyl-5-(trifluoromethyl)pyridine-3-carboxamide

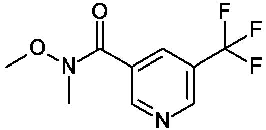

A DCM suspension (60 ml) of 5-(trifluoromethyl)pyridine-3-carboxylic acid (4 g, 20.93 mmol) was treated with 1,1'-carbonyldiimidazole (3.73 g, 23 mmol) and Et₃N (5.8 mL, 42 mmol). The reaction was stirred at RT for 3 h, diluted with H₂O, the organic phase collected, and the aq phase re-extracted with DCM. The combined organic layers were washed with sat. aq NaHCO₃, collected, dried over MgSO₄, filtered, and concentrated to obtain the title compound (3.75 g) as a yellow oil that was used without purification. MS ES+ m/z 235 [M+H]⁺.

### Preparation 74

### N-Methoxy-N,2,5-trimethyl-thiazole-4-carboxamide

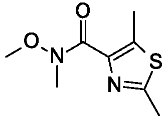

To an ice-cold soln of 2,5-dimethylthiazole-4-carboxylic acid (1.25 g, 7.95 mmol) and methoxy(methyl)ammonium chloride (1.95 g, 20.0 mmol) in DMF (10 ml) was added NEt₃ (2.85 g, 28 mmol). The mixture was stirred for 5 min then HATU (3.6 g, 9.3 mmol) was added. The reaction was stirred at RT for 16 h. The reaction was poured into H₂O, the aq soln was extracted with DCM (2X) and the combined organic layers were washed with 10% aq. LiCl, dried over MgSO₄, filtered and concentrated. The residue was purified by silica gel chromatography eluting with 0% to 30% EA in cHex to afford the title compound. The title compound was taken on to the next step.

### Preparation 75

### 1-[5-(Trifluoromethyl)pyridazin-3-yl]ethanone

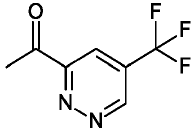

A soln of 3-(1-ethoxyethenyl)-5-(trifluoromethyl)pyridazine (1.0 g, 4.58 mmol) and conc HCl (2.26 g, 22.92 mmol) in THF (10 ml) was stirred for 1 h at RT under a N₂. The resulting mixture was diluted with H₂O (10 ml), basified to pH 8 with NaHCO₃, and extracted with MTBE (3 x 20 ml). The combined organic layers were washed with brine (1 x 10 ml), dried over anhydrous Na₂SO₄, collected, and filtered. The filtrate was concentrated to obtain the title compound (810 mg, 93%) as a yellow solid. MS ES+ m/z 191 [M+H]⁺.

The following compounds were prepared in a manner essentially analogous to the method of Preparation 75 using the appropriate reagents, adjusting reaction time to determine completion of the reaction, and adjusting the purification system as appropriate.

**Table 14**

| Prep # | Chemical Name | Structure | MS ES+ m/z |
|---|---|---|---|
| 76 | 1-(3,6-Dimethylpyrazin-2-yl)ethanone | 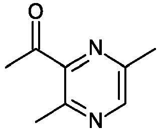 | 192 [M+H]⁺ |

### Preparation 77

### 1-(4-Cyclopropyl-5-fluoro-2-pyridyl)ethanone

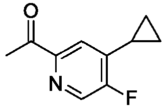

A soln of 2-chloro-4-cyclopropyl-5-fluoropyridine (617 mg, 3.60 mmol) and Pd(PPh₃)₄ (416 mg, 0.36 mmol) in toluene (10 ml) was degassed by vacuum / N₂ cycle (3x). Tributyl(1-ethoxyvinyl)tin (1.56 mL, 4.32 mmol) was added and the mixture was stirred at 100 °C under N₂ for 6 h. The reaction was treated with sat. aq KF (2.5 mL), filtered through a pad of DE, and the cake was washed with EA (30 ml). The phases from the filtrate were separated. The organic phase was washed with sat. aq NaHCO₃ (3 x 20 ml) and brine (20 ml), dried over MgSO₄, filtered, and concentrated in vacuo to afford the intermediate vinyl ether as a brown oil.

The intermediate vinyl ether was dissolved in THF (10 ml) then aq HCl (2M, 5 mL) was added, and the reaction was stirred 12 h at RT. The reaction was treated with sat. aq NaHCO₃ (30 ml), and the mixture was stirred vigorously for 10 min. The aq phase was extracted with EA (3 x 25 ml) and the combined organic layers were washed with brine (25 ml), dried over MgSO₄, filtered, concentrated in vacuo. The residue was purified by silica gel chromatography eluting with EA in heptane to afford the title compound as a colorless oil (226 mg, 35%). ES+ m/z 180.1 [M+H]⁺.

The following compounds were prepared in a manner essentially analogous to the method of Preparation 77 using the appropriate reagents, adjusting reaction time to determine completion of the reaction, and adjusting the purification system as appropriate.

**Table 15**

| Prep # | Chemical Name | Structure | MS ES+ m/z |
|---|---|---|---|
| 78 | 1-(7-Fluoro-4-isoquinolyl)ethanone | 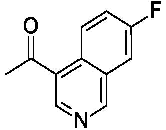 | 190.1 [M+H]⁺ |

### Preparation 79

### 1-[5-(Trifluoromethyl)-3-pyridyl]ethanone

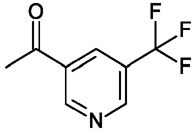

A soln of N-methoxy-N-methyl-5-(trifluoromethyl)pyridine-3-carboxamide (2 g, 6.41 mmol, 75 mass%) in THF (30 ml) at 0 °C was treated dropwise with MeMgBr (10 mL, 14 mmol, 1.4 M in toluene / THF [3:1]) and stirred for 1 h. The reaction was quenched with saturated aq NH₄Cl at 0 °C, diluted with H₂O, and extracted with EA (2x). The organic layers were combined, dried over MgSO4, filtered, and concentrated. The resulting oil was purified by silica gel chromatography eluting with a gradient of 0% to 80% EA in cHex to obtain the title compound (1.0 g, 83%) as a white solid. ¹H NMR (400 MHz, CDCl₃): δ 9.35 (d, J= 1.9 Hz, 1H), 9.07 (d, J= 1.5 Hz, 1H), 8.48-8.47 (m, 1H), 2.72 (s, 3H).

The following compounds were prepared in a manner essentially analogous to the method of Preparation 79 using the appropriate reagents, adjusting reaction time to determine completion of the reaction, and adjusting the purification system as appropriate.

**Table 16**

| Prep # | Chemical Name | Structure | ¹H NMR (400 MHz, CDCl₃) δ |
|---|---|---|---|
| 80 | 1-(2,5-Dimethylthiazol-4-yl)ethanone | 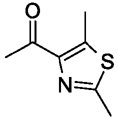 | a |
| 81¹ | Cyclopropyl-(5-fluoro-2-pyridyl)methanone | 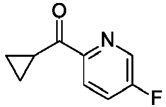 | 8.57 (d, J = 2.8 Hz, 1H), 8.12 (ddd, J = 8.7, 4.7, 0.6 Hz, 1H), 7.54 (ddd, J = 8.7, 8.0, 2.8 Hz, 1H), 3.48 (tt, J = 7.9, 4.7 Hz, 1H), 1.29 - 1.23 (m, 2H), 1.16 - 1.07 (m, 2H). |
| 82¹ | Cyclobutyl-(5-fluoro-2-pyridyl)methanone | 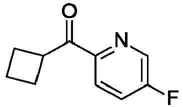 | a |

| | | | |
|---|---|---|---|
| ^{a} Material used in subsequent step without further characterization. ¹ Purified by silica gel chromatography eluting with 0% to 30% EA in cHex. | | | |

### Preparation 83

### Methyl 1-(5-bromo-2-pyridyl)-3,3-difluoro-cyclobutanecarboxylate

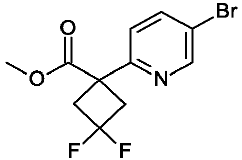

A toluene (50 mL) soln of 5-bromo-2-fluoropyridine (7.0 g, 39.78 mmol) and methyl 3,3-difluorocyclobutane-1-carboxylate (6.57 g, 43.75 mmol) was treated dropwise with KHMDS (51.7 ml, 51.71 mmol, 1.0 M in THF) at -70 °C under a nitrogen atmosphere. After stirring for 30 min at -70 °C, the reaction was quenched with saturated, aq NH₄Cl at RT, acidified to pH 1~2 with aq HCl (1.2 M) and extracted with EA (3 x 100 ml). The combined organic layers were washed with brine (2 x 100 ml), dried over anhydrous Na₂SO₄, filtered, and concentrated to obtain the title compound (11 g, crude) as a yellow solid. The crude product was used directly without purification. MS ES+ m/z (⁷⁹Br/⁸¹Br) 306/308 [M+H]⁺.

### Preparation 84

### 1-Methyl-2-(2-pyridyl)pyrrolidin-3-ol

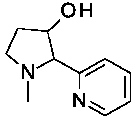

To a soln of 2-(2-pyridyl)pyrrolidin-3-ol (450 mg, 2.74 mmol) in DCM (40 ml) was added H₂CO (13.31 mol/L) in H₂O (0.65 ml). After stirring 10 minutes, Na(OAc)₃BH (0.9 g, 4 mmol) was added. The mixture was stirred overnight at RT. The reaction was quenched with saturated aq NaHCO₃. The aq phase was acidified with HCl until pH 2 then directly loaded onto a SCX column. The title compound was eluted with 2N NH₃ in MeOH to afford the title compound (200 mg, 9%), MS ES+ m/z 179 [M+H]⁺.

### Preparation 85

### 1-[1-(Trifluoromethyl)pyrazol-3-yl]ethanol

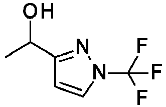

MeMgBr (0.21 ml, 1.80 mmol, 3M) was added to a soln of N-methoxy-N-methyl-1-(trifluoromethyl)pyrazole-3-carboxamide (329 mg, 2.00 mmol) in THF (2.7 ml) under N₂ at 0 °C. After stirring 1 h at 0 °C the reaction was carefully quenched with AcOH (0.13 ml, 2.25 mmol) and diluted with MeOH (2 ml). Next, NaBH₄ (102 mg, 2.70 mmol) was added in one portion, and the reaction was stirred for 40 min at 0 °C. The reaction was diluted with saturated aq NH₄Cl (4 ml), extracted with Et₂O (2 x 5 ml) and DCM (2 x 5 ml) and the combined organic phases were dried over MgSO₄, filtered, and the solvent was concentrated in vacuo to afford the title compound as a pale-yellow oil (174 mg, quantitative). ES+ m/z 180.9 [M+H]⁺.

### Preparation 86

### 1-(1-Methylpyrrolo[2,3-c]pyridin-4-yl)ethanol

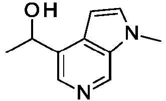

A 0.5 M soln of lithium dibutyl(methyl)magnesate was prepared by adding MeMgBr (5 ml, 15 mmol) and n-BuLi (18.75 mL, 30 mmol) to THF (6.25 ml) in a round-bottom flask at 0 °C.

The stock soln of lithium dibutyl(methyl)magnesate (10 ml, 5 mmol) was added dropwise to a soln of 4-bromo-1-methyl-1H-pyrrolo[2,3-c]pyridine (979 mg, 4.64 mmol) in THF (25 ml) at -40 °C. After 1 h acetaldehyde (2.6 ml, 46.40 mmol) was added at -40 °C. The reaction was stirred for 1 h, allowing the temperature to reach -20°C. The reaction was quenched with saturated NH₄Cl then extracted with EA. The combined organic layers were dried on MgSO₄, filtered, and concentrated in vacuo. The residue was purified by silica gel chromatography eluting with MeOH in DCM to give the title compound as beige solid (548 mg, 67%). MS ES+ m/z 177.1 [M+H]⁺.

### Preparation 87

### 1-(1-Methylpyrazolo[3,4-c]pyridin-4-yl)ethanol

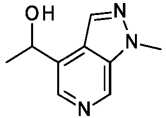

To a soln of MeMgBr (1.67 ml, 3.0 M soln, 5.0 mmol) in THF (10 ml) was added n-BuLi (6.25 ml, 1.6 M soln in hexane, 10.0 mmol,) at 0 °C under N₂. The reaction was stirred for 30 min. then cooled to -40°C. Next, 4-bromo-1-methyl-1H-pyrazolo[3,4-c]pyridine (1.01 g, 4.76 mmol) was added to the reaction. The resultant suspension was briefly (approx. 2 min) stirred at 0 °C to reach complete dissolution of the solids. The soln was re-cooled to -40°C and stirred for 1 h. Next, acetaldehyde (2.65 ml, 8.9 mmol) was added dropwise. The soln was stirred at -40 °C for 90 min. then the reaction was treated with saturated aq. NH₄Cl soln and diluted with EA. The layers were separated, and the aq layer was extracted with EA (4x). The combined organic layers were dried on MgSO4, filtered, and concentrated in vacuo. The residue was purified by silica gel chromatography eluting with MeOH in DCM to provide the title compound as beige solid (372 mg, 44%). MS ES+ m/z 178.2 [M+H]⁺.

### Preparation 88

### 1-(1-Isopropyltriazol-4-yl)ethanol

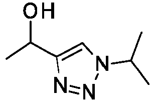

MeMgBr in Et₂O (2.5 M in Et₂O, 2.2 ml, 5.47 mmol) was added dropwise to a soln of 1-isopropyl-1H-1,2,3-triazole-4-carbaldehyde (508 mg, 3.65 mmol) in THF (5 ml) at 0 °C under N₂ and the reaction was allowed to stir at RT for 3 h. After that time, the soln was cooled to 0 °C and quenched with saturated NH₄Cl (aq., 25 ml) and extracted with DCM (3 x 25 ml). The combined organic layers were dried over MgSO₄, filtered, and concentrated in vacuo to give the title compound as a pale-yellow oil (558 mg, 99%). MS ES+ m/z 156.2 [M+H]⁺.

The following compounds were prepared in a manner essentially analogous to the method of Preparation 88 using the appropriate reagents, adjusting reaction time to determine completion of the reaction, and adjusting the purification system as appropriate.

**Table 17**

| Prep # | Chemical Name | Structure | MS ES+ m/z |
|---|---|---|---|
| 89 | 1-(2-Cyclopropyl thiazol-4-yl)ethanol | 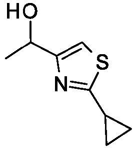 | 170 [M+H]⁺ |
| 90 | 1-(2-Methylthiazol-4-yl)propan-1-ol | 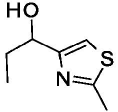 | 158 [M+H]⁺ |
| 91 | 1-(2-Bromothiazol-4-yl)ethanol | 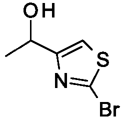 | (⁷⁹Br/⁸¹Br) 208/210 [M+H]⁺ |

### Preparation 92

### 1-(2-Isopropyltriazol-4-yl)ethanol

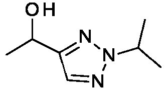

4-Bromo-2-isopropyl-2H-1,2,3-triazole (1.00 g, 5.26 mmol) was dissolved in 10 ml of THF under N₂ and cooled to -78 °C. A soln of n-BuLi (3.62 ml, 5.79 mmol, 1.6 M in hexane) was added dropwise. The soln was stirred at -78 °C for 45 min, then acetaldehyde (1.63 ml, 26.31 mmol) was added, and the reaction was slowly allowed to warm to RT then stirred for 18 h. The reaction was quenched with H₂O (30 mL), extracted with EA (3 x 15 ml) and the combined organic layers were dried over MgSO₄, filtered, and concentrated in vacuo. The residue was purified by flash silica gel chromatography eluting with MeOH in DCM to afford the title compound as a pale-yellow oil (299 mg, 37%). ¹H NMR (400 MHz, CDCl₃) δ (ppm): 7.51 (s, 1H), 5.04 (q, J = 6.5 Hz, 1H), 4.79 (hept, J = 6.7 Hz, 1H), 1.57 (t, J = 6.5 Hz, 3H), 1.56 (d, J = 6.7 Hz, 6H).

The following compounds were prepared in a manner essentially analogous to the method of Preparation 92 using the appropriate reagents, adjusting reaction time to determine completion of the reaction, and adjusting the purification system as appropriate.

**Table 18**

| Prep # | Chemical Name | Structure | MS ES+ m/z |
|---|---|---|---|
| 93¹ | 1-(6-Bromopyrazin-2-yl)ethanol | 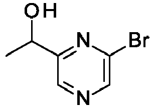 | (⁷⁹Br/⁸¹Br) 203/205 [M+H]⁺ |

| | | | |
|---|---|---|---|
| Purified by silica gel chromatography eluting with 10% to 50% EA in cHex. | | | |

### Preparation 94

### 1-[5-(Difluoromethyl)-3-pyridyl]ethanol

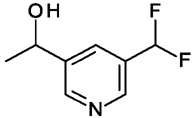

In a separate flask, a degassed (by ultrasound) soln of 1-[5-(difluoromethyl)-3-pyridyl]ethanone (856 mg, 5.00 mmol) in aq FA (2.1 g, 1.7 ml, 45 mmol) and NEt₃ (6.0 g, 60 mmol) was added [[(1S,2S)-2-amino-1,2-diphenyl-ethyl]-(p-tolylsulfonyl)amino]-chloro-ruthenium;1-isopropyl-4-methyl-benzene (29 mg, 0.05 mmol) at RT. The mixture was stirred at RT under N₂ overnight. H₂O and EtOH were added to the mixture and partially evaporated. Saturated aq NaHCO₃ was added to ensure high pH and mixture was extracted with EA (4x). Combined organic layers were dried over Na₂SO₄ and filtered. DE was added, and the suspension was evaporated. The residue was purified by silica gel chromatography eluting with 30% to 90% EA in cHex to afford the title compound (815 mg, 94%) as a green oil. MS ES+ m/z 174 [M+H]⁺.

The following compounds were prepared in a manner essentially analogous to the method of Preparation 94 using the appropriate reagents, adjusting reaction time to determine completion of the reaction, and adjusting the purification system as appropriate.

**Table 19**

| Prep # | Chemical Name | Structure | MS ES+ m/z |
|---|---|---|---|
| 95¹ | 1-[6-(Trifluoromethyl)pyrazin-2-yl] ethanol | 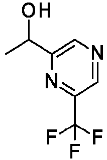 | 193 [M+H]⁺ |
| 96² | 1-(2,5-Dimethylthiazol-4-yl)ethanol | 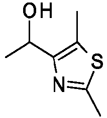 | 158 [M+H]⁺ |

| | | | |
|---|---|---|---|
| ¹ Purified by silica gel chromatography eluting with 5% EA in cHex (2 CV), 5% to 50% EA in cHex (15 CV), 50% to 100% EA in cHex (5 CV). ² Purified by silica gel chromatography eluting with 0% to 50% EA in cHex. | | | |

### Preparation 97

### 1-(2-Methoxythiazol-4-yl)ethanol

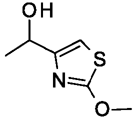

In a glass pressure flask 1-(2-bromothiazol-4-yl)ethanol (2.14 g, 9.25 mmol) in MeOH (23 ml) was added a soln of NaOMe (30 mass %) in MeOH (23 ml). The headspace was purged with N₂, and the flask was sealed. The reaction was stirred at 50 °C. After 6 h, the reaction was stirred overnight at RT. The next day heating was resumed for 4 h. Upon cooling to RT, the reaction was poured over aq saturated NH₄Cl soln (150 ml), and DCM (30 ml) was added. The organic layer was separated and the aq layer was extracted with DCM (3 x 20 ml). The combined organic layers were dried over Na₂SO₄, filtered, and concentrated. The residue was dissolved in DCM, dry-loaded onto DE and purified by silica gel column chromatography eluting with the following conditions: 100% cHex (1 CV), 0% to 35% EA in cHex (16 CV), 35% to 50% EA in cHex (14 CV). ¹H NMR (400.21 MHz, CDCl₃): δ 6.50 (d, J= 1.0 Hz, 1H), 4.82-4.77 (m, 1H), 4.08 (s, 3H), 2.37 (d, J= 4.4 Hz, 1H, OH), 1.53 (d, J= 6.4 Hz, 3H).

### Preparation 98

### 1-[5-(Trifluoromethyl)pyridazin-3-yl]ethanol

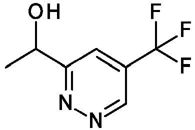

To a stirred soln of 1-[5-(trifluoromethyl)pyridazin-3-yl]ethanone (810 mg, 4.26 mmol) in MeOH (10 ml) was added NaBH₄ (48.35 mg, 1.28 mmol) at 0 °C under a N₂. The resulting mixture was stirred for 30 min at RT. The reaction was quenched with H₂O (10 ml) at 0 °C. The mixture was extracted with EA (3 x 20 ml). The combined organic layers were washed with brine (2 x 10 ml), dried over anhydrous Na₂SO₄, filtered, and concentrated to obtain the title compound (800 mg, 98%). ¹H NMR (400 MHz, DMSO-d₆) δ 9.61 (d, 1H), 8.10 (d, 1H), 5.88 (d, 1H), 5.14 - 5.08 (m, 1H), 1.50 (d, 3H).

The following compounds were prepared in a manner essentially analogous to the method of Preparation 98 using the appropriate reagents, adjusting reaction time to determine completion of the reaction, and adjusting the purification system as appropriate.

**Table 20**

| Prep # | Chemical Name | Structure | MS ES+ m/z |
|---|---|---|---|
| 99 | 1-(3,6-Dimethylpyrazin-2-yl)ethanol | 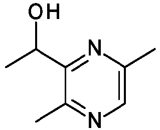 | 153 [M+H]⁺ |
| 100 | 1-(4-Cyclopropyl-5-fluoro-2-pyridyl)ethanol | 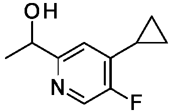 | 182.1 [M+H]⁺ |
| 101 | 1-(7-Fluoro-4-isoquinolyl)ethanol | 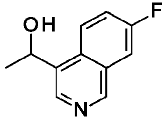 | 192.2 [M+H]+ |
| 102 | Cyclopropyl-(5-fluoro-2-pyridyl)methanol | 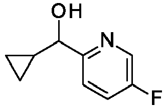 | a |
| 103 | Cyclobutyl-(5-fluoro-2-pyridyl)methanol | 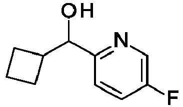 | a |

| | | | |
|---|---|---|---|
| ^{a} Material used in subsequent step without further characterization. | | | |

### Preparation 104

### [1-(5-Bromo-2-pyridyl)-3,3-difluoro-cyclobutyl]methanol

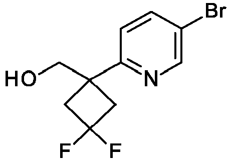

To a soln of methyl 1-(5-bromo-2-pyridyl)-3,3-difluoro-cyclobutanecarboxylate (11.0 g, crude) in THF (100 ml) was treated with LiBH₄ (0.78 g, 35.94 mmol) in portions at RT under N₂. After stirring at RT for 3 h, the reaction was quenched with sat. NH₄Cl and concentrated. The residue was purified by silica gel chromatography eluting with PE / EA (5:1) to afford the title compound (1.8 g, 16% over 2 steps) as a yellow solid. MS ES+ m/z 278/280 [M+H]⁺.

### Preparation 105

### 2-Benzyloxy-1-(3,5-difluoro-2-pyridyl)ethanol

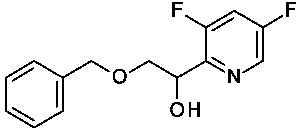

To 2-bromo-3,5-difluoropyridine (5 g, 25.78 mmol) in toluene (50 ml) was treated with i-PrMgCl (19.33 ml, 38.66 mmol, 2M in THF) at 0°C under N₂. After stirring for 3 h at RT, the reaction was cooled to 0 °C and treated with 2-(benzyloxy)acetaldehyde (3.10 g, 20.62 mmol). The reaction was stirred for 2 h at RT then quenched with saturated aq NH₄Cl (200 mL). The reaction was extracted with EA (3 x 300 ml). The combined organic layers were washed with brine (2 x 200 ml), dried over Na₂SO₄, filtered, and concentrated. The residue was purified by silica gel chromatography eluting with PE / EA (4:1) to obtain the title compound (1.6 g, 23%) as a light-yellow oil. MS ES+ m/z 266 [M+H]⁺.

### Preparation 106

### 1-(5-Fluoropyrimidin-2-yl)ethane-1,2-diol

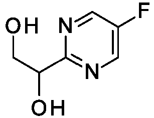

To a mixture of 2-ethenyl-5-fluoropyrimidine (15.0 g, crude) and K₂OsO₄·2H₂O (1.38 g, 3.75 mmol) in ACN : H₂O (6:1, 140 ml) was added NMO (17.56 g, 149.86 mmol) at 0 °C under N₂. The resulting mixture was stirred for 2 h at RT under N₂. The reaction was diluted with H₂O (20 ml), extracted with MTBE (3 x 200 ml). The aq layer was extracted with CHCl₃ : IPA (3:1) (3 x 250 ml). The combined organic layers were washed with brine (3 x 50 ml), dried over Na₂SO₄, filtered and concetrated to afford the title compound (6.0 g, 51%) as a black oil. MS ES+ m/z 159 [M+H]⁺.

### Preparation 107

### 1-(5-Fluoropyrimidin-2-yl)-2-(2-trimethylsilylethoxymethoxy)ethanol

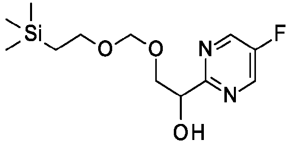

To a mixture of 1-(5-fluoropyrimidin-2-yl)ethane-1,2-diol (6.0 g, 37.94 mmol) and DIEA (14.71 g, 113.83 mmol) in DCM (60 ml) was added SEM-Cl (4.43 g, 26.56 mmol) dropwise at 0 °C under N₂. The mixture was stirred for 1 h at RT. The reaction was quenched by the addition of MeOH (20 ml) at RT. The mixture was stirred for 30 min then concentrated in vacuo. The residue was purified by silica gel chromatography eluting with PE / EA (6:1 to 4:1) to afford the title compound (3.0 g, 27%) as a yellow oil. ¹H NMR (300 MHz, DMSO-d₆) δ 8.93 (d, 2H), 4.93 - 4.86 (m, 1H), 4.78 - 4.71 (m, 2H), 4.66 - 4.60 (m, 1H), 3.83 - 3.74 (m, 2H), 3.59 - 3.41 (m, 2H), 0.84 -0.59 (m, 2H), 0.03 - 0.03 (s, 9H). MS ES+ m/z 289 [M+H]⁺.

2-(5-Fluoropyrimidin-2-yl)-2-(2-trimethylsilylethoxymethoxy)ethanol was also isolated after chromatography (600 mg, 5%). ¹H NMR (300 MHz, DMSO-d₆) δ 8.91 (d, 2H), 5.56 (d, 1H), 4.89 - 4.75 (m, 1H), 4.61 - 4.53 (m, 2H), 3.98 - 3.71 (m, 2H), 3.47 - 3.38 (m, 2H), 0.88 - 0.78 (m, 2H), 0.03 - 0.03 (s, 9H). MS ES+ m/z 289 [M+H]⁺.

### Preparation 108

### 2-[tert-Butyl(dimethyl)silyl]oxy-1-(5-fluoro-2-pyridyl)ethanol

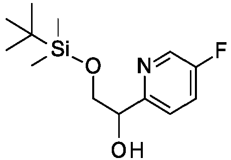

A toluene soln (10 ml) of 2-bromo-5-fluoropyridine (1.2 g, 6.82 mmol) at 0 °C was treated dropwise with i-PrMgCl (5.11 ml, 10.23 mmol, 2M in THF) under N₂. The resulting mixture was stirred for 30 min at 0 °C and treated with 2-[tert-butyl(dimethyl) silyl]oxyacetaldehyde (1.78 g, 10.23 mmol) dropwise over 10 min. The resulting mixture was stirred for 2 h at 0 °C, quenched with H₂O, and extracted with EA (3 x 20 mL). The combined organic layers were washed with brine (2 x 10 ml), dried over anhydrous Na₂SO₄, filtered, and concentrated. The residue was purified by silica gel chromatography eluting with PE / EA (10:1 to 5:1) to obtain the title compound (420 mg, 23%) as a colorless oil. MS ES+ m/z 272 [M+H]⁺.

### Preparation 109

### 2-(2-Benzyloxy-1-methoxy-ethyl)-3,5-difluoro-pyridine

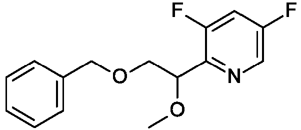

A soln of 2-benzyloxy-1-(3,5-difluoro-2-pyridyl)ethanol (1.5 g, 5.66 mmol) and CH₃I (1.20 g, 8.48 mmol) in THF (20 ml) was treated with t-BuOK (6.79 ml, 6.79 mmol, 1M in THF) at 0 °C under a N₂. The resulting mixture was stirred for 1 h at RT, quenched with saturated aq NH₄Cl (100 ml), and extracted with EA (3 x 200 ml). The combined organic layers were washed with brine (2 x 200 ml), dried over Na₂SO₄, filtered, and concentrated to obtain the title compound (1.52 g, 96%) as a light-yellow oil. MS ES+ m/z 280 [M+H]⁺.

### Preparation 110

### tert-Butyl-[2-(5-fluoro-2-pyridyl)-2-methoxy-ethoxy]-dimethyl-silane

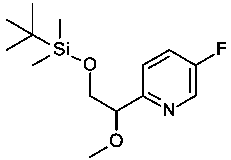

A soln (30 ml) of 2-[tert-butyl(dimethyl)silyl]oxy-1-(5-fluoro-2-pyridyl)ethanol (3.0 g, 11.05 mmol) and CH₃I (7.84 g, 55.27 mmol) in THF (30 ml) at 0 °C was treated in portions with NaH (0.53 g, 22.11 mmol, 60% wt) under N₂. After stirring at RT for 2 h, the reaction was quenched with H₂O (50ml) and extracted with EA (2 x 50 ml). The combined organic layers were washed with brine (2 x 80 ml), dried over anhydrous Na₂SO₄, filtered, and concentrated to obtain the title compound (2.9 g, 92%) as a yellow solid. MS ES+ m/z 286 [M+H]⁺.

### Preparation 111

### 2-[[2-(5-Fluoropyrimidin-2-yl)-2-methoxy-ethoxy]methoxy]ethyl-trimethyl-silane

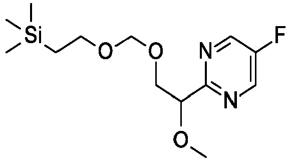

To a stirred mixture of 1-(5-fluoropyrimidin-2-yl)-2-(2-trimethylsilylethoxy methoxy)ethanol (1.01 g, 3.50 mmol) in THF (10 ml) was added CH₃I (746 mg, 5.25 mmol) and Ag₂O (2.43 g, 10.51 mmol) in portions at RT under N₂. The reaction was stirred for 4 h at 60 °C under N₂. Upon cooling to RT the reaction was concentrated in vacuo. The residue was purified by silica gel column chromatography eluting with DCM / EA (2:1 to 3:1) to afford the title compound (948 mg, 90%) as a yellow oil. MS ES+ *m*/*z* 303 [M+H]⁺.

### Preparation 112

### 5-Bromo-2-[3,3-difluoro-1-(tetrahydropyran-2-yloxymethyl)cyclobutyl]pyridine

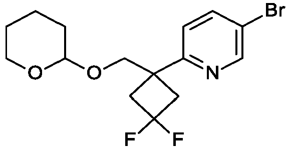

A soln of [1-(5-bromo-2-pyridyl)-3,3-difluoro-cyclobutyl]methanol (1.7 g, 6.113 mmol) and DHP (2.57 g, 30.565 mmol) in DCM (10 ml) was treated with TsOH (10.5 mg, 0.06 mmol) under N₂. After stirring at 50 °C for 2 h, the reaction was allowed to cool to RT and concentrated. The residue was purified by silica gel chromatography eluting with PE / EA (20: 1) to afford the title compound (2.1 g, 94.8%) as a yellow oil. MS ES+ m/z 362/364 [M+H]⁺.

### Preparation 113

### 2-[3,3-Difluoro-1-(tetrahydropyran-2-yloxymethyl)cyclobutyl]-5-fluoro-pyridine

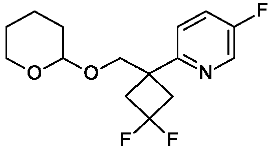

A soln of 5-bromo-2-[3,3-difluoro-1-(tetrahydropyran-2-yloxymethyl) cyclobutyl]pyridine (2.0 g, 5.5 mmol) in THF (20 ml) was treated dropwise with n-BuLi (2.87 ml, 7.18 mmol, 2.5 M in hexane) at -70 °C under N₂. The reaction was stirred for 15 min at -70 °C and then treated with NFSI (3.48 g, 11.04 mmol). The reaction was stirred for an additional hour at -70 °C then quenched with H₂O at RT. The resulting mixture was extracted with EA (3 x 100 ml), combined organic layers were washed with brine (2 x 100 ml), dried over Na₂SO₄, filtered, and concentrated. The residue was purified by silica gel chromatography, eluting with PE / EA (20:1). To afford the title compound (930 mg, crude) as a yellow oil. MS ES+ m/z 302 [M+H]⁺.

### Preparation 114

### [3,3-Difluoro-1-(5-fluoro-2-pyridyl)cyclobutyl]methanol

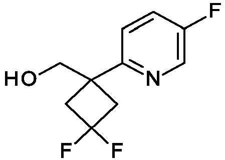

A soln of 2-[3,3-difluoro-1-(tetrahydropyran-2-yloxymethyl)cyclobutyl]-5-fluoro-pyridine (900 mg, crude) in THF (10 mL) was treated with aq HCl (2.5 ml, 12M) at RT under N₂. After stirring at RT for 3 h, the reaction was diluted with H₂O (50 ml), basified to pH 7-8 with aq NaHCO₃, and extracted with EA (3 x 50 ml). The combined organic layers were washed with brine (2 x 50 ml), dried over Na₂SO₄, filtered, and concentrated. The residue was purified by Prep-TLC: PE / EA (5:1) to afford the title compound (450 mg, 69%) as a light-yellow oil. MS ES+ m/z 218 [M+H]⁺.

### Preparation 115

### 2-(3,5-Difluoro-2-pyridyl)-2-methoxy-ethanol

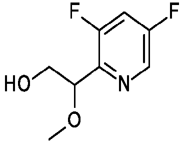

A soln of 2-benzyloxy-1-(3,5-difluoro-2-pyridyl)ethanol (1.5 g, 5.37 mmol) in TFA (15 ml) was stirred for 4 h at 80 °C under N₂. The reaction was allowed to cool to RT, diluted with H₂O (50 ml), basified to pH 9 with K₂CO₃, and extracted with EA (3 x 100 ml). The combined organic layers were washed with brine (2 x 100 ml), dried over Na₂SO₄, filtered, and concentrated. The residue was purified by silica gel chromatography eluting with PE / EA (5:1 to 4:1) to afford the title compound (545 mg, 54%) as a light-yellow oil. MS ES+ m/z 190 [M+H]⁺.

### Preparation 116

### 2-(5-Fluoro-2-pyridyl)-2-methoxy-ethanol

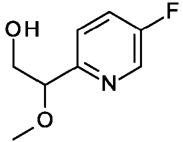

A soln of tert-butyl-[2-(5-fluoro-2-pyridyl)-2-methoxy-ethoxy]-dimethyl-silane (2.9 g, 10.16 mmol) in DCM (5 ml) was treated dropwise with 4M HCl in 1,4-dioxane (30 ml) at RT under N₂. When complete, the reaction was concentrated and the residue dissolved in 50 ml of H₂O, basified to pH 9 with saturated aq Na₂CO₃, and extracted with EA (2 x 60 ml). The combined organic layers were washed with brine (2 x 40 ml), dried over Na₂SO₄, and concentrated to obtain the title compound (1.6 g, crude) as a yellow oil. MS ES+ m/z 172 [M+H]⁺.

### Preparation 117

### 2-(5-Fluoropyrimidin-2-yl)-2-methoxy-ethanol

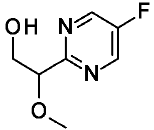

To 2-[[2-(5-fluoropyrimidin-2-yl)-2-methoxy-ethoxy]methoxy]ethyl-trimethylsilane (925 mg, 3.06 mmol) in MeOH (9 ml) was added conc. HCl (3 ml) dropwise at RT under N₂. The reaction was stirred for 2 h at RT then quenched with H₂O (100 ml). The mixture was basified to pH 8 with NaHCO₃. The aqueous layer was extracted with EA (3 x 100 ml). The combined organic layers were concentrated in vacuo to afford the title compound as a white solid (450 mg, 85%). MS ES+ m/z 173 [M+H]⁺.

### Preparation 118

### 1-[5-(Trifluoromethyl)-3-pyridyl]ethanamine

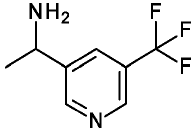

A soln of 1-[5-(trifluoromethyl)-3-pyridyl]ethanone (0.77 g, 4.06 mmol) in EtOH (30 ml) was treated with NH₄OAc (4.7 g, 61 mmol) and stirred at RT for 20 min. The reaction was treated with NaBH₃CN (0.54 g, 8.12 mmol) and stirred at RT for 1 h and then at 85 °C for 1.25 h. The reaction was allowed to cool to RT and concentrated. The resulting oil was loaded onto an SCX cartridge. Non-basic impurities were washed off the cartridge with MeOH then the title compound was eluted with methanolic ammonia (2M). The resulting oil was purified by reversed phase (Claricep C) chromatography eluting with 20% to 50% aq NH₄CO₃ in ACN to obtain the title compound (1.9 g, 77%) as a yellow oil. MS ES+ m/z 191 [M+H]⁺.

### Preparation 119

### N-Benzyl-3,3,3-trifluoro-2-(5-fluoro-2-pyridyl)propan-1-amine

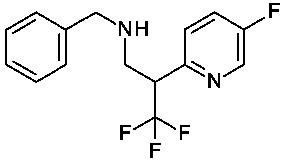

A THF soln (30 ml) of 5-Fluoro-2-[1-(trifluoromethyl)vinyl]pyridine (3.4 g, 17.79 mmol) was treated with benzylamine (3.81 g, 35.58 mmol) under N₂. After stirring at 50 °C for 2 h, the reaction was cooled to RT, diluted with H₂O (100 ml) and extracted with EA (3 x 100 ml). The combined organic layers were washed with brine (100 ml), dried over Na₂SO₄, filtered, and concentrated. The residue was purified by silica gel chromatography, eluting with PE / EA (20:1 to 10:1), to afford the title compound (4.0 g, 75%) as a colorless oil. MS ES+ m/z 299 [M+H]⁺.

### Preparation 120

### 3,3,3-Trifluoro-2-(5-fluoro-2-pyridyl)propan-1-amine

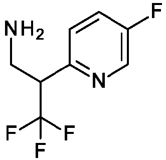

To a soln of N-benzyl-3,3,3-trifluoro-2-(5-fluoro-2-pyridyl)propan-1-amine (3.50 g, 11.73 mmol) in IPA (50 ml) was added Pd/C (2.50 g, 23.47 mmol) and Pd(OH)₂/C (2.82 g, 20.11 mmol) under N₂. The resulting mixture was stirred for 2 h at RT under H₂. The mixture was filtered and washed with MeOH (3 x 10 ml). The filtrate was concentrated to afford the title compound (2.0 g, crude). MS ES+ m/z 209 [M+H]⁺.

### Preparation 121

### 3-(1-Chloroethyl)-5-(trifluoromethyl)pyridine

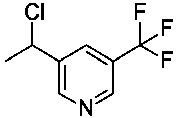

To a stirred mixture of 1-[5-(trifluoromethyl)pyridin-3-yl]ethanol (36.00 g, 188 mmol) in DCM (400 ml) was add SOCl₂ (44.81 g, 376 mmol) at RT under N₂. The resulting mixture was stirred for 2 h at RT. The reaction was quenched with H₂O (500 ml). The mixture was extracted with DCM (3 x 500 ml). The combined organic layers were washed with brine (1 x 500 ml), dried over anhydrous Na₂SO₄. After filtration, the filtrate was concentrated under reduced pressure to afford the title compound (40 g, crude) as a yellow oil. ES+ m/z 210 [M+H]⁺.

### Preparation 122

### 4-(1-Chloroethyl)-2-isopropyl-triazole

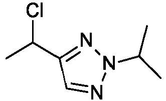

1-(2-Isopropyltriazol-4-yl)ethanol (344 mg, 2.22 mmol) was dissolved in SOCl₂ (3 mL) and toluene (3 ml), then refluxed for 1 h. The reaction was concentrated in vacuo to obtain the title compound as a brown oil (365 mg, quantitative). The material was immediately used in the next step.

The following compounds were prepared in a manner essentially analogous to the method of Preparation 122 using the appropriate reagents, adjusting reaction time to determine completion of the reaction, and adjusting the purification system as appropriate.

**Table 21**

| Prep # | Chemical Name | Structure | ¹H NMR (400 MHz, DMSO-d₆) δ |
|---|---|---|---|
| 123¹ | 4-(1-Chloroethyl)-1-isopropyl-triazole | 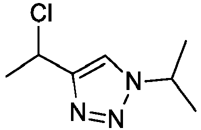 | 8.29 (s, 1H), 5.44 (q, J = 6.8 Hz, 1H), 4.80 (hept, J = 6.7 Hz, 1H), 1.84 (d, J = 6.8 Hz, 3H), 1.48 (d, J = 6.7 Hz, 5H) |
| 124^{2,3} | 4-(1-Chloroethyl)-1-methyl-pyrazolo[3,4-c]pyridine hydrochloride | 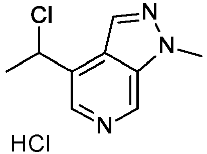 | 9.47 (s, 1H), 8.62 (s, 1H), 8.45 (s, 1H), 5.83 (q, J = 6.8 Hz, 1H), 4.27 (s, 3H), 2.00 (d, J = 6.9 Hz, 3H) |
| 125^{3,4} | 4-(1-Chloroethyl)-1-methyl-pyrrolo[2,3-c]pyridine hydrochloride | 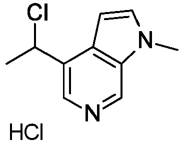 | 9.39 (s, 1H), 8.46 (s, 1H), 8.34 (d, J = 2.9 Hz, 1H), 7.14 (dd, J = 2.9, 0.6 Hz, 1H), 5.88 (q, J = 6.9 Hz, 1H) , 4.09 (s, 3H), 1.99 (d, J = 6.9 Hz, 3H) |
| 126^{2,3} | 4-(1-Chloroethyl)-7-fluoroisoquinoline hydrochloride | 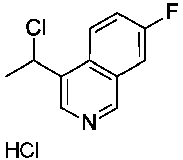 | 9.39 (s, 1H), 8.74 (s, 1H), 8.45 (dd, J = 9.3, 5.2 Hz, 1H), 8.08 (dd, J = 9.1, 2.7 Hz, 1H), 7.90 (td, J = 9.0, 2.7 Hz, 1H), 6.17 (q, J = 6.9 Hz, 1H), 2.05 (d, J = 6.8 Hz, 3H) |

| | | | |
|---|---|---|---|
| ¹ Reaction refluxed in a 1: 1 mixture SOCl₂:toluene. ² Reaction run in a 1:1 mixture SOCl₂:DCM at RT . ³ Reaction was evaporated and co-evaporated with toluene. ⁴ Reaction run in a 1:5 mixture SOCl₂:DCM at RT. | | | |

### Preparation 127

### 4-[1-Chloroethyl]-2-cyclopropyl-thiazole

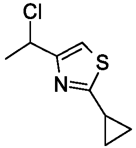

MsCl (0.16 ml, 2.1 mmol) was added dropwise to a soln of 1-(2-cyclopropylthiazol-4-yl)ethanol (320.5 mg, 1.76 mmol) and NEt₃ (0.49 ml, 3.5 mmol) in DCM (7 ml) cooled to 0 °C under N₂. The reaction was stirred at 0 °C for 1 h then the cooling bath was removed. After 3.5 h the reaction was re-cooled to 0 °C and quenched by slow addition of H₂O (5 ml). The layers were separated, and the aq layer was extracted with DCM (3 ml). The combined organics layers were washed with brine (5 ml), dried over Na₂SO₄, filtered, and concentrated to afford the title compound (316 mg, 95%) as a brown oil. ¹HNMR (400 MHz, CDCl₃): δ 7.03 (s, 1H), 5.18 (qd, J= 6.8, 0.6 Hz, 1H), 2.38-2.33 (m, 1H), 1.90 (d, J= 6.8 Hz, 3H), 1.18-1.15 (m, 4H).

The following compounds were prepared in a manner essentially analogous to the method of Preparation 127 using the appropriate reagents, adjusting reaction time to determine completion of the reaction, and adjusting the purification system as appropriate.

**Table 22**

| Prep # | Chemical Name | Structure | ¹H NMR (400 MHz, CDCl₃) δ |
|---|---|---|---|
| 128 | 4-(1-Chloropropyl)-2-methyl-thiazole | 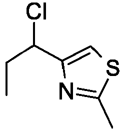 | 7.11 (d, J= 0.6 Hz, 1H), 4.96 (dd, J= 6.0, 7.9 Hz, 1H), 2.73 (3, 3H), 2.32-2.24 (m, 2H), 1.05 (t, J= 7.3 Hz, 3H) |
| 129 | 4-(1-Chloroethyl)-2-methoxy-thiazole | 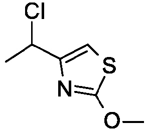 | 6.65 (d, J= 0.7 Hz, 1H), 5.04 (qd, J= 6.8, 0.6 Hz, 1H), 4.10 (s, 3H), 1.86 (d, J= 6.8 Hz, 3H) |

### Preparation 130

### 1-(3,6-Dimethylpyrazin-2-yl)ethyl methanesulfonate

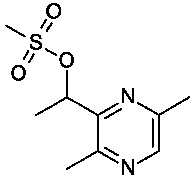

MsCl (903.09 mg, 7.88 mmol) was added dropwise at 0 °C to a stirred mixture of 1-(3,6-dimethylpyrazin-2-yl)ethanol (1 g, 6.57 mmol) and DIEA (127.38 mg, 0.99 mmol) in DCM (10 ml) under N₂ and stirred for 2 h. The reaction was removed from the cooling bath, quenched with H₂O (10 ml), and extracted with DCM (3 x 10 ml). The combined organic layers were washed with brine (3 x 10 ml), dried over Na₂SO₄, filtered, and concentrated to obtain the title compound (1.1 g, 73%) as a brown oil. MS ES+ *m*/*z 231* [M+H]⁺.

The following compounds were prepared in a manner essentially analogous to the method of Preparation 130 using the appropriate reagents, adjusting reaction time to determine completion of the reaction, and adjusting the purification system as appropriate.

**Table 23**

| Prep # | Chemical Name | Structure | MS ES+ m/z |
|---|---|---|---|
| 131 | [(1S)-1-(2-Pyridyl)ethyl] methanesulfonate | 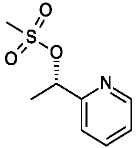 | 202 [M+H]⁺ |
| 132 | 1-[1-(Trifluoromethyl) pyrazol-3-yl]ethyl methanesulfonate | 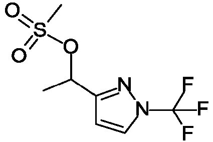 | a |
| 133 | 1-(4-Cyclopropyl-5-fluoro-2-pyridyl)ethyl methanesulfonate | 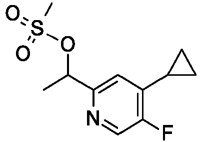 | a |
| 134 | 1-[5-(Difluoromethyl)-3-pyridyl]ethyl methanesulfonate | 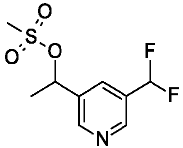 | 252 [M+H]⁺ |
| 135 | [Cyclopropyl-(5-fluoro-2-pyridyl)methyl] methanesulfonate | 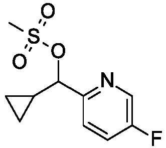 | a |
| 136 | 1-(6-Bromopyrazin-2-yl)ethyl methanesulfonate | 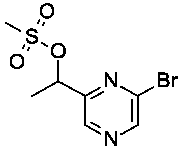 | (⁷⁹ Br/⁸¹Br) 281/283 [M+H]⁺ |
| 137 | [Cyclobutyl-(5-fluoro-2-pyridyl)methyl] methanesulfonate | 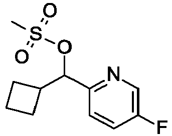 | a |
| 138 | 1-(2,5-Dimethylthiazol-4-yl)ethyl methanesulfonate | 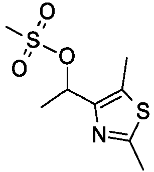 | a |
| 139 | 1-[6-(Trifluoromethyl)pyrazin-2-yl]ethyl methanesulfonate | 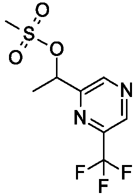 | 271 [M+H]⁺ |
| 140 | [2-Methyl-2-(2-pyridyl)propyl] methanesulfonate | 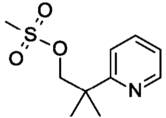 | a |

| | | | |
|---|---|---|---|
| ^{a} Material used in subsequent step without further characterization. | | | |

### Preparation 141

### 2-Bromo-1-(5-fluoro-2-pyridyl)pyridine

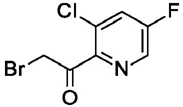

NBS (1.14 g, 6.38 mmol) was added in small portions over a 5-10 min period to a soln of 3-chloro-2-(1-ethoxyvinyl)-5-fluoro-pyridine (1.22 g, 6.06 mmol) in a mixture of THF (12 ml) and H₂O (3 ml) at 0° C. The reaction was stirred at 0° C for 1 hr then diluted with EA (50 ml), washed with H₂O (50 ml) and brine (50 ml), dried over MgSO₄, filtered, concentrated in vacuo. The residue was purified by silica gel chromatography eluting with EA in heptane to afford the title compound as a pale yellow oil (1.394 g, 91%). ¹H NMR (400 MHz, CDCl₃) δ (ppm): 8.44 (d, J = 2.3 Hz, 1H), 7.61 (dd, J = 7.9, 2.4 Hz, 1H), 4.69 (s, 2H).

The following compounds were prepared in a manner essentially analogous to the method of Preparation 141 using the appropriate reagents, adjusting reaction time to determine completion of the reaction, and adjusting the purification system as appropriate.

**Table 24**

| Prep # | Chemical Name | Structure | MS ES+ m/z |
|---|---|---|---|
| 142¹ | 2-Bromo-1-(4-chloro-5-fluoro-2-pyridyl)ethanone | 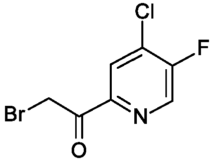 | (⁷⁹ Br/⁸¹Br) 251.8/253.9 [M+H]⁺ |

| | | | |
|---|---|---|---|
| ¹ Purified by silica gel chromatography eluting with EA in heptane. | | | |

### Preparation 143

### 2-Bromo-1-(3,5-difluoro-2-pyridyl)ethanone

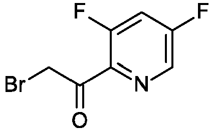

A soln of phenyltrimethylammonium bromide (51.36 g, 133 mmol) in THF (423 ml) was treated with a THF soln (181 mL) of 1-(3,5-difluoro-2-pyridyl)ethanone (20 g, 120.93 mmol) at RT under N₂. After stirring at 60 °C for 1.5 h, the mixture was cooled to RT and stirred for 1 h. The resulting suspension was filtered, and the solids were washed with THF (2 x 100 ml). The combined filtrates were concentrated to obtain a brown oil which was purified by silica gel chromatography eluting with a gradient of 10% to 50% DCM in cHex to obtain the title compound (15 g, 42%) as a yellow oil. MS ES+ *m*/*z* (⁷⁹Br/⁸¹Br) 237 [M+H]⁺.

The following compounds were prepared in a manner essentially analogous to the method of Preparation 143 using the appropriate reagents, adjusting reaction time to determine completion of the reaction, and adjusting the purification system as appropriate.

**Table 25**

| Prep # | Chemical Name | Structure | MS ES+ m/z |
|---|---|---|---|
| 144¹ | 2-Bromo-1-(3-chloro-5-fluoro-2-pyridyl)ethanone | 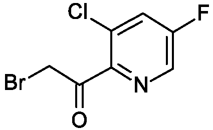 | 252/254 (⁷⁹Br/⁸¹Br) [M+H]⁺ |

| | | | |
|---|---|---|---|
| ¹ Purified by silica gel chromatography eluting with a gradient of 5% to 30% DCM in cHex. | | | |

### Preparation 145

### 2-Chloro-1-(5-fluoropyrimidin-2-yl)ethanone

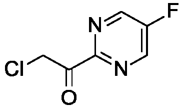

To a stirred solution of 2-bromo-5-fluoropyrimidine (3.0 g, 16.95) in toluene (30 ml) was added dropwise i-PrMgCl (1 M in THF)(10.17 mL, 20.340 mmol) at 0 °C under N₂. The reaction was stirred for 2 h at RT. To the above mixture was added 2-chloro-N-methoxy-N-methylacetamide (2.33 g, 16.95 mmol) dropwise over a 20 min period at 0 °C under N₂. The reaction was stirred for 1 h at RT then quenched with saturated aq NH₄Cl (200 ml). The mixture was extracted with EA (2 x 300 ml). The combined organic layers were washed with brine (2 x 300 ml), dried over Na₂SO₄, filtered and concetrated in vacuo to afford the title compound (2.3 g, 78%) as a yellow oil. MS ES+ m/z 175 [M+H]⁺.

### Preparation 146

### 6-Bromo-3-iodo-4-methoxy-pyrazolo[1,5-a]pyridine

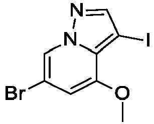

6-Bromo-4-methoxypyrazolo[1,5-a]pyridine (0.41 g, 1.81 mmol) and NIS (609 mg, 2.71 mmol) were dissolved in ACN (8 mL) and stirred at RT for 1 hr. The suspension was filtered, and the filtrate was concentrated in vacuo. The residue was purified by silica gel chromatography eluting with a linear gradient of 0% to 100% EA in heptane. The fractions containing the title compound was concentrated in vacuo and combined with the filtered solid to afford the title compound (0.60 g, 94.1 %). ES/MS *m*/*z* (⁷⁹Br/⁸¹Br) 352.6/354.6 [M+H]⁺.

### Preparation 147

### 6-Bromo-4-methoxy-3-(trifluoromethyl)pyrazolo[1,5-a]pyridine

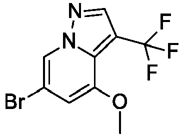

6-Bromo-3-iodo-4-methoxy-pyrazolo[1,5-a]pyridine (2 g, 5.67 mmol) was dissolved in DMF (28 ml) and treated with CuI (3.8 g, 20 mmol). N₂ was bubbled through the mixture for 5 min, then methyl 2,2-difluoro-2-(fluorosulfonyl)acetate (3.9 g, 20 mmol) was added, and the reaction was stirred at 80 °C for 2 h. Upon cooling to RT, the reaction was diluted with EA, filtered, and solids were washed with EA. The filtrate was diluted with H₂O and the layers were separated. The organic layer was dried over MgSO₄, filtered, and concentrated to obtain the title compound (582 mg, 29%) as a yellow solid. MS ES+ m/z 295/297 [M+H]⁺.

### Preparation 148

### 6-Bromo-3-fluoro-4-methoxy-pyrazolo[1,5-a]pyridine

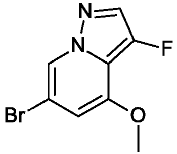

A soln of 6-bromo-4-methoxypyrazolo[1,5-a]pyridine (5 g, 22.021 mmol) and 1-chloromethyl-4-fluoro-1,4-diazoniabicyclo[2.2.2]octane bis(tetrafluoroborate) (7.04 g, 22.02 mmol) in ACN (50 ml) was stirred overnight at RT under N₂. The resulting mixture was diluted with H₂O (50 ml) and extracted with EA (3 x 100 ml). The combined organic layers were washed with brine (2 x 100 ml), dried over anhydrous Na₂SO₄, filtered, and concentrated. The residue was purified by reversed phase C18 flash chromatography with the following conditions: Column, C18; mobile phase, eluting with a gradient of 30% to 40% ACN in H₂O (0.1% FA); 254 nm to obtain the title compound (810 mg, 15%) as a yellow solid. ¹H NMR (300 MHz, DMSO-d₆) δ 8.56 (t, 1H), 8.04 (d, 1H), 6.77 (d, 1H), 3.97 (s, 3H).

### Preparation 149

### 6-Bromo-4-methoxy-3-methyl-pyrazolo[1,5-a]pyridine

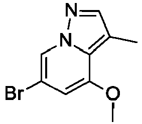

Et₃Si (8.0 ml, 50.20 mmol) was added dropwise to a solution of (6-bromo-4-methoxypyrazolo[1,5-a]pyridin-3-yl)methanol (6.45 ml, 25.10 mmol) in TFA (50 ml) at 0 °C. The reaction was allowed to warm to RT and stirred overnight. The reaction was concentrated in vacuo and the residue was dissolved in DCM, washed with saturated aq Na₂CO₃ (2x), dried over MgSO₄, filtered, and concentrated. The residue was purified by silica gel chromatography eluting with EA in heptane to afford the title compound as a white solid (3.81 g, 63%). MS ES+ m/z (³⁵Cl/³⁷Cl) 241/243 [M+H]⁺.

### Preparation 150

### 7-Chloro-5-methoxy-imidazo[1,2-a]pyridine

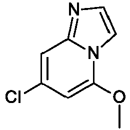

A soln of 4-chloro-6-methoxypyridin-2-amine (7.00 g, 44.14 mmol), chloroacetaldehyde (8.32 g, 52.99 mmol, 50%) and NaHCO₃ (11.12 g, 132.42 mmol) in n-BuOH (140 ml) was divided into fourteen batches and stirred overnight at 65 °C in sealed tubes. The soln was cooled to RT, diluted with H₂O (200 ml) and extracted with EA (3 x 200 mL). The organic layers were combined, dried over Na₂SO₄, and concentrated under reduced pressure. The residue was purified by silica gel chromatography eluting with 50% EA in PE to give the title compound as a light-brown solid (6.1 g, 76%). ES/MS m/z 183 [M+H]+.

### Preparation 151

### 6-Bromo-4-hydroxy-pyrazolo[1,5-a]pyridine-3-carbonitrile

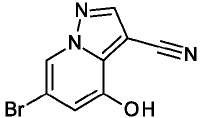

A suspension of 6-bromo-4-methoxy-pyrazolo[1,5-a]pyridine-3-carbonitrile (100 g, 396.72 mmol) in 1 L of DMA was treated with a soln of NaOH (31.7 g, 793 mmol) in H₂O (100 ml) in one portion at 40°C followed by treatment with NDM (161 g, 793 mmol). After stirring at 50°C for 1 h, the reaction was cooled to 0°C, quenched with 37% aq HCl (100 ml) to give a final pH of 5. The reaction was diluted with H₂O (4L) and the resulting solids were collected by filtration, washed with H₂O (1L) and heptane (500 ml) to obtain the title compound (85.5 g, 82%) as a beige solid. MS ES+ *m*/*z* 238 [M+H]⁺.

### Preparation 152

### 5,7-Dichloro-3-iodo-imidazo[1,2-a]pyridine

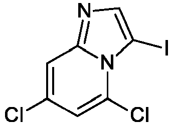

A soln of 5,7-dichloroimidazo[1,2-a]pyridine (5.00 g, 26.74 mmol) in DCM (50 ml) was treated in portions with NIS (12.03 g, 53.47 mmol) at RT under N₂. After stirring for 2 h at RT, the reaction was diluted with H₂O (100 ml) and extracted with DCM (3 x 50 ml). The combined organic layers were washed with brine (3 x 50 ml), dried over Na₂SO₄, filtered, and concentrated to obtain the title compound (12.0 g, crude) as a dark yellow solid. MS ES+ m/z 313 [M+H]⁺.

### Preparation 153

### 5,7-Dichloroimidazo[1,2-a]pyridine-3-carbonitrile

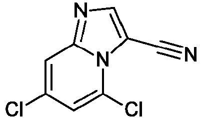

A DMF soln of 5,7-dichloro-3-iodo-imidazo[1,2-a]pyridine (11.00 g, 35.15 mmol) was treated in portions with CuCN (6.30 g, 70.31 mmol) at RT under N₂. After stirring at 100 °C for 2 h, the reaction was allowed to cool to RT, treated dropwise with NH₄OH (100 ml) over 5 min, diluted with H₂O (300 ml), and stirred at RT for 2 h. The reaction was extracted with DCM (3 x 400 mL), and the combined organic layers were washed with brine (3 x 300 mL), dried over Na₂SO₄, filtered, and concentrated. The residue was purified by silica gel chromatography eluting with PE / EA (5:1 to 1:2) to obtain the title compound (3.5 g, 47%) as a yellow solid. MS ES+ m/z (³⁵Cl/³⁷Cl) 212/214 [M+H]⁺.

### Preparation 154

### 7-Chloro-5-[2-(3,5-difluoro-2-pyridyl)-2-methoxy-ethoxy]imidazo[1,2-a]pyridine

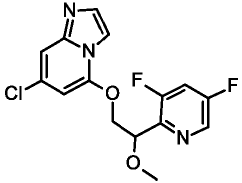

A stirred mixture of 2-(3,5-difluoro-2-pyridyl)-2-methoxy-ethanol (6.57 g, 34.76 mmol) and 5,7-dichloroimidazo[1,2-a]pyridine (6.5 g, 34.76 mmol) in THF (120 ml) was treated with t-BuOK (48.66 ml, 48.66 mmol, 1M in THF) dropwise at 0 °C under N₂. After stirring at RT for 2 h, the reaction was quenched with saturated aq NH₄Cl, extracted with EA (2 x 500 ml), washed with brine (2 x 200 ml), dried over Na₂SO₄, filtered, and concentrated. The residue was purified by reversed phase C18 flash chromatography eluting with a gradient of 40% to 50% ACN in H₂O to afford the title compound as a light-yellow solid (1.75 g, 15%). MS ES+ m/z 340 [M+H]⁺.

### Preparation 155

### 6-bromo-4-[1-[5-(trifluoromethyl)-3-pyridyl]ethoxy]pyrazolo[1,5-a]pyridine-3-carbonitrile, Isomer 2

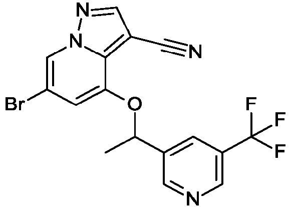

To 6-bromo-4-hydroxypyrazolo[1,5-a]pyridine-3-carbonitrile (45.43 g, 190.84 mmol) in DMF (350 ml) were added K₂CO₃ (79.13 g, 572.52 mmol) and 3-(1-chloroethyl)-5-(trifluoromethyl)pyridine (40 g, crude) in portions over 2 min at 80 °C. The reaction was stirred for 2 h at RT under N₂. The resulting mixture was diluted with H₂O (1L), extracted with EA (3 x 1.5 L). The combined organic layers were washed with brine (5 x 1L), dried over Na₂SO₄, and filtered. The filtrate was concentrated. The residue was purified by silica gel column chromatography, eluting with PE / EA (10:1 to 2:1) to afford the racemate of the title compound (32 g, 40.78%) as a yellow solid. MS ES+ m/z (⁷⁹Br/⁸¹Br) 411/413 [M+H]⁺.

The racemate of the title compound (32 g) was subjected to the following chromatography conditions: SFC, Column: NB_CHIRAL ART Cellulose-SB; 5 x 25 cm, 5 µm, eluting with 15% IPA (0.2% DEA) in CO₂ to afford the title compound, Isomer 2 (13.5 g, 42.19%), t_{R} is 6.58 min with 99% ee. MS ES+ m/z (⁷⁹Br/⁸¹Br) 411/413 [M+H]⁺.

### Preparation 156

### 6-Bromo-4-[(1R)-1-(2-pyridyl)ethoxy]pyrazolo[1,5-a]pyridine-3-carbonitrile tosylate

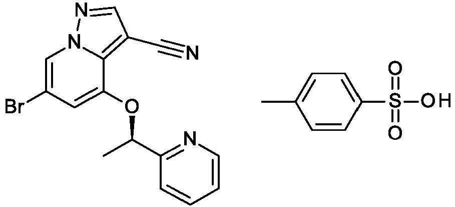

A THF soln (300 ml) of PPh₃ (34.1 g, 130 mmol) was treated dropwise over 4 min with DIAD (23.2 g, 115 mmol) at RT. After stirring at RT for 30 min, a THF soln (100 ml) of 6-bromo-4-hydroxy-pyrazolo[1,5-a]pyridine-3-carbonitrile (20 g, 76.5 mmol) and (1S)-1-(2-pyridyl)ethanol (11.3 g, 91.8 mmol) was added dropwise over 6 min. After stirring at RT for 45 min, the reaction was concentrated in vacuo. The residue was slurried in hexane / MTBE (4:1; 250 ml) and allowed to sit overnight at RT. The resulting solid was collected by filtration, washed with hexanes (2 x 100 ml) and purified by silica gel chromatography eluting with a gradient of 0% to 15% acetone in hexanes. The resulting solid was dissolved in MTBE (100 mL) and treated with 4-methylbenznesulfonic acid hydrate (8.85 g, 46.5 mmol). The resulting suspension was stirred overnight at RT. The solid was collected by filtration and washed with MTBE (3 x 50 mL) to obtain the title compound (43.4 g, 87%) as a white solid. MS ES+ (⁷⁹Br/⁸¹Br) m/z 343/345 [M+H]⁺.

### Preparation 157

### 6-Bromo-3-chloro-4-[2-(5-fluoropyrimidin-2-yl)-2-methoxy-ethoxy]pyrazolo[1,5-a]pyridine

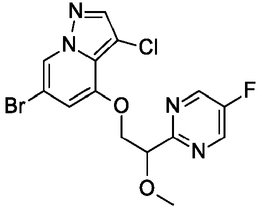

To PPh₃ (3.50 g, 13.34 mmol) in THF (10 ml) was added dropwise DIAD (2.52 g, 12.45 mmol) at 0 °C under N₂. The reaction was stirred for 0.5 h at 0 °C. Next, 6-bromo-3-chloropyrazolo[1,5-a]pyridin-4-ol (1.1 g, 4.45 mmol) and 2-(5-fluoropyrimidin-2-yl)-2-methoxy-ethanol (918.26 mg, 5.334 mmol, 1.2 equiv) in THF (10 mL) were added at RT. The reaction was stirred at RT for 2 h before being concentrated in vacuo. The residue was purified by reverse flash chromatography eluting with the following conditions: column, C18; mobile phase, 50% to 55% ACN in H₂O to afford the title compound (700 mg, 39%) as a yellow solid. MS ES+ m/z 402/403 [M+H]⁺.

### Preparation 158

### 6-Bromo-4-[2-(5-fluoro-2-pyridyl)-2-oxo-ethoxy]pyrazolo[1,5-a]pyridine-3-carbonitrile

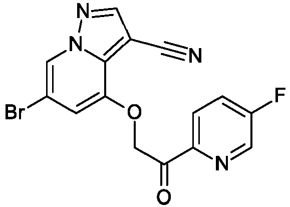

A mixture of 6-bromo-4-hydroxy-pyrazolo[1,5-a]pyridine-3-carbonitrile (4 g, 16.80 mmol) and Cs₂CO₃ (8.21 g, 25.2 mmol) in ACN (20 ml) was stirred at RT under N₂ for 0.5 h. A soln of 2-bromo-1-(5-fluoro-2-pyridyl)ethanone (4.6 g, 20 mmol) in ACN (20 ml) was added and the reaction was allowed to stir at RT for 1 h. The reaction was diluted with H₂O (100 ml) that resulted in a suspension. The solid was collected by filtration and washed with H₂O to obtain the title compound (5.86 g, 93%) as a white solid. MS ES+ m/z 375/377 [M+H]⁺.

The following compounds were prepared in a manner essentially analogous to the method of Preparation 158 using the appropriate reagents, adjusting reaction time to determine completion of the reaction, and adjusting the purification system as appropriate.

**Table 26**

| Prep # | Chemical Name | Structure | MS ES+ m/z |
|---|---|---|---|
| 159^{1, 2} | 2-(6-Bromopyrazolo[1,5-a]pyridin-4-yl)oxy-1-(5-fluoro-2-pyridyl)ethanone | 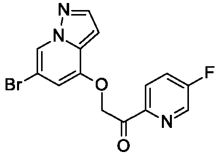 | (⁷⁹Br/⁸¹Br) 350/352 [M+H]⁺ |
| 160^{1,3,4} | 2-(6-Bromo-3-chloro-pyrazolo[1,5-a]pyridin-4-yl)oxy-1-(5-fluoro-2-pyridyl)ethanone | 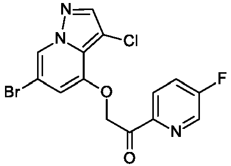 | (⁷⁹Br/⁸¹Br) 384/386 [M+H]⁺ |
| 161^{1,5,6} | 2-(6-Bromo-3-chloro-pyrazolo[1,5-a]pyridin-4-yl)oxy-1-(5-fluoropyrimidin-2-yl)ethanone | 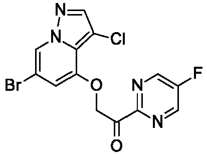 | (⁷⁹Br/⁸¹Br) 385/387 [M+H]⁺ |

| | | | |
|---|---|---|---|
| ¹ DIPEA used as base for this transformation. ² Workup: Reaction was concentrated in vacuo. Residue dissolved into MeOH and triturated with H₂O. Precipitated product was collected by filtration. ³ Reaction was heated at 80 °C. ⁴ Workup: Reaction was concentrated in vacuo. Residue triturated in H₂O. Precipitated product was collected by filtration. ⁵ Workup: Reaction was diluted with H₂O, extracted with EA, dried over Na₂SO₄, filtered and concentrated in vacuo. ⁶ Purified by silica gel chromatography eluting with EA in DCM (1:30 to 1:15). | | | |

### Preparation 162

### 1-(6-Bromopyrazolo[1,5-a]pyridin-4-yl)oxy-2-(5-fluoro-2-pyridyl)propan-2-ol

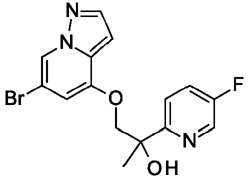

A soln of 2-(6-bromopyrazolo[1,5-a]pyridin-4-yl)oxy-1-(5-fluoro-2-pyridyl)ethenone (3 g, 8.57 mmol) in THF (10 ml) was added to a stirred soln of MeMgBr (3M in Et₂O, 14.3 ml, 42.84 mmol) in THF (30 ml) at RT under N₂ and the reaction was allowed to stir for 2 h. The soln was quenched with H₂O (50 ml) and extracted with EA (3 x 50 ml). The combined organic layers were washed with brine (3 x 50 ml), dried over Na₂SO₄, filtered, and concentrated in vacuo to obtain the title compound (3 g, crude) as a brown oil. MS ES+ m/z (⁷⁹Br/⁸¹Br) 366/368 [M+H]⁺.

The following compounds were prepared in a manner essentially analogous to the method of Preparation 162 using the appropriate reagents, adjusting reaction time to determine completion of the reaction, and adjusting the purification system as appropriate.

**Table 27**

| Prep # | Chemical Name | Structure | MS ES+ m/z |
|---|---|---|---|
| 163^{1,2} | 1-(6-Bromo-3-chloro-pyrazolo[1,5-a]pyridin-4-yl)oxy-2-(5-fluoro-2-pyridyl)propan-2-ol | 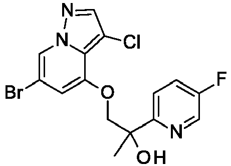 | (⁷⁹Br/⁸¹Br) 400/402 [M+H]⁺ |
| 164³ | 1-(6-Bromo-3-chloro-pyrazolo[1,5-a]pyridin-4-yl)oxy-2-(5-fluoropyrimidin-2-yl)propan-2-ol | 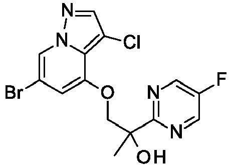 | (⁷⁹Br/⁸¹Br) 401/403 [M+H]⁺ |

| | | | |
|---|---|---|---|
| ¹ Reaction quenched with sat. aq NH4Cl (30 ml) at 0°C. ² Purified by silica gel chromatography eluting with DCM / PE (1:2 - 2: 1). ³ Purified by Prep-TLC PE / EA (8:1). | | | |

### Preparation 165

### 6-Bromo-4-[2-(5-fluoro-2-pyridyl)-2-hydroxy-ethoxy]pyrazolo[1,5-a]pyridine-3-carbonitrile

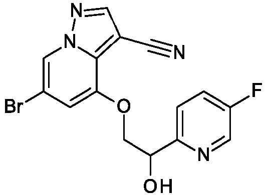

A suspension of 6-bromo-4-[2-(5-fluoro-2-pyridyl)-2-oxo-ethoxy]pyrazolo[1,5-a]pyridine-3-carbonitrile (2.18 g, 5.82 mmol) in MeOH (50 ml) was cooled in an ice bath and then treated with NaBH₄ (0.85 g, 22.47 mmol) in one portion. The reaction was allowed to stir at 0 °C for 5 min and then stirred at RT for 1 h. The reaction was concentrated, and the residue taken up in DCM and washed with H₂O and brine. The organic layer was collected, dried over MgSO₄, filtered, and concentrated to obtain the title compound (1.98 g, 83%) as a beige solid. MS ES+ m/z 377/379 [M+H]⁺.

The following compounds were prepared in a manner essentially analogous to the method of Preparation 165 using the appropriate reagents, adjusting reaction time to determine completion of the reaction, and adjusting the purification system as appropriate.

**Table 28**

| Prep # | Chemical Name | Structure | MS ES+ m/z |
|---|---|---|---|
| 166¹ | 2-(6-Bromo-3-chloro-pyrazolo[1,5-a]pyridin-4-yl)oxy-1-(5-fluoro-2-pyridyl)ethanol | 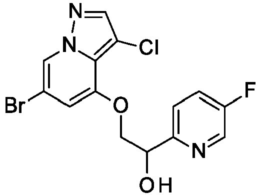 | (⁷⁹Br/⁸¹Br) 385/387 [M+H]⁺ |
| 167 | 2-(6-Bromo-3-chloro-pyrazolo[1,5-a]pyridin-4-yl)oxy-1-(5-fluoropyrimidin-2-yl)ethanol | 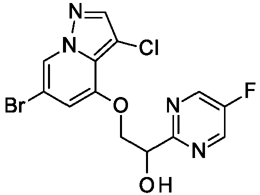 | (⁷⁹Br/⁸¹Br) 387/389 [M+H]⁺ |

| | | | |
|---|---|---|---|
| ¹ Workup: Reaction was concentrated in vacuo. Residue triturated in H₂O and the precipitate was collected by filtration. | | | |

### Preparation 168

### 6-Bromo-3-chloro-4-[2-(5-fluoro-2-pyridyl)-2-methoxy-ethoxy]pyrazolo[1,5-a]pyridine

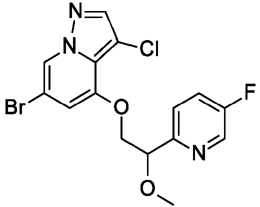

To 2-(6-bromo-3-chloro-pyrazolo[1,5-a]pyridin-4-yl)oxy-1-(5-fluoro-2-pyridyl)ethanol (1.62 g, 4.19 mmol) in THF (10 ml) was added NaH (335 mg, 8.38 mmol, 60%) in portions at 0 °C under N₂. To the mixture was added CH₃I (2.97 g, 20.95 mmol) dropwise at 0 °C. The reaction was stirred for 2 h at RT then quenched with H₂O (50 ml). The mixture was extracted with EA (3 x 100 ml), organic layers were combined, washed with brine (3 x 50 ml), dried over Na₂SO₄, filtered and concetrated in vacuo. The residue was purified by silica gel column chromatography, eluting with PE / EA (10:1 to 5:1 to afford the title compound (1.01 g, 60%) as a light yellow solid. MS ES+ m/z 402 [M+H]⁺.

### Preparation 169

### 6-Bromo-4-[2-(5-fluoro-2-pyridyl)-2-methoxy-ethoxy]pyrazolo[1,5-a]pyridine-3-carbonitrile

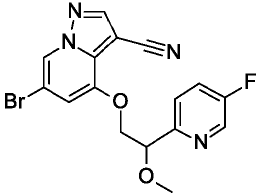

To a stirred solution of 6-bromo-4-[2-(5-fluoro-2-pyridyl)-2-hydroxy-ethoxy]pyrazolo[1,5-a]pyridine-3-carbonitrile (3 g, 7.95 mmol) and CH₃I (2.26 g, 15.91 mmol) in THF (30 mL) was added NaH (60%) (636 mg, 15.91 mmol) in portions at 0 °C under N₂. The mixture was stirred for 2 hr at RT then quenched with H₂O (20 mL). The mixture was extracted with EA (3 x 40 mL). The combined organic layers were washed with brine (2 x 20 mL), dried over Na₂SO₄, filtered, and concentrated in vacuo. The residue was purified by silica gel column chromatography, eluted with PE / EA (4:1 to1:1) to afford the title compound (2.9 g, 93%) as a yellow solid. MS ES+ m/z (⁷⁹Br/⁸¹Br) 391.0/393.0 [M+H]⁺.

### Preparation 170

### 7-Chloro-5-[[3,3,3-trifluoro-2-(5-fluoro-2-pyridyl)propyl]amino]imidazo[1,2-a]pyridine-3-carbonitrile

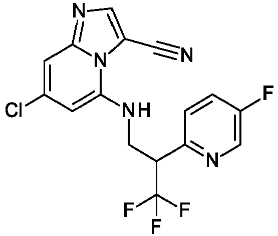

A stirred soln of 3,3,3-Trifluoro-2-(5-fluoro-2-pyridyl)propan-1-amine (2.0 g, 9.6 mmol) and 5,7-dichloroimidazo[1,2-a]pyridine-3-carbonitrile (2.64 g, 12.5 mmol) in DMF (5 ml) was treated with DIEA (3.72 g, 28.8 mmol) under N₂. After stirring at 100 °C for 24 h, the reaction was purified by reversed flash C18 chromatography with the following conditions: Column, C18; eluting with a gradient of 50% to 70% ACN in H₂O to afford the title compound (1.2 g, 34%). MS ES+ m/z 384 [M+H]⁺.

### Preparation 171

### 6-Bromo-4-[1-[5-(trifluoromethyl)-3-pyridyl]ethylamino]pyrazolo[1,5-a]pyridine-3-carbonitrile

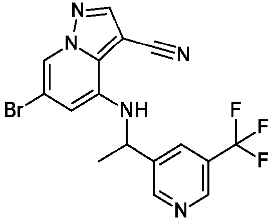

A pressure flask was charged with Pd₂(dba)₃ (0.05 g, 0.05 mmol), dppf (0.06 g, 0.1 mmol), K₃PO₄ (1.3 g, 6 mmol), and DME (5 ml). The suspension was degassed by bubbling N₂ through the mixture for several min then 1-[5-(trifluoromethyl)-3-pyridyl]ethanamine (0.40 g, 1.89 mmol) and 4,6-dibromopyrazolo[1,5-a]pyridine-3-carbonitrile (0.52 g, 1.7 mmol) were added and N₂ was bubbled through the mixture for another couple of min. The tube was capped, and the reaction heated at 207°C for 15 min. After stirring overnight at RT, the reaction was recharged with DME (5 mL), Pd₂(dba)₃ (0.05 g, 0.05 mmol), and dppf (0.06 g, 0.1 mmol). After degassing the mixture, the reaction was capped and heated at 100 °C for 8 h. The reaction was cooled to RT, diluted with H₂O, and extracted with EA (3x). The combined organic layers were dried over MgSO₄, filtered, and concentrated. The residue was purified by silica gel chromatography eluting with a gradient of 0% to 50% EA in cHex to obtain the title compound (393 mg) which was used in the next synthetic reaction without further purification. MS ES+ m/z 410/412 [M+H]⁺.

The following compounds were prepared in a manner essentially analogous to the method of Preparation 171 using the appropriate reagents, adjusting reaction time to determine completion of the reaction, and adjusting the purification system as appropriate.

**Table 29**

| Prep # | Chemical Name | Structure | MS ES+ m/z |
|---|---|---|---|
| 172¹ | 6-Bromo-4-[[2-(5-fluoro-2-pyridyl)-2-methoxyethyl]amino]pyrazolo[1,5-a]pyridine-3-carbonitrile | 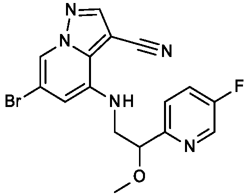 | (⁷⁹Br/⁸¹Br) 390/392 [M+H]⁺ |
| 173² | 6-Bromo-4-[3-hydroxy-3-(2-pyridyl)pyrrolidin-1-yl]pyrazolo[1,5-a]pyridine-3-carbonitrile | 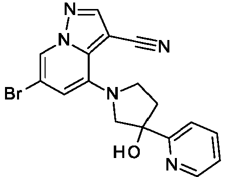 | (⁷⁹Br/⁸¹Br) 384/386 [M+H]⁺ |

| | | | |
|---|---|---|---|
| ¹ Purified by silica gel chromatography eluted with 0% to 100% EA in cHex. ² Purified by silica gel chromatography eluting with 0% to 50% EA in cHex. | | | |

### Preparation 174

### 6-(4,4,5,5-Tetramethyl-1,3,2-dioxaborolan-2-yl)-4-[1-[5-(trifluoromethyl)-3-pyridyl]ethylamino]pyrazolo[1,5-a]pyridine-3-carbonitrile

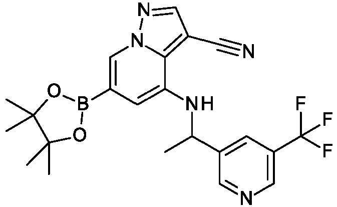

A high-pressure flask was charged with 6-bromo-4-[[2-(5-fluoro-2-pyridyl)-2-methoxy-ethyl]amino]pyrazolo[1,5-a]pyridine-3-carbonitrile (0.39 g, 0.55 mmol), bis(pinacolato)diboron (0.21 g, 0.82 mmol), KOAc (0.21 g, 2.18 mmol), [1,1'-bis(diphenylphosphino)ferrocene] dichloropalladium(II) (0.01 g, 0.01 mmol), and 1,4-dioxane (4 ml). N₂ was bubbled through the mixture for a few minutes, the flask was then closed, and the reaction was heated at 90 °C for 5.5 h. After cooling to RT, the reaction was diluted with H₂O and extracted with EA (3x). The combined organic layers were dried over MgSO4, filtered, concentrated, and the residue purified by silica gel chromatography eluting with 0% to 80% EA in cHex to obtain the title compound (0.39 g, 82%) as a thick brown oil. MS ES+ m/z 376 [M+H]⁺ as the boronic acid.

The following compounds were prepared in a manner essentially analogous to the method of Preparation 174 using the appropriate reagents, adjusting reaction time to determine completion of the reaction, and adjusting the purification system as appropriate.

**Table 30**

| Prep # | Chemical Name | Structure | MS ES+ m/z |
|---|---|---|---|
| 175 | 4-[[2-(5-Fluoro-2-pyridyl)-2-methoxyethyl]amino]-6-(4,4,5,5-tetramethyl-1,3,2-dioxaborolan-2-yl) pyrazolo[1,5-a]pyridine-3-carbonitrile | 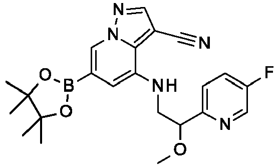 | 438 [M+H]⁺ |
| 176 | 4-[3-Hydroxy-3-(2-pyridyl)pyrrolidin-1-yl]-6-(4,4,5,5-tetramethyl-1,3,2-dioxaborolan-2-yl)pyrazolo[1,5-a]pyridine-3-carbonitrile | 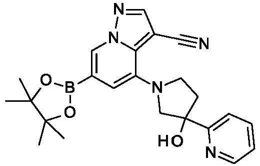 | 432 [M+H]⁺ |

### Preparation 177

### 4-Methoxy-6-(4,4,5,5-tetramethyl-1,3,2-dioxaborolan-2-yl)pyrazolo[1,5-a]pyridine

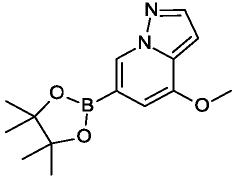

A soln of 6-bromo-4-methoxy-pyrazolo[1,5-a]pyridine (50.0 g, 220.2 mmol) and 4,4,5,5-tetramethyl-2-(4,4,5,5-tetramethyl-1,3,2-dioxaborolan-2-yl)-1,3,2-dioxaborolane (61.0 g, 240.3 mmol) in 1,4-dioxane (500 ml) was degassed with N₂ at RT for 15 min. KOAc (64.0 g, 652.1 mmol) was added followed by the addition of Pd(dppf)Cl₂ (6.0 g, 8.2 mmol) in portions at 25 °C under N₂. Mixture was degassed with N₂ for 15 min. The reaction was stirred at 80 °C for 12 h under N₂. The reaction was cooled to 25 °C and filtered through DE. Filter cake was washed with 1,4-dioxane (3 x 100 ml). The filtrate was concentrated in vacuo at 45 °C to afford an oily black residue. The residue was purified by silica gel plug filtration (300 g, 12 cm column diameter). The plug was eluted with 20:1 to 3:1 c-Hex: EA to afford the title compound (62.7 g, 206 mmol) as a pale-yellow solid. MS ES+ m/z 275 [M+H]⁺.

### Preparation 178

### 4-Methoxy-6-(4,4,5,5-tetramethyl-1,3,2-dioxaborolan-2-yl)pyrazolo[1,5-a]pyridine-3-carbonitrile

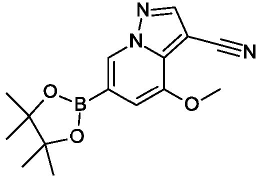

A stirred 1,4-dioxane soln (200 ml) of 6-bromo-4-methoxy-pyrazolo[1,5-a]pyridine-3-carbonitrile (20.0 g, 79.34 mmol) was treated with bis(pinacolato)diboron (30.22 g, 119.01 mmol), KOAc (23.36 g, 238.03 mmol), and Pd(dppf)Cl₂ (2.90 g, 3.97 mmol) at RT under N₂. After stirring the reaction at 80 °C for 1 h, the crude reaction was taken on to the next synthetic step without purification. MS ES+ *m*/*z* 300 [M+H]⁺.

The following compounds were prepared in a manner essentially analogous to the method of Preparation 178 using the appropriate reagents, adjusting reaction time to determine completion of the reaction, and adjusting the purification system as appropriate.

**Table 31**

| Prep # | Chemical Name | Structure | MS ES+ m/z |
|---|---|---|---|
| 179 | 3-Fluoro-4-methoxy-6-(4,4,5,5-tetramethyl-1,3,2-dioxaborolan-2-yl)pyrazolo[1,5-a]pyridine | 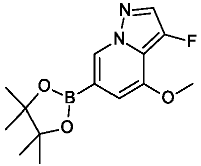 | 293 [M+H]⁺ |
| 180a | (5-Methoxyimidazo[1,2-a] pyridine-7-yl)boronic acid | 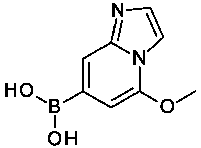 | 193 [M+H]⁺ |
| 181³ | 4-[(1R)-1-(2-Pyridyl)ethoxy]-6-(4,4,5,5-tetramethyl-1,3,2-dioxaborolan-2-yl)pyrazolo [1,5-a]pyridine-3-carbonitrile | 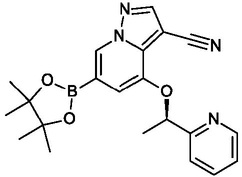 | 391 [M+H]⁺ |
| 182^{1,2} | 6-(4,4,5,5-Tetramethyl-1,3,2-dioxaborolan-2-yl)-4-[1-[5-(trifluoromethyl)-3-pyridyl]ethoxy]pyrazolo[1,5-a]pyridine-3-carbonitrile, Isomer 2 | 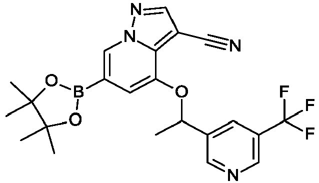 | 459 [M+H]⁺ |
| 183⁴ | 4-Methoxy-6-(4,4,5,5-tetramethyl-1,3,2-dioxaborolan-2-yl)-3-(trifluoromethyl)pyrazolo [1,5-a]pyridine | 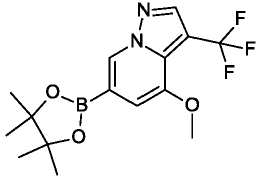 | 261 [M+H]⁺ |
| 184 | 4-[2-(5-Fluoro-2-pyridyl)-2-hydroxy-ethoxy]-6-(4,4,5,5-tetramethyl-1,3,2-dioxaborolan-2-yl)pyrazolo[1, 5-a]pyridine-3-carbonitrile | 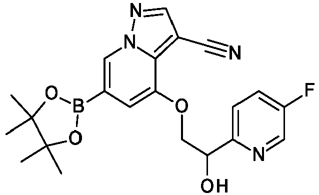 | a |
| 185 | 2-(5-Fluoro-2-pyridyl)-1-[6-(4,4,5,5-tetramethyl-1,3,2-dioxaborolan-2-yl)pyrazolo[1,5-a]pyridin-4-yl]oxy-propan-2-ol | 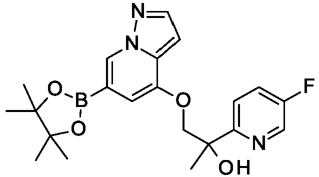 | a |
| 186⁵ | [5-[2-(3,5-Difluoro-2-pyridyl)-2-methoxy-ethoxy]imidazo[1,2-a]pyridin-7-yl]boronic acid | 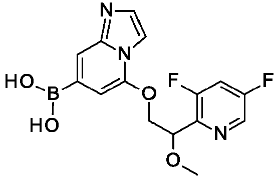 | 350 [M+H]⁺ |
| 187^{6,7} | 1-[3-Chloro-6-(4,4,5,5-tetramethyl-1,3,2-dioxaborolan-2-yl)pyrazolo[1,5-a]pyridin-4-yl]oxy-2-(5-fluoro-2-pyridyl)propan-2-ol | 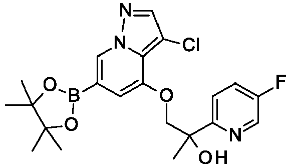 | 448 [M+H]⁺ |
| 188⁸ | 4-Methoxy-3-methyl-6-(4,4,5,5-tetramethyl-1,3,2-dioxaborolan-2-yl)pyrazolo[1,5-a]pyridine | 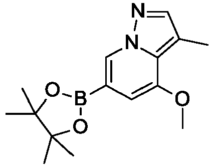 | 289 [M+H]⁺ |

| | | | |
|---|---|---|---|
| ¹ Purified by silica gel chromatography eluting with 0% to 50% EA in cHex. ² Purified by silica gel chromatography eluting with 0% to 80% EA in cHex. ³ Compound degrades on MS. MS data indicates mixture of boronate ester and boronic acid. ⁴ Compound degrades on MS. MS data consistent for boronic acid. ⁵ Xphos Palladacycle Gen 4 used as catalyst for this transformation. ⁶ Pd(dppf)Cl₂.CH₂Cl₂ used as catalyst for this transformation. ⁷ Reaction was heated at 100 °C. ^{a} Material used in subsequent step without further characterization. ⁸ Purified by silica gel chromatography eluting with EA in heptane. | | | |

### Preparation 189

### tert-Butyl (3S,4R)-4-[4-[3-chloro-4-[2-(5-fluoropyrimidin-2-yl)-2-methoxy-ethoxy]pyrazolo[1,5-a]pyridin-6-yl]-5-methyl-triazol-1-yl]-3-fluoro-piperidine-1-carboxylate

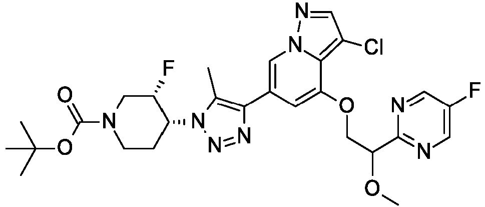

To 6-bromo-3-chloro-4-[2-(5-fluoropyrimidin-2-yl)-2-methoxy-ethoxy]pyrazolo[1,5-a]pyridine (626 mg, 1.56 mmol) and bis(pinacolato)diboron (475 mg, 1.87 mmol) in dioxane (7 ml) were added Pd(dppf)Cl₂ · CH₂Cl₂ (64 mg, 0.08 mmol) and KOAc (459 mg, 4.68 mmol) in portions at RT under N₂. The reaction was stirred for 2 h at 100 °C under N₂ then allowed to cool down to RT. Next, tert-butyl (3 S,4R)-4-(4-bromo-5-methyl-triazol-1-yl)-3-fluoro-piperidine-1-carboxylate (534 mg, 1.47 mmol), K₂CO₃ (554 mg, 4.01 mmol), PdCl₂(DtBPF) (44 mg, 0.07 mmol) and H₂O (2 ml) were added at RT under N₂. The mixture was stirred overnight at 100 °C. Upon cooling to RT, the reaction was quenched by the addition of H₂O (100 ml). The mixture was extracted with EA (3 x 100 ml), organic layers were combined, dried over Na₂SO₄, filtered and the filrtate concentrated in vacuo. The residue was purified by silica gel chromatography, eluting with PE / EA (10:1 to 100% EA). To afford the title compound (585 mg, 72%) as a brown solid. MS ES+ *m*/*z* 605 [M+H]⁺.

The following compounds were prepared in a manner essentially analogous to the method of Preparation 189 using the appropriate reagents, adjusting reaction time to determine completion of the reaction, and adjusting the purification system as appropriate.

**Table 32**

| Prep # | Chemical Name | Structure | MS ES+ m/z |
|---|---|---|---|
| 190¹ | tert-Butyl (3S,4R)-4-[4-[3-chloro-4-[2-(5-fluoropyrimidin-2-yl)-2-hydroxy-propoxy]pyrazolo[1, 5-a]pyridin-6-yl]-5-methyl-triazol-1-yl]-3-fluoro-piperidine-1-carboxylate | 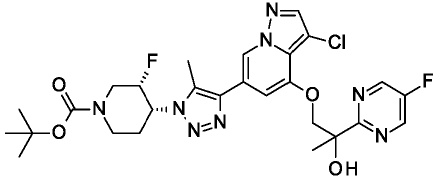 | 605 [M+H]⁺ |
| 191² | tert-Butyl (3S,4R)-4-[4-[3-chloro-4-[2-(5-fluoro-2-pyridyl)-2-methoxy-ethoxy]pyrazolo[1,5-a]pyridin-6-yl]-5-methyl-triazol-1-yl]-3-fluoro-piperidine-1-carboxylate | 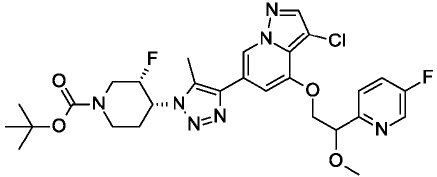 | 604 [M+H]⁺ |
| 192³ | tert-Butyl (3S,4R)-4-[4-[3-chloro-4-[2-(5-fluoropyrimidin-2-yl)-2-hydroxy-ethoxy]pyrazolo[1,5-a]pyridin-6-yl]-5-methyl-triazol-1-yl]-3-fluoro-piperidine-1-carboxylate | 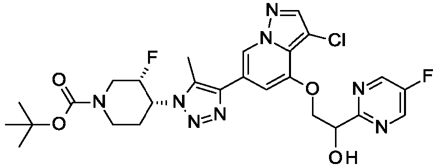 | 591 [M+H]⁺ |
| 193⁴ | tert-Butyl (3S,4R)-4-[4-[3-cyano-4-[2-(5-fluoro-2-pyridyl)-2-methoxy-ethoxy]pyrazolo[1,5-a]pyridin-6-yl]-5-methyl-triazol-1-yl]-3-fluoro-piperidine-1-carboxylate | 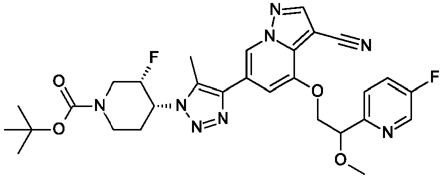 | 595 |

| | | | |
|---|---|---|---|
| ¹ Purified by silica gel chromatography, eluted with PE / EA (2:3 to 1:4). ² Purified by silica gel chromatography, eluted with PE / EA (1:3 to 1:4). ³ Purified by silica gel chromatography, eluted with PE / EA (1:2 to 1:3). ⁴ Purified by silica gel chromatography, eluted with PE / EA (1:2 to 1:3). | | | |

### Preparation 194

### tert-Butyl 4-[4-(3-cyano-4-methoxy-pyrazolo[1,5-a]pyridine-6-yl)-5-methyl-triazol-1-yl]piperidine-1-carboxylate

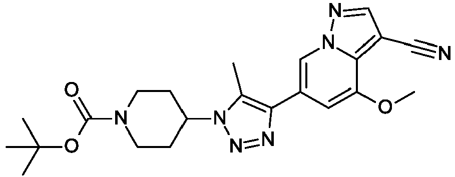

The mixture obtained from the synthesis of 4-methoxy-6-(4,4,5,5-tetramethyl-1,3,2-dioxaborolan-2-yl)pyrazolo[1,5-a]pyridine-3-carbonitrile (20.0 g, 79.34 mmol) was treated with tert-butyl 4-(4-bromo-5-methyl-triazol-1-yl)piperidine-1-carboxylate (27.70 g, 80.23 mmol), K₂CO₃ (27.72 g, 200.57 mmol), PdCl₂(DtBPF) (2.18 g, 3.34 mmol), and H₂O (50 mL) at RT under N₂. The reaction was stirred at 80 °C for 1 h and allowed to cool to RT. The reaction was diluted with H₂O (300 ml) and extracted with EA (3 x 300 ml). The combined organic layers were washed with brine (3 x 300 ml), dried over anhydrous Na₂SO₄, filtered, and concentrated to obtain the title compound (15.31 g, 44% two step yield) as a yellow solid. MS ES+ *m*/*z* 438 [M+H]⁺.

### Preparation 195

### tert-Butyl 4-[4-(4-methoxypyrazolo[1,5-a]pyridine-6-yl)-5-methyl-triazol-1-yl]piperidine-1-carboxylate

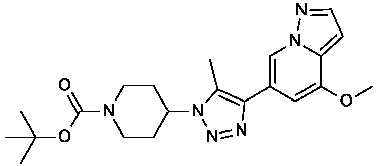

To 4-methoxy-6-(4,4,5,5-tetramethyl-1,3,2-dioxaborolan-2-yl)pyrazolo[1,5-a]pyridine (5.160 g, 18.83 mmol) and K₂CO₃ (5.0 g, 36.18 mmol) was added a soln of tert-butyl 4-(4-bromo-5-methyl-triazol-1-yl)piperidine-1-carboxylate (7.0 g, 20.28 mmol) in toluene (50 ml) followed by the addition of H₂O (12.5 ml). The mixture was degassed with N₂ for 10 min then PdCl₂(DtBPF) (1.0 g, 1.54 mmol) was added. Degassing with N₂ continued for an additional 5 min. The reaction was heated at 90 °C for 18 h. Upon cooling to RT, the reaction was concentrated in vacuo to remove bulk of toluene. The residue was diluted with H₂O to afford a brown solid. The solid was decanted from the H₂O. EA (50 ml) was added to the solid then evaporated. This process was repeated three times. EA was added (25 ml) and mixture was heated to 50 °C then cooled to RT. A yellow solid was collected by filtration to afford the title compound (4.6 g, 53%). MS ES+ m/z 413.2 [M+H]⁺. The filtrate was concentrated, and the residue was purified by silica gel chromatography eluting with 10% to 100% EA in c-Hex to afford the title compound (2.0 g, 24%) as an oily yellow solid. MS ES+ m/z 413.2 [M+H]⁺.

### Preparation 196

### tert-Butyl 4-[4-[3-cyano-4-[(1R)-1-(2-pyridyl)ethoxy]pyrazolo[1,5-a]pyridine-6-yl]-5-methyl-pyrazol-1-yl]piperidine-1-carboxylate

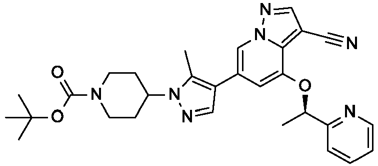

A mixture of 4-[(1R)-1-(2-Pyridyl)ethoxy]-6-(4,4,5,5-tetramethyl-1,3,2-dioxaborolan-2-yl)pyrazolo [1,5-a]pyridine-3-carbonitrile (5.0 g, 12.81 mmol), tert-butyl 4-(4-bromo-5-methyl-pyrazol-1-yl)piperidine-1-carboxylate (5.0 g, 14.53 mmol) in toluene (50 ml) was degassed with bubbling N₂ for 5 min. Next, a soln of K₃CO₂ (4.0 g, 28.94 mmol) in H₂O (12.5 ml) was added, and the mixture was degassed for an additional 5 min. PdCl₂(DtBPF) (500 mg, 0.77 mmol) was added and the mixture was degassed with N₂ while the reaction was heated to 60 °C. At this point N₂ bubbling was stopped. Then reaction was heated to 100 °C under N₂.

After 2 h the reaction was cooled to RT and DE was added (6 g) with stirring. 30 min later the mixture was filtered over a DE plug, and solids were washed with H₂O (50 ml) and toluene (150 ml). The layers from the filtrate were separated and the organic layer dried over MgSO4, filtered, and concentrated to afford the title compound (7.0 g, 98%) as oily brown material. MS ES+ *m*/*z* 528 [M+H]⁺.

The following compounds were prepared in a manner essentially analogous to the method of Preparation 196 using the appropriate reagents, adjusting reaction time to determine completion of the reaction, and adjusting the purification system as appropriate. K₃PO₄ can be used in place of K₃CO₂.

**Table 33**

| Prep # | Chemical Name | Structure | MS ES+ m/z |
|---|---|---|---|
| 197¹ | tert-Butyl 4-[4-(3-fluoro-4-methoxy-pyrazolo[1,5-a]pyridine-6-yl)-5-methyl-triazol-1-yl]piperidine-1-carboxylate | 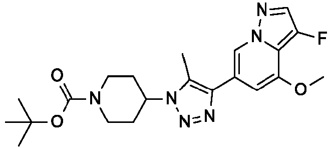 | 431 [M+H]⁺ |
| 198^{2,6} | tert-Butyl 4-[4-(5-methoxyimidazo[1,2-a]pyridine-7-yl)-5-methyl-triazol-1-yl]piperidine-1-carboxylate | 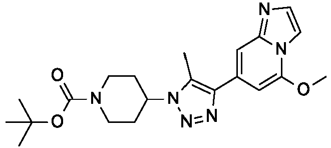 | 413 [M+H]⁺ |
| 199³ | tert-Butyl 3-[4-(4-methoxypyrazolo[1,5-a]pyridine-6-yl)-5-methyl-triazol-1-yl]azetidine-1-carboxylate | 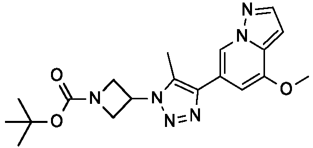 | 385 [M+H]⁺ |
| 200 | tert-Butyl (3R)-3-[4-[3-cyano-4-[(1R)-1-(2-pyridyl)ethoxy]pyrazolo[ 1,5-a]pyridin-6-yl]-5-methyl-pyrazol-1-yl]piperidine-1-carboxylate | 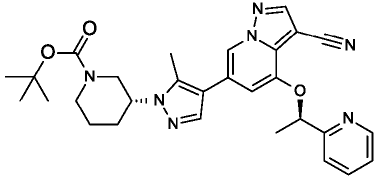 | 528 [M+H]⁺ |
| 201 | tert-Butyl (3S)-3-[4-[3-cyano-4-[(1R)-1-(2-pyridyl)ethoxy]pyrazolo[ 1,5-a]pyridin-6-yl]-5-methyl-pyrazol-1-yl]piperidine-1-carboxylate | 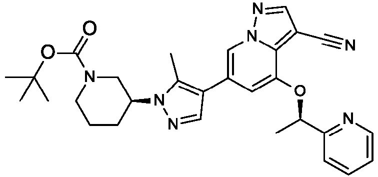 | 528 [M+H]⁺ |
| 202⁴ | tert-Butyl 6-[4-[3-cyano-4-[(1R)-1-(2-pyridyl)ethoxy]pyrazolo[ 1,5-a]pyridin-6-yl]-5-methyl-pyrazol-1-yl]-2-azaspiro[3.3]heptane-2-carboxylate | 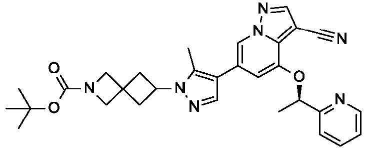 | 540 [M+H]⁺ |
| 203⁵ | tert-Butyl 3-[4-[3-cyano-4-[1-[5-(trifluoromethyl)-3-pyridyl]ethoxy]pyrazolo[ 1,5-a]pyridin-6-yl]-5-methyl-triazol-1-yl]azetidine-1-carboxylate, Isomer 2 | 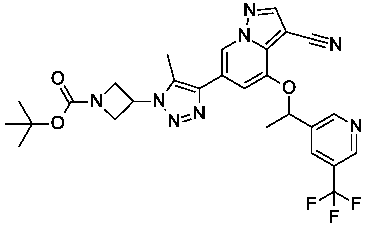 | 513 [M+H-tBu]⁺ |
| 204^{7,8,9,10} | tert-Butyl (3S,4R)-3-fluoro-4-[4-[4-[2-(5-fluoro-2-pyridyl)-2-hydroxy-propoxy]pyrazolo[1,5-a]pyridin-6-yl]-5-methyl-triazol-1-yl]piperidine-1-carboxylate | 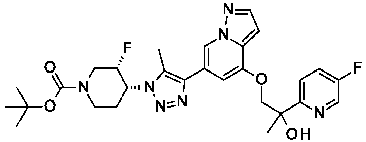 | 570 [M+H]⁺ |
| 205^{7,9,11} | tert-Butyl (3S,4S)-3-fluoro-4-[4-[4-[2-(5-fluoro-2-pyridyl)-2-hydroxy-propoxy]pyrazolo[1,5-a]pyridin-6-yl]-5-methyl-triazol-1-yl]piperidine-1-carboxylate | 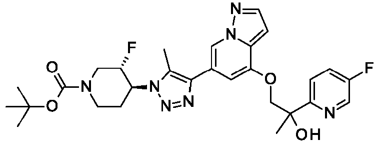 | 570 [M+H]⁺ |
| 206¹² | 3-Fluoro-4-methoxy-6-[5-methyl-1-[1-(oxetan-3-yl)-4-piperidyl]triazol-4-yl]pyrazolo[1,5-a]pyridine | 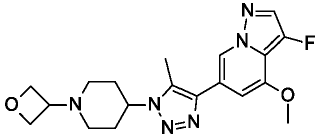 | 387 [M+H]⁺ |
| 207^{7,9,13} | tert-Butyl 4-[4-[5-[2-(3,5-difluoro-2-pyridyl)-2-methoxy-ethoxy]imidazo[1,2-a]pyridin-7-yl]-5-methyl-triazol-1-yl]-4-methylpiperidine-1-carboxylate | 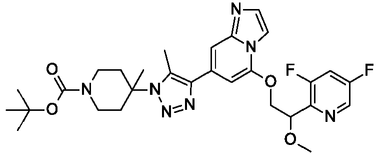 | 584 [M+H]⁺ |
| 208^{7,14,15} | tert-Butyl (4R)-3,3-difluoro-4-[4-[4-[2-(5-fluoro-2-pyridyl)-2-hydroxy-propoxy]pyrazolo[1,5-a]pyridin-6-yl]-5-methyl-triazol-1-yl]piperidine-1-carboxylate | 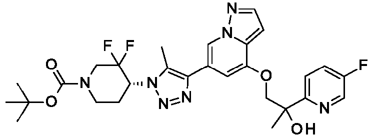 | 588 [M+H]⁺ |
| 209^{7,16,17} | tert-Butyl (3R,4S)-4-[4-[3-chloro-4-[2-(5-fluoro-2-pyridyl)-2-hydroxy-propoxy]pyrazolo[1,5-a]pyridin-6-yl]-5-methyl-triazol-1-yl]-3-fluoro-piperidine-1-carboxylate | 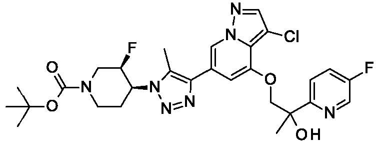 | 604 [M+H]⁺ |
| 210^{7,16,18} | tert-Butyl (3R,4R)-3-fluoro-4-[4-[4-[2-(5-fluoro-2-pyridyl)-2-hydroxy-propoxy]pyrazolo[1,5-a]pyridin-6-yl]-5-methyl-triazol-1-yl]piperidine-1-carboxylate | 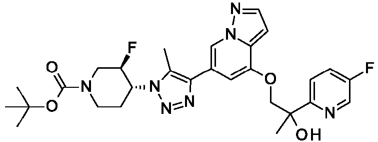 | 570 [M+H]⁺ |
| 211^{19,20} | tert-Butyl 4-[4-(4-methoxy-3-methyl-pyrazolo[1,5-a]pyridin-6-yl)-5-methyl-triazol-1-yl]piperidine-1-carboxylate | 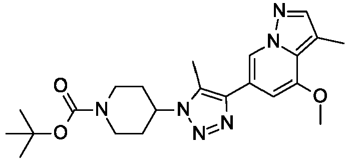 | 427 [M+H]⁺ |
| 212²¹ | tert-Butyl 4-[3-[4-[5-[2-(3,5-difluoro-2-pyridyl)-2-methoxy-ethoxy]imidazo[1,2-a]pyridin-7-yl]-5-methyl-triazol-1-yl]cyclobutyl]piperazine-1-carboxylate | 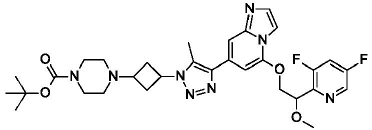 | 625 [M+H]⁺ |
| 213²² | tert-Butyl 4-((1r,3r)-3-(4-(5-(2-(3,5-difluoropyridin-2-yl)-2-methoxyethoxy) imidazo[1,2-a]pyridin-7-yl)-5-methyl-1H-1,2,3-triazol-1-yl)cyclobutyl) piperazine-1-carboxylate | 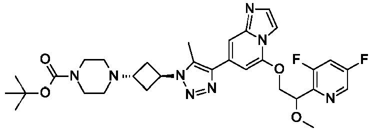 | 625 [M+H]⁺ |

| | | | |
|---|---|---|---|
| ¹ Silica gel column chromatography, eluting with PE / EA (2:1 to 1:1). ² Pd(PPh₃)₄ used as catalyst for this transformation ³ Purified by silica gel chromatography eluting with 10% to 100% EA in cHex. ⁴ Purified by silica gel chromatography eluting with 0% to 100% EA in hex followed by 0% to 20% MeOH in EA. ⁵ Purified by silica gel chromatography eluting with 0% to 100% EA in cHex. ⁶ Purified by silica gel chromatography, eluting with EA / EtOH (30:1). ⁷ Dioxane used as solvent. ⁸ Reaction was heated at 80°C ⁹ Workup: Reaction extracted with EA. Combined organic layers washed with brine, dried over Na₂SO₄, filtered and concentrated. ¹⁰ Purified by silica gel column chromatography, eluting with PE / EA (10:1 to 3:1). ¹¹ Purified by silica gel column chromatography, eluting with 50% EA in PE. ¹² Workup: Reaction concentrated. Resude was slurried in H₂O and the resultant insoluble material was collected by filtration. ¹³ Purified by reversed phase C18 chromatography eluting with 40% to 60% ACN in H₂O. ¹⁴ Workup: Reaction diluted with H₂O and extracted with EA. Combined organic layers washed with brine, dried over Na₂SO₄, filtered and concentrated. ¹⁵ Purified by silica gel chromatography, eluting with 20% EA in PE. ¹⁶ Workup: Reaction concentrated. ¹⁷ Purified by silica gel column chromatography, eluting with 50% to 75% EA in PE. ¹⁸ Purified by silica gel column chromatography, eluting with 10% MeOH in DCM. ¹⁹ Pd(dppf)Cl₂ was used as catalyst for this transformation. Reaction was heated at 90 °C. ²⁰ Purified by silica gel chromatography eluting with MeOH in DCM. ²¹ Purified by silica gel column chromatography, eluting with DCM / MeOH (15:1 to 10:1). ²² Purified by silica gel column chromatography, eluting with DCM / MeOH (10:1) | | | |

### Preparation 214a

### tert-Butyl 2-[4-[3-cyano-4-[(1R)-1-(2-pyridyl)ethoxy]pyrazolo[1,5-a]pyridin-6-yl]-5-methyl-pyrazol-1-yl]-7-azaspiro[3.5]nonane-7-carboxylate

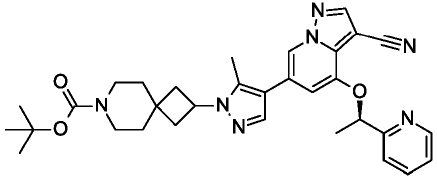

and

### Preparation 214b

### tert-Butyl 2-[4-[3-cyano-4-[(1R)-1-(2-pyridyl)ethoxy]pyrazolo[1,5-a]pyridin-6-yl]-3-methyl-pyrazol-1-yl]-7-azaspiro[3.5]nonane-7-carboxylate

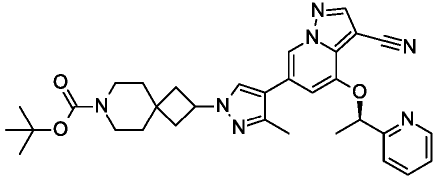

A mixture of tert-butyl 2-(4-bromo-5-methyl-pyrazol-1-yl)-7-azaspiro[3.5]nonane-7-carboxylate, tert-butyl 2-(4-bromo-3-methyl-pyrazol-1-yl)-7-azaspiro[3.5]nonane-7-carboxylate (1.68 g, 3.11 mmol), and 4-[(1R)-1-(2-pyridyl)ethoxy]-6-(4,4,5,5-tetramethyl-1,3,2-dioxaborolan-2-yl)pyrazolo[1,5-a]pyridine-3-carbonitrile (1.33 g, 3.41 mmol) in toluene (40 ml) and H₂O (4 ml) and treated with K₂CO₃ (1.14 g, 8.25 mmol). N₂ was bubbled through the reaction for 2 min before adding PdCl₂(DtBPF) (135 mg, 0.21 mmol) and the tube was sealed. After stirring at 90 °C for 20 h, the reaction was allowed to cool and diluted with EA (50 ml) and H₂O (20 ml). The layers were separated, and the aq layer was extracted with EA. The combined organic layers were concentrated. The residue was purified by silica gel chromatography eluting with a gradient of 0% to 10% acetone in DCM to obtain a mixture of the title compounds (621 mg, 74%) as a thick yellow oil. MS ES+ *m*/*z* 568 [M+H]⁺.

The following compounds were prepared in a manner essentially analogous to the method of Preparation for 214a and 214b using the appropriate reagents, adjusting reaction time to determine completion of the reaction, and adjusting the purification system as appropriate.

**Table 34**

| Prep # | Chemical Name | Structure | MS ES+ m/z |
|---|---|---|---|
| 215a | tert-Butyl 4-[4-[3-cyano-4-[(1R)-1-(2-pyridyl)ethoxy]pyrazolo[1,5-a]pyridin-6-yl]-5-methyl-pyrazol-1-yl]azepane-1-carboxylate | 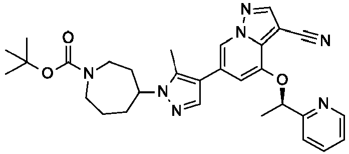 | 542 [M+H]⁺ |
| 215b | tert-Butyl 4-[4-[3-cyano-4-[(1R)-1-(2-pyridyl)ethoxy]pyrazolo[1,5-a]pyridin-6-yl]-3-methyl-pyrazol-1-yl]azepane-1-carboxylate | 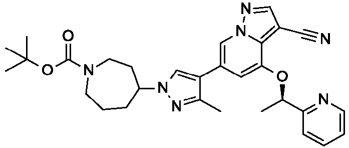 | 542 [M+H]⁺ |

### Preparation 216

### 4-Methoxy-6-[5-methyl-1-[1-(oxetan-3-yl)-4-piperidyl]triazol-4-yl]pyrazolo[1,5-a]pyridine-3-carbonitrile

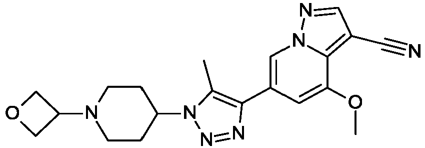

N₂ was bubbled through a mixture of 4-methoxy-6-(4,4,5,5-tetramethyl-1,3,2-dioxaborolan-2-yl)pyrazolo[1,5-a]pyridine-3-carbonitrile (39 g, 117 mmol 90 mass%), K₂CO₃ (40 g, 289 mmol), and 4-(4-bromo-5-methyl-triazol-1-yl)-1-(oxetan-3-yl)piperidine (42.6 g, 135 mmol) in toluene (390 ml) and H₂O (116 mL). After 10 min, PdCl₂(DtBPF) (0.68 g, 1.04 mmol) was added and N₂ was bubbled through the reaction for an additional 5 min. The reaction was stirred at 90 °C for 8 h then stirred at RT for approximately 60 h. The reaction was diluted with H₂O (100 ml) and the mixture was concentrated to remove toluene. The resulting suspension was stirred at RT for 15 min, filtered, and the solids were washed with H₂O (2 x 30 mL) to obtain the title compound (42.6 g, 83%). MS ES+ m/z 394 [M+H]⁺.

The following compounds were prepared in a manner essentially analogous to the method of Preparation 216 using the appropriate reagents, adjusting reaction time to determine completion of the reaction, and adjusting the purification system as appropriate.

**Table 35**

| Prep # | Chemical Name | Structure | MS ES+ m/z |
|---|---|---|---|
| 217 | 4-Methoxy-6-[5-methyl-1-[1-(oxetan-3-yl)-4-piperidyl] triazol-4-yl]pyrazolo[1,5-a]pyridine | 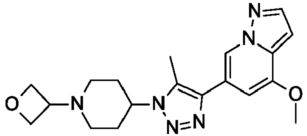 | 369 [M+H]⁺ |
| 218¹ | 4-Methoxy-6-[5-methyl-1-[1-(oxetan-3-yl)-4-piperidyl] triazol-4-yl]-3-(trifluoromethyl)pyrazolo[1,5-a]pyridine | 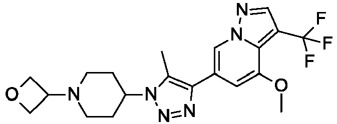 | 437 [M+H]⁺ |

| | | | |
|---|---|---|---|
| Purified by silica gel chromatography eluting with 0% to 100% EA in cHex followed by 0% to 10% MeOH in DCM. | | | |

### Preparation 219

### tert-Butyl 4-[4-(3-chloro-4-methoxy-pyrazolo[1,5-a]pyridin-6-yl)-5-methyl-triazol-1-yl]piperidine-1-carboxylate

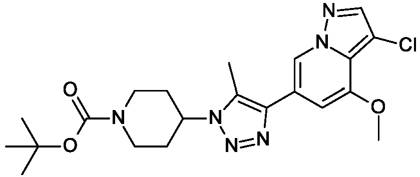

A soln of tert-butyl 4-[4-(4-methoxypyrazolo[1,5-a]pyridin-6-yl)-5-methyl-triazol-1-yl]piperidine-1-carboxylate (4 g, 9.12 mmol) in DCM (50 ml) was treated with NCS (700 mg, 5.24 mmol). After stirring at RT for 24 h, another portion of NSC (700 mg, 5.24 mmol) was added and stirring continued for another 24 h. The reaction was treated with H₂O (100 ml) and the layers were separated. The organic layer was dried over MgSO₄, filtered, and concentrated. The residue was purified by silica gel chromatography eluting with a gradient of 0% and 100% EA in cHex to obtain the title compound as a white solid (3.4 g, 82%). MS ES+ m/z 447/449 [M+H]⁺.

### Preparation 220

### 3-Chloro-4-methoxy-6-[5-methyl-1-[1-(oxetan-3-yl)-4-piperidyl]triazol-4-yl]pyrazolo[1,5-a]pyridine

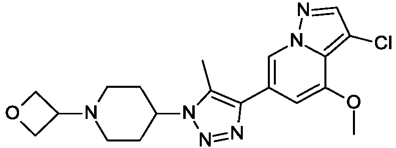

4-Methoxy-6-[5-methyl-1-[1-(oxetan-3-yl)-4-piperidyl]triazol-4-yl]pyrazolo[1,5-a]pyridine (34 g, 83.06 mmol) was dissolved in DCM (500 ml) and treated with NCS (12 g, 89.86 mmol) under N₂. After stirring at RT for 18 h, added more NCS (6.5 g, 49 mmol) and allowed to stir overnight. Diluted with H₂O (200 ml) and separated layers, dried organic layer over Na₂SO₄, filtered, and concentrated. The residue was purified by silica gel chromatography eluting with a gradient of 0% to 10% MeOH in DCM to obtain the title compound (16 g, 45%) as an amber oil. MS ES+ m/z 403/405 [M+H]⁺.

The following compounds were prepared in a manner essentially analogous to the method of Preparation 220 using the appropriate reagents, adjusting reaction time to determine completion of the reaction, and adjusting the purification system as appropriate.

**Table 36**

| Prep # | Chemical Name | Structure | MS ES+ m/z |
|---|---|---|---|
| 221¹ | tert-Butyl 3-[4-(3-chloro-4-methoxy-pyrazolo[1,5-a]pyridin-6-yl)-5-methyl-triazol-1-yl]azetidine-1-carboxylate | 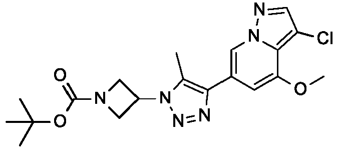 | (³⁵Cl/³⁷Cl) 419/421 [M+H]⁺ |
| 222^{2,3} | tert-Butyl (3S,4R)-4-[4-[3-chloro-4-[2-(5-fluoro-2-pyridyl)-2-hydroxy-propoxy]pyrazolo[1,5 -a]pyridin-6-yl]-5-methyl-triazol-1-yl]-3-fluoro-piperidine-1-carboxylate | 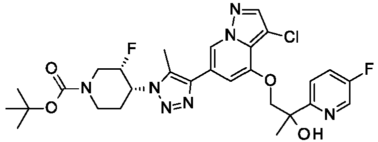 | 604 [M+H]⁺ |
| 223⁵ | tert-Butyl (3S,4S)-4-[4-[3-chloro-4-[2-(5-fluoro-2-pyridyl)-2-hydroxy-propoxy]pyrazolo[1,5 -a]pyridin-6-yl]-5-methyl-triazol-1-yl]-3-fluoro-piperidine-1-carboxylate | 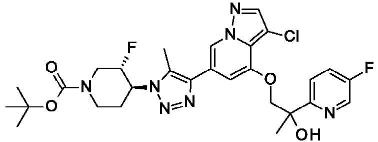 | 604 [M+H]⁺ |
| 224^{2,4} | tert-Butyl (4R)-4-[4-[3-chloro-4-[2-(5-fluoro-2-pyridyl)-2-hydroxy-propoxy]pyrazolo[1,5 -a]pyridin-6-yl]-5-methyl-triazol-1-yl]-3,3-difluoro-piperidine-1-carboxylate | 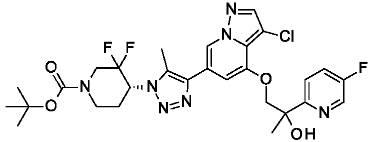 | 622 [M+H]⁺ |
| 225^{3,6} | tert-Butyl (3R,4R)-4-[4-[3-chloro-4-[2-(5-fluoro-2-pyridyl)-2-hydroxy-propoxy]pyrazolo[1,5 -a]pyridin-6-yl]-5-methyl-triazol-1-yl]-3-fluoro-piperidine-1-carboxylate | 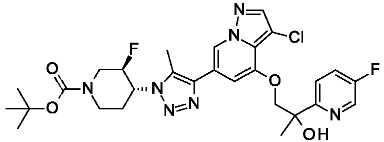 | 604 [M+H]⁺ |
| 226^{7,10} | tert-Butyl 4-[4-(3-bromo-4-methoxy-pyrazolo[1,5-a]pyridin-6-yl)-5-methyl-triazol-1-yl]piperidine-1-carboxylate | 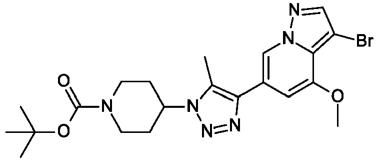 | (⁷⁹Br/⁸¹Br) 491/493 [M+H]⁺ |
| 227^{4, 11} | tert-Butyl 4-[3-[4-[3-chloro-5-[2-(3,5-difluoro-2-pyridyl)-2-methoxy-ethoxy]imidazo[1,2-a]pyridin-7-yl]-5-methyl-triazol-1-yl]cyclobutyl]piperaz ine-1-carboxylate | 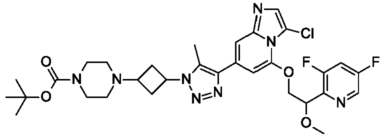 | 659 [M+H]⁺ |
| 228^{8, 9} | tert-Butyl 4-((1r,3r)-3-(4-(3-chloro-5-(2-(3,5-difluoropyridin - 2-yl)-2-methoxy ethoxy)imidazo[1,2-a]pyridin-7-yl)-5-methyl-1H-1,2,3-triazol-1-yl) cyclobutyl) piperazine-1-carboxylate | 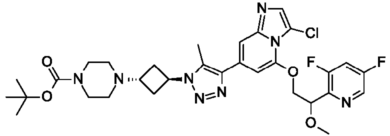 | 659 [M+H]⁺ |

| | | | |
|---|---|---|---|
| Purified by silica gel chromatography eluting with 0% to 100% EA in cHex. ² Reaction was heated at 50 °C ³ Purified by Prep TLC: PE / EA (1:1) ⁴ Purified by Prep TLC: DCM / MeOH (20:1) ⁵ Residue taken on to next step without purification. ⁶ Workup: Reaction concentrated. ⁷ Workup: Reaction quenched with 40% aq NaHSO₃, layers separated, organic layer dried over MgSO₄, filtered and concentrated. ⁸ Purified by reversed flash, eluted with 40% to 50% MeOH in H₂O. ⁹ Column: XB-Phenyl, 50 x 250 mm, 10 µm; eluting with 60 to 70% ACN in H₂O (10mM NH3-H2O). ¹⁰NBS used in place of NCS. ¹¹ Purified by reversed flash, eluted with 45% to 55% ACN in H₂O. | | | |

### Preparation 229

### 4-Hydroxy-6-[5-methyl-1-[1-(oxetan-3-yl)-4-piperidyl]triazol-4-yl]pyrazolo[1,5-a]pyridine-3-carbonitrile

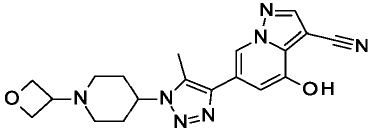

A mixture of 4-Methoxy-6-[5-methyl-1-[1-(oxetan-3-yl)-4-piperidyl]triazol-4-yl]pyrazolo[1,5-a]pyridine-3-carbonitrile (20 g, 45.75 mmol) in DMA (250 ml) was added NDM (21.9 ml, 91.51 mmol) and aq NaOH (7.25 ml, 137.3 mmol, 18.94 mol/L) under N₂. The reaction was degassed with N₂ for 15 min then heated at 50 °C for 2 h. Upon cooling to RT, H₂O (2L) was added. After stirring 15 min the pH was adjusted to 6.9 by the addition of aq HCL (10% w/w) then K₂HPO₄. The reaction was stirred 15 min. The insoluble material was collected by filtration, washed with H₂O (2 x 25 ml) to afford the title compound as pale grey solid (11.0 g, 62% Yield). MS ES+ m/z 380 [M+H]⁺.

### Preparation 230

### tert-Butyl 4-[4-(3-chloro-4-hydroxy-pyrazolo[1,5-a]pyridine-6-yl)-5-methyl-triazol-1-yl]piperidine-1-carboxylate

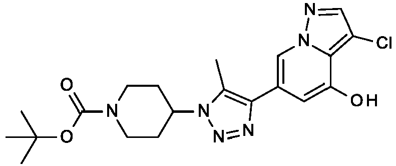

To tert-butyl 4-[4-(3-chloro-4-methoxy-pyrazolo[1,5-a]pyridin-6-yl)-5-methyl-triazol-1-yl]piperidine-1-carboxylate (3.4 g, 7.5 mmol) in DMA (20 ml) under N₂ atmosphere was added NDM (5.5 ml, 23 mmol) and NaOH (18.94 mol/L) in H₂O (1.4 ml). The reaction was heated overnight at 60 °C. Upon cooling to RT, the mixture was diluted with H₂O (100 ml) and the pH was adjusted to pH = 5 with aq HCl. A cream colored solid resulted. After stirring for 15 minutes the insoluble material was collected by filtration and the solids were rinsed with H₂O to afford the title compound (1.4 g, 43%). MS ES+ m/z 433 [M+H]⁺.

### Preparation 231

### tert-Butyl 4-[4-(3-cyano-4-hydroxy-pyrazolo[1,5-a]pyridine-6-yl)-5-methyl-triazol-1-yl]piperidine-1-carboxylate

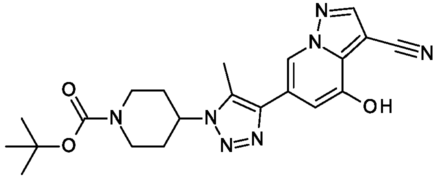

NaOH (50% aq.) (36.57 g, 457.14 mmol) was added dropwise to a stirred mixture of tert-butyl 4-(4-[3-cyano-4-methoxypyrazolo[1,5-a]pyridin-6-yl]-5-methyl-1,2,3-triazol-1-yl)piperidine-1-carboxylate (40.00 g, 91.43 mmol) and NDM (55.51 g, 274.28 mmol) in DMA (400 ml) at 0 °C. The mixture is stirred for 8 h at 50 °C. The mixture was diluted with H₂O (400 ml), acidified to pH 6 with FA, filtered, and the filter cake was washed with H₂O, and dried under vacuum. The solid was triturated in hexanes (200 ml) and Et₂O (200 ml), filtered, then stirred in MeOH (400 ml) for 2 h at 60 °C. The mixture was filtered, and the filter cake was concentrated under vacuum to give the title compound as a light-yellow solid (32 g, 82.6%). ES/MS m/z 424.3 [M+H]⁺.

The following compounds were prepared in a manner essentially analogous to the method of Preparation 231 using the appropriate reagents, adjusting reaction time to determine completion of the reaction, and adjusting the purification system as appropriate.

**Table 37**

| Prep # | Chemical Name | Structure | MS ES+ m/z |
|---|---|---|---|
| 232 | tert-Butyl 4-[4-(3-fluoro-4-hydroxy-pyrazolo[1,5-a]pyridine-6-yl)-5-methyl-triazol-1-yl]piperidine-1-carboxylate | 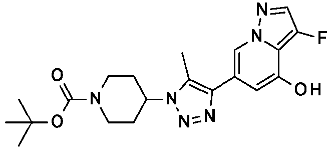 | 417 [M+H]⁺ |
| 233 | tert-Butyl 4-[4-(5-hydroxyimidazo[1,2-a]pyridine-7-yl)-5-methyl-triazol-1-yl]piperidine-1-carboxylate | 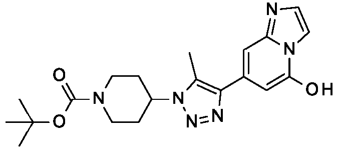 | 399 [M+H]⁺ |
| 234 | 3-Chloro-6-[5-methyl-1-[1-(oxetan-3-yl)-4-piperidyl]triazol-4-yl]pyrazolo[1,5-a]pyridine-4-ol | 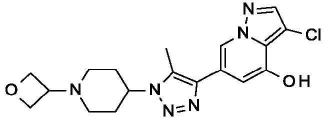 | 389 [M+H]⁺ |
| 235 | tert-Butyl 3-[4-(3-chloro-4-hydroxy-pyrazolo[1,5-a]pyridin-6-yl)-5-methyl-triazol-1-yl]azetidine-1-carboxylate | 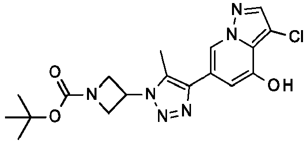 | 405/407 [M+H]⁺ |
| 236 | 6-[5-Methyl-1-[1-(oxetan-3-yl)-4-piperidyl]triazol-4-yl]-3-(trifluoromethyl)pyrazolo[1,5-a]pyridin-4-ol | 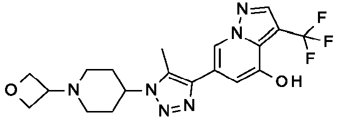 | 423 [M+H]⁺ |
| 237¹ | 3-Fluoro-6-[5-methyl-1-[1-(oxetan-3-yl)-4-piperidyl] triazol-4-yl]pyrazolo [1,5-a]pyridin-4-ol | 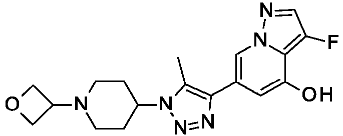 | 373 [M+H]⁺ |
| 238² | tert-Butyl 4-[4-(4-hydroxy-3-methyl-pyrazolo[1,5-a]pyridin-6-yl)-5-methyl-triazol-1-yl]piperidine-1-carboxylate | 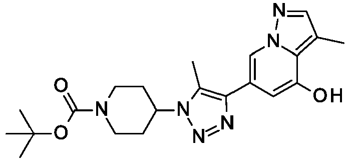 | 413 [M+H]⁺ |
| 239^{3,4} | tert-Butyl 4-[4-(3-bromo-4-hydroxy-pyrazolo[1,5-a]pyridin-6-yl)-5-methyl-triazol-1-yl]piperidine-1-carboxylate | 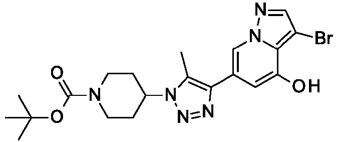 | (⁷⁹Br/⁸¹Br) 477/479 [M+H]⁺ |

| | | | |
|---|---|---|---|
| ¹ Workup: Reaction cooled to RT then aq citrus acid (3%) was added until pH approx 5. Resultant insoluble material was isolated by filtration, triterated in cHex, filtered and rinsed with cHEx followed by MTBE. ² Workup: Reaction was neutralized with NH₄Cl solution to pH 7-8 and concentrated in vacuo. The residue was treated with DCM and H₂O, layers were separated, organic layer dried over MgSO₄, filtered, and concentrated. Residue was triturated in heptane, filtered and washed with heptane. ³ Workup: Reaction cooled to RT then aq citrus acid (3%) was added. Resultant precipitate was isolated by filtration, washed with H₂O then cHex. The solids were dissolved into DCM then cHex was added. The soln was sonicated until a precipitate had formed. The precipitate was collected by filtration. ⁴ Purified by silica gel chromatography eluting with 0% to 50% acetone in DCM. | | | |

### Preparation 240

### 4-Hydroxy-6-[5-methyl-1-(4-piperidyl)triazol-4-yl]pyrazolo[1,5-a]pyridine-3-carbonitrile hydrochloride

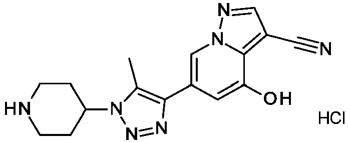

A soln of tert-butyl 4-[4-(3-cyano-4-hydroxy-pyrazolo[1,5-a]pyridin-6-yl)-5-methyl-triazol-1-yl]piperidine-1-carboxylate (50 g, 118.07 mmol) in MeOH (100 ml) was treated with 4M HCl in MeOH (300 ml) at RT under N₂. The reaction was concentrated to obtain the title compound (40 g, HCl salt, crude) as a white solid. MS ES+ *m*/*z* 417 [M+H]⁺.

The following compounds were prepared in a manner essentially analogous to the method of Preparation 240 using the appropriate reagents, adjusting reaction time to determine completion of the reaction, and adjusting the purification system as appropriate.

**Table 38**

| Prep # | Chemical Name | Structure | MS ES+ m/z |
|---|---|---|---|
| 241^{a} | 6-[1-(Azetidin-3-yl)-5-methyl-triazol-4-yl]-3-chloro-pyrazolo[1,5-a]pyridin-4-ol hydrochloride | 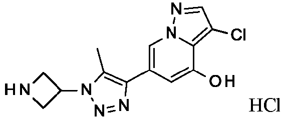 | 305/307 [M+H]⁺ |

| | | | |
|---|---|---|---|
| ^{a}HCl in 2-propanol was used in this transformation. | | | |

### Preparation 242

### 4-Hydroxy-6-[5-methyl-1-[1-(oxetan-3-yl)-4-piperidyl]triazol-4-yl]pyrazolo[1,5-a]pyridine-3-carbonitrile

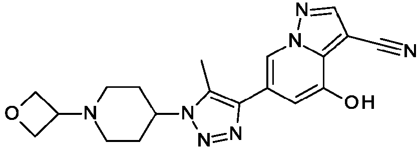

A soln of 4-hydroxy-6-[5-methyl-1-(4-piperidyl)triazol-4-yl]pyrazolo[1,5-a]pyridine-3-carbonitrile hydrochloride (40 g, HCl salt, crude) in MeOH (300 ml) was basified to pH 12 with NaOH (5 g), then acidified to pH 5-6 with AcOH (10 g), then treated with 3-oxetanone (53.49 g, 742.21 mmol) at RT under N₂. After stirring at 50 °C for 2 h, treated the reaction with NaBH₃CN (23.32 g, 371.10 mmol) at RT and then stirred at 50 °C for 2 h. After cooling to RT, the reaction was concentrated, and the residue was suspended in H₂O (500 ml) and basified to pH 8 with solid NaHCO₃. The suspension was filtered, the solids washed with MTBE (3 x 100 ml), and the solids lyophilized to obtain the title compound (30 g, 64%) as a white solid. MS ES+ m/z 380 [M+H]⁺.

The following compound was prepared in a manner essentially analogous to the method of Preparation 242 using the appropriate reagents, adjusting reaction time to determine completion of the reaction, and adjusting the purification system as appropriate.

**Table 39**

| Prep # | Chemical Name | Structure | MS ES+ m/z |
|---|---|---|---|
| 243 | 3-Chloro-6-[5-methyl-1-[1-(oxetan-3-yl)azetidin-3-yl]triazol-4-yl]pyrazolo[1,5-a]pyridin-4-ol | 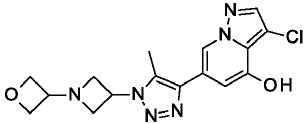 | 361/363 [M+H]⁺ |

### Preparation 244

### 4-[2-(5-fluoro-2-pyridyl)-2-oxo-ethoxy]-6-[5-methyl-1-[1-(oxetan-3-yl)-4-piperidyl]triazol-4-yl]pyrazolo[1,5-a]pyridine-3-carbonitrile

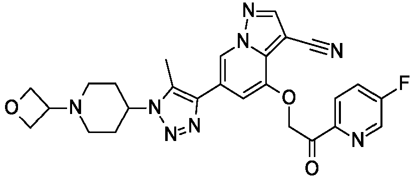

To a suspension of 4-hydroxy-6-[5-methyl-1-[1-(oxetan-3-yl)-4-piperidyl]triazol-4-yl]pyrazolo[1,5-a]pyridine-3-carbonitrile (1 g, 2.64 mmol) in ACN (10 ml) was added Cs₂CO₃ (1.12 g, 3.43 mmol). The suspension was stirred at RT for 30 minutes. Next a solution of 2-bromo-1-(5-fluoro-2-pyridyl)ethanone (0.747 g, 3.43 mmol) in ACN (3 ml) was added dropwise at RT over a 30-minute period. The reaction was stirred vigorously stirred at RT for approximately 8 h. H₂O was added and the suspension was stirred for 10 min then was left standing overnight. Suspension was further diluted with H₂O then filtered. The solids were rinsed with EA followed by c-Hex then dried in vacuo to obtain the title compound (1.2 g, 83%). MS ES+ m/z 517 [M+H]⁺.

### Preparation 245

### tert-Butyl 4-[4-[3-chloro-4-[2-(5-fluoro-2-pyridyl)-2-oxo-ethoxy]pyrazolo[1,5-a]pyridine-6-yl]-5-methyl-triazol-1-yl]piperidine-1-carboxylate

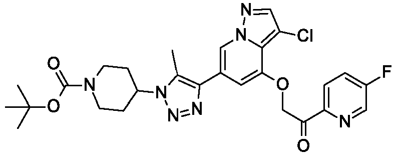

A soln of tert-butyl 4-[4-(3-chloro-4-hydroxy-pyrazolo[1,5-a]pyridine-6-yl)-5-methyl-triazol-1-yl]piperidine-1-carboxylate (1.5 g, 3.47 mmol) and 2-bromo-1-(5-fluoro-2-pyridyl)pyridine (1.51 g, 6.93 mmol) in ACN (30 ml) was treated with DIPEA (1.34 g, 10.40 mmol) at RT under N₂. After stirring at 50 °C for 1 h, the reaction was allowed to cool to RT, concentrated, and the residue purified by silica gel column chromatography eluting with PE / EA (1:1 to 1:2) to obtain the title compound (1.1 g, 56%) as a brown solid. MS ES+ m/z 570 [M+H]⁺.

The following compounds were prepared in a manner essentially analogous to the method of Preparation 245 using the appropriate reagents, adjusting reaction time to determine completion of the reaction, and adjusting the purification system as appropriate. Cs₂CO₃ can be used in place of DIPEA

**Table 40**

| Prep # | Chemical Name | Structure | MS ES+ m/z |
|---|---|---|---|
| 246¹ | tert-Butyl 4-[4-[3-cyano-4-[2-(2,4-difluorophenyl)-2-oxo-ethoxy]pyrazolo[1,5-a] pyridine-6-yl]-5-methyl-triazol-1-yl]piperidine-1-carboxylate | 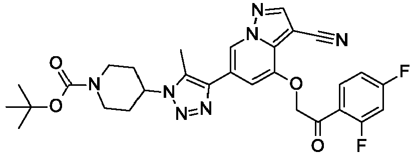 | 522 [M+H]⁺ |
| 247² | tert-Butyl 4-[4-[3-fluoro-4-[2-(5-fluoro-2-pyridyl)-2-oxo-ethoxy]pyrazolo[1,5-a] pyridine-6-yl]-5-methyl-triazol-1-yl]piperidine-1-carboxylate | 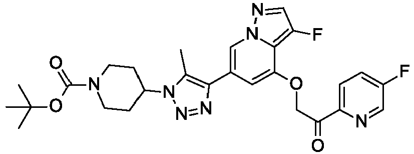 | 554 [M+H]+ |
| 248³ | tert-Butyl 4-[4-[3-cyano-4-[2-(5-fluoro-2-pyridyl)-2-oxo-ethoxy]pyrazolo[1,5-a]pyridin-6-yl]-5-methyl-triazol-1-yl]piperidine-1-carboxylate | 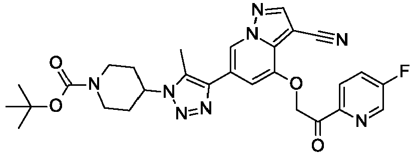 | 561 [M+H]⁺ |
| 249^{4,9} | tert-Butyl 4-[4-[3-chloro-4-[2-(3,5-difluoro-2-pyridyl)-2-oxo-ethoxy]pyrazolo[1,5-a]pyridin-6-yl]-5-methyl-triazol-1-yl]piperidine-1-carboxylate | 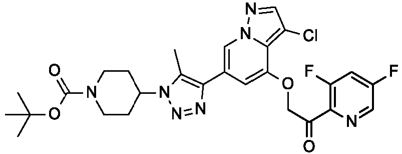 | (³⁵Cl/³⁷Cl) 588/590 [M+H]⁺ |
| 250^{4,5} | tert-Butyl 4-[4-[4-[2-(3-chloro-5-fluoro-2-pyridyl)-2-oxo-ethoxy]-3-cyano-pyrazolo[1,5-a]pyridin-6-yl]-5-methyl-triazol-1-yl]piperidine-1-carboxylate | 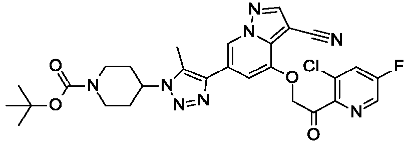 | (³⁵Cl/³⁷Cl) 595/597 [M+H]⁺ |
| 251^{4,5} | tert-Butyl 4-[4-[4-[2-(4-chloro-5-fluoro-2-pyridyl)-2-oxo-ethoxy]-3-cyano-pyrazolo[1,5-a]pyridin-6-yl]-5-methyl-triazol-1-yl]piperidine-1-carboxylate | 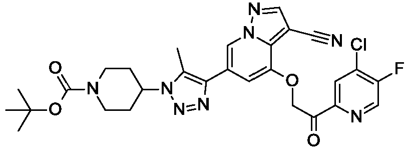 | (³⁵Cl/³⁷Cl) 595/597 [M+H]⁺ |
| 252⁶ | tert-Butyl 4-[4-[3-cyano-4-[[1-(5-fluoro-2-pyridyl)cyclopropyl] methoxy]pyrazolo[1,5-a]pyridin-6-yl]-5-methyl-triazol-1-yl]piperidine-1-carboxylate | 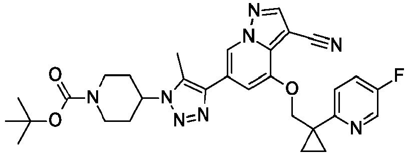 | 573 [M+H]⁺ |
| 253⁷ | 2-[3-Chloro-6-[5-methyl-1-[1-(oxetan-3-yl)azetidin-3-yl]triazol-4-yl]pyrazolo[1,5-a]pyridin-4-yl]oxy-1-(5-fluoro-2-pyridyl)ethanone | 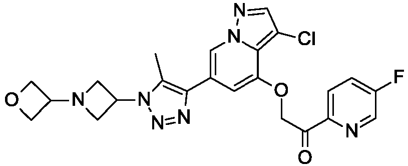 | (³⁵Cl/³⁷Cl) 498/500 [M+H]⁺ |
| 254⁷ | 2-[3-Chloro-6-[5-methyl-1-[1-(oxetan-3-yl)azetidin-3-yl]triazol-4-yl]pyrazolo[1,5-a]pyridin-4-yl]oxy-1-(3,5-difluoro-2-pyridyl)ethanone | 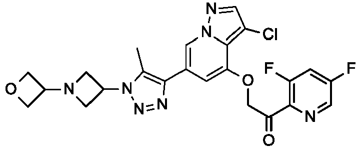 | (³⁵Cl/³⁷Cl) 516/518 [M+H]⁺ |
| 255⁸ | 4-(2-Cyclopentyl-2-oxo-ethoxy)-6-[5-methyl-1-[1-(oxetan-3-yl)-4-piperidyl]triazol-4-yl]pyrazolo[1,5-a]pyridine-3-carbonitrile | 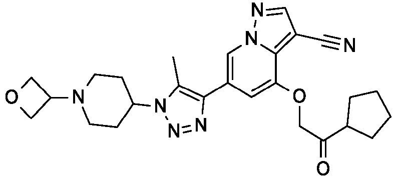 | 490 [M+H]⁺ |
| 256⁷ | 1-(5-Fluoro-2-pyridyl)-2-[6-[5-methyl-1-[1-(oxetan-3-yl)-4-piperidyl]triazol-4-yl]-3-(trifluoromethyl)pyra zolo[1,5-a]pyridin-4-yl]oxy-ethanone | 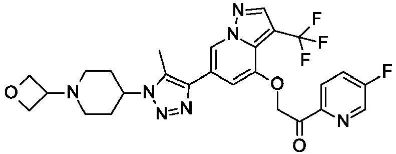 | 560 [M+H]⁺ |
| 257^{4,5} | tert-Butyl 4-[4-[3-chloro-4-(2-isothiazol-3-yl-2-oxo-ethoxy)pyrazolo[1,5-a]pyridin-6-yl]-5-methyl-triazol-1-yl]piperidine-1-carboxylate | 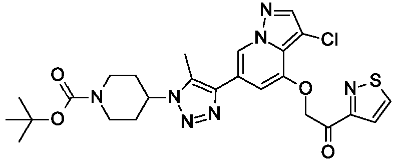 | 558 [M+H]⁺ |
| 258^{4,10} | 4-[2-(3,5-Difluoro-2-pyridyl)-2-oxo-ethoxy]-6-[5-methyl-1-[1-(oxetan-3-yl)-4-piperidyl]triazol-4-yl]pyrazolo[1,5-a]pyridine-3-carbonitrile | 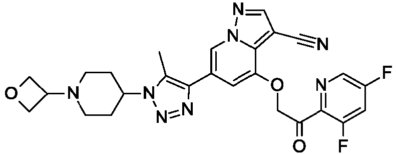 | 535 [M+H]⁺ |
| 259^{4,11} | tert-Butyl 4-[4-[3-cyano-4-[2-(3,5-difluoro-2-pyridyl)-2-oxo-ethoxy]pyrazolo [1,5-a]pyridin-6-yl]-5-methyl-triazol-1-yl]piperidine-1-carboxylate | 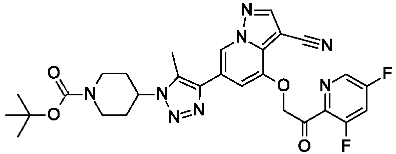 | 579 [M+H]⁺ |
| 260^{4, 12} | tert-Butyl 4-[4-[4-[2-(3,5-difluoro-2-pyridyl)-2-oxo-ethoxy]-3-fluoro-pyrazolo [1,5-a]pyridin-6-yl]-5-methyl-triazol-1-yl] piperidine-1-carboxylate | 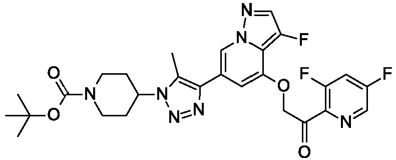 | 572 [M+H]⁺ |
| 261¹³ | 2-[3-Chloro-6-[5-methyl-1-[1-(oxetan-3-yl)-4-piperidyl]triazol-4-yl]pyrazolo[1,5-a]pyridin-4-yl]oxy-1-(3,5-difluoro-2-pyridyl)ethanone | 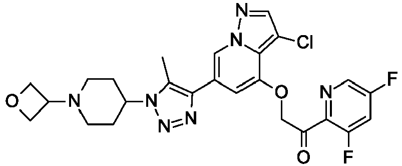 | 544/546 [M+H]⁺ |
| 262^{4,14} | 1-(3-Chloro-5-fluoro-2-pyridyl)-2-[3-fluoro-6-[5-methyl-1-[1-(oxetan-3-yl)-4-piperidyl]triazol-4-yl]pyrazolo[1,5-a]pyridin-4-yl]oxy-ethanone | 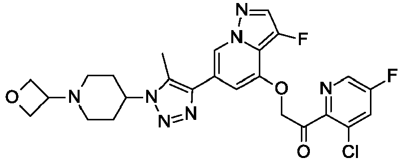 | 544 [M+H]⁺ |
| 263^{4,15} | 2-[3-Fluoro-6-[5-methyl-1-[1-(oxetan-3-yl)-4-piperidyl]triazol-4-yl]pyrazolo[1,5-a]pyridin-4-yl]oxy-1-(5-fluoro-2-pyridyl)ethanone | 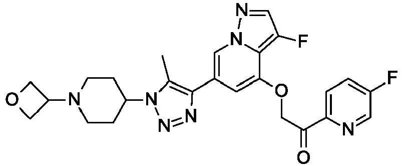 | 510 [M+H]⁺ |
| 264^{4,5} | tert-Butyl 4-[4-[4-[2-(5-fluoro-2-pyridyl)-2-oxo-ethoxy]-3-methyl-pyrazolo [1,5-a]pyridin-6-yl]-5-methyl-triazol-1-yl] piperidine-1-carboxylate | 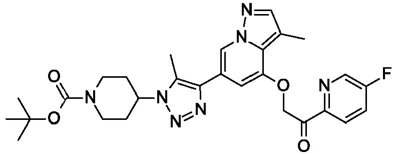 | 550 [M+H]⁺ |
| 265^{4,5} | tert-Butyl 4-[4-[4-[2-(3-chloro-2-pyridyl)-2-oxo-ethoxy]-3-cyano-pyrazolo [1,5-a]pyridin-6-yl]-5-methyl-triazol-1-yl] piperidine-1-carboxylate | 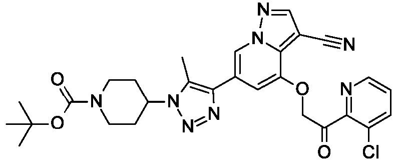 | 577 [M+H]⁺ |
| 266^{4,14,16} | tert-Butyl 4-[4-[3-cyano-4-[2-(3-methyl-2-pyridyl)-2-oxo-ethoxy]pyrazolo[1,5-a]pyridin-6-yl]-5-methyl-triazol-1-yl]piperidine-1-carboxylate | 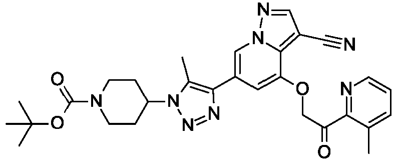 | 557 [M+H]⁺ |
| 267^{4,7,17} | 2-[3-Chloro-6-[5-methyl-1-[1-(oxetan-3-yl)-4-piperidyl]triazol-4-yl]pyrazolo[1,5-a]pyridin-4-yl]oxy-1-(5-fluoro-2-pyridyl)ethanone | 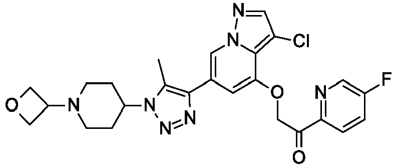 | 526 [M+H]⁺ |
| 268^{4,18} | tert-Butyl 4-[4-[3-bromo-4-[2-(5-fluoro-2-pyridyl)-2-oxo-ethoxy]pyrazolo[1,5-a]pyridin-6-yl]-5-methyl-triazol-1-yl]piperidine-1-carboxylate | 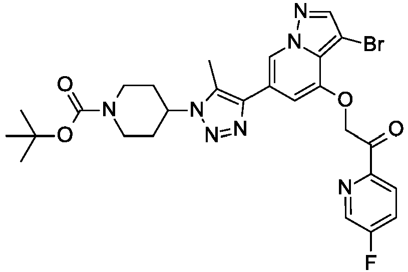 | (⁷⁹Br/⁸¹Br) 614/616 [M+H]⁺ |

| | | | |
|---|---|---|---|
| ¹ Purified by reversed phase flash C18 chromatography with the following conditions: column, C18; eluting with 75% to 80% CAN in H₂O. ² Purified by reversed phase C18 chromatography eluting with 50% to 60% CAN in H2O (0.1% FA) ³ Workup: Reaction was concentrated, diluted with H₂O and insoluble material was collected by filtration. ⁴ Cs₂CO₃ used as base in this synthesis. ⁵ Purified by silica gel chromatography eluting with MeOH in DCM. ⁶ Purified by silica gel chromatography eluting with 5% (EA / EtOH 3:1) in cHex (2 CV), 5% to 50% (EA / EtOH 3:1) in cHex (20 CV) then 100% EA / EtOH (3:1). ⁷ Workup: Reaction was concentrated, diluted with H₂O and insoluble material was collected by filtration. ⁸ Workup: EA and H₂O were added, layers separated, organic layer washed with 1N NaOH and brine. Organic layer was dried over MgSO₄, filtered and concentrated. ⁹ Workup: Reaction concentrated in vacuo. Residue was dissolved into H₂O and acetone. Solution concentrated until solid precipitated. Suspension was stirred overnight then solids collected by filtration. ¹⁰ Purified by silica gel chromatography eluting with DCM (5 CV), 2% MeOH in DCM (15 CV), 5% MeOH in DCM (10 CV) and MeOH (5 CV). ¹¹ Workup: H₂O added to reaction then concentrated. The residue was triturated in H₂O and the insoluble material was collected by filtration. ¹² Workup: Reaction diluted with H₂O and triturated for 30 min. Insoluble material was collected by filtration, washed with H₂O, MTBE and cHex. ¹³ Purified by silica gel chromatography eluting with acetone in 0% to 50% acetone in DCM (25 CV), 50% to 100% acetone in DCM (5 CV) then 100% acetone. ¹⁴ Workup: H₂O was added to the recation then concentrated. The resultant suspension diluted with H₂O then extracted with DCM. Organic layer was washed with brine, dried over Na₂SO₄, filtered and concetrated. ¹⁵ Workup: H₂O was added to the recation then concentrated. The resultant suspension diluted with H₂O, triterated and the insoluble material was collected by filtration. ¹⁶ Purified by silica gel chromatography eluting with 5% MeOH in DCM. ¹⁷ Isolated material was sonicated in aqueous 2M Na₂CO₃ for 10 min then filtered and washed with water. ¹⁸ Workup: H₂O was added and the resultant precipitate was collected by filtration. | | | |

### Preparation 269

### tert-Butyl 4-[4-[3-cyano-4-[1-(1-isopropyltriazol-4-yl)ethoxy]pyrazolo[1,5-a]pyridin-6-yl]-5-methyl-triazol-1-yl]piperidine-1-carboxylate

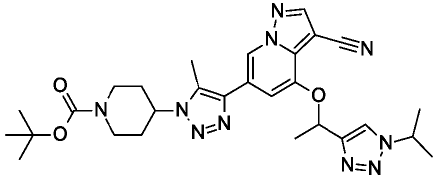

Cs₂CO₃ (1.41 g, 4.31 mmol) was added to a degassed soln of tert-butyl 4-(4-(3-cyano-4-hydroxypyrazolo[1,5-a]pyridin-6-yl)-5-methyl-1H-1,2,3-triazol-1-yl)piperidine-1-carboxylate (665 mg, 1.57 mmol) and 4-(1-chloroethyl)-1-isopropyl-1H-1,2,3-triazole (495 mg, 2.35 mmol) in DMF (5 ml) and the mixture was stirred at 65 °C for 2 h. The reaction was diluted with EA (25 ml) and washed with H₂O (25 ml) and brine (25 ml). The organic layer was dried over MgSO₄, filtered, concentrated in vacuo. The residue was purified by silica gel chromatography eluting with MeOH in DCM to give the title compound as pale-yellow oil (601 mg, 68%). MS ES+ m/z 561.5 [M+H]⁺.

The following compounds were prepared in a manner essentially analogous to the method of Preparation 269 using the appropriate reagents, adjusting reaction time to determine completion of the reaction, and adjusting the purification system as appropriate.

**Table 41**

| Prep # | Chemical Name | Structure | MS ES+ m/z |
|---|---|---|---|
| 270¹ | tert-Butyl 4-[4-[3-cyano-4-[1-(2-isopropyltriazol-4-yl)ethoxy] pyrazolo[1,5-a]pyridin-6-yl]-5-methyl-triazol-1-yl]piperidine-1-carboxylate | 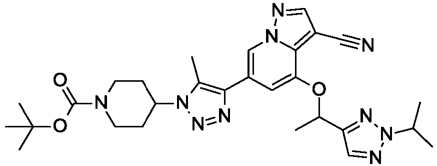 | 505.4 [M - tbutyl + H]⁺ |
| 271¹ | tert-Butyl 4-[4-[3-cyano-4-[1-(1-methylpyrazolo[3,4-c]pyridin-4-yl)ethoxy] pyrazolo[1,5-a]pyridin-6-yl]-5-methyl-triazol-1-yl] piperidine-1-carboxylate | 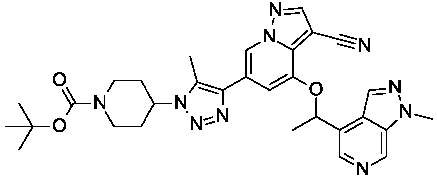 | 583.5 [M+H]⁺ |
| 272¹ | tert-Butyl 4-[4-[3-cyano-4-[1-(1-methylpyrrolo[2,3-c]pyridin-4-yl) ethoxy] pyrazolo[1,5-a]pyridin-6-yl]-5-methyl-triazol-1-yl] piperidine-1-carboxylate | 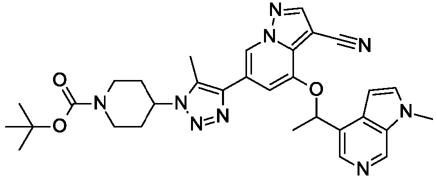 | 582.5 [M+H]⁺ |
| 273¹ | tert-Butyl 4-[4-[3-cyano-4-[1-[1-(trifluoromethyl) pyrazol-3-yl]ethoxy] pyrazolo[1,5-a]pyridin-6-yl]-5-methyl-triazol-1-yl] piperidine-1-carboxylate | 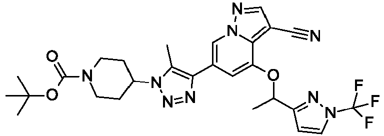 | 586.4 [M+H]⁺ |
| 274¹ | tert-Butyl 4-[4-[3-cyano-4-[1-(4-cyclopropyl-5-fluoro-2-pyridyl)ethoxy] pyrazolo [1,5-a]pyridin-6-yl]-5-methyl-triazol-1-yl] piperidine-1-carboxylate | 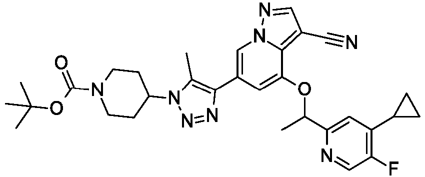 | 587.5 [M+H]⁺ |
| 275¹ | tert-Butyl 4-[4-[3-cyano-4-[1-(7-fluoro-4-isoquinolyl) ethoxy]pyrazolo[1,5-a]pyridin-6-yl]-5-methyl-triazol-1-yl]piperidine-1-carboxylate | 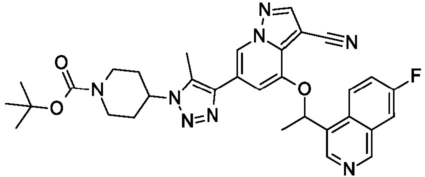 | 597.5 [M+H]⁺ |
| 276² | 4-[1-(6-Bromopyrazin-2-yl)ethoxy]-6-[5-methyl-1-[1-(oxetan-3-yl)-4-piperidyl]triazol-4-yl]pyrazolo[1,5-a]pyridine-3-carbonitrile | 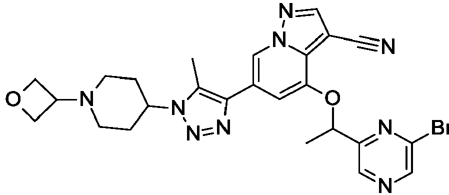 | (⁷⁹Br/⁸¹Br) 564/ 566 [M+H]⁺ |
| 277³ | tert-Butyl 4-[4-[3-cyano-4-[1-(2-cyclopropylthiazol-4-yl)ethoxy]pyrazolo[1, 5-a]pyridin-6-yl]-5-methyl-triazol-1-yl]piperidine-1-carboxylate | 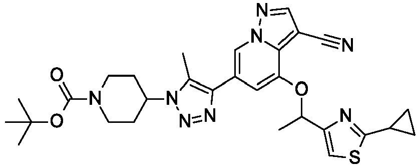 | 575 [M+H]⁺ |
| 278⁴ | tert-Butyl 4-[4-[3-cyano-4-[2-methyl-2-(2-pyridyl)propoxy]pyraz olo[1,5-a]pyridin-6-yl]-5-methyl-triazol-1-yl]piperidine-1-carboxylate | 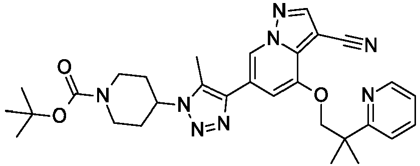 | a |
| 279⁵ | tert-Butyl 4-[4-[3-cyano-4-[1-(2-methoxythiazol-4-yl)ethoxy]pyrazolo[1, 5-a]pyridin-6-yl]-5-methyl-triazol-1-yl]piperidine-1-carboxylate | 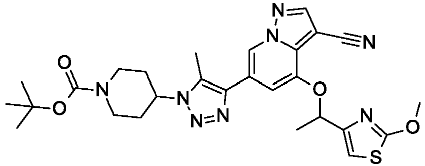 | 565 [M+H]⁺ |
| 280⁶ | tert-Butyl 4-[4-[3-cyano-4-[(1R)-1-[5-(trifluoromethyl)-3-pyridyl]ethoxy]pyrazo lo[1,5-a]pyridin-6-yl]-5-methyl-triazol-1-yl]piperidine-1-carboxylate | 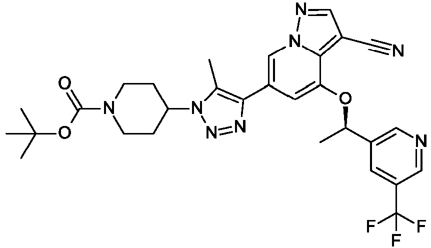 | 597 [M+H]⁺ |

| | | | |
|---|---|---|---|
| ¹ Purified by silica gel chromatography eluting with MeOH in DCM. ² Purified by silica gel chromatography eluting with 20% to 100% EA in cHex. ³ Purified by silica gel chromatography eluting with 20% to 40% (3:1 EA : EtOH) in cHex. ⁴ Purified by silica gel chromatography eluting with 5% EA in cHex (2CV) at 5%; 5% to 50% EA in cHex (20 CV), 50% to 100% EA in cHex (10CV), then 100% EA. ⁵ Purified by silica gel chromatography eluting with 10% 3:1 EA/EtOH in cHex (3 CV), 10% to 20% gradient 3:1 EA/EtOH in cHex (12 CV), 20% 3:1 EA/EtOH in cHex (2 CV), 20% to 50% gradient 3:1 EA/EtOH in cHex (18 CV). ⁶ Upon cooling to RT the reaction was treated with H₂O. Insoluble material was collected by filtration to afford title compound. ^{a} Material used in subsequent step without further characterization. | | | |

### Preparation 281

### tert-Butyl 4-[4-[4-[2-(3,5-difluoro-2-pyridyl)-2-methoxy-ethoxy]-3-fluoro-pyrazolo[1,5-a]pyridine-6-yl]-5-methyl-triazol-1-yl]piperidine-1-carboxylate

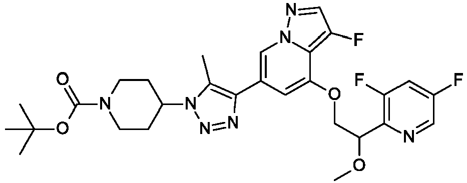

A soln of PPh3 (1.04 g, 3.96 mmol) in THF (10 ml) at 0°C was treated dropwise with DIAD (0.77 g, 3.83 mmol) under N₂. The reaction was stirred for 0.5 h at 0 °C before adding to a mixture of 2-(3,5-difluoro-2-pyridyl)-2-methoxy-ethanol (0.30 g, 1.59 mmol) and tert-butyl 4-[4-(3-fluoro-4-hydroxy-pyrazolo[1,5-a]pyridine-6-yl)-5-methyl-triazol-1-yl]piperidine-1-carboxylate (0.55 g, 1.32 mmol) in THF (10 ml). The resulting mixture was stirred for 2 h at RT then concentrated. The residue was purified by reversed phase flash C18 chromatography with the following conditions: column, C18; eluting with a gradient of 45% to 55% CAN in H₂O (0.1% FA) to afford the title compound (480 mg, 62%) as a yellow solid. MS ES+ m/z 588 [M+H]⁺.

The following compounds were prepared in a manner essentially analogous to the method of Preparation 281 using the appropriate reagents, adjusting reaction time to determine completion of the reaction, and adjusting the purification system as appropriate.

**Table 42**

| Prep # | Chemical Name | Structure | MS ES+ m/z |
|---|---|---|---|
| 282¹ | tert-Butyl 4-[4-[5-[2-(5-fluoro-2-pyridyl)-2-methoxy-ethoxy]imidazo[1,2-a]pyridine-7-yl]-5-methyl-triazol-1-yl]piperidine-1-carboxylate | 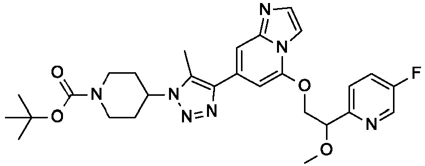 | 552 [M+H]⁺ |
| 283² | tert-Butyl 4-[4-[5-[2-(3,5-difluoro-2-pyridyl)-2-methoxy-ethoxy]imidazo[1,2-a]pyridin-7-yl]-5-methyl-triazol-1-yl]piperidine-1-carboxylate | 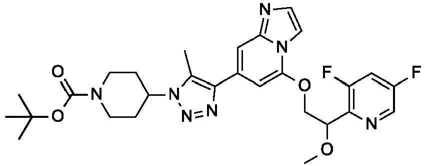 | 570 [M+H]⁺ |

| | | | |
|---|---|---|---|
| ¹ Purified by flash reversed phase C18 chromatography eluting with 30% to 50% CAN in H₂O (0.1% NH₄HCO₃). ² Purified by flash reversed phase C18 chromatography eluting with 60% to 70% CAN in H₂O. | | | |

### Preparation 284

### tert-Butyl 4-[4-[3-chloro-5-[2-(5-fluoro-2-pyridyl)-2-methoxy-ethoxy]imidazo[1,2-a]pyridin-7-yl]-5-methyl-triazol-1-yl]piperidine-1-carboxylate

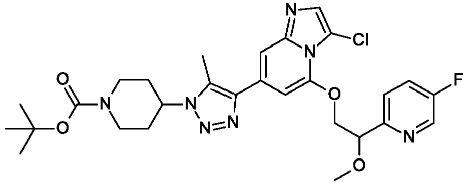

A soln of tert-butyl 4-[4-[5-[2-(5-fluoro-2-pyridyl)-2-methoxy-ethoxy]imidazo[1,2-a]pyridin-7-yl]-5-methyl-triazol-1-yl]piperidine-1-carboxylate (1.4 g, 2.54 mmol) in DMF (15 ml) at 0°C was treated with DCDMH (400 mg, 2.03 mmol). After stirring at 0 °C for 0.5 h, the residue was purified by reversed phase flash C18 chromatography with the following conditions: column, C18; eluting with a gradient of 60% to 70% CAN in H₂O to afford the title compound (700 mg, 47%) as a yellow solid. MS ES+ m/z 586 [M+H]⁺.

The following compounds were prepared in a manner essentially analogous to the method of Preparation 284 using the appropriate reagents, adjusting reaction time to determine completion of the reaction, and adjusting the purification system as appropriate.

**Table 43**

| Prep # | Chemical Name | Structure | MS ES+ m/z |
|---|---|---|---|
| 285 | tert-Butyl 4-[4-[3-chloro-5-[2-(3,5-difluoro-2-pyridyl)-2-methoxy-ethoxy]imidazo[1,2-a]pyridin-7-yl]-5-methyl-triazol-1-yl]piperidine-1-carboxylate | 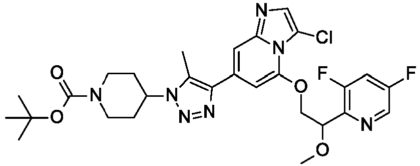 | 604 [M+H]⁺ |
| 286^{1,2} | tert-Butyl 4-[4-[3-chloro-5-[2-(3,5-difluoro-2-pyridyl)-2-methoxy-ethoxy]imidazo[1,2-a]pyridin-7-yl]-5-methyl-triazol-1-yl]-4-methylpiperidine-1-carboxylate | 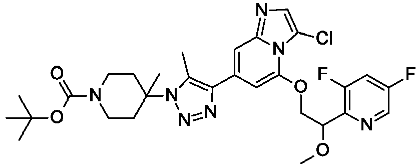 | 618 [M+H]⁺ |

| | | | |
|---|---|---|---|
| ¹ Purified by reversed phase C18 chromatography eluting with 45% to 50% AcCN in H₂O. ² Purified by Prep-TLC (PE / EA 1:3) | | | |

### Preparation 287

### tert-Butyl 4-[4-[3-cyano-4-[2-(3,5-difluoro-2-pyridyl)-2-hydroxy-ethoxy]pyrazolo[1,5-a]pyridin-6-yl]-5-methyl-triazol-1-yl]piperidine-1-carboxylate, Isomer 1

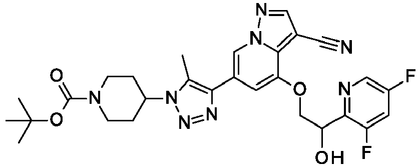

In a sealed tube, chloro(N-[(1R,2R)-2-[(S)-[2-[[1,2,3,4,5,6-η)-4-methylphenyl]methoxy]ethyl]amino]-1,2-diphenylethylmethanesulfonamidato) ruthenium(II) (25 mg, 0.04 mmol, 98 mass%) was added to a stirred mixture of tert-butyl 4-[4-[3-cyano-4-[2-(3,5-difluoro-2-pyridyl)-2-oxo-ethoxy]pyrazolo[1,5-a]pyridin-6-yl]-5-methyl-triazol-1-yl]piperidine-1-carboxylate (3.5 g, 4.2 mmol) in FA NEt₃ complex (5:2 ratio) (18 ml, 42.21 mmol) and DCM (14 ml, 218 mmol). The reaction was heated at 45 °C under N₂ for 75 minutes. Upon cooling to RT, the reaction was diluted with DCM, washed with H₂O, saturated aq NaHCO₃, H₂O and brine. The organic layer was dried over MgSO₄, filtered and concentrated. The residue was purified by silica gel chromatography eluting with 20% acetone in DCM to afford the title compound as an orange colored solid (3.23g, 99%). MS ES+ m/z 581 [M+H]⁺.

The following compounds were prepared in a manner essentially analogous to the method of Preparation 287 using the appropriate reagents, adjusting reaction time to determine completion of the reaction, and adjusting the purification system as appropriate.

**Table 44**

| Prep # | Chemical Name | Structure | MS ES+ m/z |
|---|---|---|---|
| 288¹ | tert-Butyl 4-[4-[4-[2-(3-chloro-2-pyridyl)-2-hydroxy-ethoxy]-3-cyano-pyrazolo[1,5-a]pyridin-6-yl]-5-methyl-triazol-1-yl]piperidine-1-carboxylate, Isomer 1 | 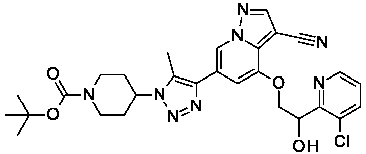 | 579 [M+H]⁺ |
| 289² | tert-Butyl 4-[4-[3-cyano-4-[2-hydroxy-2-(3-methyl-2-pyridyl)ethoxy]pyrazolo [1,5-a]pyridin-6-yl]-5-methyl-triazol-1-yl]piperidine-1-carboxylate, Isomer 1 | 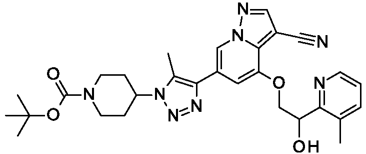 | 559 [M+H]⁺ |
| 290² | tert-Butyl 4-[4-[3-bromo-4-[2-(5-fluoro-2-pyridyl)-2-hydroxy-ethoxy]pyrazolo[1,5-a]pyridin-6-yl]-5-methyl-triazol-1-yl]piperidine-1-carboxylate, Isomer 1 | 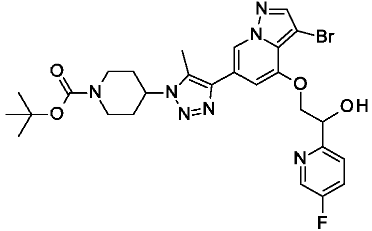 | (⁷⁹Br/⁸¹Br) 616/618 [M+H]⁺ |

| | | | |
|---|---|---|---|
| ¹ Purified by silica gel chromatography eluting with MeOH in DCM. ² Crude taken on to next step after aq workup. | | | |

### Preparation 291

### 4-[2-(5-Fluoro-2-pyridyl)-2-hydroxy-ethoxy]-6-[5-methyl-1-[1-(oxetan-3-yl)-4-piperidyl]triazol-4-yl]pyrazolo[1,5-a]pyridine-3-carbonitrile

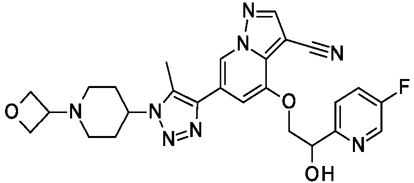

To a suspension of 4-[2-(5-fluoro-2-pyridyl)-2-oxo-ethoxy]-6-[5-methyl-1-[1-(oxetan-3-yl)-4-piperidyl]triazol-4-yl]pyrazolo[1,5-a]pyridine-3-carbonitrile (4.01 g, 6.58 mmol) in MeOH (65 ml) was added in one portion NaBH₄ (1.0 g, 26.43 mmol) at 0 °C. The mixture was stirred at 0 °C for 5 min and then at RT for 1 h. The reaction was diluted with H₂O (100 ml). The resultant precipitate was collected by filtration and washed with H₂O / MeOH (4:1) (20 ml). The resulting solid was dried at 40°C under vacuum to afford the title compound (3.40 g, 91%). MS ES+ m/z 519 [M+H]⁺.

### Preparation 292

### tert-Butyl 4-[4-[3-chloro-4-[2-(5-fluoro-2-pyridyl)-2-hydroxy-ethoxy]pyrazolo[1,5-a] pyridine-6-yl]-5-methyl-triazol-1-yl]piperidine-1-carboxylate

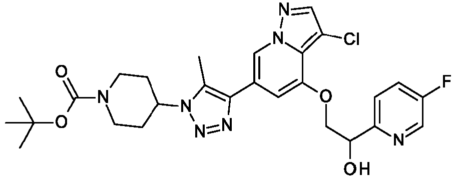

A soln of tert-butyl 4-[4-[3-chloro-4-[2-(5-fluoro-2-pyridyl)-2-oxo-ethoxy]pyrazolo[1,5-a]pyridine-6-yl]-5-methyl-triazol-1-yl]piperidine-1-carboxylate (1 g, 1.75 mmol) in MeOH (20 ml) was treated with NaBH₄ (66.37 mg, 1.75 mmol) at RT under N₂. After stirring at RT for 1 h, the reaction was quenched with H₂O (50 ml) and extracted with EA (3 x 100 ml). The combined organic layers were washed with brine (2 x 50 ml), dried over anhydrous Na₂SO₄, filtered, and concentrated to obtain the title compound (1.0 g, 100%) as a brown solid. MS ES+ m/z 572 [M+H]⁺.

The following compounds were prepared in a manner essentially analogous to the method of Preparation 292 using the appropriate reagents, adjusting reaction time to determine completion of the reaction, and adjusting the purification system as appropriate. DCM can be used as a cosolvent.

**Table 45**

| Prep # | Chemical Name | Structure | MS ES+ m/z |
|---|---|---|---|
| 293 | tert-Butyl 4-[4-[3-cyano-4-[2-(2,4-difluorophenyl)-2-hydroxy-ethoxy] pyrazolo[1,5-a]pyridine-6-yl]-5-methyl-triazol-1-yl]piperidine-1-carboxylate | 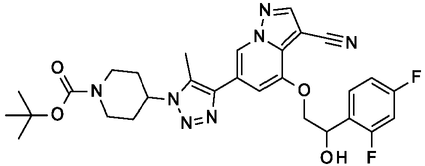 | 524 [M+H]⁺ |
| 294 | tert-Butyl 4-[4-[3-cyano-4-[2-(5-fluoro-2-pyridyl)-2-hydroxy-ethoxy] pyrazolo[1,5-a]pyridin-6-yl]-5-methyl-triazol-1-yl]piperidine-1-carboxylate | 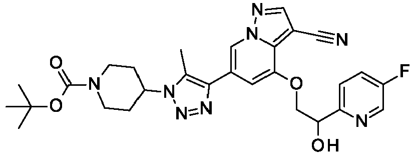 | 563 [M+H]⁺ |
| 295 | tert-Butyl 4-[4-[3-fluoro-4-[2-(5-fluoro-2-pyridyl)-2-hydroxy-ethoxy] pyrazolo[1,5-a]pyridin-6-yl]-5-methyl-triazol-1-yl]piperidine-1-carboxylate | 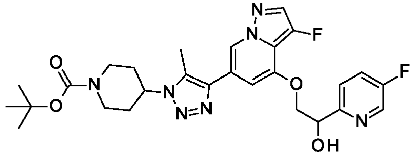 | 556 [M+H]⁺ |
| 296 | tert-Butyl 4-[4-[3-chloro-4-[2-(3,5-difluoro-2-pyridyl)-2-hydroxy-ethoxy]pyrazolo[1,5-a]pyridin-6-yl]-5-methyl-triazol-1-yl]piperidine-1-carboxylate | 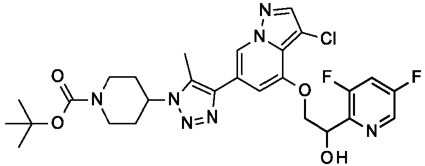 | (³⁵Cl/³⁷Cl) 590/592 [M+H]⁺ |
| 297 | 2-[3-Chloro-6-[5-methyl-1-[1-(oxetan-3-yl)azetidin-3-yl]triazol-4-yl]pyrazolo[1,5-a]pyridin-4-yl]oxy-1-(5-fluoro-2-pyridyl)ethanol | 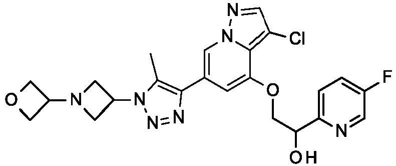 | (³⁵Cl/³⁷Cl) 500/502 [M+H]⁺ |
| 298 | 2-[3-Chloro-6-[5-methyl-1-[1-(oxetan-3-yl)azetidin-3-yl]triazol-4-yl]pyrazolo[1,5-a]pyridin-4-yl]oxy-1-(3,5-difluoro-2-pyridyl)ethanol | 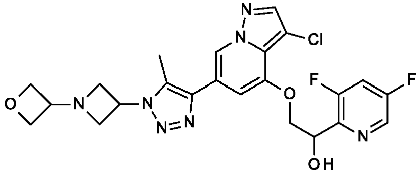 | (³⁵Cl/³⁷Cl) 518/520 [M+H]⁺ |
| 299 | tert-Butyl 4-[4-[4-[2-(3-chloro-5-fluoro-2-pyridyl)-2-hydroxy-ethoxy]-3-cyano-pyrazolo[1,5-a]pyridin-6-yl]-5-methyl-triazol-1-yl]piperidine-1-carboxylate | 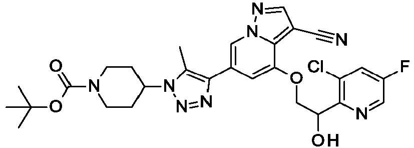 | (³⁵Cl/³⁷Cl) 597/599 [M+H]⁺ |
| 300 | tert-Butyl 4-[4-[4-[2-(4-chloro-5-fluoro-2-pyridyl)-2-hydroxy-ethoxy]-3-cyano-pyrazolo[1,5-a]pyridin-6-yl]-5-methyl-triazol-1-yl]piperidine-1-carboxylate | 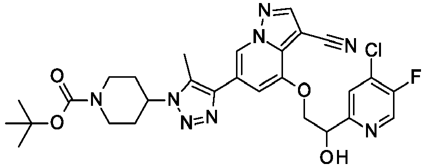 | (³⁵Cl/³⁷Cl) 597/599 [M+H]⁺ |
| 301¹ | 1-(5-Fluoro-2-pyridyl)-2-[6-[5-methyl-1-[1-(oxetan-3-yl)-4-piperidyl]triazol-4-yl]-3-(trifluoromethyl)pyrazol o[1,5-a]pyridin-4-yl]oxy-ethanol | 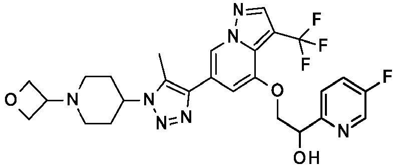 | 562 [M+H]⁺ |
| 302 | 4-(2-Cyclopentyl-2-hydroxy-ethoxy)-6-[5-methyl-1-[1-(oxetan-3-yl)-4-piperidyl]triazol-4-yl]pyrazolo[1,5-a]pyridine-3-carbonitrile | 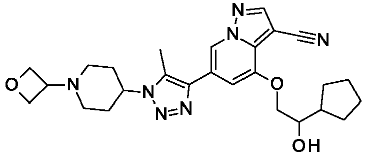 | 492 [M+H]⁺ |
| 303 | tert-Butyl 4-[4-[3-chloro-4-(2-hydroxy-2-isothiazol-3-yl-ethoxy)pyrazolo[1,5-a]pyridin-6-yl]-5-methyl-triazol-1-yl]piperidine-1-carboxylate | 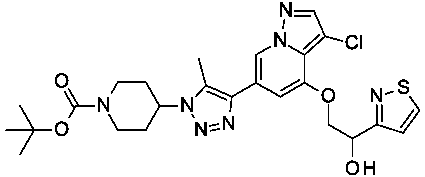 | 560 [M+H]⁺ |
| 304² | 4-[2-(3,5-Difluoro-2-pyridyl)-2-hydroxy-ethoxy]-6-[5-methyl-1-[1-(oxetan-3-yl)-4-piperidyl]triazol-4-yl]pyrazolo[1,5-a]pyridine-3-carbonitrile | 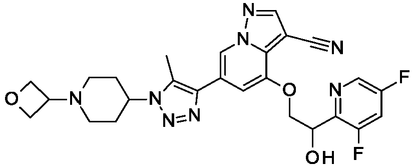 | 537 [M+H]⁺ |
| 305³ | 1-(3-Chloro-5-fluoro-2-pyridyl)-2-[3-fluoro-6-[5-methyl-1-[1-(oxetan-3-yl)-4-piperidyl]triazol-4-yl]pyrazolo[1,5-a]pyridin-4-yl]oxy-ethanol | 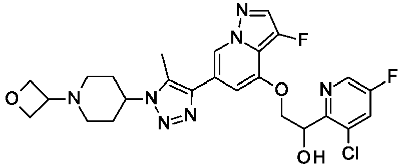 | 546 [M+H]⁺ |
| 306⁴ | 2-[3-Fluoro-6-[5-methyl-1-[1-(oxetan-3-yl)-4-piperidyl]triazol-4-yl]pyrazolo[1,5-a]pyridin-4-yl]oxy-1-(5-fluoro-2-pyridyl)ethanol | 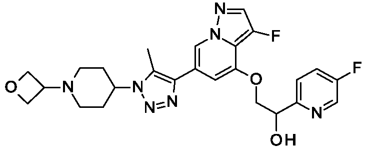 | 512 [M+H]⁺ |

| | | | |
|---|---|---|---|
| ¹Purified by silica gel chromatography eluting with 0% to 10% MeOH in DCM. ² Purified by silica gel chromatography eluting with DCM (5 CV), 2% MeOH in DCM (10 CV), 5% MeOH in DCM (10 CV). ³ Purified by silica gel chromatography eluting with 0% to 40% (10% MeOH in DCM) in DCM. ⁴ Purified by silica gel chromatography eluting with 0% to 40% (10% MeOH in DCM) in DCM. | | | |

### Preparation 307

### tert-Butyl 4-[4-[4-[2-(3,5-difluoro-2-pyridyl)-2-hydroxy-propoxy]-3-fluoro-pyrazolo[1,5-a]pyridin-6-yl]-5-methyl-triazol-1-yl]piperidine-1-carboxylate

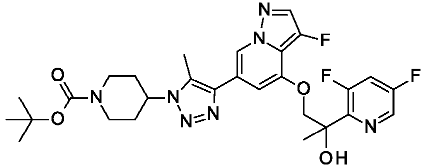

A soln of tert-butyl 4-[4-[4-[2-(3,5-difluoro-2-pyridyl)-2-oxo-ethoxy]-3-fluoro-pyrazolo[1,5-a]pyridin-6-yl]-5-methyl-triazol-1-yl]piperidine-1-carboxylate (0.61 g, 1.07 mmol) in DCM (10 ml) was slowly added to a solution of MeMgBr in THF (3 mL, 4.2 mmol, 1.4 mol/L) that was precooled cooled to 0 °C under N₂. Additional DCM (2 ml) was added, and the reaction was stirred overnight. The reaction was quenched with aq. NH₄Cl, extracted with DCM, and washed with H₂O and brine. The organic phase was concentrated to afford a residue. The residue was purified by silica gel chromatography eluting with 0% to 20% acetone in DCM to afford the title compound (175 mg, 0.25 mmol, 24%, 85 mass%) as a yellow oil. MS ES+ m/z 588 [M+H]⁺.

The following compounds were prepared in a manner essentially analogous to the method of Preparation 307 using the appropriate reagents, adjusting reaction time to determine completion of the reaction, and adjusting the purification system as appropriate. DCM can be used as a cosolvent.

**Table 46**

| Prep # | Chemical Name | Structure | MS ES+ m/z |
|---|---|---|---|
| 308¹ | tert-Butyl 4-[4-[4-[2-(5-fluoro-2-pyridyl)-2-hydroxy-propoxy]-3-methyl-pyrazolo[1,5-a]pyridin-6-yl]-5-methyl-triazol-1-yl]piperidine-1-carboxylate | 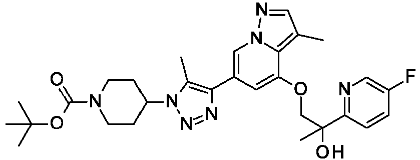 | 566 [M+H]⁺ |

| | | | |
|---|---|---|---|
| ¹Purified by silica gel chromatography eluting with 30% (10% MeOH in DCM) in DCM. | | | |

### Preparation 309

### tert-Butyl 4-[4-[3-chloro-4-[2-(3,5-difluoro-2-pyridyl)-2-methylsulfonyloxy-ethoxy]pyrazolo[1,5-a]pyridin-6-yl]-5-methyl-triazol-1-yl]piperidine-1-carboxylate

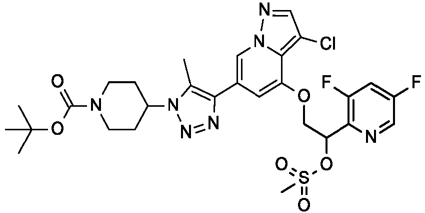

A solution of tert-butyl 4-[4-[3-chloro-4-[2-(3,5-difluoro-2-pyridyl)-2-hydroxy-ethoxy]pyrazolo[1,5-a]pyridin-6-yl]-5-methyl-triazol-1-yl]piperidine-1-carboxylate (0.60 g, 0.74 mmol) in DCM (6 ml) was successively treated at 0 °C with NEt₃ (0.152 g, 1.51 mmol) and MsCl (0.13 g, 1.2 mmol). After 15 min, the cooling bath was removed, and the mixture was allowed to warm to RT. After 1 h the mixture was washed with H₂O (1.5 ml), dried over MgSO₄, and concentrated in vacuo to afford the title compound as a green solid. The product was used in the next step without further purification. MS ES+ m/z (³⁵Cl/³⁷Cl) 668/670 [M+H]⁺.

### Preparation 310

### tert-Butyl 4-[4-[3-cyano-4-[2-(3,5-difluoro-2-pyridyl)-2-isopropoxy-ethoxy]pyrazolo[1,5-a]pyridin-6-yl]-5-methyl-triazol-1-yl]piperidine-1-carboxylate, Isomer 1

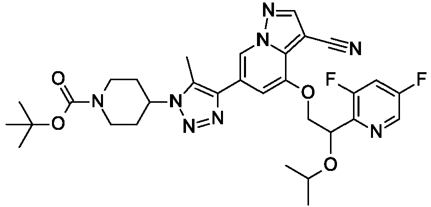

To a mixture of tert-butyl 4-[4-[3-cyano-4-[(2S)-2-(3,5-difluoro-2-pyridyl)-2-hydroxy-ethoxy]pyrazolo[1,5-a]pyridin-6-yl]-5-methyl-triazol-1-yl]piperidine-1-carboxylate (1.0 g, 1.3 mmol, 75 mass%), Ag₂O (600 mg, 2.59 mmol) and 2-iodopropane (2.6 ml, 26 mmol,) in DMF (10 ml) at RT under N₂ was added NaH in mineral oil (62 mg, 1.55 mmol, 60 mass%). The reaction was stirred at RT for 10 min then additional NaH in mineral oil (62 mg, 1.55 mmol, 60 mass%) was added. Portions of NaH in mineral oil (62 mg, 1.55 mmol, 60 mass%), were added approximately every 10-20 min. After 90 minutes and 7 additions of NaH, H₂O was added to the mixture. The mixture was extracted with EA, organic phase washed with brine (3x), dried over MgSO₄, filtered and concentrated. The residue was purified by silica gel chromatography eluting with 20% to 50% EA in cHex. The isolated material was repurified by silica gel chromatography eluting with 20% EA in MTBE. An impurity had co-eluted with the title compound (720mg, 56%, 63% purity). MS ES+ m/z 523 [M+H]⁺.

### Preparation 311

### tert-Butyl 4-[4-[3-chloro-4-[2-(3,5-difluoro-2-pyridyl)-2-methoxy-ethoxy]pyrazolo[1,5-a]pyridin-6-yl]-5-methyl-triazol-1-yl]piperidine-1-carboxylate

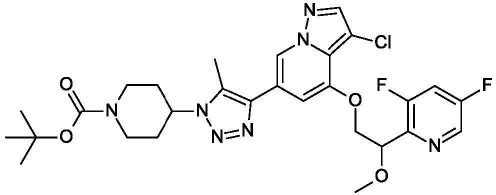

A soln of tert-butyl 4-[4-[3-chloro-4-[2-(3,5-difluoro-2-pyridyl)-2-hydroxy-ethoxy]pyrazolo[1,5-a]pyridin-6-yl]-5-methyl-triazol-1-yl]piperidine-1-carboxylate (0.63 g, 0.78 mmol) in N,N-dimethylacetamide (6 ml) was treated with NaH (65 mg, 1.63 mmol, 60% wt) followed by CH₃I (0.08 ml, 1.28 mmol) at RT. After stirring at RT for 90 min, the reaction was quenched with MeOH (0.5 ml) and purified by reversed phase C18 chromatography eluting with a gradient of 30% to 90% ACN in H₂O (NH₄HCO₃ buffer pH 9) to afford the title compound (192 mg, 38%) as an orange solid. MS ES+ m/z 604/606 [M+H]⁺.

The following compounds were prepared in a manner essentially analogous to the method of Preparation 311 using the appropriate reagents, adjusting reaction time to determine completion of the reaction, and adjusting the purification system as appropriate.

**Table 47**

| Prep # | Chemical Name | Structure | MS ES+ m/z |
|---|---|---|---|
| 312¹ | 3-Chloro-4-[2-(5-fluoro-2-pyridyl)-2-methoxy-ethoxy]-6-[5-methyl-1-[1-(oxetan-3-yl)azetidin-3-yl]triazol-4-yl]pyrazolo[1,5-a]pyridine | 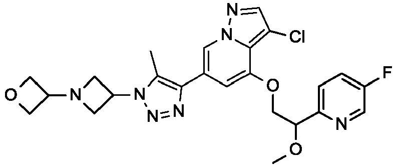 | (³⁵Cl/³⁷Cl) 514/516 [M+H]⁺ |
| 313^{2,3} | tert-Butyl 4-[4-[3-cyano-4-[2-(5-fluoro-2-pyridyl)-2-isopropoxy-ethoxy]pyrazolo [1,5-a]pyridin-6-yl]-5-methyl-triazol-1-yl]piperidine-1-carboxylate | 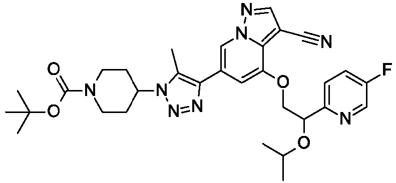 | 605 [M+H]⁺ |
| 314⁴ | 4-[2-(3-Chloro-5-fluoro-2-pyridyl)-2-methoxy-ethoxy]-3-fluoro-6-[5-methyl-1-[1-(oxetan-3-yl)-4-piperidyl]triazol-4-yl]pyrazolo[1,5-a]pyridine | 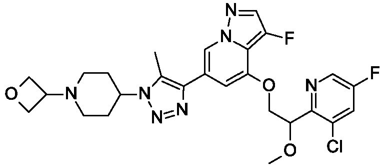 | 560 [M+H]⁺ |
| 315^{5, 6} | tert-Butyl 4-[4-[3-chloro-4-[2-(5-fluoro-2-pyridyl)-2-methoxy-ethoxy]pyrazolo [1,5-a]pyridin-6-yl]-5-methyl-triazol-1-yl] piperidine-1-carboxylate | 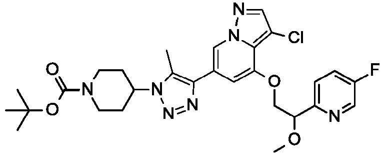 | 587 [M+H]⁺ |
| 316^{5, 7} | tert-Butyl 4-[4-[4-[2-(3-chloro-2-pyridyl)-2-methoxy-ethoxy]-3-cyano-pyrazolo[1,5-a]pyridin-6-yl]-5-methyl-triazol-1-yl]piperidine-1-carboxylate, Isomer 1 | 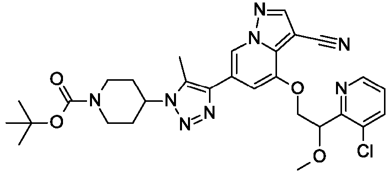 | 594 [M+H]⁺ |
| 317^{5, 6} | tert-Butyl 4-[4-[3-cyano-4-[2-methoxy-2-(3-methyl-2-pyridyl) ethoxy]pyrazolo[1,5-a]pyridin-6-yl]-5-methyl-triazol-1-yl]piperidine-1-carboxylate, Isomer 1 | 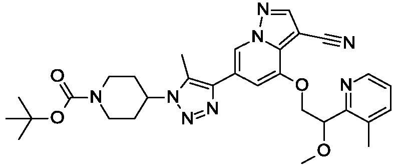 | 573 [M+H]⁺ |

| | | | |
|---|---|---|---|
| ¹ Purified by reversed phase C18 chromatography eluting with aq NH₄HCO₃ pH 9 (5 CV), then 30% to 60% ACN in aq NH₄HCO₃ pH 9. ² Ag₂O (3.95 eq) was added to reaction. ³ Purification by silica gel chromatography eluting with 0% to 50% [DCM : MeOH (9:1)] in DCM. ⁴ Purified by SCX chromatography. Non-basic impurities were washed off with DCM, 5% MeOH in DCM. The title compound was eluted with methanolic ammonia (2M). ⁵ THF was used as the solvent. ⁶ Workup: Reaction was extracted with DCM, washed with H2O (3x), brine, dried over MgSO4, filtered and concentrated. ⁷ Workup: Reaction was diluted with H₂O, extracted with DCM, washed with brine, dried over MgSO4, filtered and concentrated. | | | |

### Preparation 318

### tert-Butyl 4-[4-[3-chloro-4-[2-(3,5-difluoro-2-pyridyl)-2-(dimethylamino)ethoxy]pyrazolo[1,5-a]pyridin-6-yl]-5-methyl-triazol-1-yl]piperidine-1-carboxylate

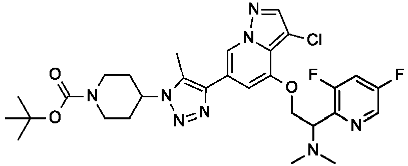

A mixture of tert-butyl 4-[4-[3-chloro-4-[2-(3,5-difluoro-2-pyridyl)-2-methylsulfonyloxy-ethoxy]pyrazolo[1,5-a]pyridin-6-yl]-5-methyl-triazol-1-yl]piperidine-1-carboxylate (0.50 g, 0.74 mmol) and dimethylamine in THF (2M soln, 2 ml) was stirred at RT for 48 h. The crude mixture was purified by silica gel chromatography eluting with a gradient of 0% to 100% acetone in DCM to afford the title compound as a yellow solid (0.15 g, 27.8%, purity >85%). MS ES+ m/z (³⁵Cl/³⁷Cl) 617/619 [M+H]⁺.

### Preparation 319

### 4-[1-(1-Isopropyltriazol-4-yl)ethoxy]-6-[5-methyl-1-(4-piperidyl)triazol-4-yl]pyrazolo[1,5-a]pyridine-3-carbonitrile hydrochloride

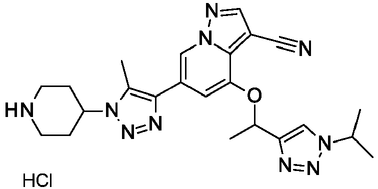

HCl in dioxane (4M, 2.0 ml, 5.98 mmol) was added to a soln of tert-butyl 4-(4-(3-cyano-4-(1-(1-isopropyl-1H-1,2,3-triazol-4-yl)ethoxy)pyrazolo[1,5-a]pyridine-6-yl)-5-methyl-1H-1,2,3-triazol-1-yl)piperidine-1-carboxylate (600 mg, 1.07 mmol) in DCM (10 ml) and the mixture was stirred at RT for 2 h. The reaction was concentrated in vacuo to afford the title compound as a hygroscopic beige solid (689 mg, quantitative). MS ES+ m/z 461.4 [M+H]⁺.

The following compounds were prepared in a manner essentially analogous to the method of Preparation 319 using the appropriate reagents, adjusting reaction time to determine completion of the reaction, and adjusting the purification system as appropriate. For compounds where the amine salt was isolated, the formation of the mono-, di-, or trivalent salt is dependent on the pKa of the amine and the acid used to form the salt. The exact mono-, di-, or trivalent salt form for each example was not identified.

**Table 48**

| Prep # | Chemical Name | Structure | MS ES+ m/z |
|---|---|---|---|
| 320 | 4-[1-(2-Isopropyltriazol-4-yl)ethoxy]-6-[5-methyl-1-(4-piperidyl)triazol-4-yl]pyrazolo[1,5-a]pyridine-3-carbonitrile hydrochloride | 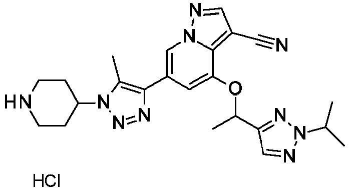 | 461 [M+H]⁺ |
| 321 | 6-[5-Methyl-1-(4-piperidyl)triazol-4-yl]-4-[1-(1-methylpyrazolo[3,4-c]pyridine-4-yl)ethoxy]pyrazolo [1,5-a]pyridine-3-carbonitrile hydrochloride | 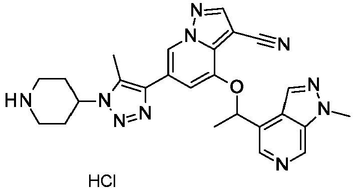 | 483 [M+H]⁺ |
| 322 | 6-[5-Methyl-1-(4-piperidyl)triazol-4-yl]-4-[1-(1-methylpyrrolo[2,3-c]pyridine-4-yl)ethoxy] pyrazolo[1,5-a]pyridine-3-carbonitrile hydrochloride | 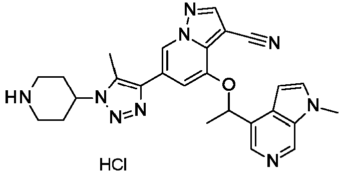 | 482 [M+H]⁺ |
| 323 | 2-[3-Fluoro-6-[5-methyl-1-(4-piperidyl)triazol-4-yl]pyrazolo[1,5-a]pyridine-4-yl]oxy-1-(5-fluoro-2-pyridyl)ethanol hydrochloride | 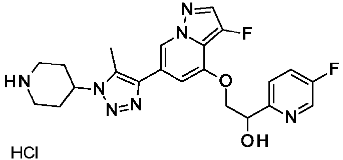 | 456 [M+H]⁺ |
| 324 | 3-Chloro-4-[2-(3,5-difluoro-2-pyridyl)-2-methoxy-ethoxy]-6-[5-methyl-1-(4-piperidyl)triazol-4-yl]pyrazolo[1,5-a]pyridine hydrochloride | 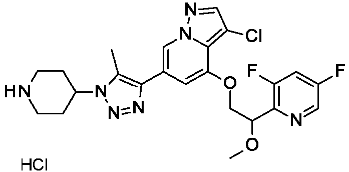 | (³⁵Cl/³⁷Cl) 504/506 [M+H]⁺ |
| 325 | 6-[5-Methyl-1-(4-piperidyl)triazol-4-yl]-4-[1-[1-(trifluoromethyl)pyrazol-3-yl]ethoxy]pyrazolo[1,5-a]pyridine-3-carbonitrile hydrochloride | 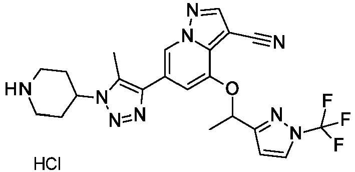 | 586 [M+H]⁺ |
| 326 | 4-[1-(4-Cyclopropyl-5-fluoro-2-pyridyl)ethoxy]-6-[5-methyl-1-(4-piperidyl)triazol-4-yl]pyrazolo[1,5-a]pyridine-3-carbonitrile hydrochloride | 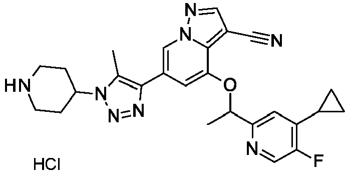 | 487 [M+H]⁺ |
| 327 | 4-[1-(7-Fluoro-4-isoquinolyl)ethoxy]-6-[5-methyl-1-(4-piperidyl)triazol-4-yl]pyrazolo[1,5-a]pyridine-3-carbonitrile hydrochloride | 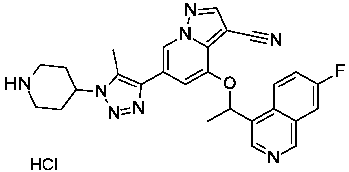 | 497 [M+H]⁺ |
| 328 | 4-[2-(3-Chloro-5-fluoro-2-pyridyl)-2-hydroxy-ethoxy]-6-[5-methyl-1-(4-piperidyl)triazol-4-yl]pyrazolo[1,5-a]pyridine-3-carbonitrile hydrochloride | 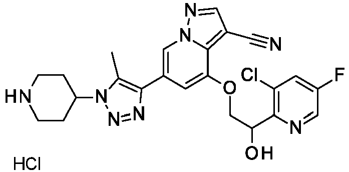 | 497 [M+H]⁺ |
| 329 | 4-[2-(4-Chloro-5-fluoro-2-pyridyl)-2-hydroxy-ethoxy]-6-[5-methyl-1-(4-piperidyl)triazol-4-yl]pyrazolo[1,5-a]pyridine-3-carbonitrile hydrochloride | 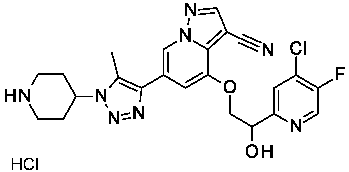 | (³⁵Cl/³⁷Cl) 497/499 [M+H]⁺ |
| 330 | 2-[3-Chloro-6-[5-methyl-1-(4-piperidyl)triazol-4-yl]pyrazolo[1,5-a]pyridin-4-yl]oxy-1-isothiazol-3-yl-ethanol hydrochloride | 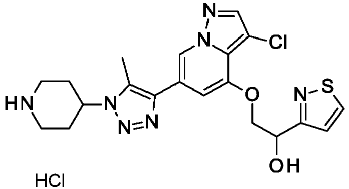 | 460 [M+H]⁺ |
| 331¹ | 2-[3-Chloro-6-[5-methyl-1-(4-piperidyl)triazol-4-yl]pyrazolo[1,5-a]pyridin-4-yl]oxy-1-(3,5-difluoro-2-pyridyl)-N,N-dimethyl-ethanamine hydrochloride | 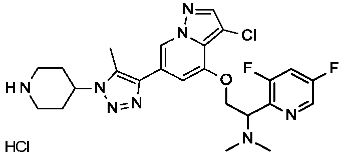 | (³⁵Cl/³⁷Cl) 517/519 [M+H]⁺ |
| 332 | 6-[5-Methyl-1-(4-piperidyl)triazol-4-yl]-4-[(1R)-1-[5-(trifluoromethyl)-3-pyridyl]ethoxy]pyrazolo[1 ,5-a]pyridine-3-carbonitrile hydrochloride | 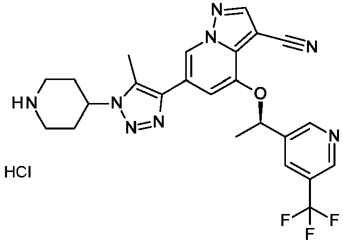 | 497 [M+H]⁺ |
| 333 | 1-[3-Chloro-6-[1-[(3S,4R)-3-fluoro-4-piperidyl]-5-methyl-triazol-4-yl]pyrazolo[1,5-a]pyridin-4-yl]oxy-2-(5-fluoro-2-pyridyl)propan-2-ol hydrochloride | 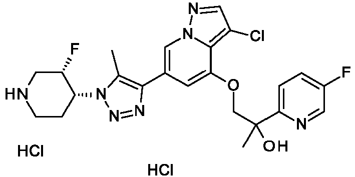 | 504 [M+H]⁺ |
| 334 | 2-(5-Fluoro-2-pyridyl)-1-[3-methyl-6-[5-methyl-1-(4-piperidyl)triazol-4-yl]pyrazolo[1,5-a]pyridin-4-yl]oxy-propan-2-ol hydrochloride | 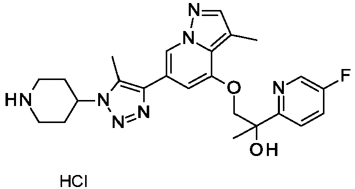 | 466 [M+H]⁺ |
| 335 | 4-[2-(3-Chloro-2-pyridyl)-2-methoxy-ethoxy]-6-[5-methyl-1-(4-piperidyl)triazol-4-yl]pyrazolo[1,5-a]pyridine-3-carbonitrile hydrochloride, Isomer 1 | 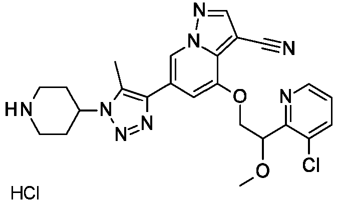 | 493 [M+H]⁺ |
| 336 | 4-[2-Methoxy-2-(3-methyl-2-pyridyl)ethoxy]-6-[5-methyl-1-(4-piperidyl)triazol-4-yl]pyrazolo[1,5-a]pyridine-3-carbonitrile hydrochloride, Isomer 1 | 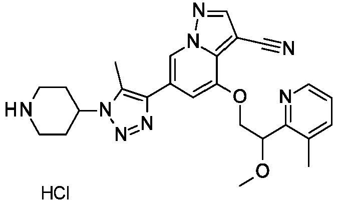 | 473 [M+H]⁺ |
| 337² | (1S)-2-[3-Bromo-6-[5-methyl-1-(4-piperidyl)triazol-4-yl]pyrazolo[1,5-a]pyridin-4-yl]oxy-1-(5-fluoro-2-pyridyl)ethanol hydrochloride, Isomer 1 | 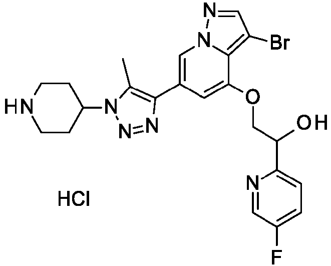 | (⁷⁹Br/⁸¹Br) 516/518 [M+H]⁺ |
| 338 | 3-Chloro-5-(2-(3,5-difluoropyridin-2-yl)-2-methoxyethoxy)-7-(5-methyl-1-((1r,3r)-3-(piperazin-1-yl)cyclobutyl)-1H-1,2,3-triazol-4-yl)imidazo[1,2-a]pyridine, hydrochloride | 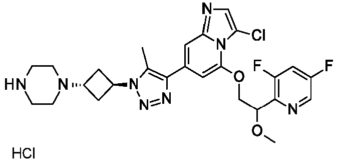 | 559 [M+H]⁺ |
| 339 | 6-[1-[(3S,4R)-3-Fluoro-4-piperidyl]-5-methyl-triazol-4-yl]-4-[2-(5-fluoro-2-pyridyl)-2-methoxy-ethoxy]pyrazolo[1,5-a]pyridine-3-carbonitrile; hydrochloride | 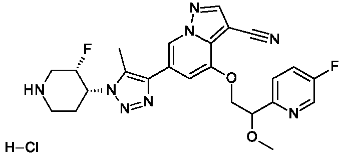 | 495 |

| | | | |
|---|---|---|---|
| ¹ Reaction was run in MeOH. Upon workup residue was triturated in EA, filtered, collected by filtration to afford title compound. ² Reaction concentrated in vacuo. The residue was diluted with EA, slurried for 1 h. The precipitate was collected by filtration. | | | |

### Preparation 340

### 2-[3-Chloro-6-[5-methyl-1-(4-piperidyl)triazol-4-yl]pyrazolo[1,5-a]pyridine-4-yl]oxy-1-(5-fluoro-2-pyridyl)ethanol, 2,2,2-trifluoroacetic acid

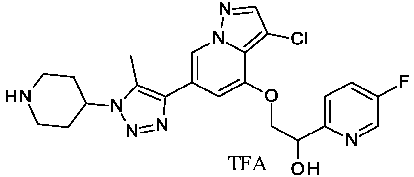

A soln of tert-butyl 4-[4-[3-chloro-4-[2-(5-fluoro-2-pyridyl)-2-hydroxy-ethoxy]pyrazolo[1,5-a]pyridine-6-yl]-5-methyl-triazol-1-yl]piperidine-1-carboxylate (1.0 g, 1.75 mmol) in DCM (10 ml) was treated with TFA (10 ml) and allowed to stir at RT for 1 h. The reaction was concentrated, and the crude product used in the next synthetic step without purification. MS ES+ m/z 472 [M+H]⁺.

The following compounds were prepared in a manner essentially analogous to the method of Preparation 340 using the appropriate reagents, adjusting reaction time to determine completion of the reaction, and adjusting the purification system as appropriate. For compounds where the amine salt was isolated, the formation of the mono-, di-, or trivalent salt is dependent on the pKa of the amine and the acid used to form the salt. The exact mono-, di-, or trivalent salt form for each example was not identified.

**Table 49**

| Prep # | Chemical Name | Structure | MS ES+ m/z |
|---|---|---|---|
| 341 | 3-Chloro-5-[2-(5-fluoro-2-pyridyl)-2-methoxy-ethoxy]-7-[5-methyl-1-(4-piperidyl)triazol-4-yl]imidazo[1,2-a]pyridine, 2,2,2-trifluoroacetic acid | 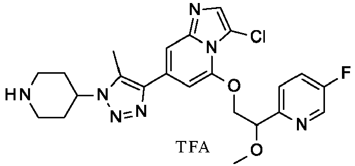 | 486 [M+H]⁺ |
| 342 | 3-Chloro-5-[2-(3,5-difluoro-2-pyridyl)-2-methoxy-ethoxy]-7-[5-methyl-1-(4-piperidyl)triazol-4-yl]imidazo[1,2-a]pyridine, 2,2,2-trifluoroacetic acid | 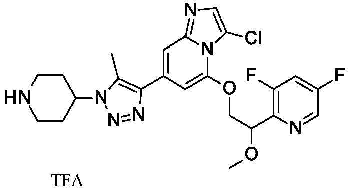 | 504 [M+H]⁺ |
| 343 | 6-[1-(2-Azaspiro[3.3]heptan-6-yl)-5-methyl-pyrazol-4-yl]-4-[(1R)-1-(2-pyridyl)ethoxy]pyrazolo[1,5-a]pyridine-3-carbonitrile, 2,2,2-trifluoroacetic acid | 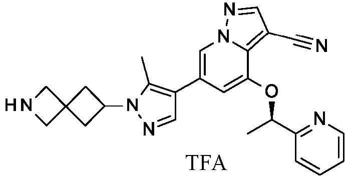 | 440 [M+H]⁺ |
| 344a | 6-[1-(Azepan-4-yl)-5-methyl-pyrazol-4-yl]-4-[(1R)-1-(2-pyridyl)ethoxy]pyrazolo[1,5-a]pyridine-3-carbonitrile, 2,2,2-trifluoroacetic acid | 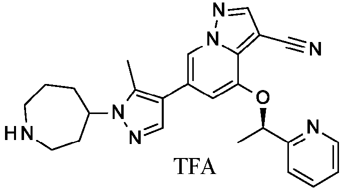 | a |
| 344b | 6-[1-(Azepan-4-yl)-3-methyl-pyrazol-4-yl]-4-[(1R)-1-(2-pyridyl)ethoxy]pyrazolo[1,5-a]pyridine-3-carbonitrile, 2,2,2-trifluoroacetic acid | 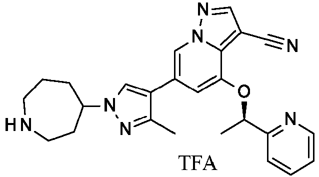 | a |
| 345 | 4-[[1-(5-Fluoro-2-pyridyl)cyclopropyl]methoxy] -6-[5-methyl-1-(4-piperidyl)triazol-4-yl]pyrazolo[1,5-a]pyridine-3-carbonitrile, 2,2,2-trifluoroacetic acid | 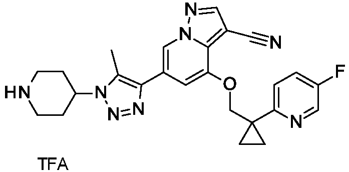 | 473 [M+H]⁺ |
| 346 | 4-[1-(2-Cyclopropylthiazol-4-yl)ethoxy]-6-[5-methyl-1-(4-piperidyl)triazol-4-yl]pyrazolo[1,5-a]pyridine-3-carbonitrile, 2,2,2-trifluoroacetic acid | 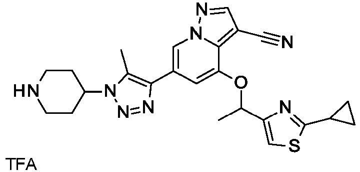 | 475 [M+H]⁺ |
| 347 | 4-[1-(2-Methoxythiazol-4-yl)ethoxy]-6-[5-methyl-1-(4-piperidyl)triazol-4-yl]pyrazolo[1,5-a]pyridine-3-carbonitrile, 2,2,2-trifluoroacetic acid | 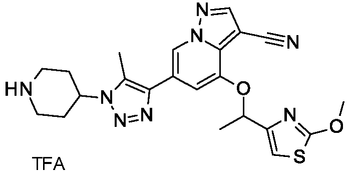 | 465 [M+H]⁺ |
| 348 | 1-[3-Chloro-6-[1-[(3S,4S)-3-fluoro-4-piperidyl]-5-methyl-triazol-4-yl]pyrazolo[1,5-a]pyridin-4-yl]oxy-2-(5-fluoro-2-pyridyl)propan-2-ol, 2,2,2-trifluoroacetic acid | 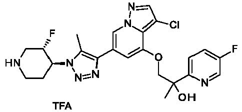 | 504 [M+H]⁺ |
| 349 | 3-Chloro-5-[2-(3,5-difluoro-2-pyridyl)-2-methoxy-ethoxy]-7-[5-methyl-1-(4-methyl-4-piperidyl)triazol-4-yl]imidazo[1,2-a]pyridine, 2,2,2-trifluoroacetic acid | 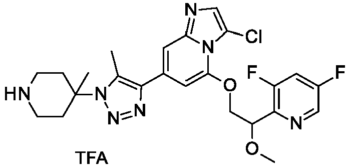 | 518 [M+H]⁺ |
| 350 | 1-[3-Chloro-6-[1-[(4R)-3,3-difluoro-4-piperidyl]-5-methyl-triazol-4-yl]pyrazolo[1,5-a]pyridin-4-yl]oxy-2-(5-fluoro-2-pyridyl)propan-2-ol, 2,2,2-trifluoroacetic acid | 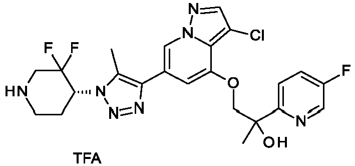 | 522 [M+H]⁺ |
| 351 | 1-[3-Chloro-6-[1-[(3R,4S)-3-fluoro-4-piperidyl]-5-methyl-triazol-4-yl]pyrazolo[1,5-a]pyridin-4-yl]oxy-2-(5-fluoro-2-pyridyl)propan-2-ol, 2,2,2-trifluoroacetic acid | 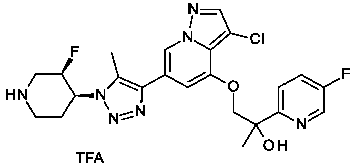 | 504 [M+H]⁺ |
| 352 | 1-[3-Chloro-6-[1-[(3R,4R)-3-fluoro-4-piperidyl]-5-methyl-triazol-4-yl]pyrazolo[1,5-a]pyridin-4-yl]oxy-2-(5-fluoro-2-pyridyl)propan-2-ol, 2,2,2-trifluoroacetic acid | 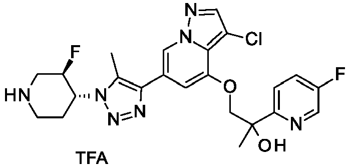 | 504 [M+H]⁺ |
| 353 | 3-Chloro-6-[1-[(3 S,4R)-3-fluoro-4-piperidyl]-5-methyl-triazol-4-yl]-4-[2-(5-fluoro-2-pyridyl)-2-methoxy-ethoxy]pyrazolo[1,5-a]pyridine, 2,2,2-trifluoroacetic acid | 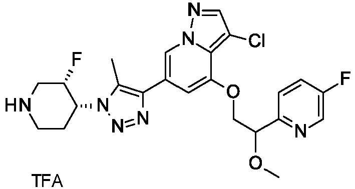 | 504 [M+H]⁺ |
| 354 | 3-Chloro-5-[2-(3,5-difluoro-2-pyridyl)-2-methoxy-ethoxy]-7-[5-methyl-1-(3-piperazin-1-ylcyclobutyl)triazol-4-yl]imidazo[1,2-a]pyridine, 2,2,2-trifluoroacetic acid | 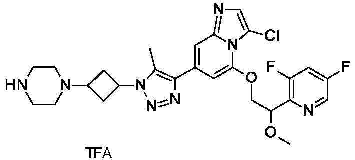 | 559 [M+H]⁺ |

| | | | |
|---|---|---|---|
| ^{a} Material used in subsequent step without further characterization. | | | |

### Preparation 355

### 4-[2-(2,4-Difluorophenyl)-2-hydroxy-ethoxy]-6-[5-methyl-1-(4-piperidyl)triazol-4-yl]pyrazolo[1,5-a]pyridine-3-carbonitrile

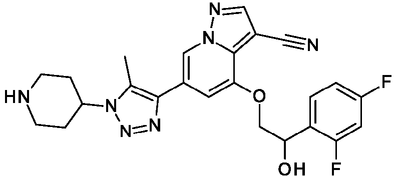

tert-Butyl 4-[4-[3-cyano-4-[2-(2,4-difluorophenyl)-2-hydroxy-ethoxy]pyrazolo[1,5-a]pyridine-6-yl]-5-methyl-triazol-1-yl]piperidine-1-carboxylate (1.0 g, 1.73 mmol) was suspended in 4M HCl in 1,4-dioxane (20 ml) and the reaction stirred at RT for 2 h. The reaction was concentrated, and the residue basified to pH 9 with saturated aq Na₂CO₃ (10 ml). The resulting solid was collected by filtration and washed with H₂O (3 x 50 ml) to obtain the title compound (0.80 g, crude) as a brown solid. MS ES+ m/z 480 [M+H]⁺.

The following compounds were prepared in a manner essentially analogous to the method of Preparation 355 using the appropriate reagents, adjusting reaction time to determine completion of the reaction, and adjusting the purification system as appropriate.

**Table 50**

| Prep # | Chemical Name | Structure | MS ES+ m/z |
|---|---|---|---|
| 356 | 4-[2-(3,5-Difluoro-2-pyridyl)-2-methoxy-ethoxy]-3-fluoro-6-[5-methyl-1-(4-piperidyl)triazol-4-yl]pyrazolo[1,5-a]pyridine | 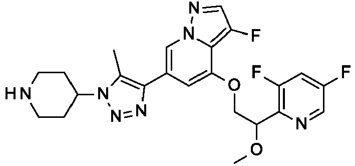 | 488 [M+H]⁺ |
| 357 | 6-[5-Methyl-1-(4-piperidyl)pyrazol-4-yl]-4-[(1R)-1-(2-pyridyl)ethoxy] pyrazolo[1,5-a]pyridine-3-carbonitrile | 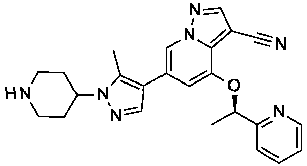 | 428 [M+H]⁺ |
| 358¹ | 6-[5-Methyl-1-(4-piperidyl)triazol-4-yl]-4-[2-methyl-2-(2-pyridyl)propoxy] pyrazolo[1,5-a]pyridine-3-carbonitrile | 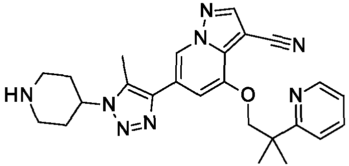 | 457 [M+H]⁺ |
| 359² | 6-[1-(Azetidin-3-yl)-5-methyl-triazol-4-yl]-4-[1-[5-(trifluoromethyl)-3-pyridyl]ethoxy]pyrazolo[1,5-a]pyridine-3-carbonitrile, Isomer 2 | 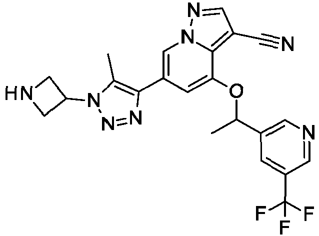 | 469 [M+H]⁺ |
| 360² | 6-[5-Methyl-1-[(3R)-3-piperidyl]pyrazol-4-yl]-4-[(1R)-1-(2-pyridyl)ethoxy] pyrazolo[1,5-a]pyridine-3-carbonitrile | 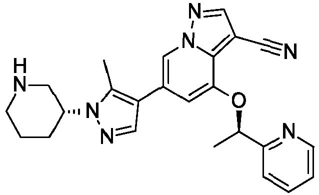 | 428 [M+H]⁺ |
| 361² | 6-[5-Methyl-1-[(3 S)-3-piperidyl]pyrazol-4-yl]-4-[(1R)-1-(2-pyridyl)ethoxy] pyrazolo[1,5-a]pyridine-3-carbonitrile | 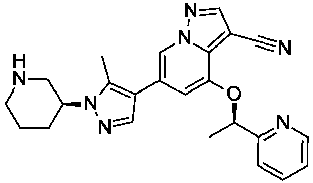 | 428 [M+H]⁺ |
| 362¹ | 4-[2-(5-Fluoro-2-pyridyl)-2-isopropoxy-ethoxy]-6-[5-methyl-1-(4-piperidyl) triazol-4-yl]pyrazolo[1,5-a]pyridine-3-carbonitrile | 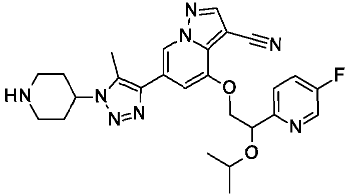 | 505 [M+H]⁺ |
| 363^{3,4} | 4-[2-(3,5-Difluoro-2-pyridyl)-2-isopropoxy-ethoxy]-6-[5-methyl-1-(4-piperidyl)triazol-4-yl] pyrazolo[1,5-a]pyridine-3-carbonitrile, Isomer 1 | 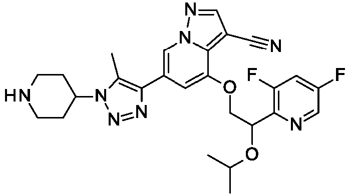 | 523 [M+H]+ |
| 364¹ | 2-(3,5-Difluoro-2-pyridyl)-1-[3-fluoro-6-[5-methyl-1-(4-piperidyl)triazol-4-yl] pyrazolo[1,5-a]pyridin-4-yl]oxy-propan-2-ol | 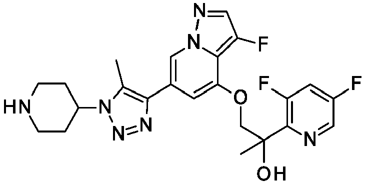 | 488 [M+H]⁺ |
| 365⁵ | 3-Chloro-4-[2-(5-fluoro-2-pyridyl)-2-methoxy-ethoxy]-6-[5-methyl-1-(4-piperidyl) triazol-4-yl]pyrazolo[1,5-a]pyridine | 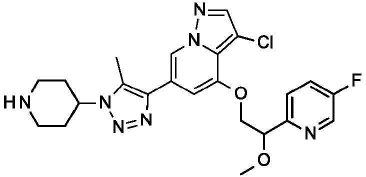 | 486 [M+H]⁺ |
| 366⁵ | 3-Chloro-6-[1-[(3 S,4R)-3-fluoro-4-piperidyl]-5-methyl-triazol-4-yl]-4-[2-(5-fluoropyrimidin-2-yl)-2-methoxy-ethoxy]pyrazolo [1,5-a]pyridine | 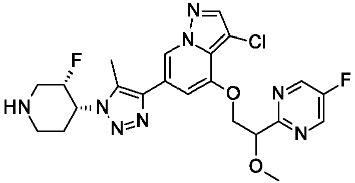 | 505 [M+H]⁺ |
| 367⁶ | 1-[3-Chloro-6-[1-[(3S,4R)-3-fluoro-4-piperidyl]-5-methyl-triazol-4-yl]pyrazolo[1,5-a]pyridin-4-yl]oxy-2-(5-fluoropyrimidin-2-yl)propan-2-ol | 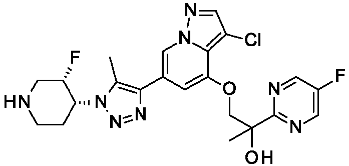 | 505 [M+H]⁺ |
| 368 | 2-[3-Chloro-6-[1-[(3 S,4R)-3-fluoro-4-piperidyl]-5-methyl-triazol-4-yl]pyrazolo[1,5-a]pyridin-4-yl]oxy-1-(5-fluoropyrimidin-2-yl)ethanol | 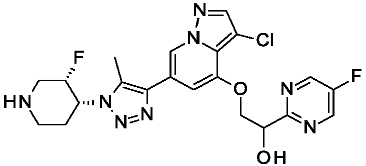 | 491 [M+H]⁺ |

| | | | |
|---|---|---|---|
| ¹ Workup: Reaction concentrated. Residue was dissolved in MeOH and applied directly onto a SCX cartridge, previously conditioned with MeOH. Non-basic impurities were washed off with MeOH then the title compound was eluted with methanolic ammonia (2M). ² Workup: Reaction concentrated. Residue was suspended in DCM and washed with aq 2M NaOH then brine. Organic layer dried over MgSO₄, filtered and concentrated. ³ Workup: Reaction concentrated. Residue was suspended in DCM and washed with aq NaOH (1M). Phases were spearated and the organic layer dried over MgSO₄, filtered and concentrated. ⁴ Product contained impurty that was carried on from previous step. ⁵ Workup: Reaction concentrated. Residue dissolved into DCM and washed with sat. aq NaHCO₃ soln, dried over MgSO₄, filtered and concentrated. ⁶ Workup: Reaction concentrated. Reside was dissolved into MeOH then treated with NaHCO₃ to pH 8. Mixture was concentrated in vacuo. | | | |

### Preparation 369a

### 6-[1-(7-Azaspiro[3.5]nonan-2-yl)-5-methyl-pyrazol-4-yl]-4-[(1R)-1-(2-pyridyl)ethoxy]pyrazolo[1,5-a]pyridine-3-carbonitrile

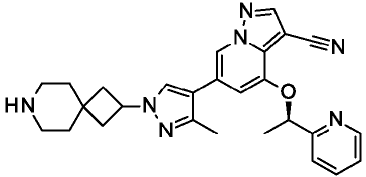

and

### Preparation 369b

### 6-[1-(7-Azaspiro[3.5]nonan-2-yl)-3-methyl-pyrazol-4-yl]-4-[(1R)-1-(2-pyridyl)ethoxy]pyrazolo[1,5-a]pyridine-3-carbonitrile

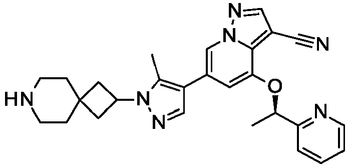

A soln of tert-butyl 2-[4-[3-cyano-4-[(1R)-1-(2-pyridyl)ethoxy]pyrazolo[1,5-a]pyridin-6-yl]-5-methyl-pyrazol-1-yl]-7-azaspiro[3.5]nonane-7-carboxylate and tert-butyl 2-[4-[3-cyano-4-[(1R)-1-(2-pyridyl)ethoxy]pyrazolo[1,5-a]pyridin-6-yl]-3-methyl-pyrazol-1-yl]-7-azaspiro[3.5]nonane-7-carboxylate (621 mg, 0.77 mmol, 70%) in DCM (6 ml) was treated with TFA (1.2 ml). After stirring at RT for 3 days, the reaction was concentrated and loaded onto a SCX column pretreated with MeOH. After washing with MeOH, the title compounds were eluted with 2 M NH₃ in MeOH to obtain a mixture of the title compounds (398 mg, 96%) as a pale-yellow solid. MS ES+ m/z 468 [M+H]⁺.

### Preparation 370

### [2-[3-Cyano-6-[5-methyl-1-[1-(oxetan-3-yl)-4-piperidyl]triazol-4-yl]pyrazolo[1,5-a]pyridin-4-yl]oxy-1-(5-fluoro-2-pyridyl)ethyl] methanesulfonate

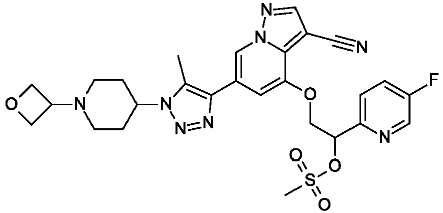

A soln of 4-[2-(5-fluoro-2-pyridyl)-2-hydroxy-ethoxy]-6-[5-methyl-1-[1-(oxetan-3-yl)-4-piperidyl]triazol-4-yl]pyrazolo[1,5-a]pyridine-3-carbonitrile (1.39 g, 2.69 mmol) and NEt₃ (0.80 ml, 5.7 mmol) in DCM (14 ml) was cooled to 0 °C and purged with N₂. The reaction was treated dropwise with MsCl (0.26 mL, 3.4 mmol). The reaction was allowed to slowly warm to RT. After 75 min, the reaction was re-cooled to 0 °C, quenched with H₂O (15 ml) and the organic layer was removed. The aq layer was extracted with DCM (2 x 5mL). The combined organic layers were dried over Na₂SO₄, filtered, and concentrated to afford the title compound as a light brown foamy solid (1.80 g, 100% yield, 90% purity) which was used without purification. MS ES+ m/z 597 [M+H]⁺.

The following compounds were prepared in a manner essentially analogous to the method of Preparation 370 using the appropriate reagents, adjusting reaction time to determine completion of the reaction, and adjusting the purification system as appropriate.

**Table 51**

| Prep # | Chemical Name | Structure | MS ES+ m/z |
|---|---|---|---|
| 371 | [2-[3-Cyano-6-[5-methyl-1-[1-(oxetan-3-yl)-4-piperidyl]triazol-4-yl]pyrazolo[1,5-a]pyridin-4-yl]oxy-1-(3,5-difluoro-2-pyridyl)ethyl] methanesulfonate | 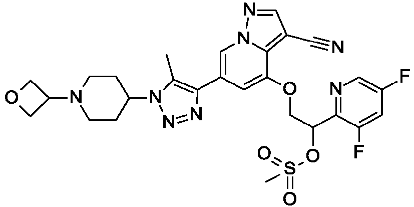 | 615 [M+H]⁺ |
| 372 | [2-[3-Fluoro-6-[5-methyl-1-[1-(oxetan-3-yl)-4-piperidyl]triazol-4-yl]pyrazolo[1,5-a]pyridin-4-yl]oxy-1-(5-fluoro-2-pyridyl)ethyl] methanesulfonate | 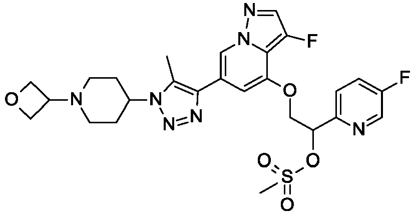 | 590 [M+H]⁺ |

### Preparation 373

### 7-[5-Methyl-1-[1-(oxetan-3-yl)-4-piperidyl]triazol-4-yl]-5-[[3,3,3-trifluoro-2-(5-fluoro-2-pyridyl)propyl]amino]imidazo[1,2-a]pyridine-3-carbonitrile

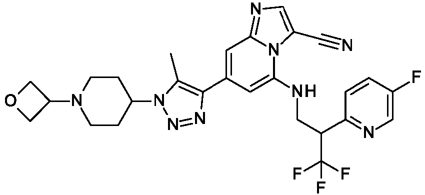

A mixture of 7-chloro-5-[[3,3,3-trifluoro-2-(5-fluoro-2-pyridyl)propyl]amino] imidazo[1,2-a]pyridine-3-carbonitrile (1.2 g, 3.28 mmol), bis(pinacolato)diboron (1.19 g, 4.69 mmol), and KOAc (0.92 g, 9.38 mmol) in dioxane (20 ml) was treated with Xphos Palladacycle Gen 4 (56.06 mg, 0.07 mmol) at 80 °C under N₂. The resulting mixture was stirred for 2 h at 80 °C. The reaction was taken on to the next step without workup or purification.

The above reaction was allowed to cool and treated with KF (0.53 g, 9.38 mmol), 4-(4-bromo-5-methyl-1,2,3-triazol-1-yl)-1-(oxetan-3-yl)piperidine (1.20 g, 3.97 mmol), H₂O (4 ml) and PdCl₂(DtBPF) (0.10 g, 0.15 mmol) under N₂. After stirring at 100 °C for 2 h, the reaction was cooled to RT, quenched with H₂O (100 ml), and extracted with EA (2 x 100 ml). The combined organic layers were washed with brine (150 ml), dried over anhydrous Na₂SO₄, filtered, and concentrated. The residue was purified by silica gel chromatography eluting with DCM / MeOH (100 to 20:1) to afford the title compound (315 mg, 18%) as a white solid. MS ES+ m/z 570 [M+H]⁺.

### Preparation 374

### (1S)-2-[3-Bromo-6-[5-methyl-1-[1-(oxetan-3-yl)-4-piperidyl]triazol-4-yl]pyrazolo[1,5-a]pyridin-4-yl]oxy-1-(5-fluoro-2-pyridyl)ethanol, Isomer 1

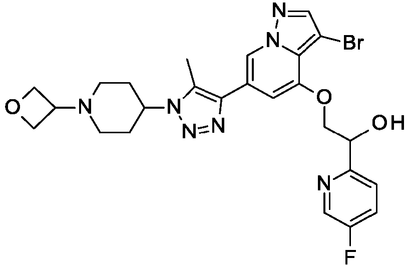

A solution of (1S)-2-[3-bromo-6-[5-methyl-1-(4-piperidyl)triazol-4-yl]pyrazolo[1,5-a]pyridin-4-yl]oxy-1-(5-fluoro-2-pyridyl)ethanol hydrochloride (7.57 g, 11.0 mmol) in MeOH (76 ml) at RT was treated with oxetan-3-one (1.97 g, 27.3 mmol), AcOH (1.60 mL, 27.9 mmol), and NaCNBH₃ (2.80 g, 44.6 mmol). After 48 h, the reaction was quenched with aq. 1 N K₂CO₃ (150 ml), and extracted with DCM (300 ml). The organic phase was dried MgSO₄, filtered and concentrated in vacuo. The residue was purified by silica gel chromatography eluting with 0% to 100% acetone in DCM to afford the title compound as a white solid (0.352 g, 6 %).

### Example 1

### 4-[[2-(5-Fluoro-2-pyridyl)-2-methoxy-ethyl]amino]-6-[5-methyl-1-[1-(oxetan-3-yl)-4-piperidyl]triazol-4-yl]pyrazolo[1,5-a]pyridine-3-carbonitrile

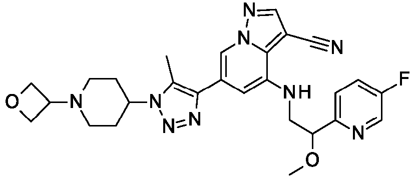

To a vial was added 4-[[2-(5-fluoro-2-pyridyl)-2-methoxy-ethyl]amino]-6-(4,4,5,5-tetramethyl-1,3,2-dioxaborolan-2-yl)pyrazolo[1,5-a]pyridine-3-carbonitrile (0.51 mmol, 224 mg), 4-(4-bromo-5-methyl-triazol-1-yl)-1-(oxetan-3-yl)piperidine (278 mg, 0.92 mmol ), K₂CO₃ (142 mg, 1.03 mmol), toluene (2 mL), and H₂O (200 ul). The vial was flushed with N₂ then PdCl₂(DtBPF) (51 mg, 0.08 mmol) was added. The reaction was heated at 100 °C for 3 h. The mixture was filtered through DE, the filtrate was loaded onto an SCX column pretreated with MeOH. The column was washed with MeOH. The product was eluted with 2 M NH₃ in MeOH and concentrated. The residue was purified by silica gel chromatography eluting with 0% to 100% (25% EtOH in EA) in cHex to afford the title compound (0.40 g, 16%). MS ES+ m/z 532 [M+H]⁺.

### Example 2

### 4-[1-(7-Fluoro-4-isoquinolyl)ethoxy]-6-[5-methyl-1-[1-(oxetan-3-yl)-4-piperidyl]triazol-4-yl]pyrazolo[1,5-a]pyridine-3-carbonitrile

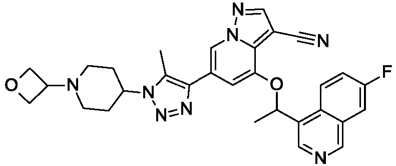

4-[1-(7-fluoro-4-isoquinolyl)ethoxy]-6-[5-methyl-1-(4-piperidyl)triazol-4-yl]pyrazolo[1,5-a]pyridine-3-carbonitrile hydrochloride (305 mg, 0.54 mmol) and DIPEA (0.28 ml, 1.61 mmol) were dissolved in MeOH (5 ml) and activated 4A molecular sieves was added. To that soln, 3-oxetanone (0.17 ml, 2.70 mmol), NaBH₃CN (172 mg, 2.74 mmol) and AcOH (0.37 ml, 6.43 mmol) were sequentially added, and the reaction was stirred at 70 °C for 2 h. Upon completion, the reaction was cooled to RT and diluted with EA. The mixture was washed with H₂O (25 ml) and brine (25 ml), dried over MgSO₄, filtered, concentrated in vacuo. The residue was purified by silica gel chromatography eluting with MeOH in DCM to afford the title compound as an off-white solid (35 mg, 15%). MS ES+ m/z 553.3 [M+H]⁺.

The following compounds were prepared in a manner essentially analogous to the method of Example 2 using the appropriate reagents, adjusting reaction time to determine completion of the reaction, and adjusting the purification system as appropriate.

**Table 52**

| Ex # | Chemical Name | Structure | MS ES+ m/z |
|---|---|---|---|
| 3^{1,2} | 6-[5-Methyl-1-(1-tetrahydropyran-4-ylazetidin-3-yl)triazol-4-yl]-4-[1-[5-(trifluoromethyl)-3-pyridyl]ethoxy]pyrazo lo[1,5-a]pyridine-3-carbonitrile, Isomer 2 | 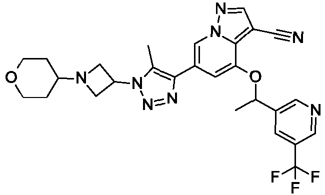 | 553 [M+H]⁺ |
| 4^{1,4} | 4-[[1-(5-Fluoro-2-pyridyl)cyclopropyl]m ethoxy]-6-[5-methyl-1-[1-(oxetan-3-yl)-4-piperidyl]triazol-4-yl]pyrazolo[1,5-a]pyridine-3-carbonitrile | 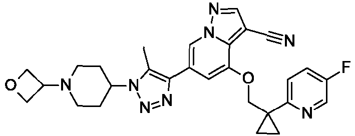 | 529 [M+H]⁺ |
| 5^{1,3} | 6-[5-Methyl-1-[1-(oxetan-3-yl)-4-piperidyl]triazol-4-yl]-4-[2-methyl-2-(2-pyridyl)propoxy]pyraz olo[1,5-a]pyridine-3-carbonitrile | 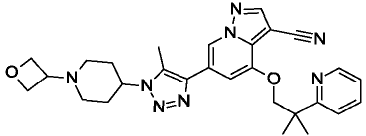 | 513 [M+H]⁺ |
| 6^{1,2} | 6-[5-Methyl-1-[1-(oxetan-3-yl)azetidin-3-yl]triazol-4-yl]-4-[1-[5-(trifluoromethyl)-3-pyridyl]ethoxy]pyrazo lo[1,5-a]pyridine-3-carbonitrile, Isomer 2 | 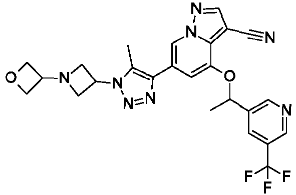 | 525 [M+H]⁺ |
| 7⁵ | 6-[5-Methyl-1-[(3R)-1-(oxetan-3-yl)-3-piperidyl]pyrazol-4-yl]-4-[(1R)-1-(2-pyridyl)ethoxy]pyrazo lo[1,5-a]pyridine-3-carbonitrile | 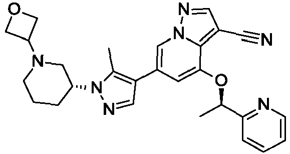 | 484 [M+H]⁺ |
| 8⁵ | 6-[5-Methyl-1-[(3S)-1-(oxetan-3-yl)-3-piperidyl]pyrazol-4-yl]-4-[(1R)-1-(2-pyridyl)ethoxy]pyrazo lo[1,5-a]pyridine-3-carbonitrile | 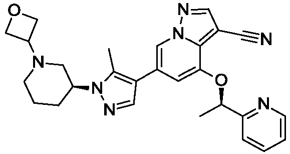 | 484 [M+H]⁺ |
| 9^{6,7} | 4-[2-(3,5-Difluoro-2-pyridyl)-2-isopropoxy-ethoxy]-6-[5-methyl-1-[1-(oxetan-3-yl)-4-piperidyl]triazol-4-yl]pyrazolo[1,5-a]pyridine-3-carbonitrile, Isomer 2 | 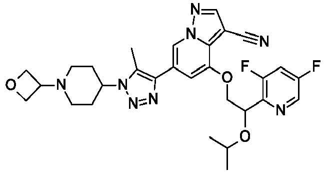 | 579 [M+H]⁺ |
| 10⁸ | 4-[2-Methoxy-2-(3-methyl-2-pyridyl)ethoxy]-6-[5-methyl-1-[1-(oxetan-3-yl)-4-piperidyl]triazol-4-yl]pyrazolo[1,5-a]pyridine-3-carbonitrile, Isomer 1 | 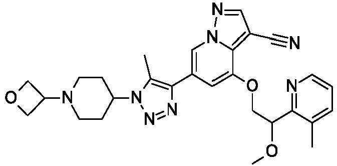 | 529 [M+H]⁺ |
| 11^{9, 10} | 4-[2-(3-Chloro-2-pyridyl)-2-methoxy-ethoxy]-6-[5-methyl-1-[1-(oxetan-3-yl)-4-piperidyl]triazol-4-yl]pyrazolo[1,5-a]pyridine-3-carbonitrile, Isomer 1 | 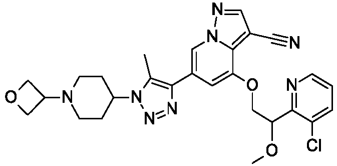 | 550 [M+H]⁺ |

| | | | |
|---|---|---|---|
| ¹ 4A molecular sieves and DIPEA were excluded in the reaction. ² Purified by silica gel chromatography eluting with 0% to 10% MeOH in DCM. ³ Purified by silica gel chromatography eluting with 5% EA in cHex (2 CV), 5% to 50% EA in cHex (20 CV), 50% to 100% EA in cHex (5 CV), 100% EA (10 CV). ⁴ Purified by C18 reversed phase C18 chromatography: Column: XBridge C18, 19 x 150mm, 5 µm, eluting with 45% to 65% ACN in H₂O (10mM NH₄HCO₃ buffer, pH 9). ⁵ Column: Luna Hilic, 30 x 150mm, 5 µm, eluting with 10% to 20%: MeOH (10mM NH₄HCO₃ pH 8). ⁶ Purified by silica gel chromatography eluting with 10% to 50% acetone in DCM. ⁷ Column: Luna Omega Polar, 21 x 150mm, 5µm; eluting with 25% to 55% ACN (0.1% FA) in H₂O (0.1% FA). ⁸ Purified by silica gel chromatography eluting with 0% to 10% MeOH in DCM. ⁹ Purified by silica gel chromatography eluting with 50% (10% MeOH in DCM) in DCM. ¹⁰ Chiral method: LUX-2PROP-AMY-CELL-1Amy2-iAmy1. | | | |

### Example 12

### 4-[2-(5-Fluoro-2-pyridyl)-2-oxo-ethoxy]-6-[5-methyl-1-[1-(oxetan-3-yl)-4-piperidyl]triazol-4-yl]pyrazolo[1,5-a]pyridine-3-carbonitrile

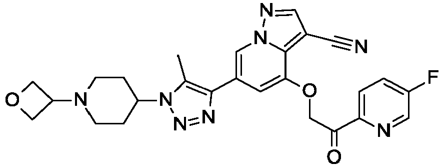

A suspension of 4-hydroxy-6-[5-methyl-1-[1-(oxetan-3-yl)-4-piperidyl]triazol-4-yl]pyrazolo[1,5-a]pyridine-3-carbonitrile (0.162 g , 0.43 mmol) in ACN (1.8 ml) was treated with K₂CO₃ (53 mg, 0.53 mmol) and 2-bromo-1-(5-fluoropyridin-2-yl)ethanone (100 mg, 0.44 mmol). The suspension was stirred at 80 °C during 16 h. Upon cooling to RT, EA and H₂O were added and the mixture was filtered. The layers from the filtrate were separated and washed with 2N NaOH, H₂O and brine, dried MgSO₄, filtered and concentrated. The residue was purified by silica gel chromatography eluting with 0% to 10% EtOH in EA to afford the title compound (91 mg, 47%). MS ES+ m/z 517 [M+H]⁺.

### Example 13

### 4-[2-(5-Fluoro-2-pyridyl)-2-hydroxy-ethoxy]-6-[5-methyl-1-[1-(oxetan-3-yl)-4-piperidyl]triazol-4-yl]pyrazolo[1,5-a]pyridine-3-carbonitrile, Isomer 1

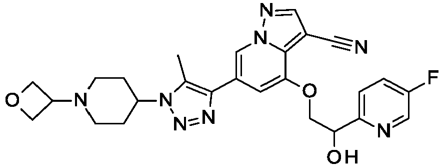

and

### Example 14

### 4-[2-(5-Fluoro-2-pyridyl)-2-hydroxy-ethoxy]-6-[5-methyl-1-[1-(oxetan-3-yl)-4-piperidyl]triazol-4-yl]pyrazolo[1,5-a]pyridine-3-carbonitrile, Isomer 2

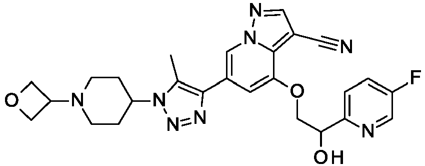

NaBH₄ (3.0 mg, 0.08 mmol) was added in one portion to a soln of 4-[2-(5-fluoro-2-pyridyl)-2-oxo-ethoxy]-6-[5-methyl-1-[1-(oxetan-3-yl)-4-piperidyl]triazol-4-yl]pyrazolo[1,5-a]pyridine-3-carbonitrile (28.3 mg, 0.05 mmol) in EtOH (0.78 mL) and DCM (0.55 ml) at RT for 3 h. Reaction was quenched with saturated NH₄Cl. The layers were separated, organic layer was washed with brine, dried over MgSO₄, filtered through DE. The filtrate was concentrated to afford 16 mg. Combined aq layers were extracted with DCM and the organic layer was concentrated to afford 5mg. Both lots were combined to afford 21 mg. This material was subjected to the following chiral chromatography conditions: Column: Chiralpak AD, 30 x 250 mm, 5 µm; eluting with 35% IPA (0.5% DMEA) in CO₂ to afford the title compound, Isomer 1 (9.0 mg, 32%), t_{R} is 1.28 min with 98% ee. MS ES+ m/z 519 [M+H]⁺ and title compound, Isomer 2, (9.2 mg, 32%), t_{R} is 1.60 min with 85% ee. MS ES+ m/z 519 [M+H]⁺. The retention times were obtained using analytical method A. (Refer to Table A for specific analytical conditions).

### Example 15

### 4-[[2-(5-Fluoro-2-pyridyl)oxetan-2-yl]methoxy]-6-[5-methyl-1-[1-(oxetan-3-yl)-4-piperidyl]triazol-4-yl]pyrazolo[1,5-a]pyridine-3-carbonitrile, Isomer 2

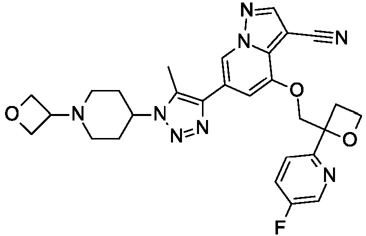

t-BuOK (260 mg, 2.29 mmol) was added to a suspension of trimethylsulfoxonium iodide (545 mg, 2.40 mmol) in 2-methyl-2-butanol (0.1 M) at RT. The sealed vial was stirred at 50 °C for 90 min under N₂. The suspension was cooled to RT and 4-[2-(5-fluoro-2-pyridyl)-2-oxo-ethoxy]-6-[5-methyl-1-[1-(oxetan-3-yl)-4-piperidyl]triazol-4-yl]pyrazolo[1,5-a]pyridine-3-carbonitrile (400 mg, 0.77 mmol) was added in one portion. The resulting mixture was vigorously stirred at RT overnight in a sealed tube under N₂. Next, the suspension was heated at 50 °C for 3 days then stirred at RT for 3 days.

In a separate tube, t-BuOK (260 mg, 2.29 mmol) was added to a suspension of trimethylsulfoxonium iodide (545 mg, 2.40 mmol) in 2-methyl-2-butanol (0.1 M) at RT. The sealed vial was stirred at 50 °C for 90 min under N₂. The above reaction was added in one portion to this suspension and the resulting mixture was stirred at 50 °C in a sealed tube under N₂ overnight. The reaction was poured into sat. aq NH₄Cl, extracted with DCM and the aq layer extracted with DCM. The combined organic layers were washed with 1N NaOH (2x), then with H₂O, followed by brine and dried over MgSO₄. The resultant residue was purified by reversed phase chromatography: Column; Claricep C-series eluted with 30% ACN in H₂O (NH₄HCO₃ pH 9)(2 CV); then a linear gradient from 30% to 60% ACN in H₂O (NH₄HCO₃ pH 9)(8 CV) and 60% ACN in H₂O (NH₄HCO₃ pH 9). Fractions containing the title compound were partially evaporated, DCM was added, and the two-layer mixture was passed through a hydrophobic filter. Filtrate was dried over MgSO₄, filtered, and evaporated.

The isolated material after reversed phase chromatography was subjected to the following chiral chromatography conditions: Column: Chiralpak IA, 20 x 250 mm, 5 µm; eluting with 40% IPA (0.5% DMEA) in CO₂ to afford the title compound (29 mg, 7%), t_{R} is 2.82 (Method P), ee > 98% ee; MS ES+ m/z 545 [M+H]⁺. The retention time was obtained using analytical method P.

### Example 16

### 6-[5-Methyl-1-[1-(oxetan-3-yl)-4-piperidyl]triazol-4-yl]-4-[1-[5-(trifluoromethyl)pyridazin-3-yl]ethoxy]pyrazolo[1,5-a]pyridine-3-carbonitrile, Isomer 2

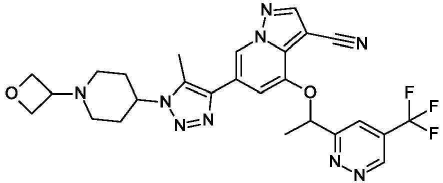

DIAD (384 mg, 1.90 mmol) was added dropwise to a soln of PPh₃ (539 mg, 2.06 mmol) in THF (10 ml) at 0°C under N₂. The resulting mixture was stirred for 0.5 h at 0 °C and then added to 4-hydroxy-6-[5-methyl-1-[1-(oxetan-3-yl)-4-piperidyl]triazol-4-yl]pyrazolo[1,5-a]pyridine-3-carbonitrile (600 mg, 1.58 mmol) and 1-[5-(trifluoromethyl)pyridazin-3-yl]ethanol (456 mg, 2.37 mmol) in THF (10 ml) and allowed to stir overnight. The reaction was acidified to pH 5 with conc HCl, diluted with H₂O (80 ml), and extracted with EA (3 x 50 ml). The aq phase was basified to pH 8 with saturated aq NaHCO₃, extracted with CHCl₃: IPA (3:1) (3 x 100 mL), dried over anhydrous Na₂SO₄, filtered, and concentrated. The residue was purified by reversed phase chromatography using the following conditions: Column: XB-C18, 50 x 250 mm, 10 µm; eluting with a gradient of 35% to 55% ACN in H₂O (10mM FA) to afford the racemate of the title compound (345 mg, 39%) as a green solid. MS ES+ *m*/*z* 554 [M+H]⁺.

The racemate (345 mg) was subjected to chiral chromatography using the following conditions: Column: CHIRALPAK IC, 2 x 25 cm, 5 µm; eluting with 50% MeOH in MTBE (10mM NH₃-MeOH), 248/208 nm; to afford the title compound (117mg, 34%), t_{R} is 9.82 min with 100% ee. MS ES+ m/z 554 [M+H]⁺.

The following compound was prepared in a manner essentially analogous to the method of Example 16 using the appropriate reagents, adjusting reaction time to determine completion of the reaction, and adjusting the purification system as appropriate and separation of enantiomers where applicable.

If the retention time was obtained from an analytical column the method will be listed in the final column. Refer to Table A for specific analytical conditions for each method.

**Table 53**

| Ex # | Chemical Name | Structure | MS ES+ m/z | t_{R} (min) |
|---|---|---|---|---|
| 17^{1,2} | 4-[[3,3-Difluoro-1-(5-fluoro-2-pyridyl) cyclobutyl]methoxy ]-6-[5-methyl-1-[1-(oxetan-3-yl)-4-piperidyl]triazol-4-yl]pyrazolo[1,5-a]pyridine-3-carbonitrile | 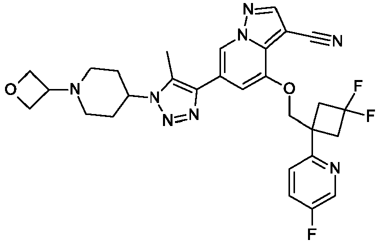 | 579 [M+H]⁺ | - |
| 18^{3,4} | 6-[5-Methyl-1-[1-(oxetan-3-yl)-4-piperidyl]triazol-4-yl]-4-[1-methyl-2-(2-pyridyl) pyrrolidin-3-yl]oxy-pyrazolo [1,5-a]pyridine-3-carbonitrile, Isomer 1 | 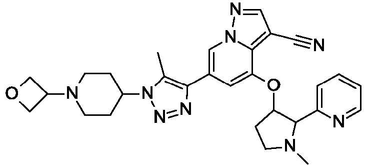 | [M+H]⁺ | 2.0 B |

| | | | | |
|---|---|---|---|---|
| ¹ Purified by silica gel chromatography eluting with DCM : MeOH (20 to 1) ² Purified by reversed chromatography: Column: welch-XB C18, 50 X 250mm, 10 µm; eluting with 28% to 35% ACN in H₂O (0.1% NH₄HCO₃). ³ Residue was dissolved in MeOH and treated with TFA then applied directly onto a SCX cartridge, previously conditioned with MeOH. Non-basic impurities were washed off with MeOH then the title compound was eluted with methanolic ammonia (2M). ⁴ Column: Chiralpak IA, 20 x 250 mm, 5 µm; eluting with 35% EtOH (0.5% DMEA) in CO₂. | | | | |

### Example 19

### 4-[1-(3,6-Dimethylpyrazin-2-yl)ethoxy]-6-[5-methyl-1-[1-(oxetan-3-yl)-4-piperidyl]triazol-4-yl]pyrazolo[1,5-a]pyridine-3-carbonitrile, Isomer 1

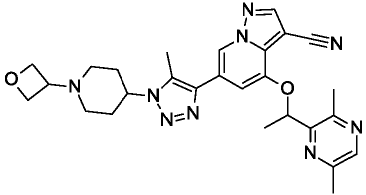

A mixture of 1-(3,6-dimethylpyrazin-2-yl)ethyl methanesulfonate (395 mg, 1.71 mmol), K₂CO₃ (546 mg, 3.95 mmol) and 4-hydroxy-6-{5-methyl-1-[1-(oxetan-3-yl)piperidin-4-yl]-1,2,3-triazol-4-yl}pyrazolo[1,5-a]pyridine-3-carbonitrile (500 mg, 1.32 mmol) in ACN (10 ml) was stirred for 2 h at 80 °C under N₂. The resultant mixture was filtered, and the filter cake was washed with DCM (3 x 10 mL). The filtrate was concentrated in vacuo. The residue was purified by reversed flash C18 chromatography eluting with 20% to 40% ACN in H₂O to afford the title compound (450 mg, 66%) as a brown solid.

The brown solid was subjected to chiral chromatography using the following conditions: Column: CHIRALPAK AD-H, 3 x 25 cm, 5 µm; eluting with 40% MeOH in CO₂; to afford the title compound (163 mg, 36%), t_{R} is 5.77 min with 100% ee.

The following compounds were prepared in a manner essentially analogous to the method of Example 19 using the appropriate reagents, adjusting reaction time to determine completion of the reaction, and adjusting the purification system as appropriate. Cs₂CO₃ and DMF can be used.

If the retention time was obtained from an analytical column the method will be listed in the final column. Refer to Table A for specific analytical conditions for each method.

**Table 54**

| Ex # | Chemical Name | Structure | MS ES+ m/z | t_{R} (min) and method |
|---|---|---|---|---|
| 20^{1,2} | 3-Chloro-4-[1-[5-(difluoromethyl)-3-pyridyl]ethoxy]-6-[5-methyl-1-[1-(oxetan-3-yl)-4-piperidyl]triazol-4-yl]pyrazolo[1,5-a]pyridine, Isomer 2 | 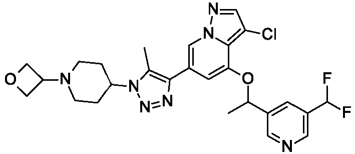 | 544 [M+H]⁺ | 2.11 B |
| 21^{3,4} | 4-[Cyclopropyl-(5-fluoro-2-pyridyl)methoxy]-6-[5-methyl-1-[1-(oxetan-3-yl)-4-piperidyl]triazol-4-yl]pyrazolo[1,5-a]pyridine-3-carbonitrile, Isomer 1 | 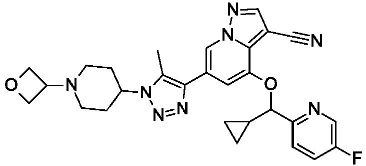 | 527 [M-H]⁺ | 2.61 C |
| 22^{3,5} | 4-[Cyclobutyl-(5-fluoro-2-pyridyl)methoxy]-6-[5-methyl-1-[1-(oxetan-3-yl)-4-piperidyl]triazol-4-yl]pyrazolo[1,5-a]pyridine-3-carbonitrile, Isomer 1 | 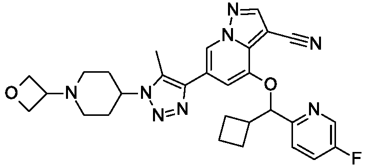 | 541 [M-H]⁺ | 1.57 D |
| 23^{6,7} | 4-[1-(2,5-Dimethylthiazol-4-yl)ethoxy]-6-[5-methyl-1-[1-(oxetan-3-yl)-4-piperidyl]triazol-4-yl]pyrazolo[1,5-a]pyridine-3-carbonitrile, Isomer 1 | 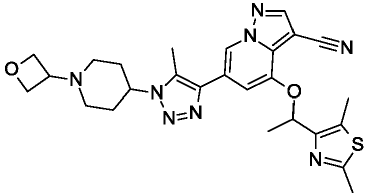 | 519.2 [M+H]⁺ | 2.70 E |
| 24^{8,9} | 6-[5-Methyl-1-[1-(oxetan-3-yl)-4-piperidyl]triazol-4-yl]-4-[1-[6-(trifluoromethyl) pyrazin-2-yl]ethoxy] pyrazolo[1,5-a]pyridine-3-carbonitrile, Isomer 1 | 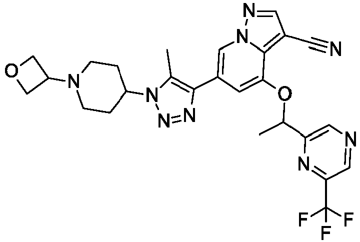 | 554 [M+H]⁺ | 1.45 F |
| 25^{10,11} | 6-[5-Methyl-1-[1-(oxetan-3-yl)-4-piperidyl]triazol-4-yl]-4-[1-(2-methylthiazol-4-yl)propoxy]pyrazo lo[1,5-a]pyridine-3-carbonitrile, Isomer 1 | 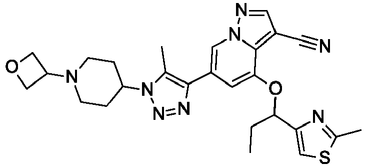 | 519 [M+H]⁺ | 2.47 C |

| | | | | |
|---|---|---|---|---|
| ¹ Purified by silica gel chromatography eluting with 0% to 50% EA (25% EtOH) in cHex. ² Column: Chiralpak IA, 20 x 250 mm, 5 µm; eluting with 35% IPA (0.5% DMEA) in CO2. ³ Purified by flash reversed phase C18 chromatography eluting with 40% to 70% ACN in H₂O (NH₅CO₃ pH 9 buffer). ⁴ Chiralpak AD, 20 x 250 mm, 5 µm; eluting with 35% IPA (0.5% DMEA) in CO₂. ⁵ Chiralcel OJ, 20 x 250 mm, 5 µm; eluting with 20% MeOH (0.5% DMEA) in CO₂. ⁶ Purified by flash reversed phase C18 chromatography eluting with 30% to 60% ACN in H₂O (NH₅CO₃ pH 9 buffer). ⁷ Column: Chiralcel OJ, 20 x 150 mm, 5 µm; eluting with MeOH (0.5% DMEA) in CO₂. ⁸ Purified by silica gel chromatography eluting with 20% to 60% EA in cHex. ⁹ Column: Chiralcel OJ, 20 x 250 mm, 5 µm; eluting with 30% MeOH (0.5% DMEA) in CO₂, flow rate: 80 (ml/min). ¹⁰ Purified by silica gel chromatography eluting with 20% 3:1 EA / EtOH in cHex (2 CV), 20% to 65% gradient 3:1 EA / EtOH in cHex (25 CV), 65% 3:1 EA / EtOH in cHex (10 CV), 65% to 100% gradient 3:1 EA / EtOH in cHex (15 CV), 100% 3:1 EA / EtOH in cHex (10 CV). ¹¹ Chiralpak AD, 20 x 250 mm, 5 µm; eluting with 35% EtOH (0.5% DMEA) in CO₂. | | | | |

### Example 26

### 4-[2-(5-Fluoro-2-pyridyl)-2-pyrrolidin-1-yl-ethoxy]-6-[5-methyl-1-[1-(oxetan-3-yl)-4-piperidyl]triazol-4-yl]pyrazolo[1,5-a]pyridine-3-carbonitrile, Isomer 1

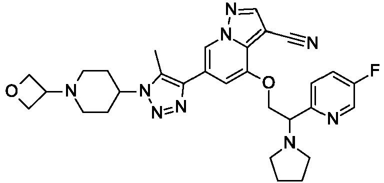

and

### Example 27

### 4-[2-(5-Fluoro-2-pyridyl)-2-pyrrolidin-1-yl-ethoxy]-6-[5-methyl-1-[1-(oxetan-3-yl)-4-piperidyl]triazol-4-yl]pyrazolo[1,5-a]pyridine-3-carbonitrile, Isomer 2

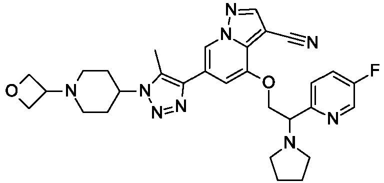

[2-[3-Cyano-6-[5-methyl-1-[1-(oxetan-3-yl)-4-piperidyl]triazol-4-yl]pyrazolo[1,5-a]pyridin-4-yl]oxy-1-(5-fluoro-2-pyridyl)ethyl] methanesulfonate (155 mg, 0.26 mmol) was placed in a screw-cap vial and dissolved in ACN (0.65 ml) under N₂. Pyrrolidine (183 mg, 0.002 mmol) was added. The reaction mixture was heated at 50 °C for 46 h. The reaction was concentrated, and the residue was treated with DCM (4 ml) and brine (2 ml). The organic layer was separated, washed with brine (2 x 2 ml), dried over Na₂SO₄, and filtered. The filtrate was concentrated to a brown oil (160 mg).

The brown oil was subjected to the following chiral chromatography conditions: Column: Amylose-1, 30 x 250 mm, 5 µm; eluting with 40% IPA (0.5% DMEA) in CO₂ to afford the title compound, Isomer 1 (59 mg, 37 %), t_{R} is 1.83, ee > 98% ee; 572 [M+H]⁺ and title compound, Isomer 2 (47 mg, 29 %), t_{R} is 2.58, ee > 98% ee. MS ES+ m/z 572 [M+H]⁺. The retention times were obtained using analytical method G (Refer to Table A for specific analytical conditions).

The following compound was prepared in a manner essentially analogous to the method of Examples 26 and 27 using the appropriate reagents, adjusting reaction time to determine completion of the reaction, and adjusting the purification system as appropriate.

If the retention time was obtained from an analytical column the method will be listed in the final column. Refer to Table A for specific analytical conditions for each method.

**Table 55**

| Ex # | Chemical Name | Structure | MS ES+ m/z | t_{R} (min) and method |
|---|---|---|---|---|
| 28^{1,2} | 4-[2-(Dimethyl amino)-2-(5-fluoro-2-pyridyl) ethoxy]-6-[5-methyl-1-[1-(oxetan-3-yl)-4-piperidyl]triazol-4-yl]pyrazolo[1,5-a]pyridine-3-carbonitrile, Isomer 2 | 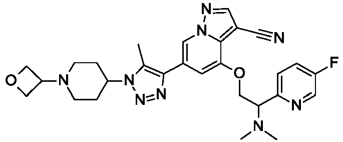 | 546 [M+H]⁺ | 2.76 H |
| 29^{3,4,5} | 4-[2-(3,3-Difluoroazetidin-1-yl)-2-(3,5-difluoro-2-pyridyl)ethoxy]-6-[5-methyl-1-[1-(oxetan-3-yl)-4-piperidyl] triazol-4-yl] pyrazolo [1,5-a] pyridine-3-carbonitrile, Isomer 1 | 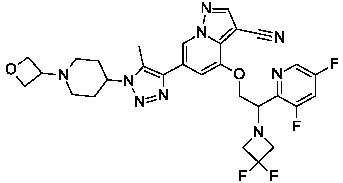 | 612 [M+H]⁺ | 2.43 J |
| 30^{6,7,8} | 2-[3-Fluoro-6-[5-methyl-1-[1-(oxetan-3-yl)-4-piperidyl] triazol-4-yl] pyrazolo[1,5-a] pyridin-4-yl]oxy-1-(5-fluoro-2-pyridyl)-N,N-dimethyl-ethanamine, Isomer 2 | 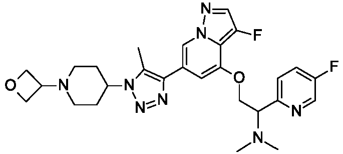 | 539 [M+H]⁺ | 2.82 P |

| | | | | |
|---|---|---|---|---|
| ¹ Reaction run in dimethylamine (2M) in THF at RT. ² Column: Chiralpak AD, 20 x 250 mm, 5 µm; eluting with 35% IPA (0.5% DMEA) in CO₂. ³ Cs₂CO₃ used as base ⁴ Column: XBridge C18, 19 x 150mm, 5 µm; eluting with 45% to 75% ACN in H₂O (NH₄HCO₃ 10mM pH 9). ⁵ Column: Chiralpak AD, 20 x 250 mm, 5 µm; eluting with 45% EtOH (0.5% DMEA) in CO₂. ⁶ Refluxed for 5 h then stirred at RT for 10 days. ⁷ Purified by reversed phase chromatography eluting with 0% to 50% (10% MeOH in DCM) in DCM (20 CV) then 50% (10% MeOH in DCM) in DCM (20 CV). ⁸ Column: Chiral Art Amylose C, 30 x 250 mm, 5 µm; eluting with 40% IPA (0.5% DMEA) in CO₂. | | | | |

### Example 31

### 4-[4-[4-[3-Chloro-4-[2-(5-fluoro-2-pyridyl)-2-methoxy-ethoxy]pyrazolo[1,5-a]pyridin-6-yl]-5-methyl-triazol-1-yl]-1-piperidyl]tetrahydrofuran-3-ol, Isomer 1

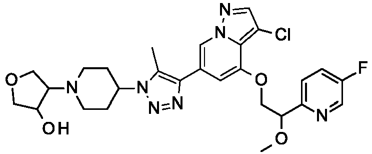

and

### Example 32

### 4-[4-[4-[3-Chloro-4-[2-(5-fluoro-2-pyridyl)-2-methoxy-ethoxy]pyrazolo[1,5-a]pyridin-6-yl]-5-methyl-triazol-1-yl]-1-piperidyl]tetrahydrofuran-3-ol, Isomer 2

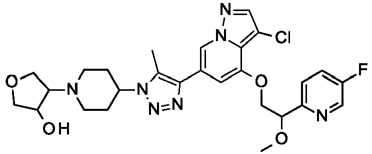

3,6-dioxabicyclo[3.1.0]hexane (157 mg, 1.73 mmol) was added to a soln of 3-chloro-4-[2-(5-fluoro-2-pyridyl)-2-methoxy-ethoxy]-6-[5-methyl-1-(4-piperidyl)triazol-4-yl]pyrazolo[1,5-a]pyridine (350 mg, 0.72 mmol) in EtOH (3 ml) and it was stirred overnight at 80°C. Additional 3,6-dioxabicyclo[3.1.0]hexane (157 mg, 1.73 mmol) was added, and the reaction was stirred overnight. The reaction was diluted with DCM (50 ml), washed with H₂O (2 x 20 ml) and brine (20 ml). The crude material was purified by silica gel chromatography eluting with MeOH in DCM to afford a brown colored oil. The oil was subjected to SFC eluting with 55% (50% 2-propanol in ACN) in CO₂ (0.1% DEA). After the chiral separation both diastereomeric pairs were individually purified by flash chromatography and eluted with a gradient of MeOH in DCM. The isolated material from each purification was triturated in pentane, washed with pentane followed by Et₂O to afford title compound, Isomer 1, (36 mg, 8%), t_{R} is 6.46 min with 96% EE, MS ES+ m/z 572 [M+H]⁺ and title compound, Isomer 2, (45 mg, 11%), t_{R} is 6.86 min with 96% EE, MS ES+ m/z 572 [M+H]⁺.

### Example 33

### 2-[3-Chloro-6-[5-methyl-1-[1-(oxetan-3-yl)-4-piperidyl]triazol-4-yl]pyrazolo[1,5-a]pyridin-4-yl]oxy-1-(5-fluoro-2-pyridyl)ethanol, Isomer 2

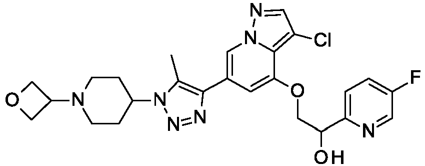

To a stirred mixture of 2-[3-Chloro-6-[5-methyl-1-(4-piperidyl)triazol-4-yl]pyrazolo[1,5-a] yridine-4-yl]oxy-1-(5-fluoro-2-pyridyl)ethanol 2,2,2 trifluoroacetic acid (800 mg, 1.37 mmol) in MeOH (20 ml) was added 3-oxetanone (611 mg, 8.48 mmol) at RT. The resulting mixture was stirred for 1 h at 50 °C under N₂. To the above mixture was added NaBH₃CN (426 mg, 6.78 mmol) in portions at RT. The resulting mixture was stirred for additional 2 h at 50°C under N₂. Upon cooling to RT the reaction was diluted with H₂O (50 ml) and the mixture was basified to pH 9 with saturated aq Na₂CO₃. The mixture was extracted with EA (3 x 150 ml). The combined organic layers were washed with brine (2 x50 ml), dried over anhydrous Na₂SO₄, filtered, and the filtrate was concentrated in vacuo. The residue was purified by reversed flash chromatography with the following conditions: column, C18; eluting with 30% to 35% ACN in H₂O (0.1% NH₃·H₂O) to afford the racemate of the title compound (420 mg, 58%) as a yellow solid. MS ES+ m/z 528 [M+H]⁺.

The yellow solid was subjected to chiral chromatography using the following conditions:
CHIRAL ART Amylose-SA, 2 x 25 cm, 5 µm; Eluting with 50% MeOH in MtBE (10mM NH³-MeOH); flow rate: 20 mL/min; 214/244 nm; to afford the title compound (140 mg, 19 %) t_{R} = 10.92, ee =100%. MS ES+ m/z 528 [M+H]⁺.

### Example 34

### 4-[1-(1-Isopropyltriazol-4-yl)ethoxy]-6-[5-methyl-1-[1-(oxetan-3-yl)-4-piperidyl]triazol-4-yl]pyrazolo[1,5-a]pyridine-3-carbonitrile, Isomer 1

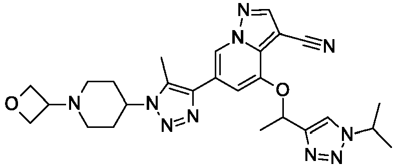

4-[1-(1-Isopropyltriazol-4-yl)ethoxy]-6-[5-methyl-1-(4-piperidyl)triazol-4-yl]pyrazolo[1,5-a]pyridine-3-carbonitrile hydrochloride (689 mg, 1.29 mmol) and DIPEA (0.67 ml, 3.87 mmol) were dissolved in MeOH (10 ml) and activated 4A molecular sieves was added. 3-oxetanone (0.46 ml, 6.46 mmol), NaBH₃CN (406 mg, 6.46 mmol) and AcOH (0.89 ml, 15.50 mol) were added sequentially, and the reaction was stirred at 70 °C for 90 min. Upon cooling to RT, the reaction was diluted with EA. The mixture was washed with H₂O (1 x 25 ml) and brine (1 x 25 ml), dried over MgSO₄, filtered, and concentrated in vacuo. The residue was purified by silica gel chromatography eluting with MeOH in DCM to afford an off-white solid (490 mg, 73%). The off-white solid was subjected to SFC, eluting with 50% EtOH (0.1% DEA) in CO₂ to afford the title compound (140 mg, 21%). RT is 6.93 min. MS ES+ m/z 517.3 [M+H]⁺.

The following compounds were prepared in a manner essentially analogous to the method of Example 34 using the appropriate reagents, adjusting reaction time to determine completion of the reaction, and adjusting the purification system as appropriate.

If the retention time was obtained from an analytical column the method will be listed in the final column. Refer to Table A for specific analytical conditions for each method.

**Table 56**

| Ex # | Chemical Name | Structure | MS ES+ m/z | t_{R} (min) and method |
|---|---|---|---|---|
| 35^{1,2} | 4-[1-(2-Isopropyltriazol-4-yl)ethoxy]-6-[5-methyl-1-[1-(oxetan-3-yl)-4-piperidyl]triazol-4-yl]pyrazolo [1,5-a]pyridine-3-carbonitrile, Isomer 1 | 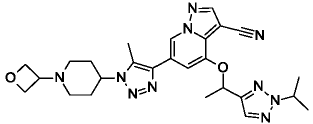 | 517.3 [M+H]⁺ | 6.61 |
| 36^{1,2} | 6-[5-Methyl-1-[1-(oxetan-3-yl)-4-piperidyl] triazol-4-yl]-4-[1-(1-methyl pyrazolo [3,4-c]pyridin-4-yl)ethoxy] pyrazolo[1,5-a]pyridine-3-carbonitrile, Isomer 2 | 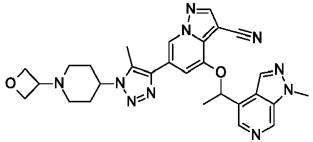 | 539.3 [M+H]⁺ | 9.46 |
| 37^{1,3} | 6-[5-Methyl-1-[1-(oxetan-3-yl)-4-piperidyl] triazol-4-yl]-4-[1-(1-methyl pyrrolo [2,3-c]pyridin-4-yl) ethoxy]pyrazolo[ 1,5-a] pyridine-3-carbonitrile, Isomer 2 | 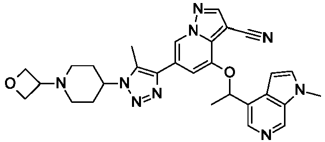 | 538.3 [M+H]⁺ | 10.2 |
| 38^{1,3} | 6-[5-Methyl-1-[1-(oxetan-3-yl)-4-piperidyl] triazol-4-yl]-4-[1-[1-(trifluoro methyl) pyrazol-3-yl]ethoxy] pyrazolo[1,5-a]pyridine-3-carbonitrile, Isomer 1 | 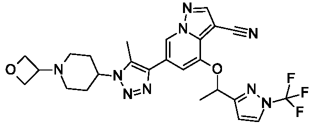 | 542.2 [M+H]⁺ | 6.90 |
| 39^{1,3} | 4-[1-(4-Cyclopropyl-5-fluoro-2-pyridyl) ethoxy]-6-[5-methyl-1-[1-(oxetan-3-yl)-4-piperidyl]triazol-4-yl]pyrazolo [1,5-a]pyridine-3-carbonitrile, Isomer 2 | 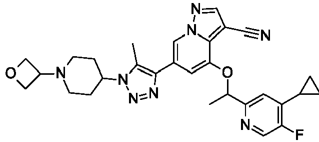 | 543.3 [M+H]⁺ | 8.97 |
| 40⁴ | 4-[2-(3,5-Difluoro-2-pyridyl)-2-methoxy-ethoxy]-3-fluoro-6-[5-methyl-1-[1-(oxetan-3-yl)-4-piperidyl] triazol-4-yl]pyrazolo[1,5-a]pyridine, Isomer 2 | 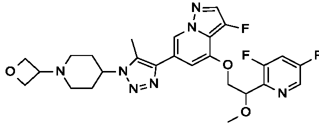 | 544 [M+H]⁺ | 22.0 |
| 41^{5,6} | 3-Chloro-5-[2-(5-fluoro-2-pyridyl)-2-methoxy-ethoxy]-7-[5-methyl-1-[1-(oxetan-3-yl)-4-piperidyl]triazol-4-yl]imidazo [1,2-a]pyridine, Isomer 2 | 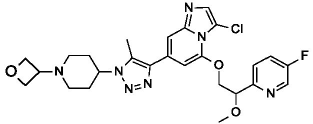 | 542 [M+H]⁺ | 20.2 |
| 42^{7,8} | 3-Chloro-5-[2-(3,5-difluoro-2-pyridyl)-2-methoxy-ethoxy]-7-[5-methyl-1-[1-(oxetan-3-yl)-4-piperidyl]triazol-4-yl]imidazo [1,2-a]pyridine, Isomer 1 | 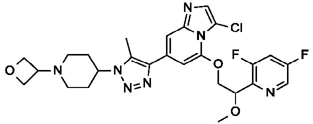 | 560 [M+H]⁺ | 4.45 |
| 43^{9,10} | 3-Chloro-4-[2-(3,5-difluoro-2-pyridyl)-2-methoxy-ethoxy]-6-[5-methyl-1-[1-(oxetan-3-yl)-4-piperidyl]triazol-4-yl]pyrazolo [1,5-a]pyridine, Isomer 1 | 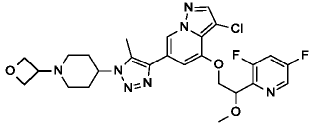 | (³⁵Cl/³⁷Cl) 560/562 [M+H]⁺ | 2.51 H |
| 44^{9,10} | 3-Chloro-4-[2-(3,5-difluoro-2-pyridyl)-2-methoxy-ethoxy]-6-[5-methyl-1-[1-(oxetan-3-yl)-4-piperidyl]triazol-4-yl]pyrazolo [1,5-a]pyridine, Isomer 2 | 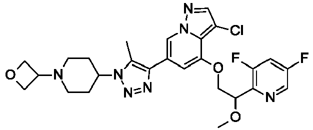 | (³⁵Cl/³⁷Cl) 560/562 [M+H]⁺ | 2.62 H |
| 45^{11,12} | 4-[1-(2-Cyclopropyl thiazol-4-yl)ethoxy]-6-[5-methyl-1-[1-(oxetan-3-yl)-4-piperidyl]triazol-4-yl]pyrazolo [1,5-a]pyridine-3-carbonitrile, Isomer 1 | 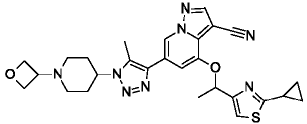 | 531 [M+H]⁺ | 1.12 I |
| 46^{13,14} | 4-[1-(2-methoxy thiazol-4-yl)ethoxy]-6-[5-methyl-1-[1-(oxetan-3-yl)-4-piperidyl]triazol-4-yl]pyrazolo [1,5-a] pyridine-3-carbonitrile, Isomer 1 | 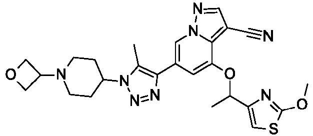 | 521 [M+H]⁺ | 2.63 J |
| 47¹⁵ | 4-[2-(5-Fluoro-2-pyridyl)-2-isopropoxy-ethoxy]-6-[5-methyl-1-[1-(oxetan-3-yl)-4-piperidyl]triazol-4-yl]pyrazolo [1,5-a]pyridine-3-carbonitrile, Isomer 2 | 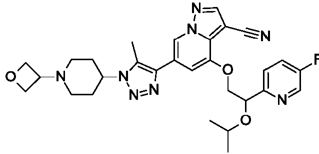 | 561 [M+H]⁺ | 1.68 H |
| 48^{16,17} | 4-[2-(2,4-Difluorophenyl)-2-hydroxy-ethoxy]-6-[5-methyl-1-[1-(oxetan-3-yl)-4-piperidyl]triazol-4-yl]pyrazolo [1,5-a]pyridine-3-carbonitrile | 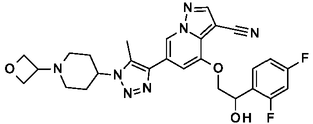 | 536 [M+H]⁺ | 1.47 |
| 49¹⁸ | 2-[3-Fluoro-6-[5-methyl-1-[1-(oxetan-3-yl)-4-piperidyl]triazol-4-yl]pyrazolo [1,5-a]pyridin-4-yl]oxy-1-(5-fluoro-2-pyridyl) ethanol, Isomer 2 | 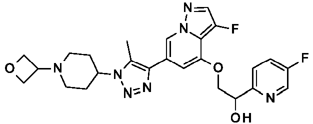 | 512 [M+H]⁺ | 9.67 |
| 50^{1,19} | 2-[3-Chloro-6-[5-methyl-1-[1-(oxetan-3-yl)-4-piperidyl] triazol-4-yl] pyrazolo[1,5-a] pyridin-4-yl]oxy-1-isothiazol-3-yl-ethanol, Isomer 2 | 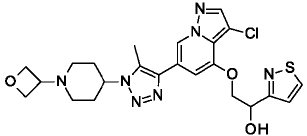 | (³⁵Cl/³⁷Cl) 516/518 [M+H]⁺ | 7.99 |
| 51^{9,20} | 2-[3-Chloro-6-[5-methyl-1-[1-(oxetan-3-yl)-4-piperidyl]triazol-4-yl]pyrazolo [1,5-a]pyridin-4-yl]oxy-1-(3,5-difluoro-2-pyridyl)-N,N-dimethyl-ethanamine, Isomer 1 | 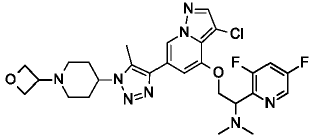 | (³⁵Cl/³⁷Cl) 573/575 [M+H]⁺ | 1.06 J |
| 52^{21,22,23} | 1-[3-Chloro-6-[1-[(3S,4R)-3-fluoro-1-(oxetan-3-yl)-4-piperidyl]-5-methyl-triazol-4-yl]pyrazolo[1,5-a]pyridin-4-yl]oxy-2-(5-fluoro-2-pyridyl) propan-2-ol, Isomer 1 | 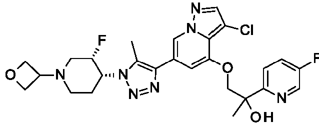 | 560 [M+H]⁺ | 7.00 |
| 53^{21,24,25} | 1-[3-Chloro-6-[1-[(3S,4S)-3-fluoro-1-(oxetan-3-yl)-4-piperidyl]-5-methyl-triazol-4-yl]pyrazolo[1,5-a]pyridin-4-yl]oxy-2-(5-fluoro-2-pyridyl) propan-2-ol, Isomer 1 | 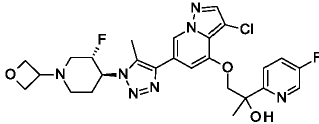 | 560 [M+H]⁺ | 5.43 |
| 54^{26,27,28} | 2-(3,5-Difluoro-2-pyridyl)-1-[3-fluoro-6-[5-methyl-1-[1-(oxetan-3-yl)-4-piperidyl]triazol-4-yl]pyrazolo [1,5-a]pyridin-4-yl]oxy-propan-2-ol, Isomer 1 | 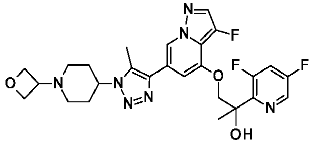 | 544 [M+H]⁺ | 2.28 S |
| 55^{21,29,30,31,32} | 3-Chloro-5-[2-(3,5-difluoro-2-pyridyl)-2-methoxy-ethoxy]-7-[5-methyl-1-[4-methyl-1-(oxetan-3-yl)-4-piperidyl]triazol-4-yl]imidazo [1,2-a]pyridine, Isomer 2 | 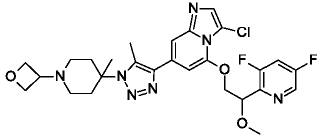 | 574 [M+H]⁺ | 9.70 |
| 56^{29,31,33,34,35} | 1-[3-Chloro-6-[1-[(4R)-3,3-difluoro-1-(oxetan-3-yl)-4-piperidyl]-5-methyl-triazol-4-yl]pyrazolo[1,5-a]pyridin-4-yl]oxy-2-(5-fluoro-2-pyridyl)propan-2-ol, Isomer 1 | 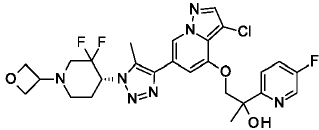 | 578 [M+H]⁺ | 5.31 |
| 57^{29,34,36,37} | 1-[3-Chloro-6-[1-[(3R,4S)-3-fluoro-1-(oxetan-3-yl)-4-piperidyl]-5-methyl-triazol-4-yl]pyrazolo[1,5-a]pyridin-4-yl]oxy-2-(5-fluoro-2-pyridyl)propan-2-ol, Isomer 1 | 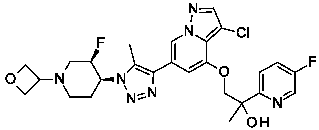 | 560 [M+H]⁺ | 4.49 |
| 58^{21,29,37,38,39} | 1-[3-Chloro-6-[1-[(3R,4R)-3-fluoro-1-(oxetan-3-yl)-4-piperidyl]-5-methyl-triazol-4-yl]pyrazolo[1,5-a]pyridin-4-yl]oxy-2-(5-fluoro-2-pyridyl)propan-2-ol, Isomer 1 | 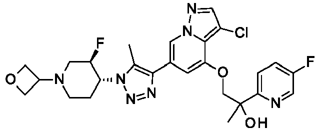 | 560 [M+H]⁺ | 5.20 |
| 59^{40,41} | 2-(5-Fluoro-2-pyridyl)-1-[3-methyl-6-[5-methyl-1-[1-(oxetan-3-yl)-4-piperidyl]triazol-4-yl]pyrazolo [1,5-a]pyridin-4-yl]oxy-propan-2-ol, Isomer 2 | 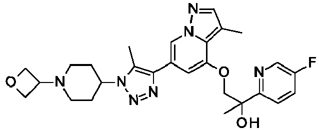 | 522 [M+H]⁺ | 8.30 |
| 60^{21,43,44} | 3-Chloro-6-[1-[(3S,4R)-3-fluoro-1-(oxetan-3-yl)-4-piperidyl]-5-methyl-triazol-4-yl]-4-[2-(5-fluoropyrimidin-2-yl)-2-methoxy-ethoxy]pyrazolo[ 1,5-a]pyridine, Isomer 2 | 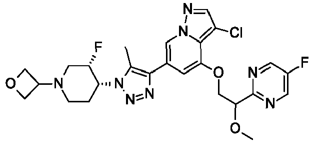 | 561 [M+H]⁺ | 19.87 |
| 61^{5, 45} | 1-[3-Chloro-6-[1-[(3S,4R)-3-fluoro-1-(oxetan-3-yl)-4-piperidyl]-5-methyl-triazol-4-yl]pyrazolo[1,5-a]pyridin-4-yl]oxy-2-(5-fluoropyrimidin-2-yl)propan-2-ol, Isomer 1 | 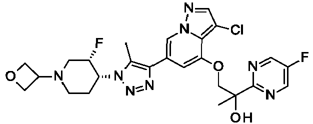 | 561 [M+H]⁺ | 13.5 |
| 62^{46,47} | 3-Chloro-6-[1-[(3S,4R)-3-fluoro-1-(oxetan-3-yl)-4-piperidyl]-5-methyl-triazol-4-yl]-4-[2-(5-fluoro-2-pyridyl)-2-methoxy-ethoxy]pyrazolo[ 1,5-a]pyridine, Isomer 2 | 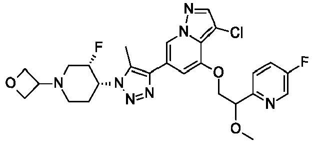 | 560 [M+H]⁺ | 6.3 |
| 63^{54,48} | 2-[3-Chloro-6-[1-[(3S,4R)-3-fluoro-1-(oxetan-3-yl)-4-piperidyl]-5-methyl-triazol-4-yl]pyrazolo[1,5-a]pyridin-4-yl]oxy-1-(5-fluoropyrimidin-2-yl)ethanol, Isomer 2 | 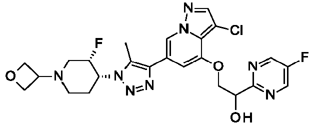 | 547 [M+H]⁺ | 19.0 |
| 64^{49,50,51} | 3-Chloro-5-[2-(3,5-difluoro-2-pyridyl)-2-methoxy-ethoxy]-7-[5-methyl-cis-1-[3-[4-(oxetan-3-yl)piperazin-1-yl]cyclobutyl]tria zol-4-yl] imidazo[1,2-a]pyridine, Isomer 1 | 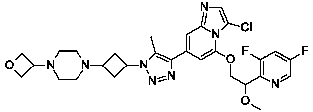 | 615 [M+H]⁺ | 9.2 |
| 65^{21,42,52,53} | 3-Chloro-5-(2-(3,5-difluoropyridin-2-yl)-2-methoxyethoxy)-7-(5-methyl-1-((1r,3r)-3-(4-(oxetan-3-yl)piperazin-1-yl)cyclobutyl)-1H-1,2,3-triazol-4-yl) imidazo [1,2-a]pyridine, Isomer 2 | 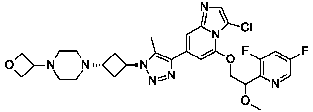 | 615 [M+H]⁺ | 15.7 |

| | | | | |
|---|---|---|---|---|
| ¹ Purified by silica gel chromatography eluting with MeOH in DCM. ² SFC, eluting with 60% MeOH (0.1% DEA) in CO₂. ³ SFC, eluting with 50% EtOH (0.1% DEA) in CO₂. ⁴ Column: Chiralpak IG, 3 x 25 cm column, 5µm; eluting with 60% ACN in H₂O (0.5% DEA). ⁵ Purified by Prep-TLC (DCM / MeOH 20: 1) ⁶ Column: Chiralpak IA, 2 x 25 cm column, 5 µm; eluting with 20% MeOH in 1:1 hexane / MTBE (0.5% 2M NH₃ in MeOH). ⁷ Purified by reversed phase chromatography: Column: AQ - C18, 250 x 50mm, 10µm; eluting with 20% to 35% ACN in aq FA. ⁸ Column: Chiralpak IG, 2 x 25 cm column, 5 µm; eluting with 25% MeOH in MTBE (10 mM NH₃-MeOH). ⁹ Purified by silica gel chromatography, eluting with 0% to 100% acetone in DCM. ¹⁰ Column: Amylose-C, 20 x 250 mm column, 5 µm; eluting with 45% EtOH (0.5% DMEA) in CO₂. ¹¹Purified by reversed phase C18 chromatography eluting with 30% to 60% ACN in H₂O ( NH₄CO₃ pH 9 buffer). ¹² Column: Chiralpak AD, 20 x 250 mm, 5 µm; eluting with 45% EtOH (0.5% DMEA) in CO₂. ¹³ Purified by silica gel chromatography eluting with 100% DCM (2CV), 0% to 2% gradient MeOH in DCM(13 CV), 2% MeOH in DCM (10 CV), 2% to 5% gradient MeOH in DCM (10 CV), 5% to 10% gradient MeOH in DCM (2 CV), 10% MeOH in DCM (15 CV). ¹⁴ Column: Chiralart Amylose C, 30 x 250 mm, 5 µm; eluting with 40% EtOH (0.5% DMEA) in CO₂. ¹⁵ Column: Chiralpak AD, 30 x 250mm, 5 µm; eluting with 40% IPA (0.5% DMEA) in CO₂. ¹⁶ Purified by silica gel chromatography eluting with DCM / MeOH (10:1). ¹⁷ Column: CHIRALPAK IE-3, 4.6 x 50 cm, 3µm; eluting with 50% EtOH in MTBE (0.1% DEA). ¹⁸ Column: Chiralpak IA, 2 x 25 cm column, 5µm; eluting with 50% EtOH in MTBE (10mM NH₃-MeOH). ¹⁹ SFC, eluting with 50% IPA (0.1% DEA) in CO₂. ²⁰ Column: Chiralpak AD, 20 x 250 mm, 5 µm; eluting with 35% EtOH in CO₂. ²¹ 4A molecular sieves, DIPEA, and AcOH were excluded in the reaction. ²² Purified by Prep-TLC (PE / EA 1:1) ²³ Column: CHIRALPAK IE, 3 x 25 cm, 5 µm; eluting with 50% MeOH in MTBE (0.5% 2M NH₃-MeOH). ²⁴ Purified by reversed phase C18 chromatography eluting with 50% to 60% ACN in H₂O ²⁵ Column: CHIRALPAK ID, 2 x 25 cm, 5 µm; eluting with 30% MeOH in MTBE (10mM NH₃-MeOH). ²⁶ Workup: Reaction concentrated. Residue was dissolved into DCM and aq Na₂CO₃ then filtered through a phase separator. Organic phase dried over MgSO₄, filtered and concentrated. ²⁷ Purified by reversed phase flash chromatography eluting with 30% ACN in aq NH₄HCO₃ pH 9 (2 CV), 30% to 60% ACN in aq NH₄HCO₃ pH 9 (8 CV), 60% ACN in aq NH₄HCO₃ pH 9 (2 CV) then 100% ACN. ²⁸ Chiralpak AD, 20 x 250mm, 5µm; eluting with 45% IPA (0.5% DMEA) in CO₂. ²⁹ Reaction conducted at 50°C ³⁰ Workup: Reaction quenched at RT with H₂O, pH adjusted to 8 with aq NaHCO₃, extracted with EA (3 x 30 ml), washed with brine (2 x 20 ml), dried over Na₂SO₄, filtered, and concentrated. ³¹ Purified by Prep-TLC (5% MeOH in DCM). ³² Column: CHIRALPAK ID, 3 x 25 cm, 5 µm; eluting with 30% EtOH in 2:1 DCM / MeOH (0.1% 2M NH₃-MeOH). ³³ 4A molecular sieves and DIPEA were excluded in the reaction. ³⁴ Workup: Reaction diluted at RT with saturated K₂CO₃ (30 ml), extracted with EA (3 x 50 ml), washed with brine (3 x 50 ml), dried over Na₂SO₄, filtered, and concentrated. ³⁵ Column: CHIRALPAK IE, 2 x 25 cm, 5 µm; eluting with 50% MeOH in MTBE (0.5% 2M NH₃-MeOH). ³⁶ Workup: Reaction quenched at RT with saturated NaHCO₃ (20 ml), extracted with 25% i-PrOH in CHCl₃ (3 x 30 ml), washed with brine (2 x 20 ml), dried over Na₂SO₄, filtered, and concentrated. ³⁷ Column: CHIRALPAK IE, 2 x 25 cm, 5 µm; eluting with 50% MeOH in MTBE (10mM NH₃-MeOH). ³⁸ Workup: Reaction was concentrated and diluted with H₂O (10 ml), extracted with 20% i-PrOH on CHCl₃ (3 x 30 ml), dried over Na₂SO₄, filtered, and concentrated. ³⁹ Purified by Prep-TLC EA 100%. ⁴⁰ Purified by silica gel chromatography eluting with 40% (10% MeOH in DCM) in DCM. ⁴¹ SFC chromatography eluting with 40% EtOH (1% DEA) in 60% CO₂. ⁴² Workup: Reaction quenched at RT with H₂O, pH adjusted to 8 with aq NaHCO₃, extracted with CHCl₃:IPA, dried over Na₂SO₄, filtered, and concentrated. ⁴³ Purified by silica gel chromatography eluting with MeOH in EA (1:30 to 1:20). ⁴⁴ Column: CHIRALPAK IE, 2 x 25 cm, 5 µm; eluting with 30% to 50% MeOH in MTBE (0.5% 2M NH₃-MeOH). ⁴⁵ Column: Chiralpak IA, 2 x 25 cm column, 5 µm; eluting with 15% ACN:EtOH (2:1) in MTBE (10 mM NH₃ in MeOH). ⁴⁶ Purified by reversed phase C18 chromatography: Column: Ultimate XB-C18, 50 x 250 mm, 10µm; eluting with 20% to 50% ACN in H₂O (0.5% NH₃H₂O). ⁴⁷ Column: CHIRALPAK ID, 2 x 25 cm, 5 µm; eluting with 50% [MeOH in DCM (1 : 1) (2 M NH₃-MeOH)] in MeOH. ⁴⁸ Column: CHIRALPAK ID, 2 x 25 cm, 5 µm; eluting with 50% [MeOH in DCM (1 : 1) (0.1% 2M NH₃-MEOH)] in MeOH. ⁴⁹ Workup: Reaction quenched at RT with H₂O, pH adjusted between 8 and 9 with aq Na₂CO₃, extracted with i-PrOH in CHCl₃, washed with brine, dried over Na₂SO₄, filtered, and concentrated. ⁵⁰ Purified by Prep-TLC MeOH in DCM (1 to 15). ⁵¹ Column: CHIRAL ART Cellulose-SC, 2 x 25 cm, 5 µm; eluting with 50% MeOH in MTBE (10mM NH3-MeOH). ⁵² Purified by Prep-TLC (DCM / MeOH 10:1). ⁵³ Column: Chiralpak IA, 2 x 25 cm column, 5µm; eluting with 50% MeOH in MTBE (10mM NH₃-MeOH). ⁵ Purified by reversed phase C18 chromatography eluting with 10% to 40% ACN in H₂O (10 mmol/L NH₃H₂O). | | | | |

### Example 66

### 6-[3,5-Dimethyl-1-[1-(oxetan-3-yl)-4-piperidyl]pyrazol-4-yl]-4-[1-[5-(trifluoromethyl)-3-pyridyl]ethoxy]pyrazolo[1,5-a]pyridine-3-carbonitrile, Isomer 2

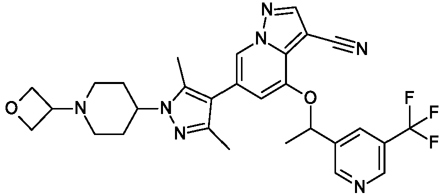

A mixture of 6-(4,4,5,5-tetramethyl-1,3,2-dioxaborolan-2-yl)-4-[1-[5-(trifluoromethyl)-3-pyridyl]ethoxy]pyrazolo[1,5-a]pyridine-3-carbonitrile (95 mg, 0.21 mmol), 4-(4-bromo-3,5-dimethyl-pyrazol-1-yl)-1-(oxetan-3-yl)piperidine (73 mg, 0.23 mmol), and K₂CO₃ (78 mg, 0.56 mmol) in toluene (0.85 ml) and H₂O (0.21 ml) was degassed with sonication under N₂. PdCl₂(DtBPF) (9 mg, 0.14 mmol) was added, the vessel was sealed, and stirred at 80°C overnight. The reaction was diluted with EA and H₂O, the layers were separated, the organic layer was dried over MgSO₄, filtered, and concentrated. The resulting residue was purified by silica gel chromatography eluting with 0% to 60% EtOH in EA (1:3) in EA to obtain the title compound (91 mg, 76%) as a green solid. MS ES+ m/z 566 [M+H]⁺.

The following compound was prepared in a manner essentially analogous to the method of Example 66 using the appropriate reagents, adjusting reaction time to determine completion of the reaction, and adjusting the purification system as appropriate.

**Table 57**

| Ex # | Chemical Name | Structure | MS ES+ m/z |
|---|---|---|---|
| 67¹ | 6-[5-Methyl-1-[1-(oxetan-3-yl)-4-piperidyl]triazol-4-yl]-4-[1-[5-(trifluoromethyl)-3-pyridyl]ethoxy]pyrazolo[1,5-a]pyridine-3-carbonitrile, Isomer 2 | 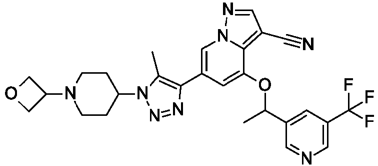 | 553 [M+H]⁺ |

| | | | |
|---|---|---|---|
| ¹Work up: Reaction concentrated. Residue diluted with H₂O and 35% aq HCl was added to adjust pH to 1. i-PrOH was gradually added until soln resulted. Charcoal added and reaction was stirred 1 h. DE was added and stirred 30 min. Suspension filtered and solids were washed with i-PrOH:H₂O (1:2). pH of filtrate adjusted to 10 with aq NaOH (10M). Resultant insoluble material was collected by filtration to afford title compound. | | | |

### Example 68

### 6-[5-Methyl-1-[1-(oxetan-3-yl)-4-piperidyl]triazol-4-yl]-4-[1-[5-(trifluoromethyl)-3-pyridyl]ethylamino]pyrazolo[1,5-a]pyridine-3-carbonitrile, Isomer 1

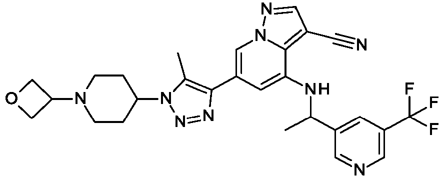

A pressure tube was charged with 6-(4,4,5,5-tetramethyl-1,3,2-dioxaborolan-2-yl)-4-[1-[5-(trifluoromethyl)-3-pyridyl]ethylamino]pyrazolo[1,5-a]pyridine-3-carbonitrile (0.39 g, 0.45 mmol), 4-(4-bromo-5-methyl-triazol-1-yl)-1-(oxetan-3-yl)piperidine (0.17 g, 0.55 mmol), K₂CO₃ (0.16 g, 1.12 mmol), toluene (8 ml) and H₂O (1 ml) was degassed by bubbling N₂ through the mixture for several min and then added PdCl₂(DtBPF) (0.02 g, 0.03 mmol) and degassed for another 2 min. The tube was capped, and the reaction was heated to 95 °C for 6 h. The reaction was cooled to RT, diluted with H₂O, and extracted with EA (3x). The combined organic layers were dried over MgSO₄, filtered, and concentrated. The residue was purified by silica gel chromatography and was eluted with 0% to 100% EA in cHex and then 0% to 5% MeOH in DCM to afford a brown foam (184 mg, 65%). MS ES+ m/z 552 [M+H]⁺.

The brown foam was subjected to chiral chromatography using the following conditions: Column: Chiralpak IH, 3 x 100mm, 3 µm; eluting with 10% to 50% EtOH (0.2% IPAm) in CO₂ to afford the title compound (69 mg, 24%), t_{R} is 1.70, ee > 98%. MS ES+ m/z 552 [M+H]⁺. The retention time was obtained using analytical method K. (Refer to Table A for specific analytical conditions).

The following compounds were prepared in a manner essentially analogous to the method of Example 68 using the appropriate reagents, adjusting reaction time to determine completion of the reaction, and adjusting the purification system as appropriate. K₃PO₄ and IPA may also be used.

If the retention time was obtained from an analytical column the method will be listed in the final column. Refer to Table A for specific analytical conditions for each method.

**Table 58**

| Ex # | Chemical Name | Structure | MS ES+ m/z | t_{R} (min) and method |
|---|---|---|---|---|
| 69^{1,2} | 6-[5-Methyl-1-[1-(oxetan-3-yl)azepan-4-yl]triazol-4-yl]-4-[1-[5-(trifluoromethyl)-3-pyridyl]ethoxy]pyrazo lo[1,5-a]pyridine-3-carbonitrile, Isomer 1 | 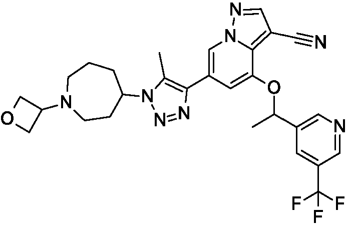 | 567 [M+H]⁺ | 1.09 Q |
| 70^{1,2} | 6-[5-Methyl-1-[1-(oxetan-3-yl)azepan-4-yl]triazol-4-yl]-4-[1-[5-(trifluoromethyl)-3-pyridyl]ethoxy]pyrazo lo[1,5-a]pyridine-3-carbonitrile, Isomer 2 | 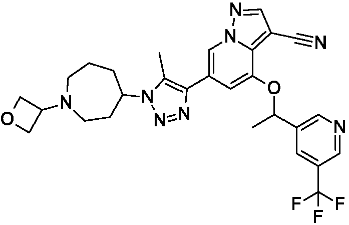 | 567 [M+H]⁺ | 2.32 Q |
| 71^{3,4} | 4-[2-(5-Fluoro-2-pyridyl)-2-hydroxy-ethoxy]-6-[5-methyl-1-[(1S,5R)-8-(oxetan-3-yl)-8-azabicyclo [3.2.1]octan-3-yl]triazol-4-yl]pyrazolo[1,5-a]pyridine-3-carbonitrile | 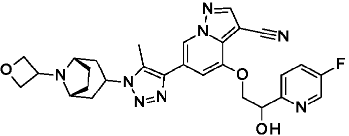 | 545 [M+H]⁺ | |
| 72^{5,6} | 4-[3-Hydroxy-3-(2-pyridyl)pyrrolidin-1-yl]-6-[2-methyl-3-[1-(oxetan-3-yl)-4-piperidyl]cyclopenta-1,4-dien-1-yl]pyrazolo[1,5-a]pyridine-3-carbonitrile, Isomer 2 | 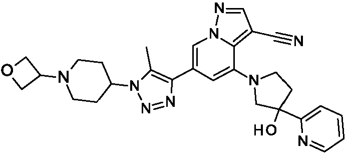 | 526 [M+H]⁺ | 1.24 R |

| | | | | |
|---|---|---|---|---|
| ¹ Purified by silica gel chromatography eluted with 0% to 100% (25% EtOH: EA) in cHex. ² Column: Chiralpak AD, 20 x 250 mm, 5 µm; eluting with 45% MeOH (0.2% DMEA) in CO₂. ³Column: Chiralpak IH, 3 x 100mm, 3 µm; eluting with 10% to 50% EtOH (0.2% IPAm) in CO₂. ⁴ Column: Chiralpak IA, 3 x 100mm, 3 µm; eluting with 25% to 50% EtOH (0.2% IPAm) in CO₂. ⁵ Purified by reversed phase flash C18 chromatography with the following conditions: column, C18; eluting with 20% to 60% ACN in H2O (NH₅CO₃ pH 9). ⁶ Column: Chiralcel OD, 20 x 250 mm, 5 µm; eluting with 45% MeOH (0.5% DMEA) in CO₂. | | | | |

### Example 73

### 6-[5-Methyl-1-[1-(2,2,6,6-tetramethyltetrahydropyran-4-yl)-4-piperidyl]pyrazol-4-yl]-4-[(1R)-1-(2-pyridyl)ethoxy]pyrazolo[1,5-a]pyridine-3-carbonitrile

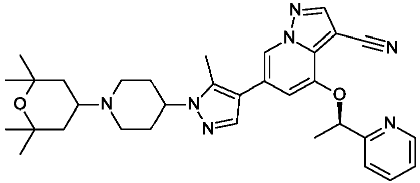

A soln of 6-[5-methyl-1-(4-piperidyl)pyrazol-4-yl]-4-[(1R)-1-(2-pyridyl)ethoxy]pyrazolo[1,5-a]pyridine-3-carbonitrile (55 mg, 0.13 mmol) and 2,2,6,6-tetramethyltetrahydropyran-4-one (36 mg, 0.23 mmol) in MeOH (3 ml) was treated with NEt₃ (90 µl, 0.65 mmol) and ZnCl₂ (10 ml, 0.02 mmol, 1.9 M in MeTHF) and the reaction stirred at 50°C. After stirring overnight, the reaction was allowed to cool to RT and treated with NaBH₃CN (33 mg, 0.53 mmol). After stirring at 50°C for 3 days, the reaction was quenched with H₂O and EA. The phases were separated, and the aq phase was extracted with EA. The organic layers were combined, dried over MgSO4, filtered, and concentrated. The resulting residue was purified by silica gel chromatography eluting with 5% MeOH in DCM to obtain the title compound (50 mg, 66%) as an amber oil. MS ES+ m/z 568 [M+H]⁺.

### Example 74

### 6-[5-Methyl-1-[2-(oxetan-3-yl)-2-azaspiro[3.3]heptan-6-yl]pyrazol-4-yl]-4-[(1R)-1-(2-pyridyl)ethoxy]pyrazolo[1,5-a]pyridine-3-carbonitrile

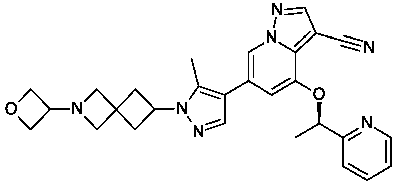

6-[1-(2-Azaspiro[3.3]heptan-6-yl)-5-methyl-pyrazol-4-yl]-4-[(1R)-1-(2-pyridyl)ethoxy]pyrazolo[1,5-a]pyridine-3-carbonitrile, 2,2,2-trifluoroacetic acid (106 mg, 0.16 mmol) was dissolved into DCE (3 ml) and treated with NEt₃ (100 µl, 0.72 mmol), 3-oxetanone (25 µL, 0.43 mmol), and Na(OAc)₃BH (89 mg, 0.42 mmol) and allowed the reaction to stir for 16 h at RT. An additional aliquot of 3-oxetanone (25 µl, 0.43 mmol) was added and stirred the reaction at 50 °C for 4 h. The reaction was diluted with EA (25 ml) and the organic layer was washed with saturated aq NaHCO₃ (50 ml) and brine (50 ml). The organic layer was collected, dried over MgSO4, filtered, and concentrated. The resulting residue was purified by silica gel chromatography eluting with a gradient of 0% to 15% MeOH in DCM to obtain the title compound (26 mg, 31%) as an off-white solid. MS ES+ m/z 496 [M+H]⁺.

### Example 75

### 2-[3-Chloro-6-[5-methyl-1-[1-(oxetan-3-yl)azetidin-3-yl]triazol-4-yl]pyrazolo[1,5-a]pyridin-4-yl]oxy-1-(3,5-difluoro-2-pyridyl)ethanol, Isomer 2

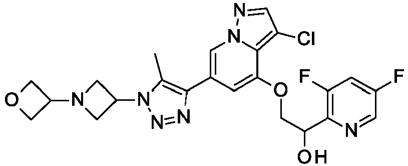

2-[3-Chloro-6-[5-methyl-1-[1-(oxetan-3-yl)azetidin-3-yl]triazol-4-yl]pyrazolo[1,5-a]pyridin-4-yl]oxy-1-(3,5-difluoro-2-pyridyl)ethanol (126 mg) was subjected to the following chiral chromatography conditions: Column: Amylose-C, 30 x 250 mm column, 5 µm; eluting with 40% in IPA in CO₂ to afford the title compound (32 mg, 25%), t_{R} is 2.51 with 90% ee, MS ES+ m/z 518.2 [M+H]⁺. The retention time was obtained using analytical method H. (Refer to Table A for specific analytical conditions).

The following compounds were prepared in a manner essentially analogous to the method of Example 75 using the appropriate reagents, adjusting reaction time to determine completion of the reaction and adjusting the purification system as appropriate.

**Table 59**

| Ex # | Chemical Name | Structure | MS ES+ m/z | t_{R} (min) and method |
|---|---|---|---|---|
| 76^{1,2} | 4-[2-(3-chloro-5-fluoro-2-pyridyl)-2-hydroxy-ethoxy]-6-[5-methyl-1-[1-(oxetan-3-yl)-4-piperidyl] triazol-4-yl]pyrazolo [1,5-a]pyridine-3-carbonitrile, Isomer 1 | 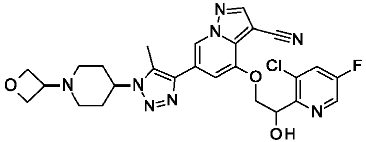 | 553.2 [M+H]⁺ | 3.85 |
| 77^{1,2} | 4-[2-(3-Chloro-5-fluoro-2-pyridyl)-2-hydroxy-ethoxy]-6-[5-methyl-1-[1-(oxetan-3-yl)-4-piperidyl] triazol-4-yl]pyrazolo [1,5-a]pyridine-3-carbonitrile, Isomer 2 | 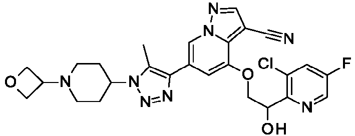 | 553.2 [M+H]⁺ | 4.85 |
| 78^{1,3} | 4-[2-(4-Chloro-5-fluoro-2-pyridyl)-2-hydroxy-ethoxy]-6-[5-methyl-1-[1-(oxetan-3-yl)-4-piperidyl]triazol-4-yl]pyrazolo[1,5-a]pyridine-3-carbonitrile, Isomer 1 | 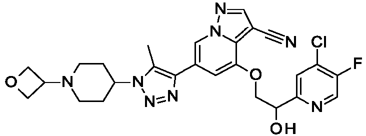 | 553.2 [M+H]⁺ | 4.42 |
| 79⁴ | 4-[2-(2,4-Difluorophenyl)-2-hydroxy-ethoxy]-6-[5-methyl-1-[1-(oxetan-3-yl)-4-piperidyl] triazol-4-yl]pyrazolo [1,5-a]pyridine-3-carbonitrile,Isomer 2 | 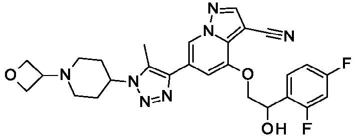 | 536 [M+H]⁺ | 2.22 |
| 80⁵ | 7-[5-Methyl-1-[1-(oxetan-3-yl)-4-piperidyl]triazol-4-yl]-5-[[3,3,3-trifluoro-2-(5-fluoro-2-pyridyl) propyl]amino] imidazo[1,2-a]pyridine-3-carbonitrile, Isomer 1 | 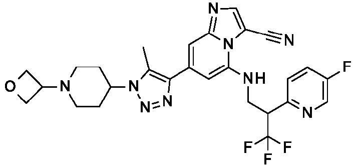 | 570 [M+H]⁺ | 10.92 |
| 81^{6,7} | 1-(5-Fluoro-2-pyridyl)-2-[6-[5-methyl-1-[1-(oxetan-3-yl)-4-piperidyl]triazol-4-yl]-3-(trifluoromethyl) pyrazolo[1,5-a]pyridin-4-yl]oxy-ethanol, Isomer 2 | 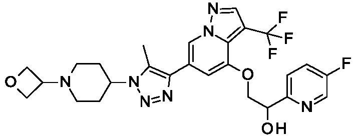 | 562 [M+H]⁺ | 1.83 I |
| 82⁷ | 3-Chloro-4-[2-(5-fluoro-2-pyridyl)-2-methoxy-ethoxy]-6-[5-methyl-1-[1-(oxetan-3-yl)azetidin-3-yl]triazol-4-yl]pyrazolo[1,5-a]pyridine, Isomer 2 | 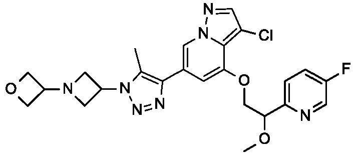 | (³⁵Cl/³⁷Cl) 514/516 [M+H]⁺ | 2.68 P |
| 83⁸ | 4-(2-Cyclopentyl-2-hydroxy-ethoxy)-6-[5-methyl-1-[1-(oxetan-3-yl)-4-piperidyl] triazol-4-yl]pyrazolo [1,5-a]pyridine-3-carbonitrile, Isomer 1 | 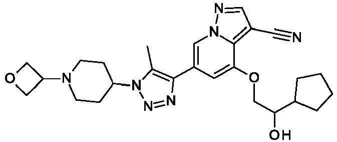 | 492 [M+H]⁺ | 1.08 L |
| 84⁸ | 4-(2-Cyclopentyl-2-hydroxy-ethoxy)-6-[5-methyl-1-[1-(oxetan-3-yl)-4-piperidyl] triazol-4-yl]pyrazolo [1,5-a]pyridine-3-carbonitrile, Isomer 2 | 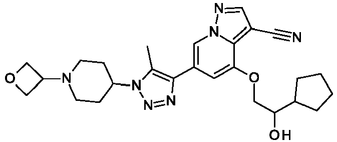 | 492 [M+H]⁺ | 1.68 L |
| 85⁹ | 2-[3-Chloro-6-[5-methyl-1-[1-(oxetan-3-yl)azetidin-3-yl]triazol-4-yl]pyrazolo[1,5-a]pyridin-4-yl]oxy-1-(5-fluoro-2-pyridyl) ethanol, Isomer 1 | 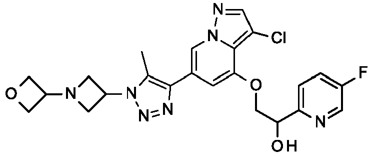 | (³⁵Cl/³⁷Cl) 500/502 [M+H]+ | 1.25 A |
| 86⁹ | 2-[3-Chloro-6-[5-methyl-1-[1-(oxetan-3-yl)azetidin-3-yl]triazol-4-yl]pyrazolo[1,5-a]pyridin-4-yl]oxy-1-(5-fluoro-2-pyridyl) ethanol, Isomer 2 | 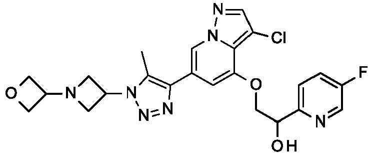 | (³⁵Cl/³⁷Cl) 500/502 [M+H]⁺ | 1.54 A |
| 87¹⁰ | 1-(3-Chloro-5-fluoro-2-pyridyl)-2-[3-fluoro-6-[5-methyl-1-[1-(oxetan-3-yl)-4-piperidyl]triazol-4-yl]pyrazolo[1,5-a]pyridin-4-yl]oxy-ethanol, Isomer 2 | 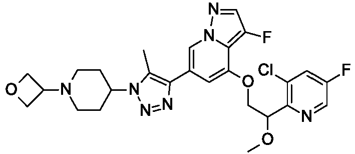 | 560 [M+H]⁺ | 2.8 S |

| | | | | |
|---|---|---|---|---|
| ¹ Purified by silica gel chromatography eluting with MeOH in DCM. ² SFC chromatography eluting with 60% 2-propanol (0.1% DEA) in 40% CO₂. ³ SFC chromatography eluting with 60% MeOH (0.1% DEA) in 40% CO₂. ⁴ Column: Chiralpak IE-3; 4.6 x 50 cm column, 3 µm; eluting with 50% EtOH in MTBE (0.1% DEA). ⁵ Column: Chiral ART Cellulose-SB, 2 x 25 cm column, 5 µm; eluting with 10% EtOH in MTBE (10mM NH₃-MeOH). ⁶ Column: Chiral Art AmylC, 30 x 250 mm, 5 µm; eluting with 45% EtOH (0.5% DMEA) in CO₂. ⁷ Column: Amylose-C, 30 x 250 mm column, 5 µm; eluting with 45% EtOH (0.5% DMEA) in CO₂. ⁸ Column: Chiralpak AD, 30 x 250 mm column, 5 µm; eluting with 55% IPA (0.5% DMEA) in CO₂. ⁹ Column: Chiral Art AmylC, 20 x 250 mm column, 5 µm; eluting with 60% IPA (0.5% DMEA) in CO₂. ¹⁰ Column: Chiral Art Amylose C, 30 x 250 mm, 5 µm; eluting with 40% IPA(0.5% DMEA) in CO₂. | | | | |

### Example 88

### 1-[3-Chloro-6-[5-methyl-1-[1-(oxetan-3-yl)-4-piperidyl]triazol-4-yl]pyrazolo[1,5-a]pyridin-4-yl]oxy-2-(3,5-difluoro-2-pyridyl)propan-2-ol, Isomer 1

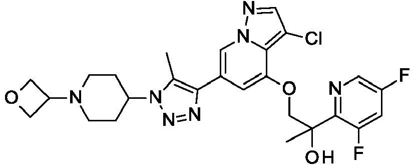

To an ice-cooled soln of 2-[3-chloro-6-[5-methyl-1-[1-(oxetan-3-yl)-4-piperidyl]triazol-4-yl]pyrazolo[1,5-a]pyridin-4-yl]oxy-1-(3,5-difluoro-2-pyridyl)ethanone (190 mg, 0.19 mmol, 55 mass%) in DCM (4 ml) under N₂, was added dropwise a soln of MeMgBr (0.26 ml, 0.78 mmol, 3 M) over 5 minutes. The resulting mixture was stirred for 3 h then diluted with DCM and slowly quenched with MeOH (2 mL). NaBH₄ (5 mg, 0.132161 mmol) was added, and the mixture stirred for 20 min. The reaction was purified by silica gel chromatography eluting with 0% to 50% (10% MeOH in DCM) in DCM. Material crashed out inside the column, 50% MeOH in DCM was used to dissolve and elute the title compound along with impurities. The residue was further purified by reversed phase chromatography, column: XBridge C18, 19 x150mm, 5 µm; eluting with 45% to 75% ACN in H₂O (10mM NH₄HCO₃, pH 9).

The isolated material after reversed phase chromatography was subjected to the following chiral chromatography conditions: Chiral Art Amylose C, 30 x 250 mm, 5 µm; eluting with 40% IPA (0.5% DMEA) in CO₂ to afford the title compound, Isomer 1 (7.5 mg, 7%), t_{R} is 2.36, ee > 98% ee; MS ES+ m/z 560/562 [M+H]⁺.

### Example 89

### 1-[3-Chloro-6-[5-methyl-1-[1-(oxetan-3-yl)-4-piperidyl]triazol-4-yl]pyrazolo[1,5-a]pyridin-4-yl]oxy-2-(5-fluoro-2-pyridyl)butan-2-ol, Isomer 2

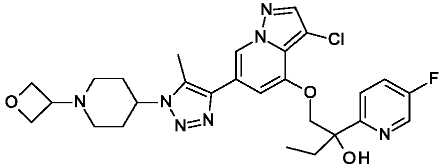

EtMgBr (0.25 ml, 0.75 mmol, 3 M in Et2O) was treated with ZnCl₂ (0.09 ml, 0.2 mmol,1.9M in MeTHF) at 0°C under N₂. After stirring at 0 °C for 1 h, the reaction was treated dropwise with a soln of 2-[3-chloro-6-[5-methyl-1-[1-(oxetan-3-yl)-4-piperidyl]triazol-4-yl]pyrazolo[1,5-a]pyridin-4-yl]oxy-1-(5-fluoro-2-pyridyl)ethanone (304 mg, 0.58 mmol) in DCM (1 ml) and allowed to stir at 0 °C for 2 h. The reaction was diluted with DCM and slowly quenched with aq NH₄Cl. The reaction was passed through a phase separator and washed with DCM. The eluent was concentrated, and the residue dissolved in MeOH (5 ml), treated with NaBH₄ (15 mg, 0.40 mmol), and stirred at RT for 1 h. The reaction was quenched with H₂O, the MeOH was removed in vacuo, extracted with DCM, washed with brine, dried over MgSO₄, filtered, and concentrated. The residue was purified by silica gel chromatography eluting with a gradient of 0% to 5% MeOH in DCM to afford a colorless oil (125.6 mg, 35%). The colorless oil was subjected to the following chiral chromatography conditions: Column: Cellulose-1, 30 x 250 mm column, 5 µm; eluting with 35% IPA (0.5% DMEA) in CO₂ to afford the title compound (18 mg, 6%), t_{R} is 2.64 with 98% ee. MS ES+ m/z (³⁵Cl/³⁷Cl) 556/558 [M+H]⁺. The retention time was obtained using analytical method M.

The following compounds were prepared in a manner essentially analogous to the method of Example 89 using the appropriate reagents, adjusting reaction time to determine completion of the reaction, and adjusting the purification system as appropriate.

If the retention time was obtained from an analytical column the method will be listed in the final column. Refer to Table A for specific analytical conditions for each method.

**Table 60**

| Ex # | Chemical Name | Structure | MS ES+ m/z | t_{R} (min) and method |
|---|---|---|---|---|
| 90^{1,2} | 2-[3-Chloro-6-[5-methyl-1-[1-(oxetan-3-yl)-4-piperidyl]triazol-4-yl]pyrazolo[1,5-a] pyidine-4-yl]oxy-1-cyclopropyl-1-(5-fluoro-2-pyridyl)ethanol, Isomer 1 | 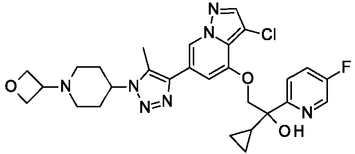 | (³⁵Cl/³⁷Cl) 568/570 [M+H]⁺ | 2.29 N |
| 91^{1,2} | 2-[3-Chloro-6-[5-methyl-1-[1-(oxetan-3-yl)-4-piperidyl]triazol-4-yl]pyrazolo[1,5-a] pyridine-4-yl]oxy-1-cyclopropyl-1-(5-fluoro-2-pyridyl)ethanol, Isomer 2 | 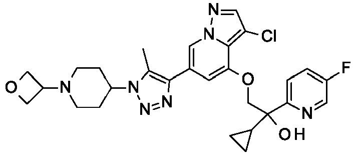 | (³⁵Cl/³⁷Cl) 568/570 [M+H]⁺ | 2.40 N |

| | | | | |
|---|---|---|---|---|
| ¹ Purified by silica gel chromatography eluting with 0% to 40% (9:1 DCM in MeOH) in DCM. ² Column: Chiralpak IH, 20 x 250 mm column (5 µm) eluting with 35% MeOH (0.5% DMEA) in CO₂. | | | | |

### Example 92

### 4-[1-(6-Cyclopropylpyrazin-2-yl)ethoxy]-6-[5-methyl-1-[1-(oxetan-3-yl)-4-piperidyl]triazol-4-yl]pyrazolo[1,5-a]pyridine-3-carbonitrile, Isomer 2

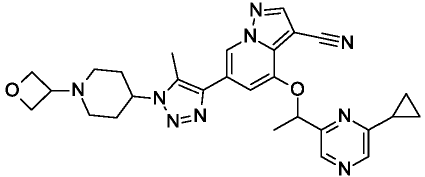

4-[1-(6-Bromopyrazin-2-yl)ethoxy]-6-[5-methyl-1-[1-(oxetan-3-yl)-4-piperidyl]triazol-4-yl]pyrazolo[1,5-a]pyridine-3-carbonitrile (182.2 mg, 0.32 mmol), cyclopropylboronic acid (48 mg, 0.56 mmol) were dissolved in 1,4-dioxane (3 ml) and CsF (197 mg, 1.30 mmol) was added. The mixture was degassed then PdCl₂(DtBPF) (21 mg, 0.03 mmol) was added, and the mixture was stirred at 90 °C overnight. Upon cooling to RT, EA was added to the reaction. The mixture was washed with sat. aq NaHCO₃ and brine. The organic phase was dried over MgSO₄, filtered, and concentrated. The residue was purified by silica gel chromatography eluting with 20% to 100% EA in cHex to afford a pale-yellow solid (120 mg, 67%). The yellow solid was subjected to chiral chromatography using the following conditions: Column: Chiralpak IH, 20 x 250 mm, 5 µm; eluting with 25% MeOH (0.5% DMEA) in CO₂ to afford the title compound (47 mg, 28%), t_{R} is 2.32 min with 97% ee. MS ES+ m/z 526 [M+H]⁺. The retention time was obtained using analytical method N.

### Example 93

### 6-[5-Methyl-1-[7-(oxetan-3-yl)-7-azaspiro[3.5]nonan-2-yl]pyrazol-4-yl]-4-[(1R)-1-(2-pyridyl)ethoxy]pyrazolo[1,5-a]pyridine-3-carbonitrile

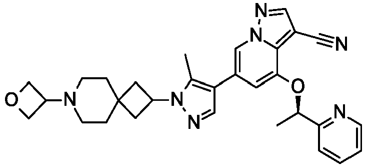

and

### Example 94

### 6-[3-Methyl-1-[7-(oxetan-3-yl)-7-azaspiro[3.5]nonan-2-yl]pyrazol-4-yl]-4-[(1R)-1-(2-pyridyl)ethoxy]pyrazolo[1,5-a]pyridine-3-carbonitrile

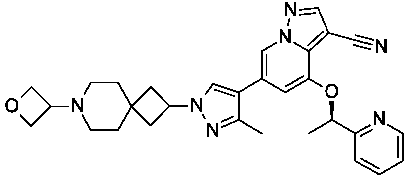

A soln of 6-[1-(7-azaspiro[3.5]nonan-2-yl)-5-methyl-pyrazol-4-yl]-4-[(1R)-1-(2-pyridyl)ethoxy]pyrazolo[1,5-a]pyridine-3-carbonitrile, 6-[1-(7-azaspiro[3.5]nonan-2-yl)-3-methyl-pyrazol-4-yl]-4-[(1R)-1-(2-pyridyl)ethoxy]pyrazolo[1,5-a]pyridine-3-carbonitrile (101 mg, 0.19 mmol), and oxetan-3-one (20 mg, 0.28 mmol) in MeOH (5 ml) was treated with AcOH (21 µl, 0.37 mmol) and NaBH₃CN (35 mg, 0.56 mmol) and stirred at RT for 2 h and then at 50°C for 18 h. The reaction was allowed to cool then concentrated. The residue was suspended in saturated aq NaHCO₃ and extracted with DCM (2X). The combined organic layers were dried over MgSO₄, filtered, and concentrated. The residue was purified by silica gel chromatography eluting with a gradient of 0% to 10% acetone in DCM to obtain a pale-yellow solid containing the two compounds. The compounds were separated using the following conditions: Column, Chiralpak IH, 20 x 250 mm, 5 µm; column eluting with 20% MeOH in CO₂ to obtain 6-[3-methyl-1-[7-(oxetan-3-yl)-7-azaspiro[3.5]nonan-2-yl]pyrazol-4-yl]-4-[(1R)-1-(2-pyridyl)ethoxy]pyrazolo[1,5-a]pyridine-3-carbonitrile (27 mg; 27%). MS ES+ m/z 524 [M+H]⁺, t_{R} = 1.80 min (analytical chiral chromatography method N, Table A), ¹H NMR (400 MHz, DMSO-d₆): 8.60-8.58 (m, 3H), 7.85 (td, J= 7.8, 1.8 Hz, 1H), 7.69-7.62 (m, 2H), 7.34 (ddd, J= 7.5, 4.9, 1.1 Hz, 1H), 6.92 (s, 1H), 5.87 (q, J= 6.4 Hz, 1H), 4.89 (quintet, J= 8.2 Hz, 1H), 4.51 (t, J= 6.5 Hz, 2H), 4.41 (t, J= 6.1 Hz, 2H), 2.29-2.18 (m, 11H), 1.71 (d, J= 6.4 Hz, 5H), 1.60 (t, J= 5.4 Hz, 2H) and 6-[5-methyl-1-[7-(oxetan-3-yl)-7-azaspiro[3.5]nonan-2-yl]pyrazol-4-yl]-4-[(1R)-1-(2-pyridyl)ethoxy]pyrazolo[1,5-a]pyridine-3-carbonitrile (14 mg, 14%) MS ES+ m/z 524 [M+H]⁺, t_{R} = 1.69 min (analytical chiral chromatography method N, Table A), ¹H NMR (400 MHz, DMSO-d₆): 8.60-8.52 (m, 3H), 8.13 (s, 1H), 7.85 (td, J= 7.7, 1.7 Hz, 1H), 7.65 (d, J= 7.8 Hz, 1H), 7.35 (ddd, J= 7.5, 4.8, 1.2 Hz, 1H), 7.03 (s, 1H), 5.89 (q, J= 6.4 Hz, 1H), 4.78 (quintet, J= 8.4 Hz, 1H), 4.51 (t, J= 6.5 Hz, 2H), 4.41 (t, J= 6.1 Hz, 2H), 2.33-2.18 (m, 11H), 1.72-1.61 (m, 7H) as thick colorless oils.

The following compounds were prepared in a manner essentially analogous to the method of Example 94 using the appropriate reagents, adjusting reaction time to determine completion of the reaction, and adjusting the purification system as appropriate.

**Table 61**

| Ex # | Chemical Name | Structure | MS ES+ m/z | t_{R} min |
|---|---|---|---|---|
| 95^{1,a} | 6-[5-Methyl-1-[1-(oxetan-3-yl)azepan-4-yl]pyrazol-4-yl]-4-[(1R)-1-(2-pyridyl)ethoxy]pyra zolo[1,5-a]pyridine-3-carbonitrile | 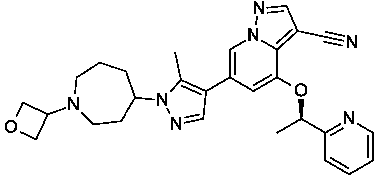 | 498 [M+H]⁺ | 2.71 |
| 96^{1, c} | 6-[5-Methyl-1-[1-(oxetan-3-yl)azepan-4-yl]pyrazol-4-yl]-4-[(1R)-1-(2-pyridyl)ethoxy]pyra zolo[1,5-a]pyridine-3-carbonitrile, Isomer 1 | 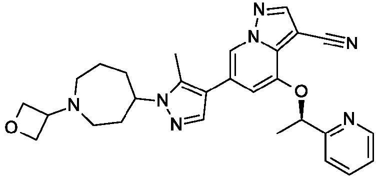 | 498 [M+H]⁺ | 2.65 |
| 97^{1,b} | 6-[3-Methyl-1-[1-(oxetan-3-yl)azepan-4-yl]pyrazol-4-yl]-4-[(1R)-1-(2-pyridyl)ethoxy]pyra zolo[1,5-a]pyridine-3-carbonitrile | 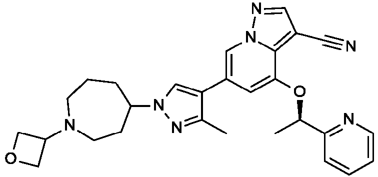 | 498 [M+H]⁺ | 2.49 |

| | | | | |
|---|---|---|---|---|
| ¹ Column: Chiralpak AD, 20 x 250 mm column, 5 µm; eluting with 35% EtOH (0.5% DMEA) in CO₂. ^{a 1}H NMR (400 MHz, DMSO-d₆): 8.60-8.58 (m, 2H), 8.50 (d, J= 1.0 Hz, 1H), 8.14 (s, 1H), 7.85 (td, J= 7.7, 1.9 Hz, 1H), 7.66 (d, J= 7.8 Hz, 1H), 7.35 (ddd, J= 7.6, 4.9, 1.2 Hz, 1H), 7.07 (s, 1H), 5.91 (q, J= 6.4 Hz, 1H), 4.54 (td, J= 6.5, 2.4 Hz, 2H), 4.42-4.35 (m, 3H), 3.66 (quintet, J= 6.4 Hz, 1H), 2.45-2.41 (m, 4H), 2.19 (s, 7H), 1.88-1.79 (m, 1H), 1.71 (d, J= 6.4 Hz, 4H). ^{b 1}H NMR (400 MHz, DMSO-d₆): 8.60-8.58 (m, 2H), 8.50 (d, J= 1.0 Hz, 1H), 8.14 (s, 1H), 7.85 (td, J= 7.7, 1.9 Hz, 1H), 7.66 (d, J= 7.8 Hz, 1H), 7.35 (ddd, J= 7.6, 4.9, 1.2 Hz, 1H), 7.07 (s, 1H), 5.91 (q, J= 6.4 Hz, 1H), 4.54 (td, J= 6.5, 2.4 Hz, 2H), 4.42-4.35 (m, 3H), 3.66 (quintet, J= 6.4 Hz, 1H), 2.45-2.41 (m, 4H), 2.19 (s, 7H), 1.88-1.79 (m, 1H), 1.71 (d, J= 6.4 Hz, 4H). ^{c 1}H NMR (400 MHz, DMSO): 8.68 - 8.56 (m, 2H), 8.50 (d, J = 1.0 Hz, 1H), 8.14 (s, 1H), 7.85 (td, J = 7.7, 1.8 Hz, 1H), 7.66 (dt, J = 7.9, 1.1 Hz, 1H), 7.37 - 7-34 (dd, J = 7.5, 1.2 Hz, 1H), 7.36 - 7.32 (dd, J = 7.5, 1.2 Hz, 1H), 7.07 (d, J = 1.1 Hz, 1H), 5.91 (q, J = 6.4 Hz, 1H), 4.55 (td, J = 6.5, 2.5 Hz, 2H), 4.44 - 4.32 (m, 2H), 3.66 (p, J = 6.4 Hz, 1H), 2.50 - 2.38 (m, 4H), 2.19 (s, 3H), 2.15 - 1.95 (m, 4H), 1.88 - 1.77 (m, 1H), 1.71 (d, J = 6.4 Hz, 3H), 1.69 - 1.58 (m, 2H). | | | | |

### Example 98

### 1-(5-Fluoro-2-pyridyl)-2-[6-[5-methyl-1-[1-(oxetan-3-yl)-4-piperidyl]triazol-4-yl]-3-(1-methylpyrazol-4-yl)pyrazolo[1,5-a]pyridin-4-yl]oxy-ethanol, Isomer 1

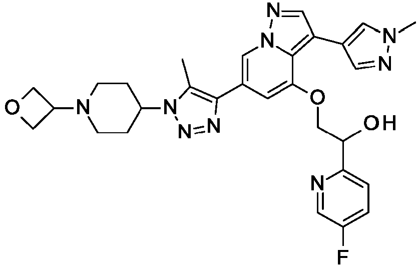

(1S)-2-[3-Bromo-6-[5-methyl-1-[1-(oxetan-3-yl)-4-piperidyl]triazol-4-yl]pyrazolo[1,5-a]pyridin-4-yl]oxy-1-(5-fluoro-2-pyridyl)ethanol (0.066 g, 0.10 mmol),1-methyl-4-(4,4,5,5-tetramethyl-1,3,2-dioxaborolan-2-yl)-1H-pyrazole (0.115 g, 0.54 mmol), and XPhos Pd G4 (0.02 g, 0.02 mmol) in toluene (1.3 ml) was treated at RT with aq K₂CO₃ (1M, 0.55 mL, 0.55 mmol), and then heated to 90 °C. After 5 h additional 1-methyl-4-(4,4,5,5-tetramethyl-1,3,2-dioxaborolan-2-yl)-1h-pyrazole (0.35 g, 1.62 mmol), XPhos Pd G4 (0.02 g, 0.02 mmol), and H₂O (0.65 ml) were added. After 7 h the reaction cooled to RT and EA (10 ml) was added. The phases were separated, and the organic phase was dried, filtered, and concentrated. The residue was purified by reversed phase chromatography eluting with 20% to 60% ACN in aq. NH₄CO₃ (pH =9) to afford the title compound as a white solid (0.017 mg, 28%). MS ES+ m/z 574 [M+H]⁺.

### Example 99

### 6-[1-[(3S,4R)-3-Fluoro-1-(oxetan-3-yl)-4-piperidyl]-5-methyl-triazol-4-yl]-4-[2-(5-fluoro-2-pyridyl)-2-methoxy-ethoxy]pyrazolo[1,5-a]pyridine-3-carbonitrile

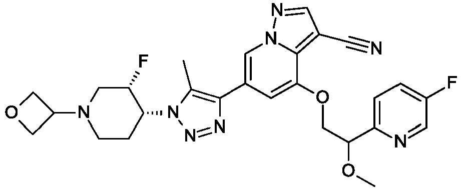

To a mixture of 6-[1-[(3S,4R)-3-Fluoro-4-piperidyl]-5-methyl-triazol-4-yl]-4-[2-(5-fluoro-2-pyri dyl)-2-methoxy-ethoxy]pyrazolo[1,5-a]pyridine-3-carbonitrile; hydrochloride (2 g, crude) in MeOH (20 mL) was added 3-oxetanone (1.49 g, 20.63) at RT under N₂. The resulting mixture was stirred for 60 min at 50 °C. Next, NaBH₃CN (432 mg, 6.88 mmol) was added at RT. The mixture was stirred for 2 hr at 50 °C. Upon cooling to RT, the reaction was quenched with H₂O (30 mL) and the mixture was basified to pH 9 with saturated aq Na₂CO₃. The mixture was extracted with EA (2 x 80 mL). The combined organic layers were washed with brine (2 x 20 mL), dried over Na₂SO₄, filtered and concentrated in vacuo. The residue was purified by C18 reversed flash chromatography, eluted with 30% to 50% ACN in H₂O (0.1% NH₃.H₂O) to afford the title compound (1.4 g, 73.97%) as a white solid. MS ES+ m/z 551 [M+H]⁺.

### Biological Assays

The following assays demonstrate that compounds provided herein are FGFR2 inhibitors. The following assays demonstrate that certain compounds provided herein selectively target FGFR2 over FGFR1.

### FGFR2 and FGFR1 Enzyme Assay

FGFR1 and FGFR2 proteins were purchased from ThermoFisher Scientific (Cat. No. PR4660A and PR5332A, respectively). Enzyme activity was monitored using the KinEASETM-TK Assay Kit (CisBio, Cat. No. 62TK0PEC) according to the manufacturer's instructions. All assays were performed at the respective KmATP for each kinase in KinEASETM Kinase Buffer. Reactions were performed in a white, small volume polystyrene 384 well plate.

An incubation was conducted with each of the proteins, 50 nM TK-Biotin Substrate (CisBio), 6.25 nM Streptavidin-XL665 (CisBio), 0.25 x Anti-Phosphorylate TK-Biotin-Cryptate (CisBio). Final enzyme concentrations were 0.08 nM for FGFR1, and 0.04 nM for FGFR2, in 10 µL reactions. Titration of compounds was performed using 1:3 serial dilutions in 100% dimethyl sulfoxide (DMSO) starting at 2.5 µM. Prior to the initiation of the reaction by adenosine triphosphate (ATP), each protein and compounds were pre-incubated for 15 minutes at room temperature. Reactions proceeded for 30 min at 30°C. Plates were quenched by the addition of the Anti-TK cryptate antibody/Streptavidin-XL665 mixture. After 1 hour in the stopping solution, the plates were read on the Envision plate reader ((PerkinElmer) (Ex. Filter. 320 nm and Em1 665 nm/ Em2 615 nm)).

Ratios were converted to a percent of control (POC) using a ratiometric emission factor. One hundred POC was determined using no test compound (DMSO alone), and 0 POC was determined in the presence of 2.5 µM of an appropriate control inhibitor. A 4-parameter logistic curve was fit to the POC values as a function of the concentration of compound, and the IC50 value was the point where the best fit curve crossed 50 POC.

### FGFR2 and FGFR1 Cell-Based Assay

HEK293 cells transfected with doxycycline (dox)-inducible human wild type FGFR1, and human wild type FGFR2, were plated at 10,000 cells/well in poly-D-lysine coated 384 well plates (Becton Dickinson, Cat. No. 356663) in Dulbecco's Modified Eagle Medium (Sigma, Cat. No. D5796) containing 10% FBS (Sigma, Cat. No. F2442), 1% Penicillin-Streptomycin (Gibco, Cat. No. 15140) and 1 µg/ml doxycycline, and allowed to attach for 24 hours at 37°C, 5% CO₂. NCI-H716 cells (ATCC, Cat. No. CCL-251) were plated at 5,000 cells/well in poly-D-lysine coated 384 well plates (Becton Dickinson, Cat. No. 356663) in RPMI 1640 medium (Gibco, Cat. No. A10491) containing 10% FBS and 1% Penicillin-Streptomycin, and allowed to attach for 24 hours at 37°C, 5% CO₂.

Cells were treated with compounds using 1:3 serial dilutions with a maximum final concentration of 3 µM. Compounds were incubated on cells for 1 hour at 37°C, 5% CO₂. Cells were fixed with formaldehyde for 20 minutes at room temperature and permeabilized with 0.3% Triton-X 100 for 15 minutes at room temperature. Then cells were blocked with 1% bovine serum albumin in 0.05% Tween for 1 hour at room temperature, and they were incubated overnight at 4 °C with anti-phospho-FGFR primary antibody (Cell Signaling, Cat. No.52928 for HEK293 FGFR1; Millipore, Cat. No.06-1433 for the rest of the cell lines), in blocking solution. The next day, cells were incubated with goat anti-rabbit IgG Alexa 488 labeled secondary antibody (Invitrogen, Cat. No. A11008) for 1 hour at room temperature, and a solution containing 0.4 ug/ml DAPI (Sigma, Cat. No. D9564) and 50 µg/mL Ribonuclease A (Sigma, Cat. No. R-6513) was added to stain nuclei. Fluorescence plates were scanned with the Acumen Explorer laser-scanning fluorescence microplate cytometer (SPT Labtech) to quantify the number of cells and the phosphorylation of FGFR to calculate a percentage of phospho-FGFR positive cells.

One hundred POC was determined using no test compound (DMSO alone), and 0 POC was determined in the presence of 3 µM of an appropriate control inhibitor. A 4-parameter logistic curve was fit to the POC values as a function of the concentration of compound, and the IC₅₀ value was the point where the best fit curve crossed 50 POC.

### Biological Assay Results

In the above enzyme assays the compounds of Examples 1-33, 35-60, 62-99 all exhibited IC₅₀ values of less than 200 nM for FGFR2.

In the above enzyme assays the compounds of Examples 1, 2, 4, 7, 9-11, 13-16, 18-24, 26-28, 31-35, 38, 40, 42-59, 61-67, 69,71-73, 75-77, 79-85, 87-93, 95-97 and 99 all exhibited IC₅₀ values of less than 100 nM for FGFR2 and are at least 3 fold more selective for FGFR2 than for FGFR1.

In the above enzyme assays the compounds of Examples 1, 2, 7, 9-11, 15-16, 18, 20-22, 27-28, 31, 33-34, 40, 42, 44, 47-53, 55-57, 61-67, 69, 71, 73, 79, 81-82, 87-89, 91, 92, 96 and 99 all exhibited IC₅₀ values of less than 50 nM for FGFR2 and are at least 8 fold more selective for FGFR2 than for FGFR1.

In the above cell-based assays the compounds of Examples 1-99 all exhibited IC₅₀ values of less than 60 nM for FGFR2.

In the above cell-based assays the compounds of Examples 1-14, 16-29, 31-40, 42, 44, 45, 47-71, 73-75, 77, 79, 81-82, 84 and 86-99 all exhibited IC₅₀ values of less than 30 nM for FGFR2 and are at least 5 fold more selective for FGFR2 than for FGFR1.

In the above cell-based assays the compounds of Examples 3-5, 7-9, 14, 16, 18-23, 28, 33, 42, 44, 51-53, 56, 60-63, 66-67, 69, 74-75, 77, 81, 88-89, 91 ,95-97 and 99 all exhibited IC₅₀ values of less than 10 nM for FGFR2 and are at least 10 fold more selective for FGFR2 than for FGFR1.

## Claims

1. A compound of the formula: 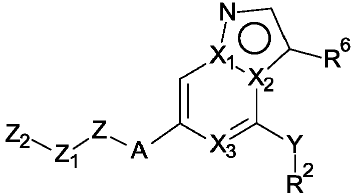 wherein
Z₂ is 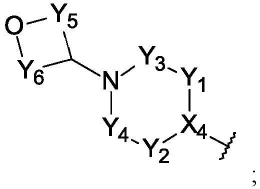
A is pyrazole, triazole, thiadiazole or oxadiazole, substituted with R¹ and R^{1A};
R¹ is hydrogen or C₁-C₃ alkyl;
R^{1A} is hydrogen, halo, CN or C₁-C₃ alkyl optionally substituted with one or more substituents independently selected from halo, OH and OCH₃;
X₁ and X₂ are independently selected from N and C, wherein when one of X₁ or X₂ is N the other is C;
X₃ is N or CH;
X₄ is N or C-R⁹;
Y is NH, O, S or a bond;
Y₁ is a bond, CHR⁷, CH₂-CHR⁷, CHR⁷-CH₂, CF₂, CH₂-CF₂ or CF₂-CH₂;
Y₂ is a bond, CHR³, CH₂-CHR³, CHR³-CH₂, CF₂, CH₂-CF₂ or CF₂-CH₂;
Y₃ is CR⁴R⁵ or CF₂;
Y₄ is CR³R⁴ or CF₂;
Y₅ is CR¹³R¹⁴, CR¹³R¹⁴-CH₂ or CH₂CR¹³R¹⁴;
Y₆ is CR¹³R¹⁴, CR¹³R¹⁴-CH₂ or CH₂CR¹³R¹⁴;
Z is a bond, CHR^{9A}, CR⁴R^{4A}, CR⁴R^{4A}-CH₂, CH₂-CR⁴R^{4A}, cyclobutyl, cyclopentyl, cyclohexyl, bicyclo(1.1.1)pentane, bicyclo(2.1.1)hexane, azetidine, pyrrolidine or piperidine;
Z₁ is a bond when Z is a bond, C_{R}⁴_{R}^{4A,} CR⁴R^{4A}-CH₂, CH₂-CR⁴R^{4A}, cyclobutyl, cyclopentyl, cyclohexyl, bicyclo(1.1.1)pentane, bicyclo(2.1.1)hexane, azetidine, pyrrolidine or piperidine, or Zi is CH₂ or CH₂-CH₂ when Z is CHR^{9A};
Z₃ is a bond, C(O), SO₂ or -NR⁴C(O);
Z₄ is a bond, C(O), SO₂ or -NR⁴C(O);
R² is C₁-C₅ alkyl or R⁸, wherein C₁-C₅ alkyl is optionally substituted with one or more substituents independently selected from halo, OH, CN, oxo, -OC₁-C₄ alkyl, -OC₃-C₅ cycloalkyl, -Z₃-R¹¹ and R¹⁰, wherein C₁-C₄ alky and C₃-C₅ cycloalkyl are optionally substituted with one or more substituents independently selected from halo, OH, OCH₃, methylamine, N,N-dimethylamine and CN;
R³ is hydrogen, F, OH, OCH₃, C₁-C₃ alkyl, cyclopropyl, or one R³ is fused with R⁵ or R⁷ to form CH₂, CH₂-CH₂ or CH₂OCH₂;
R⁴ is hydrogen or C₁-C₃ alkyl;
R^{4A} is hydrogen, halo, OH or C₁-C₃ alkyl;
R⁵ is hydrogen, F, OH, OCH₃, C₁-C₃ alkyl, cyclopropyl or is fused with one R³ to form CH₂, CH₂-CH₂ or CH₂OCH₂;
R⁶ is hydrogen, halo, C₁-C₅ alkyl, CN, 3-6 membered cycloalkyl, 4-6 membered heterocycloalkyl, 5-6 membered aryl or 5-6 membered heteroaryl, wherein 3-6 membered cycloalkyl, 4-6 membered heterocycloalkyl, 5-6 membered aryl and 5-6 membered heteroaryl are optionally substituted with one or more substituents independently selected from halo, methyl, halomethyl, OH or OCH₃ and wherein C₁-C₅ alkyl is optionally substituted with one or more substituents independently selected from halo, OH and OCH₃;
R⁷ is hydrogen, F, OH, OCH₃, C₁-C₃ alkyl or is fused with one R³ to form CH₂, CH₂-CH₂ or CH₂OCH₂;
R⁸ is 3-6 membered cycloalkyl, 4-6 membered heterocycloalkyl, 5-6 membered aryl or 5-6 membered heteroaryl, optionally fused or substituted with R^{8A};
R^{8A}; is 3-6 membered cycloalkyl, 4-6 membered heterocycloalkyl, 5-6 membered aryl or 5-6 membered heteroaryl;
R⁹ is hydrogen, C₁-C₃ alkyl, or is fused with R^{9A} to form CH₂ or CH₂-CH₂;
R¹⁰ is 3-6 membered cycloalkyl, 4-6 membered heterocycloalkyl, 5-6 membered aryl or 5-6 membered heteroaryl, optionally fused or substituted with R^{8A};
R₁₁ is C₁-C₄ alkyl, NH₂, NHC₁-C₃ alkyl, NHC₃-C₅ cycloalkyl or N(C₁-C₃ alkyl)₂, wherein C₁-C₄ alkyl, C₁-C₃ alkyl and C₃-C₅ cycloalkyl are optionally substituted with one or more substituents independently selected from halo, OH, OCH₃, methylamine, N,N-dimethylamine and CN;
R¹² is C₁-C₄ alkyl, C₃-C₅ cycloalkyl, NH₂, NHC₁-C₃ alkyl, NHC₃-C₅ cycloalkyl or N(C₁-C₃ alkyl)₂, wherein C₁-C₄ alky, C₁-C₃ alkyl and C₃-C₅ cycloalkyl are optionally substituted with one or more substituents independently selected from halo, OH, OCH₃, methylamine, N,N-dimethylamine and CN;
R¹³ is hydrogen, halo or C₁-C₃ alkyl;
R¹⁴ is hydrogen, halo or C₁-C₃ alkyl; and
R⁸, R¹⁰ and R^{8A}; are optionally substituted with one or more substituents independently selected from halo, OH, CN, -OC₁-C₄ alkyl, -OC₃-C₅ cycloalkyl and -Z₄-R¹² wherein C₁-C₄ alky and C₃-C₅ cycloalkyl are optionally substituted with one or more substituents independently selected from halo, OH, OCH₃, methylamine, N,N-dimethylamine and CN;
or a pharmaceutically acceptable salt thereof.

2. The compound according to claim 1 wherein X₁ is N, and X₂ is C, or a pharmaceutically acceptable salt thereof.

3. The compound according to claim 1 or 2, wherein X₃ is CH, or a pharmaceutically acceptable salt thereof.

4. The compound according to any one of claims 1-3, wherein A is pyrazole or triazole, substituted with R¹ and R^{1A}, or a pharmaceutically acceptable salt thereof.

5. The compound according to claim 4, wherein A is triazole, substituted with R¹ and R^{1A}, or a pharmaceutically acceptable salt thereof.

6. The compound according to any one of claims 1-5, wherein R^{1A} is hydrogen, or a pharmaceutically acceptable salt thereof.

7. The compound according to any one of claims 1-6, wherein R¹ is CH₃, or a pharmaceutically acceptable salt thereof.

8. The compound according to any one of claims 1-7, wherein Y is O, or a pharmaceutically acceptable salt thereof.

9. The compound according to any one of claims 1-8, wherein R⁶ is CN, F or Cl, or a pharmaceutically acceptable salt thereof.

10. The compound according to any one of claims 1-9, wherein Z is a bond, cyclobutyl, azetidine or piperidine, or a pharmaceutically acceptable salt thereof.

11. The compound according to claim 10, wherein Z is a bond, or a pharmaceutically acceptable salt thereof.

12. The compound according to any one of claims 1-11, wherein Z₁ is a bond, or a pharmaceutically acceptable salt thereof.

13. The compound according to any one of claims 1-11, wherein X₄ is C-R⁹, wherein R⁹ is hydrogen or CH₃, or a pharmaceutically acceptable salt thereof.

14. The compound according to any one of claims 1-13, wherein Y₁ is a bond, CHR⁷, CH₂-CHR⁷ or CHR⁷-CH₂, wherein R⁷ is selected from hydrogen, F, OH and CH₃, or a pharmaceutically acceptable salt thereof.

15. The compound according to any one of claims 1-14, wherein Y₂ is a bond, CHR³, CH₂-CHR³ or CHR³-CH₂, wherein R³ is selected from hydrogen, F, OH and CH₃, or a pharmaceutically acceptable salt thereof.

16. The compound according to any one of claims 1-15, wherein Y₃ is CR⁴R⁵ or CF₂, wherein R⁴ is hydrogen or CH₃ and R⁵ is hydrogen, F, OH or CH₃, or a pharmaceutically acceptable salt thereof.

17. The compound according to any one of claims 1-16, wherein Y₄ is CR³R⁴ or CF₂ wherein R⁴ is hydrogen or CH₃ and R³ is hydrogen, F, OH or CH₃, or a pharmaceutically acceptable salt thereof.

18. The compound according to any one of claims 1-17, wherein Y₅ and Y₆ are CH₂, or a pharmaceutically acceptable salt thereof.

19. The compound according to any one of claims 1-18, wherein R² is C₁-C₃ alkyl optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, or a pharmaceutically acceptable salt thereof.

20. The compound according to any one of claims 1-19, wherein R² is selected from: 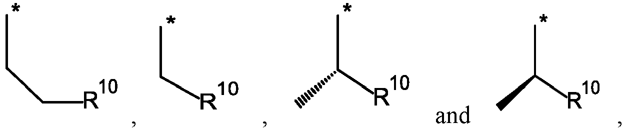 optionally substituted with one, two, three or four substituents independently selected from halo, OH, CN, oxo, -OC₁-C₄ alkyl, -OC₃-C₅ cycloalkyl, -Z₃-R¹¹ and R¹⁰, wherein C₁-C₄ alkyl and C₃-C₅ cycloalkyl are optionally substituted with one or more substituents independently selected from halo, OH, OCH₃, methylamine, N,N-dimethylamine and CN, wherein * indicates the connection point to Y, or a pharmaceutically acceptable salt thereof.

21. The compound according to any one of claims 1-20, wherein R¹⁰ is 4-6 membered heterocycloalkyl or 5-6 membered heteroaryl, optionally substituted or fused with R^{8A}, or a pharmaceutically acceptable salt thereof.

22. The compound according to any one of claims 1-20, wherein R¹⁰ is independently selected from cyclopropane, cyclobutane, cyclopropane, pyrrolidine, thiazole, pyrazole, triazole, phenyl, pyridine, pyrazine and pyridazine, optionally substituted or fused with R^{8A}, or a pharmaceutically acceptable salt thereof.

23. The compound according to any one of claims 1-22, wherein R¹⁰ and R^{8A}; are optionally substituted with one or two substituents independently selected from F, Cl, C₁-C₃ alkyl, CH₂F, CHF₂, CF₃ and -OCH₃, or a pharmaceutically acceptable salt thereof.

24. The compound according to Claim 1, selected from: 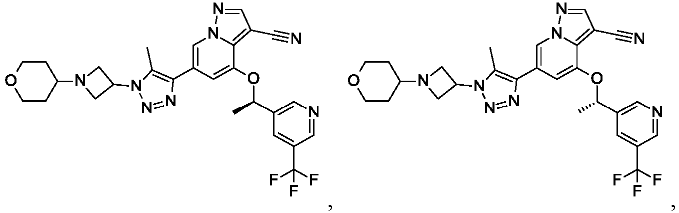 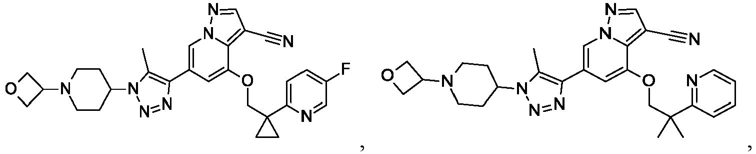 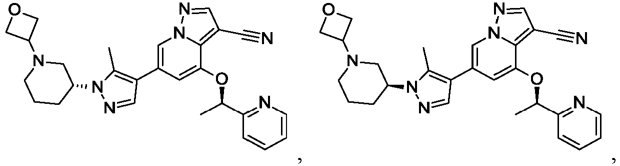 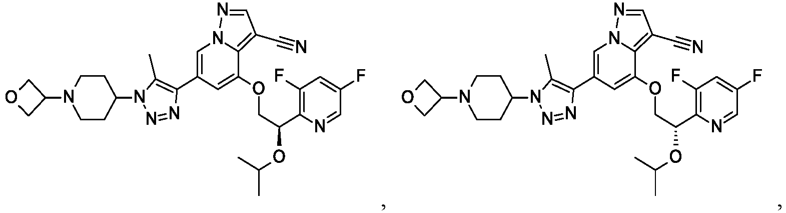 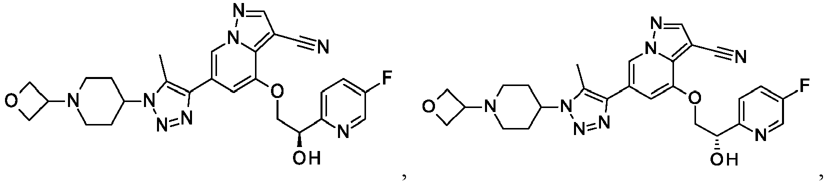 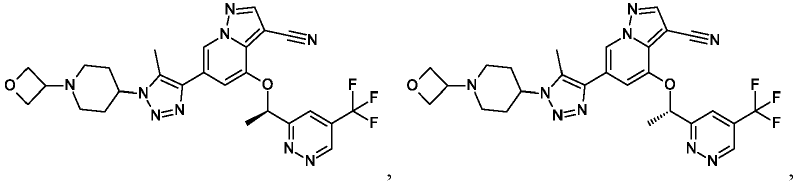 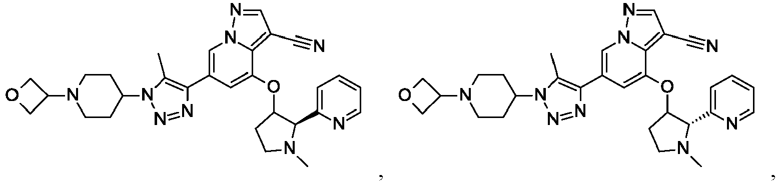 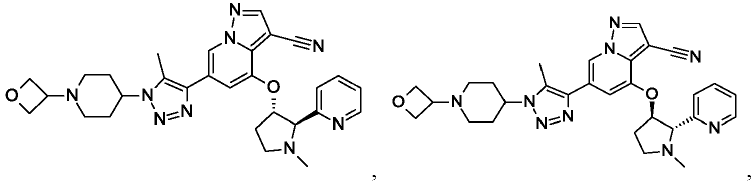 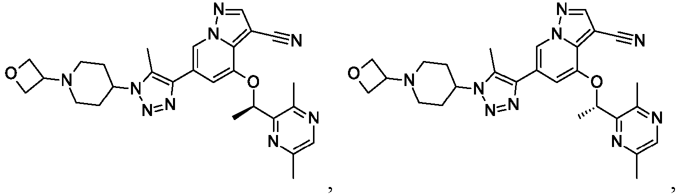 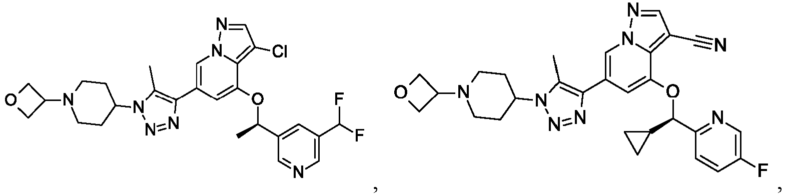 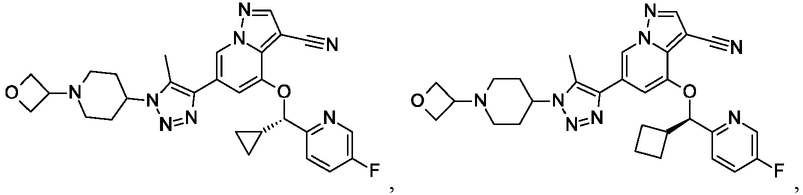 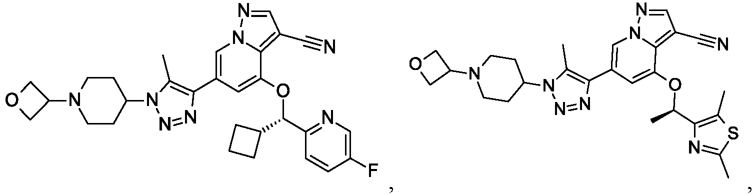 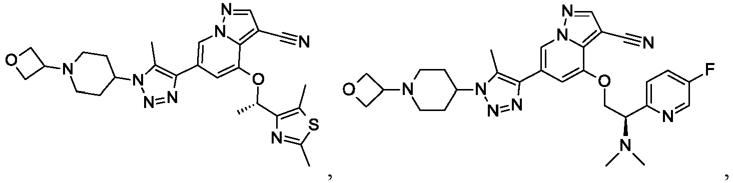 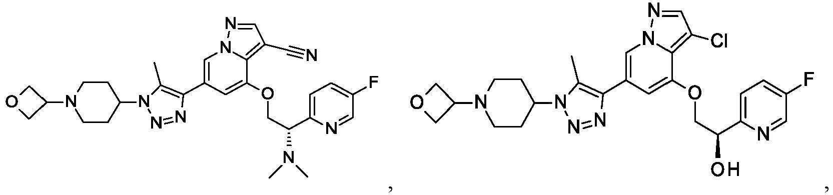 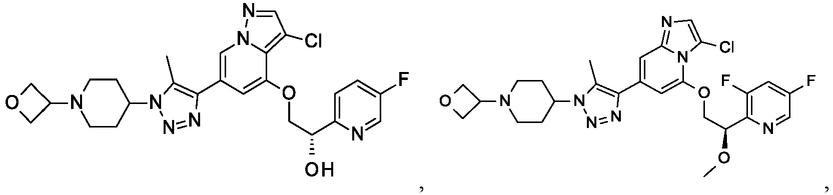 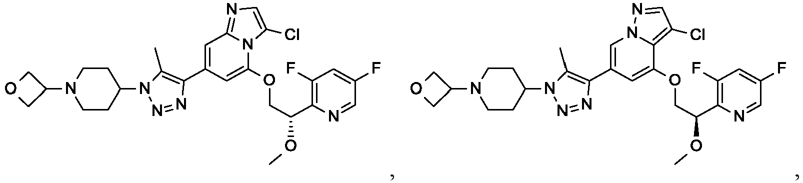 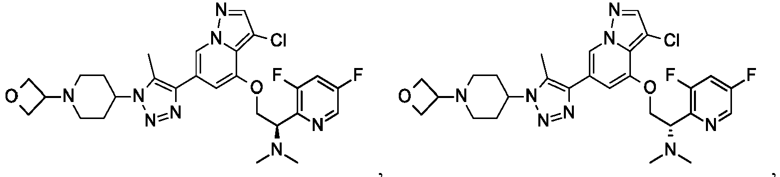 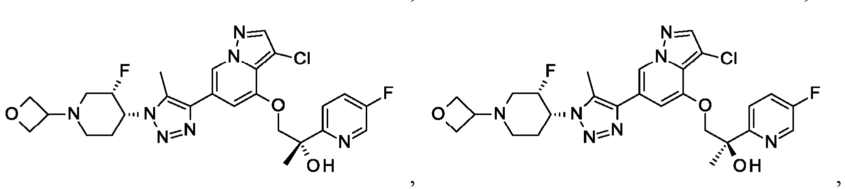 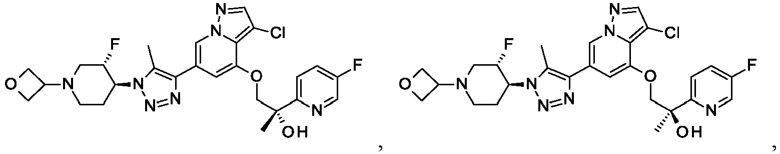 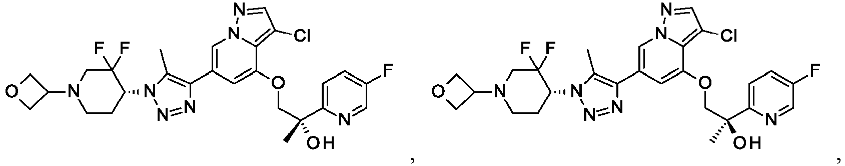 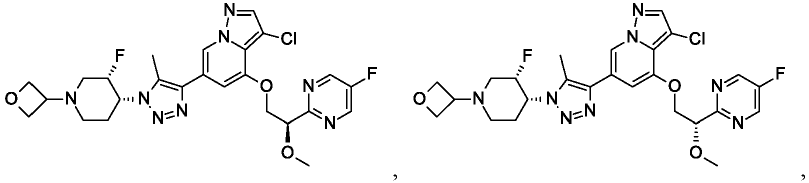 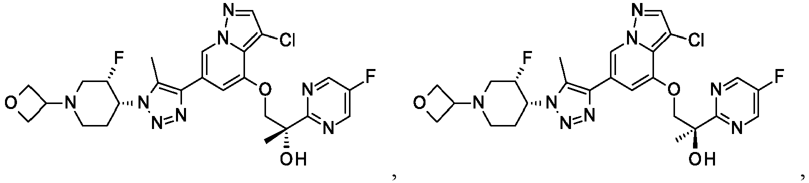 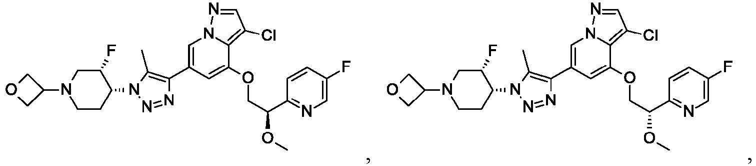 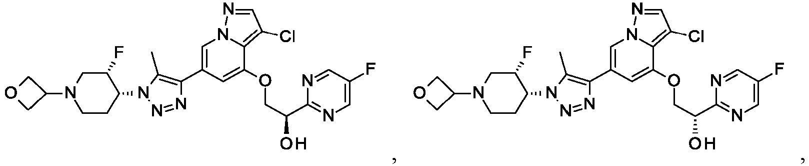 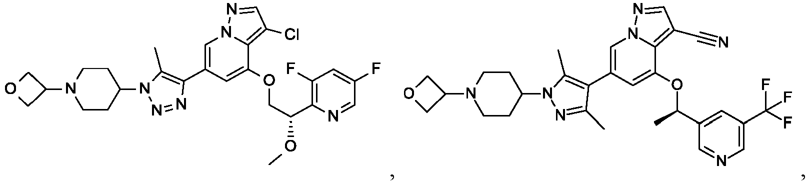 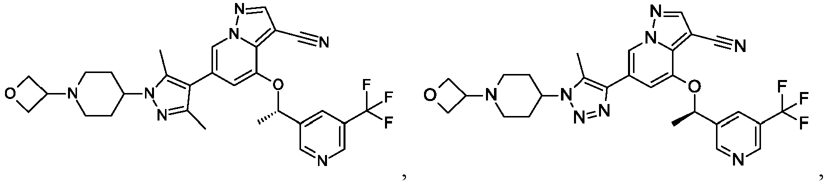 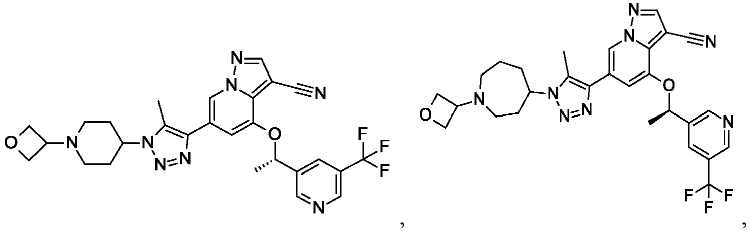 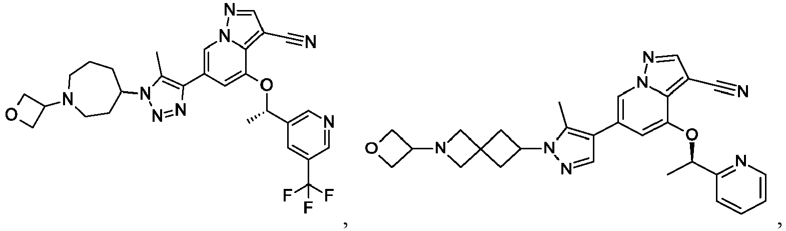 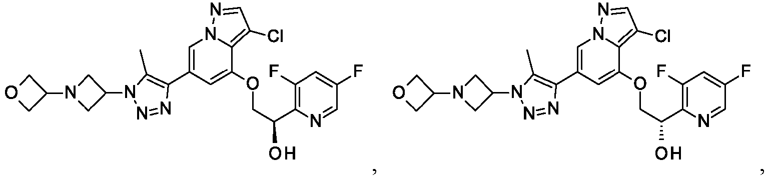 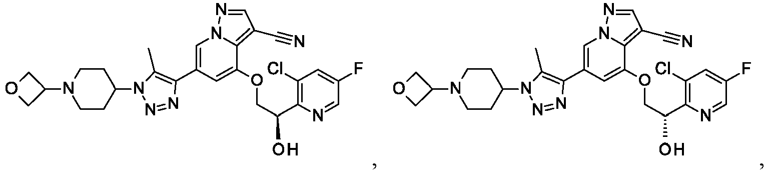 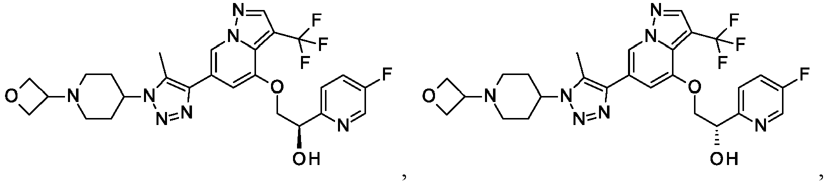 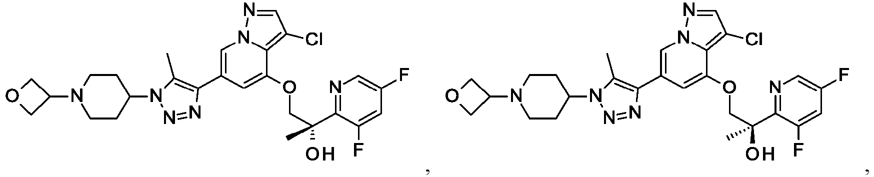 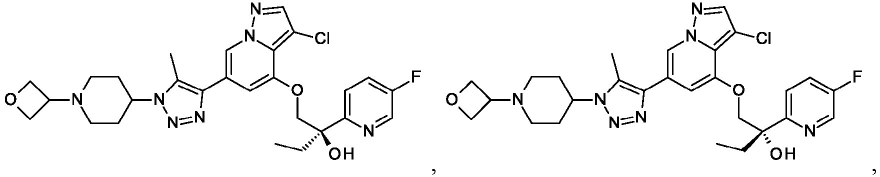 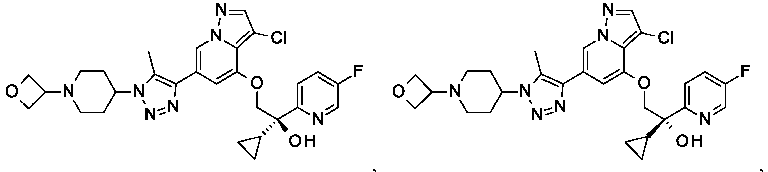 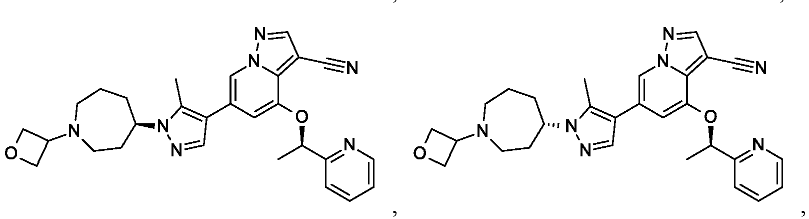 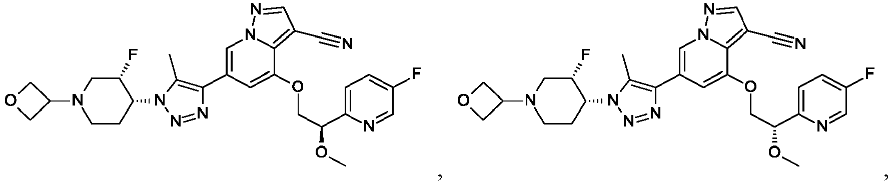 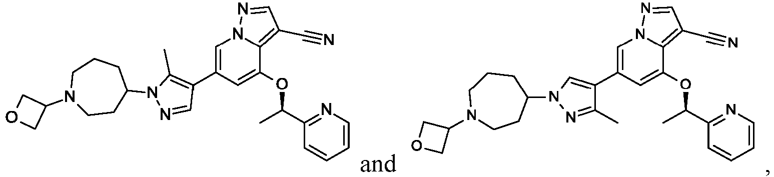 or a pharmaceutically acceptable salt thereof.

25. A pharmaceutical composition comprising a compound, or a pharmaceutically acceptable salt thereof, according to any one of claims 1-24, and a pharmaceutically acceptable carrier, diluent or excipient.

26. A compound, or a pharmaceutically acceptable salt thereof, according to any one of claims 1-24, for use in therapy.

27. A compound, or a pharmaceutically acceptable salt thereof, according to any one of claims 1-24, for use in the treatment of cancer.

28. The compound, or a pharmaceutically acceptable salt thereof, for use in the treatment of cancer according to claim 27, wherein the cancer is selected from the group consisting of hepatobiliary cancer, cancer of unknown primary, gallbladder cancer, gallbladder adenocarcinoma, bile duct cancer, intrahepatic bile duct cancer, extrahepatic bile duct cancer, breast cancer, invasive ductal cancer, invasive lobular cancer, liver cancer, hepatocellular cancer, fibrolamellar hepatocellular cancer, skin cancer, squamous cell skin cancer, melanoma, cutaneous melanoma, endometrial cancer and endometrial endometrioid adenocarcinoma.

## Patentansprüche

1. Verbindung der Formel: 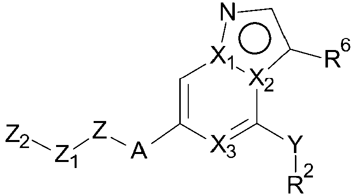 wobei
Z₂ ist 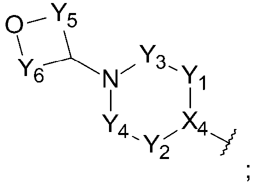
A Pyrazol, Triazol, Thiadiazol oder Oxadiazol ist, das substituiert mit R¹ und R^{1A} ist;
R¹ Hydrogen oder C₁-C₃-Alkyl ist;
R^{1A} Wasserstoff, Halogen, CN oder C₁-C₃-Alkyl ist, das optional mit einem oder mehreren Substituenten substituiert ist, die aus Halogen, OH und OCH₃ unabhängig ausgewählt sind;
X₁ und X₂ aus N und C unabhängig ausgewählt sind, wobei, wenn eines von X₁ oder X₂ N ist, das andere C ist;
X₃ N oder CH ist;
X₄ N oder C-R⁹ ist;
Y NH, O, S oder eine Bindung ist;
Y₁ eine Bindung, CHR⁷, CH₂-CHR⁷, CHR⁷-CH₂, CF₂, CH₂-CF₂ oder CF₂-CH₂ ist;
Y₂ eine Bindung, CHR³, CH₂-CHR³, CHR³-CH₂, CF₂, CH₂-CF₂ oder CF₂-CH₂ ist;
Y₃ CR⁴R⁵ oder CF₂ ist;
Y₄ CR³R⁴ oder CF₂ ist;
Y₅ ist CR¹³R¹⁴, CR¹³R¹⁴-CH₂ oder CH₂CR¹³R¹⁴;
Y₆ ist CR¹³R¹⁴, CR¹³R¹⁴-CH₂ oder CH₂CR¹³R¹⁴;
Z eine Bindung, CHR9^{A}, CR⁴R^{4A}, CR⁴R^{4A}-CH₂, CH₂-CR⁴R^{4A}, Cyclobutyl, Cyclopentyl, Cyclohexyl, Bicyclo(1.1.1)pentan, Bicyclo(2.1.1)hexan, Azetidin, Pyrrolidin oder Piperidin ist;
Z₁ eine Bindung ist, wenn Z eine Bindung ist, CR⁴R^{4A}, CR⁴R^{4A}-CH₂, CH₂-CR⁴R^{4A}, Cyclobutyl, Cyclopentyl, Cyclohexyl, Bicyclo(1.1.1)pentan, Bicyclo(2.1.1)hexan, Azetidin, Pyrrolidin oder Piperidin, oder Z₁ CH₂ oder CH₂-CH₂ ist, wenn Z CHR^{9A} ist;
Z₃ eine Bindung, C(O), SO₂ oder -NR⁴C(O) ist;
Z₄ eine Bindung, C(O), SO₂ oder -NR⁴C(O) ist;
R² C₁-C₅-Alkyl oder R⁸ ist, wobei C₁-C₅-Alkyl optional mit einem oder mehreren Substituenten substituiert ist, die aus Halogen, OH, CN, Oxo, -OC₁-C₄-Alkyl, -OC₃-C₅-Cycloalkyl, -Z₃-R¹¹ und R¹⁰ unabhängig ausgewählt sind, wobei C₁-C₄-Alkyl und C₃-C₅-Cycloalkyl optional mit einem oder mehreren Substituenten substituiert ist, die aus Halogen, OH, OCH₃, Methylamin, N,N-Dimethylamin und CN unabhängig ausgewählt sind;
R³ Wasserstoff, F, OH, OCH₃, C₁-C₃-Alkyl, Cyclopropyl ist oder ein R³ mit R⁵ oder R⁷ kondensiert ist, um CH₂, CH₂-CH₂ oder CH₂OCH₂ auszubilden;
R⁴ Hydrogen oder C₁-C₃-Alkyl ist;
R^{4A} Hydrogen, Halogen, OH oder C₁-C₃-Alkyl ist;
R⁵ Wasserstoff, F, OH, OCH₃, C₁-C₃-Alkyl, Cyclopropyl ist oder mit einem R³ kondensiert ist, um CH₂, CH₂-CH₂ oder CH₂ OCH₂ auszubilden;
R⁶ Wasserstoff, Halogen, C₁-C₅-Alkyl, CN, 3-6-gliedriges Cycloalkyl, 4-6-gliedriges Heterocycloalkyl, 5-6-gliedriges Aryl oder 5-6-gliedriges Heteroaryl ist, wobei 3-6-gliedriges Cycloalkyl, 4-6-gliedriges Heterocycloalkyl, 5-6-gliedriges Aryl und 5-6-gliedriges Heteroaryl optional mit einem oder mehreren Substituenten substituiert sind, die aus Halogen, Methyl, Halogenmethyl, OH oder OCH₃ unabhängig ausgewählt sind und wobei C₁-C₅-Alkyl optional mit einem oder mehreren Substituenten substituiert ist, die aus Halogen, OH und OCH₃ unabhängig ausgewählt sind;
R⁷ Wasserstoff, F, OH, OCH₃, C₁-C₃-Alkyl ist oder mit einem R³ kondensiert ist, um CH₂, CH₂-CH₂ oder CH₂OCH₂ auszubilden;
R⁸ 3-6-gliedriges Cycloalkyl, 4-6-gliedriges Heterocycloalkyl, 5-6-gliedriges Aryl oder 5-6-gliedriges Heteroaryl ist, das optional mit R^{8A} kondensiert oder substituiert ist;
R^{8A}; 3-6-gliedriges Cycloalkyl, 4-6-gliedriges Heterocycloalkyl, 5-6-gliedriges Aryl oder 5-6-gliedriges Heteroaryl ist;
R⁹ Wasserstoff, C₁-C₃-Alkyl ist, oder mit R^{9A} kondensiert ist, um CH₂ oder CH₂-CH₂ auszubilden;
R¹⁰ 3-6-gliedriges Cycloalkyl, 4-6-gliedriges Heterocycloalkyl, 5-6-gliedriges Aryl oder 5-6-gliedriges Heteroaryl ist, das optional mit R^{8A} kondensiert oder substituiert ist;
R₁₁ C₁-C₄-Alkyl, NH₂, NHC₁-C₃-Alkyl, NHC₃-C₅-Cycloalkyl oder N(C₁-C₃-Alkyl)₂ ist, wobei C₁-C₄-Alkyl, C₁-C₃-Alkyl und C₃-C₅-Cycloalkyl optional mit einem oder mehreren Substituenten substituiert ist, die aus Halogen, OH, OCH₃, Methylamin, N,N-Dimethylamin und CN unabhängig ausgewählt sind;
R¹² C₁-C₄-Alkyl, C₃-C₅-Cycloalkyl, NH₂, NHC₁-C₃-Alkyl, NHC₃-C₅-Cycloalkyl oder N(C₁-C₃-Alkyl)₂ ist, wobei C₁-C₄-Alkyl, C₁-C₃-Alkyl und C₃-C₅-Cycloalkyl optional mit einem oder mehreren Substituenten substituiert sind, die aus Halogen, OH, OCH₃, Methylamin, N,N-Dimethylamin und CN unabhängig ausgewählt sind;
R¹³ Hydrogen, Halogen oder C₁-C₃-Alkyl ist;
R¹⁴ Hydrogen, Halogen oder C₁-C₃-Alkyl ist; und
R⁸, R¹⁰ und R^{8A}; optional mit einem oder mehreren Substituenten substituiert sind, die aus Halogen, OH, CN, -OC₁-C₄-Alkyl, -OC₃-C₅-Cycloalkyl und -Z₄-R¹² unabhängig ausgewählt sind, wobei C₁-C₄-Alkyl und C₃-C₅-Cycloalkyl optional mit einem oder mehreren Substituenten substituiert sind, die aus Halogen, OH, OCH₃, Methylamin, N,N-Dimethylamin und CN unabhängig ausgewählt sind;
oder ein pharmazeutisch verträgliches Salz davon.

2. Verbindung nach Anspruch 1, wobei X₁ N ist und X₂ C oder ein pharmazeutisch verträgliches Salz davon ist.

3. Verbindung nach Anspruch 1 oder 2, wobei X₃ CH oder ein pharmazeutisch verträgliches Salz davon ist.

4. Verbindung nach einem der Ansprüche 1 bis 3, wobei A Pyrazol oder Triazol ist, das mit R¹ und R^{1A} oder einem pharmazeutisch verträglichen Salz davon substituiert ist.

5. Verbindung nach Anspruch 4, wobei A ein Triazol ist, das mit R¹ und R^{1A} oder einem pharmazeutisch verträglichen Salz davon substituiert ist.

6. Verbindung nach einem der Ansprüche 1 bis 5, wobei R^{1A} Hydrogen oder ein pharmazeutisch verträgliches Salz davon ist.

7. Verbindung nach einem der Ansprüche 1 bis 6, wobei R¹ CH₃ oder ein pharmazeutisch verträgliches Salz davon ist.

8. Verbindung nach einem der Ansprüche 1 bis 7, wobei Y O oder ein pharmazeutisch verträgliches Salz davon ist.

9. Verbindung nach einem der Ansprüche 1 bis 8, wobei R⁶ CN, F oder Cl oder ein pharmazeutisch verträgliches Salz davon ist.

10. Verbindung nach einem der Ansprüche 1 bis 9, wobei Z eine Bindung, Cyclobutyl, Azetidin oder Piperidin oder ein pharmazeutisch verträgliches Salz davon ist.

11. Verbindung nach Anspruch 10, wobei Z eine Bindung oder ein pharmazeutisch verträgliches Salz davon ist.

12. Verbindung nach einem der Ansprüche 1 bis 11, wobei Z₁ eine Bindung oder ein pharmazeutisch verträgliches Salz davon ist.

13. Verbindung nach einem der Ansprüche 1 bis 11, wobei X₄ C-R⁹ ist, wobei R⁹ Wasserstoff oder CH₃ oder ein pharmazeutisch verträgliches Salz davon ist.

14. Verbindung nach einem der Ansprüche 1 bis 13, wobei Y₁ eine Bindung, CHR⁷, CH₂-CHR⁷ or CHR⁷-CH₂ ist, wobei R⁷ aus Wasserstoff, F, OH und CH₃ oder einem pharmazeutisch verträglichen Salz davon ausgewählt ist.

15. Verbindung nach einem der Ansprüche 1 bis 14, wobei Y₂ eine Bindung, CHR³, CH₂-CHR³ oder CHR³-CH₂ ist, wobei R³ aus Wasserstoff, F, OH und CH₃ oder einem pharmazeutisch verträglichen Salz davon ausgewählt ist.

16. Verbindung nach einem der Ansprüche 1 bis 15, wobei Y₃ CR⁴R⁵ oder CF₂ ist, wobei R⁴ Wasserstoff oder CH₃ ist und R⁵ Wasserstoff, F, OH oder CH₃ oder ein pharmazeutisch verträgliches Salz davon ist.

17. Verbindung nach einem der Ansprüche 1 bis 16, wobei Y₄ CR³R⁴ oder CF₂ ist, wobei R⁴ Wasserstoff oder CH₃ ist und R³ Wasserstoff, F, OH oder CH₃ oder ein pharmazeutisch verträgliches Salz davon ist.

18. Verbindung nach einem der Ansprüche 1 bis 17, wobei Y₅ und Y₆ CH₂ oder ein pharmazeutisch verträgliches Salz davon sind.

19. Verbindung nach einem der Ansprüche 1 bis 18, wobei R² C₁-C₃-Alkyl ist, das optional mit einem, zwei, drei oder vier Substituenten substituiert ist, die aus Halogen, OH, CN, Oxo, -OC₁-C₄-Alkyl, -OC₃-C₅-Cycloalkyl, -Z₃-R¹¹ und R¹⁰ unabhängig ausgewählt sind, wobei C₁-C₄-Alkyl und C₃-C₅-Cycloalkyl optional mit einem oder mehreren Substituenten substituiert sind, die aus Halogen, OH, OCH₃, Methylamin, N,N-Dimethylamin und CN oder einem pharmazeutisch verträglichen Salz davon unabhängig ausgewählt sind.

20. Verbindung nach einem der Ansprüche 1 bis 19, wobei R² ausgewählt ist aus: 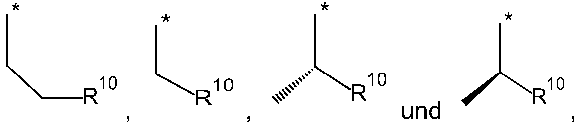 die optional mit einem, zwei, drei oder vier Substituenten substituiert sind, die aus Halogen, OH, CN, Oxo, -OC₁-C₄-Alkyl, -OC₃-C₅-Cycloalkyl, -Z₃-R¹¹ und R¹⁰ unabhängig ausgewählt sind, wobei C₁-C₄-Alkyl und C₃-C₅-Cycloalkyl optional mit einem oder mehreren Substituenten substituiert sind, die aus Halogen, OH, OCH₃, Methylamin, N,N-Dimethylamin und CN, wobei * den Verknüpfungspunkt zu Y angibt, oder einem pharmazeutisch verträglichen Salz davon unabhängig ausgewählt sind.

21. Verbindung nach einem der Ansprüche 1 bis 20, wobei R¹⁰ 4-6-gliedriges Heterocycloalkyl oder 5-6-gliedriges Heteroaryl ist, das optional mit R^{8A}; oder einem pharmazeutisch verträglichen Salz davon kondensiert ist.

22. Verbindung nach einem der Ansprüche 1 bis 20, wobei R¹⁰ aus Cyclopropan, Cyclobutan, Cyclopropan, Pyrrolidin, Thiazol, Pyrazol, Triazol, Phenyl, Pyridin, Pyrazin und Pyridazin, optional substituiert oder kondensiert mit R^{8A}, oder einem pharmazeutisch geeignetes Salz davon unabhängig ausgewählt ist.

23. Verbindung nach einem der Ansprüche 1 bis 22, wobei R¹⁰ und R^{8A} optional mit einem oder zwei Substituenten substituiert sind, die aus F, Cl, C₁-C₃ alkyl, CH₂F, CHF₂, CF₃ and -OCH₃ oder einem pharmazeutisch verträglichen Salz davon unabhängig ausgewählt sind.

24. Verbindung nach Anspruch 1, ausgewählt aus: 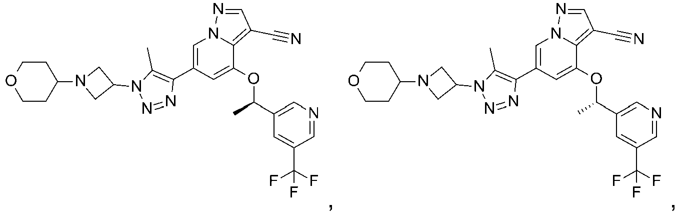 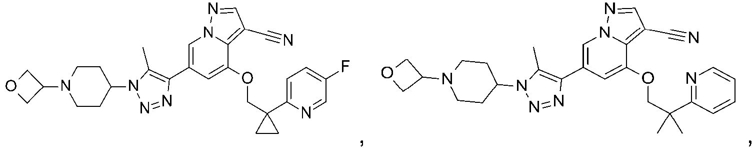 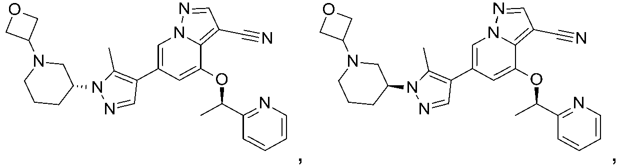 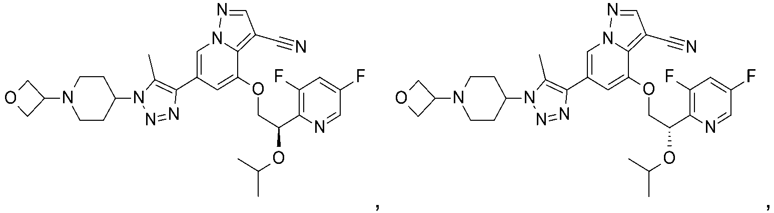 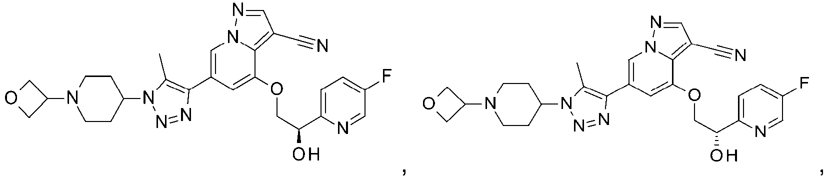 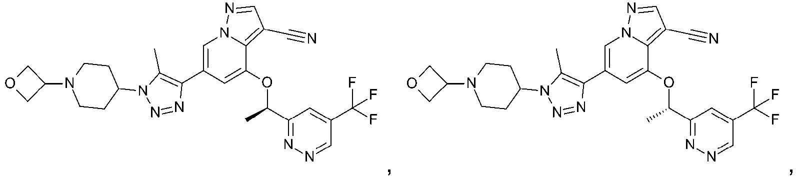 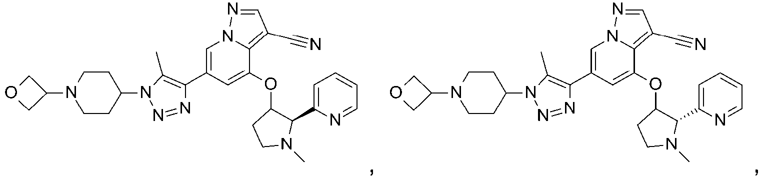 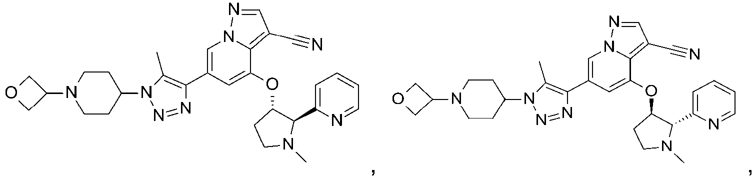 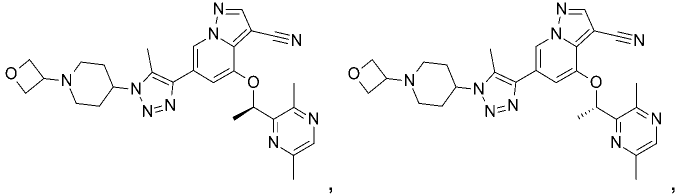 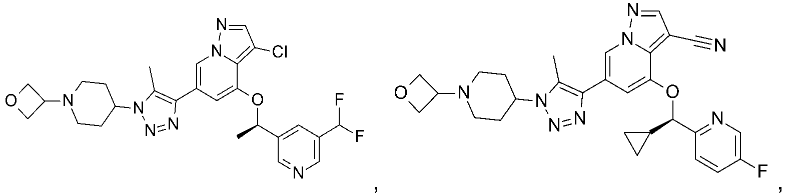 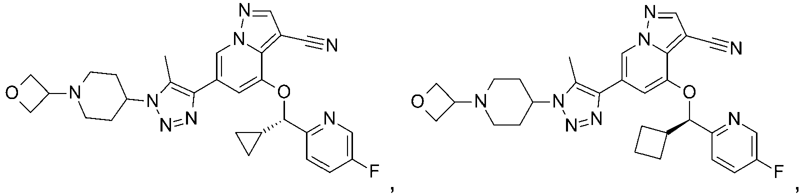 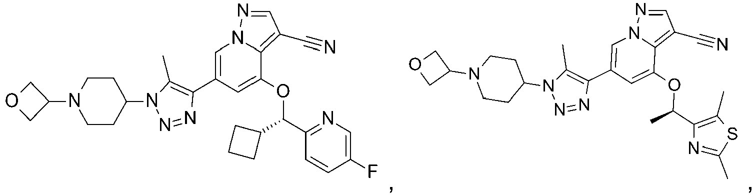 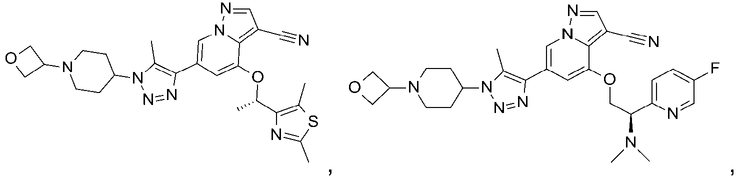 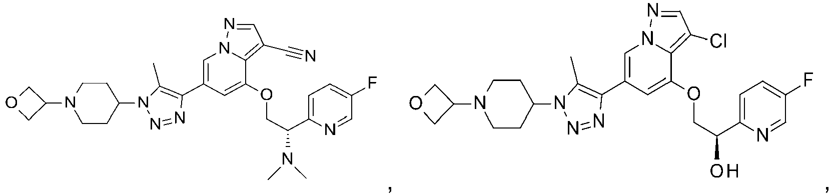 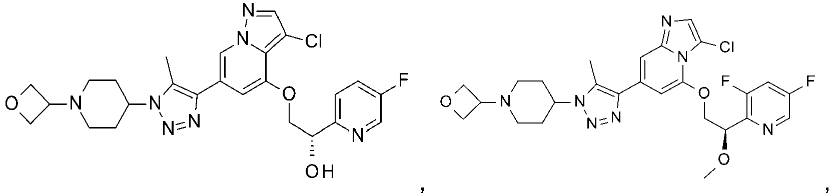 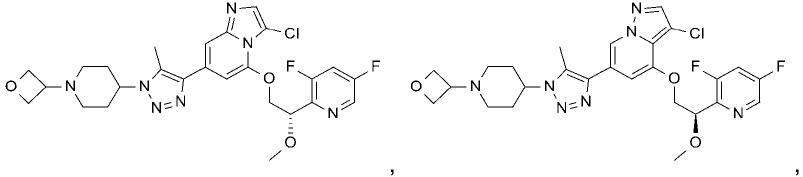 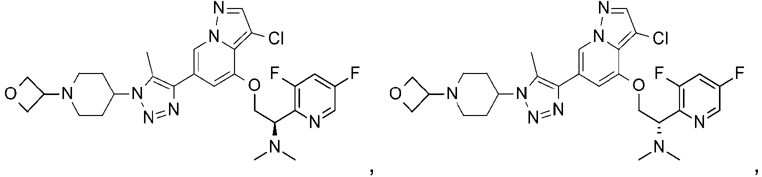 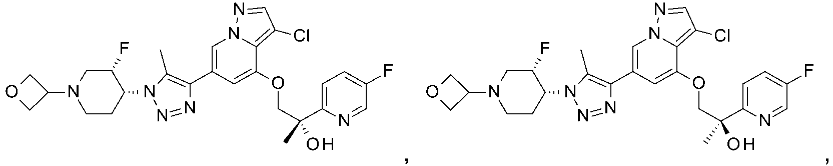 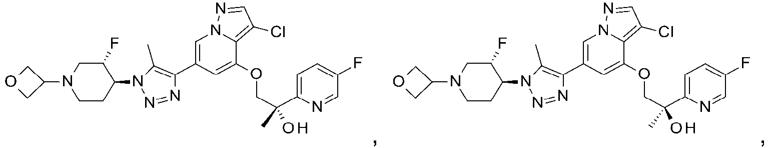 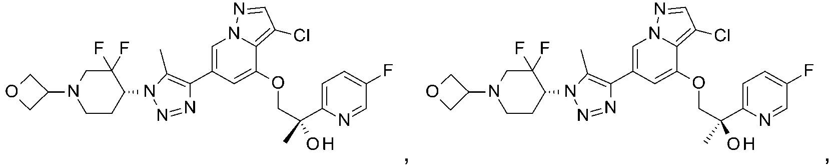 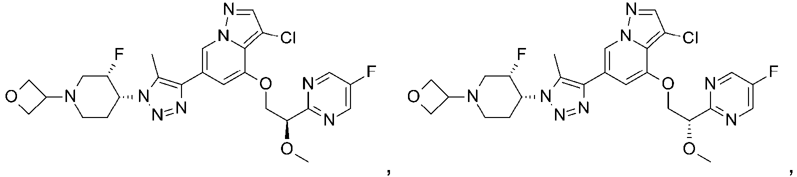 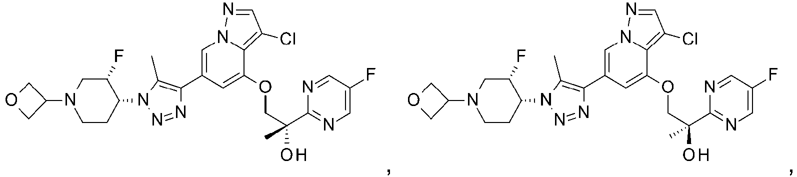 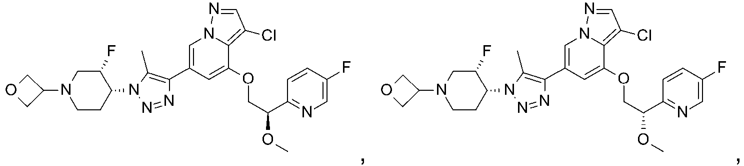 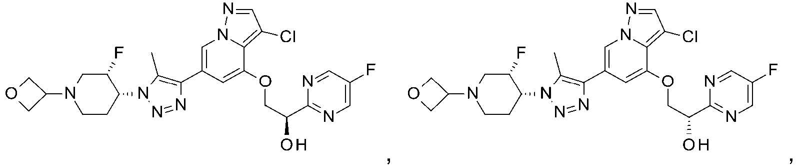 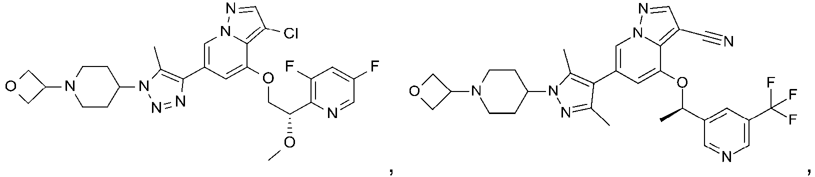 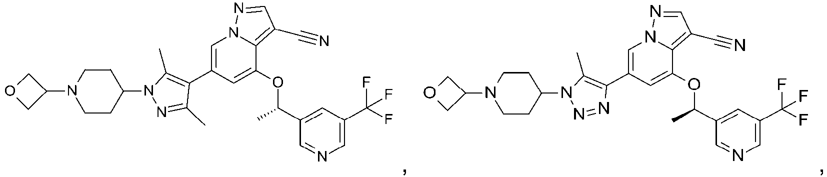 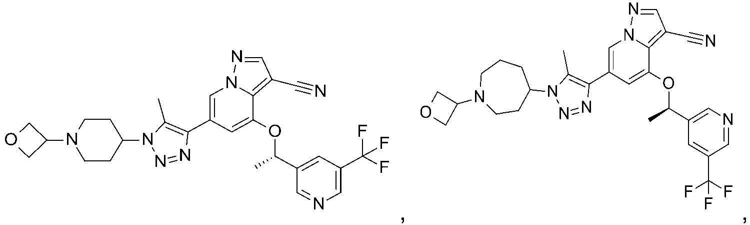 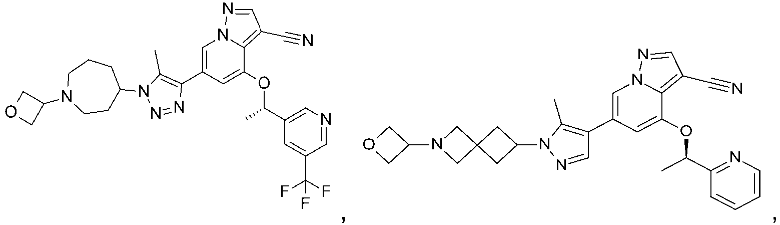 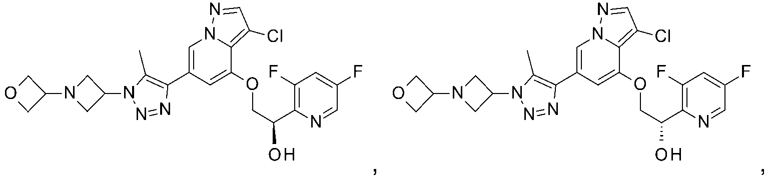 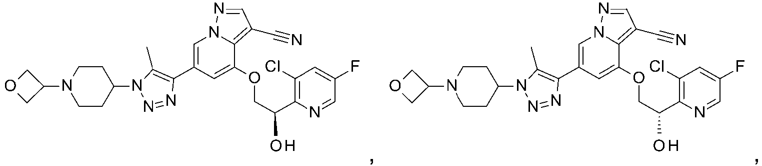 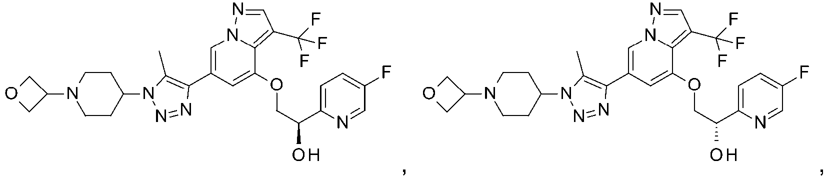 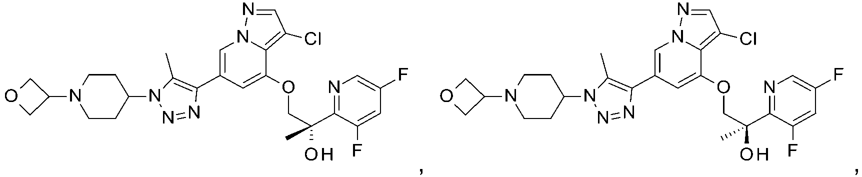 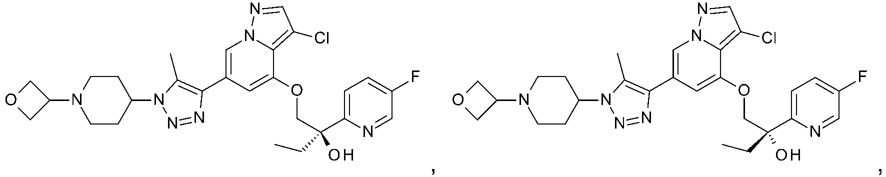 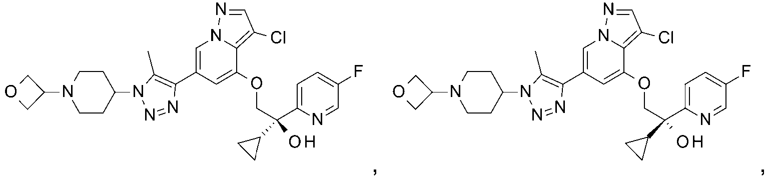 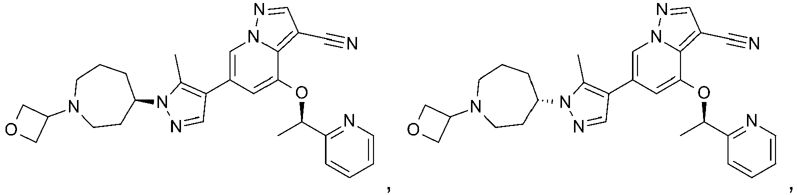 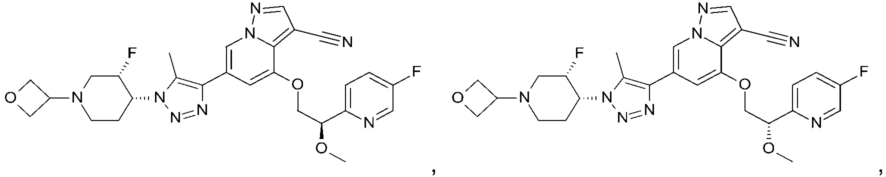 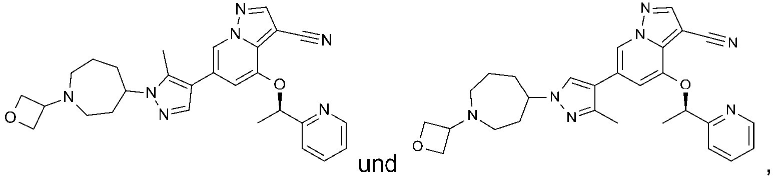 oder ein pharmazeutisch verträgliches Salz davon.

25. Pharmazeutische Zusammensetzung, umfassend eine Verbindung oder ein pharmazeutisch verträgliches Salz davon nach einem der Ansprüche 1 bis 24 und eine pharmazeutisch verträgliche Trägersubstanz, ein Verdünnungsmittel oder einen Exzipienten.

26. Verbindung oder ein pharmazeutisch verträgliches Salz davon nach einem der Ansprüche 1 bis 24 zur Verwendung bei einer Therapie.

27. Verbindung oder ein pharmazeutisch verträgliches Salz davon nach einem der Ansprüche 1 bis 24 zur Verwendung bei der Behandlung von Krebs.

28. Verbindung oder ein pharmazeutisch verträgliches Salz davon zur Verwendung bei der Behandlung von Krebs nach Anspruch 27, wobei der Krebs ausgewählt ist aus der Gruppe bestehend aus hepatobiliärem Krebs, Krebs unbekannten Primärtumors, Gallenblasenkrebs, Gallenblasenadenokarzinom, Gallengangkrebs, intrahepatischem Gallengangkrebs, extrahepatischem Gallengangkrebs, Brustkrebs, invasivem duktalem Krebs, invasivem lobulärem Krebs, Leberkrebs, hepatozellulärem Krebs, fibrolamellärem hepatozellulärem Krebs, Hautkrebs, Plattenepithelkarzinom der Haut, Melanom, kutanem Melanom, Endometriumkrebs und endometrialem endometrioidem Adenokarzinom.

## Revendications

1. Composé de formule : 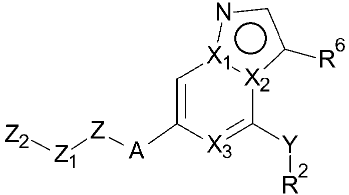 dans lequel
Z₂ représente 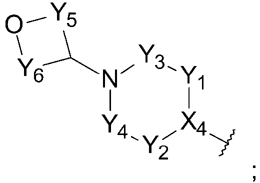
A représente pyrazole, triazole, thiadiazole ou oxadiazole, substitué par R¹ et R^{1A} ;
R¹ représente hydrogène ou alkyle en C₁-C₃ ;
R^{1A} représente hydrogène, halo, CN ou alkyle en C₁-C₃ éventuellement substitué par un ou plusieurs substituants choisis indépendamment parmi halo, OH et OCH₃ ;
X₁ et X₂ sont choisis indépendamment parmi N et C, dans lequel l'un parmi X₁ ou X₂ représente N, l'autre représente C ;
X₃ représente N ou CH ;
X₄ représente N ou C-R⁹ ;
Y représente NH, O, S ou une liaison ;
Y₁ représente une liaison, CHR⁷, CH₂-CHR⁷, CHR⁷-CH₂, CF₂, CH₂-CF₂ ou CF₂-CH₂ ;
Y₂ représente une liaison, CHR³, CH₂-CHR³, CHR³-CH₂, CF₂, CH₂-CF₂ ou CF₂-CH₂ ;
Y₃ représente CR⁴R⁵ ou CF₂ ;
Y₄ représente CR³R⁴ ou CF₂ ;
Y₅ représente CR¹³R¹⁴, CR¹³R¹⁴-CH₂ ou CH₂CR¹³R¹⁴ ;
Y₆ représente CR¹³R¹⁴, CR¹³R¹⁴-CH₂ ou CH₂CR¹³R¹⁴ ;
Z représente une liaison CHR9^{A}, C_{R}⁴_{R}^{4A,} CR⁴R^{4A}-CH₂, CH₂-CR⁴R^{4A}, cyclobutyle, cyclopentyle, cyclohexyle, bicyclo(1.1.1)pentane, bicyclo(2.1.1)hexane, azétidine, pyrrolidine ou pipéridine ;
Z₁ représente une liaison lorsque Z représente une liaison, CR⁴R^{4A}, CR⁴R^{4A}-CH₂, CH₂-CR⁴R^{4A}, cyclobutyle, cyclopentyle, cyclohexyle, bicyclo(1.1.1)pentane, bicyclo(2.1.1)hexane, azétidine, pyrrolidine ou pipéridine, ou Z₁ représente CH₂ ou CH₂-CH₂ lorsque Z représente CHR^{9A} ;
Z₃ représente une liaison, C(O), SO₂ ou -NR⁴C(O) ;
Z₄ représente une liaison, C(O), SO₂ ou -NR⁴C(O) ;
R² représente alkyle en C₁-C₅ ou R⁸, dans lequel l'alkyle en C₁-C₅ est éventuellement substitué par un ou plusieurs substituants choisis indépendamment parmi halo, OH, CN, oxo, -O-alkyle en C₁-C₄, -O-cycloalkyle en C₃-C₅, -Z₃-R¹¹ et R¹⁰, dans lequel l'alkyle en C₁-C₄ et le cycloalkyle en C₃-C₅ sont éventuellement substitués par un ou plusieurs substituants choisis indépendamment parmi halo, OH, OCH₃, méthylamine, N,N-diméthylamine et CN ;
R³ représente hydrogène, F, OH, OCH₃, alkyle en C₁-C₃, cyclopropyle, ou un R³ est fusionné avec R⁵ ou R⁷ pour former CH₂, CH₂-CH₂ ou CH₂OCH₂ ;
R⁴ représente hydrogène ou alkyle en C₁-C₃ ;
R^{4A} représente hydrogène, halo, OH ou alkyle en C₁-C₃ ;
R⁵ représente hydrogène, F, OH, OCH₃, alkyle en C₁-C₃, cyclopropyle, ou est fusionné avec un R³ pour former CH₂, CH₂-CH₂ ou CH₂OCH₂ ;
R⁶ représente hydrogène, halo, alkyle en C₁-C₅, CN, cycloalkyle de 3 à 6 chaînons, hétérocycloalkyle de 4 à 6 chaînons, aryle de 5 à 6 chaînons ou hétéroaryle de 5 à 6 chaînons, dans lequel le cycloalkyle de 3 à 6 chaînons, l'hétérocycloalkyle de 4 à 6 chaînons, l'aryle de 5 à 6 chaînons et l'hétéroaryle de 5 à 6 chaînons sont éventuellement substitués par un ou plusieurs substituants choisis indépendamment parmi halo, méthyle, halométhyle, OH ou OCH₃ et dans lequel l'alkyle en C₁-C₅ est éventuellement substitué par un ou plusieurs substituants choisis indépendamment parmi halo, OH et OCH₃ ;
R⁷ représente hydrogène, F, OH, OCH₃, alkyle en C₁-C₃, ou est fusionné avec un R³ pour former CH₂, CH₂-CH₂ ou CH₂OCH₂ ;
R⁸ représente cycloalkyle de 3 à 6 chaînons, hétérocycloalkyle de 4 à 6 chaînons, aryle de 5 à 6 chaînons ou hétéroaryle de 5 à 6 chaînons, éventuellement fusionné avec R^{8A} ou substitué par celui-ci ;
R^{8A} représente cycloalkyle de 3 à 6 chaînons, hétérocycloalkyle de 4 à 6 chaînons, aryle de 5 à 6 chaînons ou hétéroaryle de 5 à 6 chaînons ;
R⁹ représente hydrogène, alkyle en C₁-C₃, ou est fusionné avec R^{9A} pour former CH₂ ou CH₂-CH₂ ;
R¹⁰ représente cycloalkyle de 3 à 6 chaînons, hétérocycloalkyle de 4 à 6 chaînons, aryle de 5 à 6 chaînons ou hétéroaryle de 5 à 6 chaînons, éventuellement fusionné avec R⁸⁴ ou substitué par celui-ci ;
R₁₁ représente alkyle en C₁-C₄, NH₂, NH-alkyle en C₁-C₃, NH-cycloalkyle en C₃-C₅ ou N(alkyle en C₁-C₃)₂, dans lequel l'alkyle en C₁-C₄, l'alkyle en C₁-C₃ et le cycloalkyle en C₃-C₅ sont éventuellement substitués par un ou plusieurs substituants choisis indépendamment parmi halo, OH, OCH₃, méthylamine, N,N-diméthylamine et CN ;
R¹² représente alkyle en C₁-C₄, cycloalkyle en C₃-C₅, NH₂, NH-alkyle en C₁-C₃, NH-cycloalkyle en C₃-C₅ ou N(alkyle en C₁-C₃)₂, dans lequel l'alkyle en C₁-C₄, l'alkyle en C₁-C₃ et le cycloalkyle en C₃-C₅ sont éventuellement substitués par un ou plusieurs substituants choisis indépendamment parmi halo, OH, OCH₃, méthylamine, N,N-diméthylamine et CN ;
R¹³ représente hydrogène, halo ou alkyle en C₁-C₃ ;
R¹⁴ représente hydrogène, halo ou alkyle en C₁-C₃ ; et
R⁸, R¹⁰ et R^{8A} sont éventuellement substitués par un ou plusieurs substituants choisis indépendamment parmi halo, OH, CN, -O-alkyle en C₁-C₄, -O-cycloalkyle en C₃-C₅ et -Z₄-R¹², dans lequel l'alkyle en C₁-C₄ et le cycloalkyle en C₃-C₅ sont éventuellement substitués par un ou plusieurs substituants choisis indépendamment parmi halo, OH, OCH₃, méthylamine, N,N- diméthylamine et CN ;
ou sel pharmaceutiquement acceptable de celui-ci.

2. Composé selon la revendication 1, dans lequel X₁ représente N, et X₂ représente C, ou sel pharmaceutiquement acceptable de celui-ci.

3. Composé selon la revendication 1 ou 2, dans lequel X₃ représente CH, ou sel pharmaceutiquement acceptable de celui-ci.

4. Composé selon l'une quelconque des revendications 1 à 3, dans lequel A représente pyrazole ou triazole, substitué par R¹ et R^{1A}, ou sel pharmaceutiquement acceptable de celui-ci.

5. Composé selon la revendication 4, dans lequel A représente triazole, substitué par R¹ et R^{1A}, ou sel pharmaceutiquement acceptable de celui-ci.

6. Composé selon l'une quelconque des revendications 1 à 5, dans lequel R^{1A} représente hydrogène, ou sel pharmaceutiquement acceptable de celui-ci.

7. Composé selon l'une quelconque des revendications 1 à 6, dans lequel R¹ représente CH₃, ou sel pharmaceutiquement acceptable de celui-ci.

8. Composé selon l'une quelconque des revendications 1 à 7, dans lequel Y représente O, ou sel pharmaceutiquement acceptable de celui-ci.

9. Composé selon l'une quelconque des revendications 1 à 8, dans lequel R⁶ représente CN, F ou CI, ou sel pharmaceutiquement acceptable de celui-ci.

10. Composé selon l'une quelconque des revendications 1 à 9, dans lequel Z représente une liaison, cyclobutyle, azétidine ou pipéridine, ou sel pharmaceutiquement acceptable de celui-ci.

11. Composé selon la revendication 10, dans lequel Z représente une liaison, ou sel pharmaceutiquement acceptable de celui-ci.

12. Composé selon l'une quelconque des revendications 1 à 11, dans lequel Z₁ représente une liaison, ou sel pharmaceutiquement acceptable de celui-ci.

13. Composé selon l'une quelconque des revendications 1 à 11, dans lequel X₄ représente C-R⁹, dans lequel R⁹ représente hydrogène ou CH₃, ou sel pharmaceutiquement acceptable de celui-ci.

14. Composé selon l'une quelconque des revendications 1 à 13, dans lequel Y₁ représente une liaison, CHR⁷, CH₂-CHR⁷ ou CHR⁷-CH₂, dans lequel R⁷ est choisi parmi hydrogène, F, OH et CH₃, ou sel pharmaceutiquement acceptable de celui-ci.

15. Composé selon l'une quelconque des revendications 1 à 14, dans lequel Y₂ représente une liaison, CHR³, CH₂-CHR³ ou CHR³-CH₂, dans lequel R³ est choisi parmi hydrogène, F, OH et CH₃, ou sel pharmaceutiquement acceptable de celui-ci.

16. Composé selon l'une quelconque des revendications 1 à 15, dans lequel Y₃ représente CR⁴R⁵ ou CF₂, dans lequel R⁴ représente hydrogène ou CH₃ et R⁵ représente hydrogène, F, OH ou CH₃, ou sel pharmaceutiquement acceptable de celui-ci.

17. Composé selon l'une quelconque des revendications 1 à 16, dans lequel Y₄ représente CR³R⁴ ou CF₂, dans lequel R⁴ représente hydrogène ou CH₃ et R³ représente hydrogène, F, OH ou CH₃, ou sel pharmaceutiquement acceptable de celui-ci.

18. Composé selon l'une quelconque des revendications 1 à 17, dans lequel Y₅ et Y₆ représentent CH₂, ou sel pharmaceutiquement acceptable de celui-ci.

19. Composé selon l'une quelconque des revendications 1 à 18, dans lequel R² représente alkyle en C₁-C₃ éventuellement substitué par un, deux, trois ou quatre substituants choisis indépendamment parmi halo, OH, CN, oxo, -O-alkyle en C₁-C₄, -O-cycloalkyle en C₃-C₅, -Z₃-R¹¹ et R¹⁰, dans lequel l'alkyle en C₁-C₄ et le cycloalkyle en C₃-C₅ sont éventuellement substitués par un ou plusieurs substituants choisis indépendamment parmi halo, OH, OCH₃, méthylamine, N,N-diméthylamine et CN, ou sel pharmaceutiquement acceptable de celui-ci.

20. Composé selon l'une quelconque des revendications 1 à 19, dans lequel R² est choisi parmi : 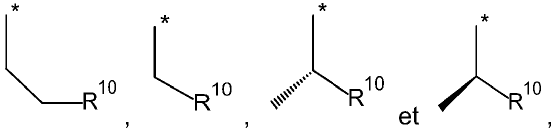 éventuellement substitué par un, deux, trois ou quatre substituants choisis indépendamment parmi halo, OH, CN, oxo, -O-alkyle en C₁-C₄, -O-cycloalkyle en C₃-C₅, -Z₃-R¹¹ et R¹⁰, dans lequel l'alkyle en C₁-C₄ et le cycloalkyle en C₃-C₅ sont éventuellement substitués par un ou plusieurs substituants choisis indépendamment parmi halo, OH, OCH₃, méthylamine, N,N-diméthylamine et CN, où * indique le point de liaison à Y, ou sel pharmaceutiquement acceptable de celui-ci.

21. Composé selon l'une quelconque des revendications 1 à 20, dans lequel R¹⁰ représente hétérocycloalkyle de 4 à 6 chaînons ou hétéroaryle de 5 à 6 chaînons, éventuellement substitué par R^{8A}; ou fusionné avec celui-ci, ou sel pharmaceutiquement acceptable de celui-ci.

22. Composé selon l'une quelconque des revendications 1 à 20, dans lequel R¹⁰ est indépendamment choisi parmi cyclopropane, cyclobutane, cyclopropane, pyrrolidine, thiazole, pyrazole, triazole, phényle, pyridine, pyrazine et pyridazine, éventuellement substitué par R^{8A}; ou fusionné avec celui-ci, ou sel pharmaceutiquement acceptable de celui-ci.

23. Composé selon l'une quelconque des revendications 1 à 22, dans lequel R¹⁰ et R^{8A}; sont éventuellement substitués par un ou deux substituants choisis indépendamment parmi F, CI, alkyle en C₁-C₃, CH₂F, CHF₂, CF₃, et -OCH₃, ou sel pharmaceutiquement acceptable de celui-ci.

24. Composé selon la revendication 1, choisi parmi : 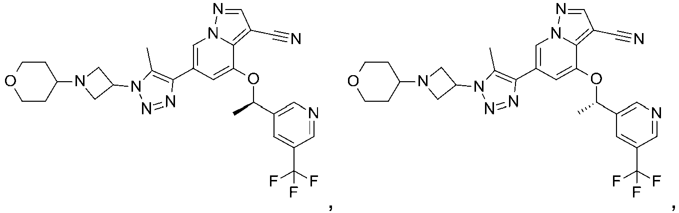 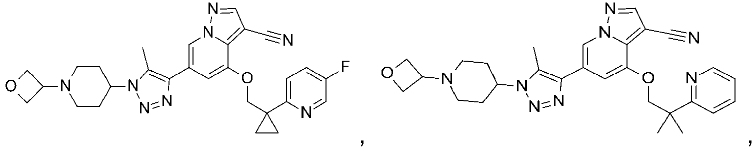 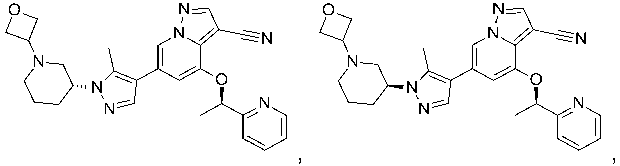 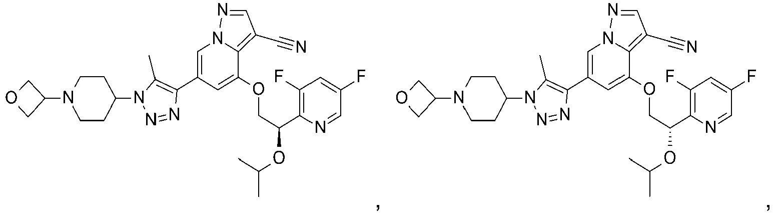 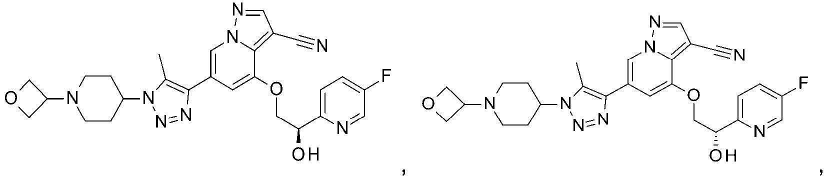 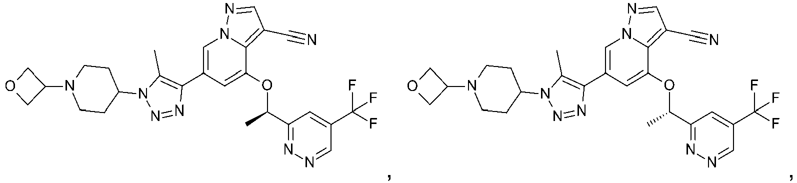 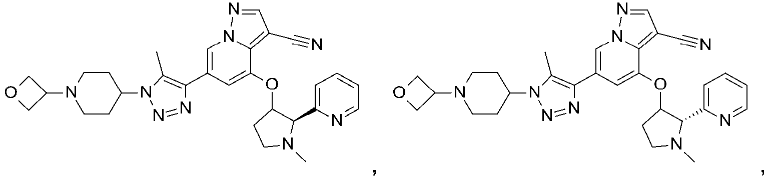 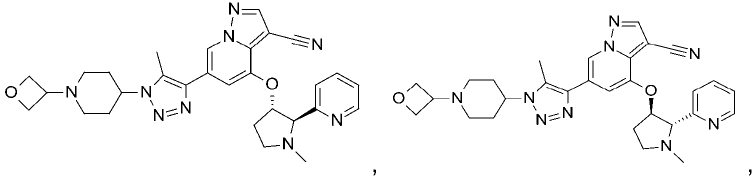 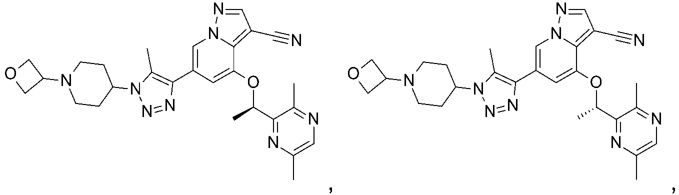 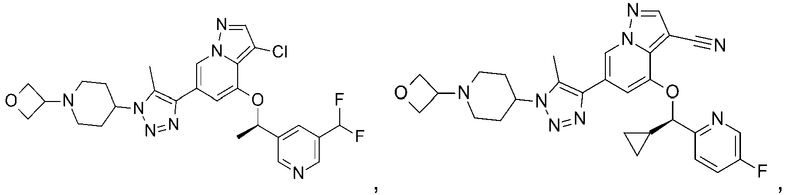 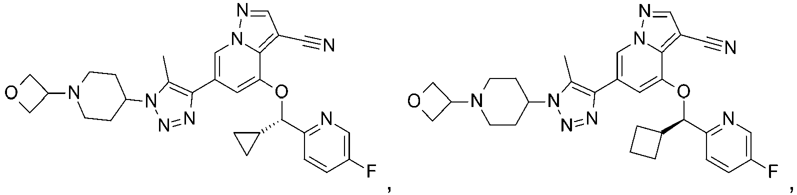 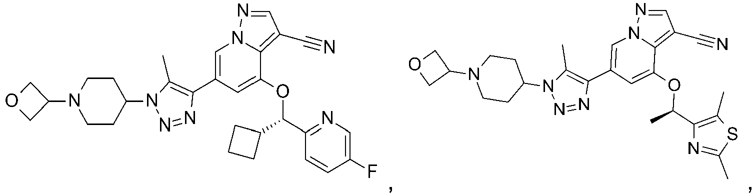 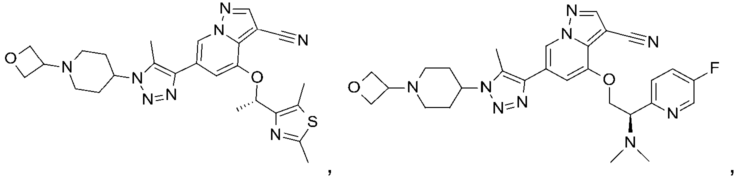 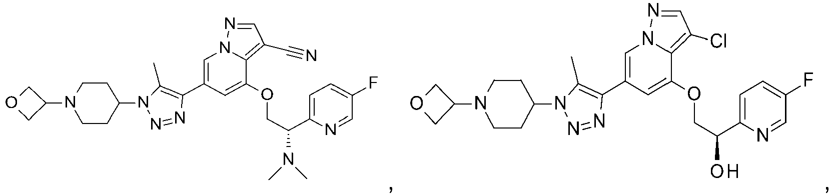 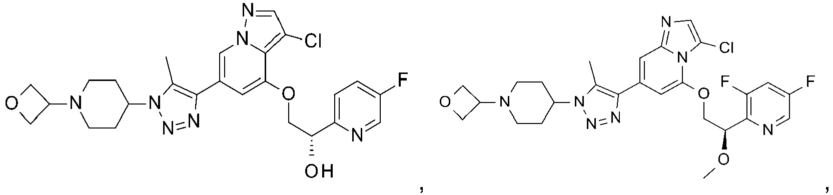 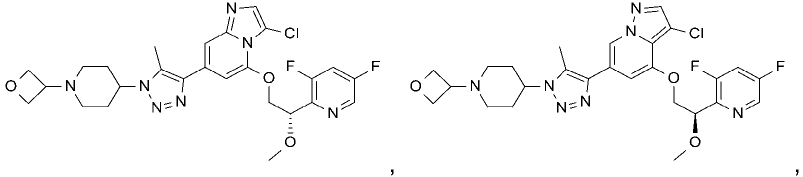 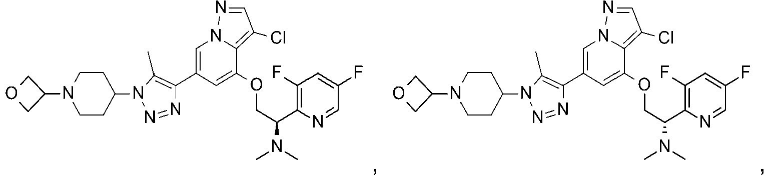 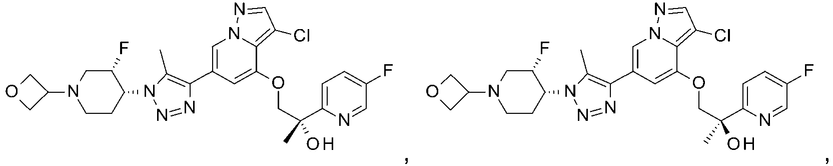 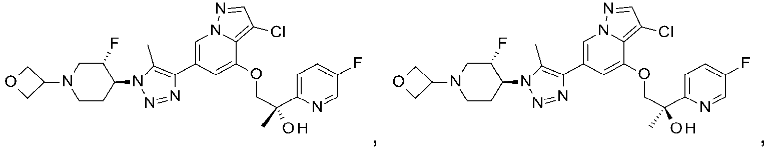 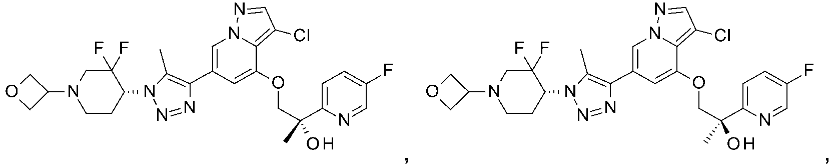 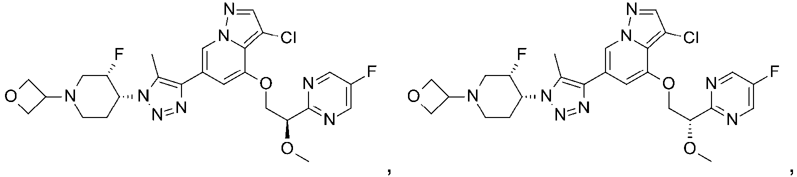 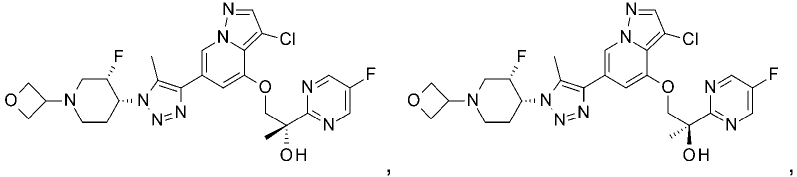 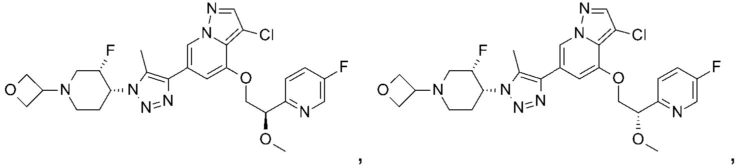 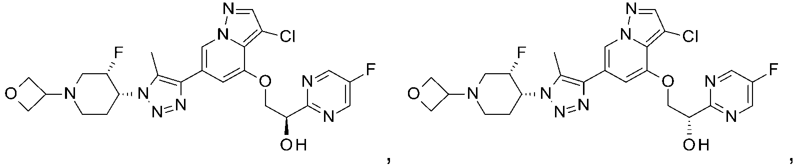 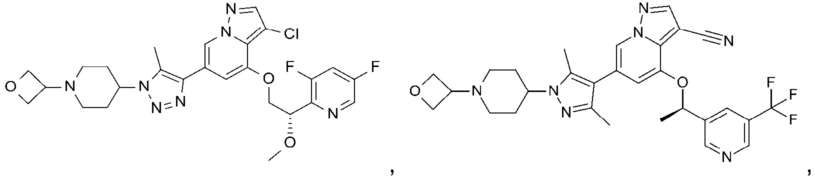 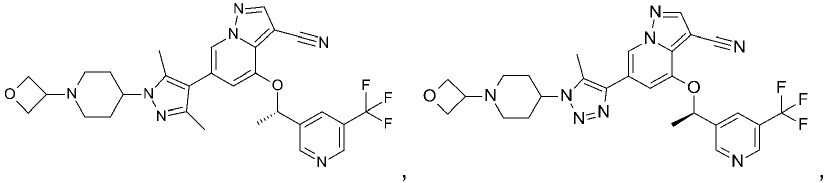 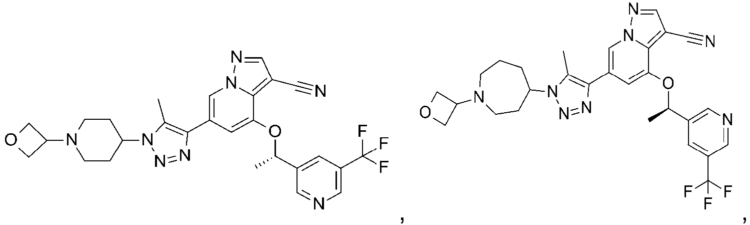 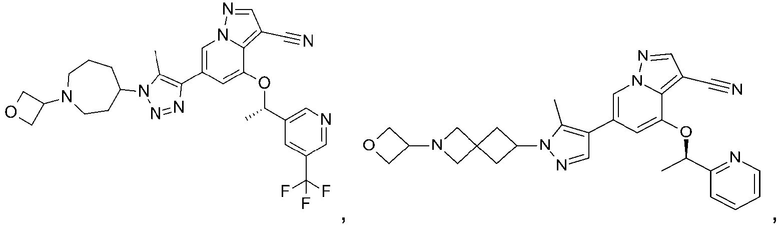 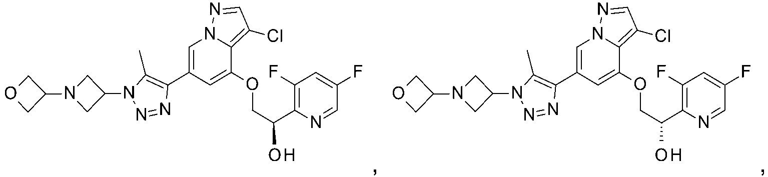 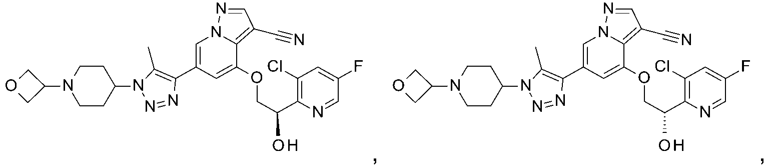 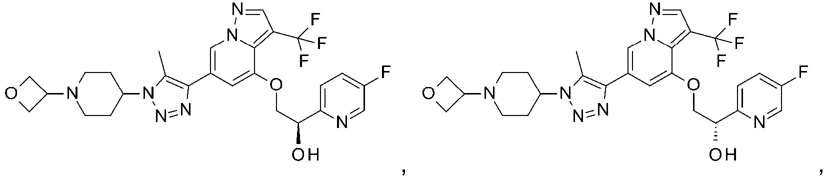 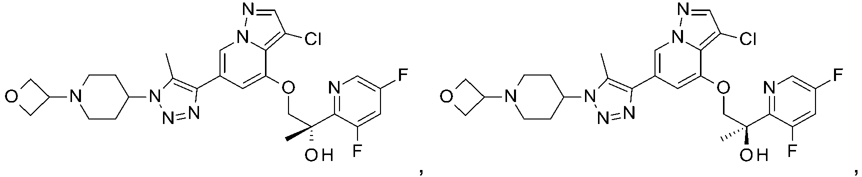 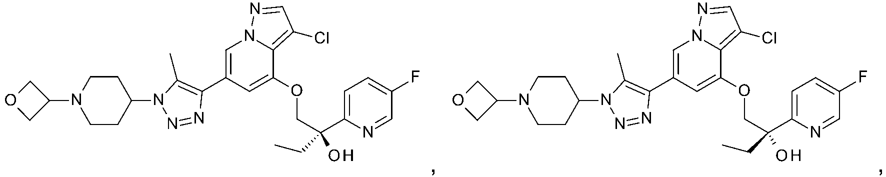 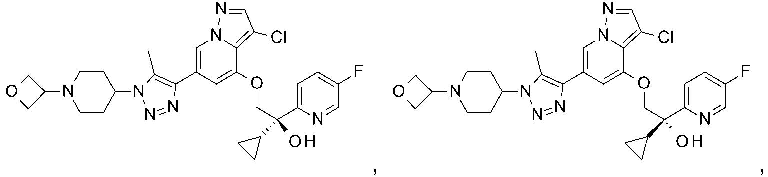 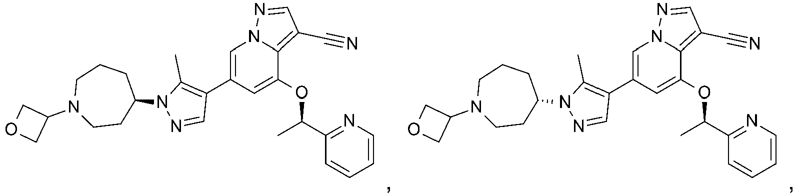 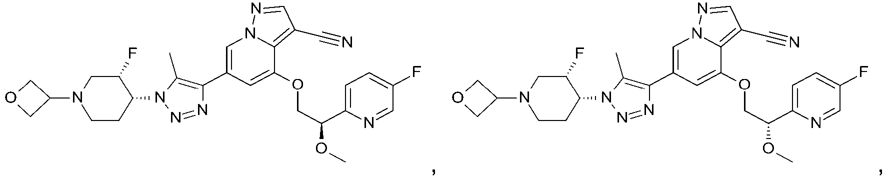 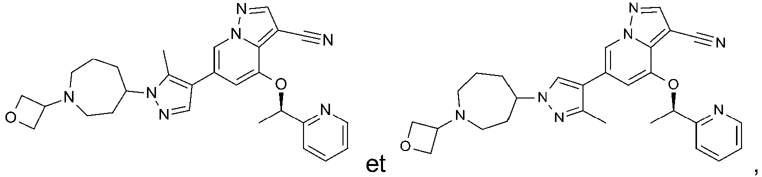 ou sel pharmaceutiquement acceptable de celui-ci.

25. Composition pharmaceutique comprenant un composé, ou un sel pharmaceutiquement acceptable de celui-ci, selon l'une quelconque des revendications 1 à 24, et un support, diluant, ou excipient pharmaceutiquement acceptable.

26. Composé, ou sel pharmaceutiquement acceptable de celui-ci, selon l'une quelconque des revendications 1 à 24, pour utilisation dans une thérapie.

27. Composé, ou sel pharmaceutiquement acceptable de celui-ci, selon l'une quelconque des revendications 1 à 24, pour utilisation dans le traitement du cancer.

28. Composé, ou sel pharmaceutiquement acceptable de celui-ci, pour utilisation dans le traitement du cancer selon la revendication 27, dans lequel le cancer est choisi dans le groupe constitué de cancer hépatobiliaire, cancer primitif inconnu, cancer de la vésicule biliaire, adénocarcinome de la vésicule biliaire, cancer des voies biliaires, cancer des voies biliaires intrahépatiques, cancer des voies biliaires extrahépatiques, cancer du sein, cancer canalaire invasif, cancer lobulaire invasif, cancer du foie, cancer hépatocellulaire, cancer hépatocellulaire fibrolamellaire, cancer de la peau, cancer de la peau épidermoïde, mélanome, mélanome cutané, cancer de l'endomètre et adénocarcinome endométrioïde de l'endomètre.
